(19) Europäisches Patentamt
European Patent Office
Office européen des brevets

(11) EP 1 873 157 A1

(12) EUROPEAN PATENT APPLICATION

(43) Date of publication:
**02.01.2008 Bulletin 2008/01**

(51) Int Cl.:
***C07D 487/04*** *(2006.01)* ***A61K 31/519*** *(2006.01)*
***A61P 35/00*** *(2006.01)*

(21) Application number: **06090113.9**

(22) Date of filing: **21.06.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Bayer Schering Pharma Aktiengesellschaft**
**13353 Berlin (DE)**

(72) Inventors:
- **Ince, Stuart**
  **10559 Berlin (DE)**
- **Prien, Olaf**
  **10711 Berlin (DE)**
- **Lu, Shoufu**
  **CA 94582 (US)**
- **Yu, Hongyi**
  **PA 19087 (US)**
- **Husemann, Manfred**
  **16450 Hohen Neuendorf (DE)**
- **Schuck, Karina**
  **10115 Berlin (DE)**

(54) **Pyrazolopyrimidines and salts thereof, pharmaceutical compositions comprising same, methods of preparing same and uses of same**

(57) The invention relates to pyrazolopyrimidines according to the general formula (I) :

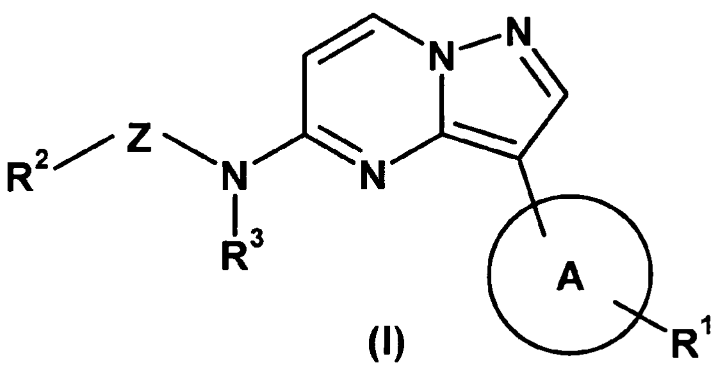

(I)

and salts thereof, to pharmaceutical compositions comprising said pyrazolopyrimidines and to a method of preparing said pyrazolopyrimidines as well as the use thereof for manufacturing a pharmaceutical composition for the treatment or prophylaxis of diseases of dysregulated vascular growth or of diseases which are accompanied with dysregulated vascular growth.

EP 1 873 157 A1

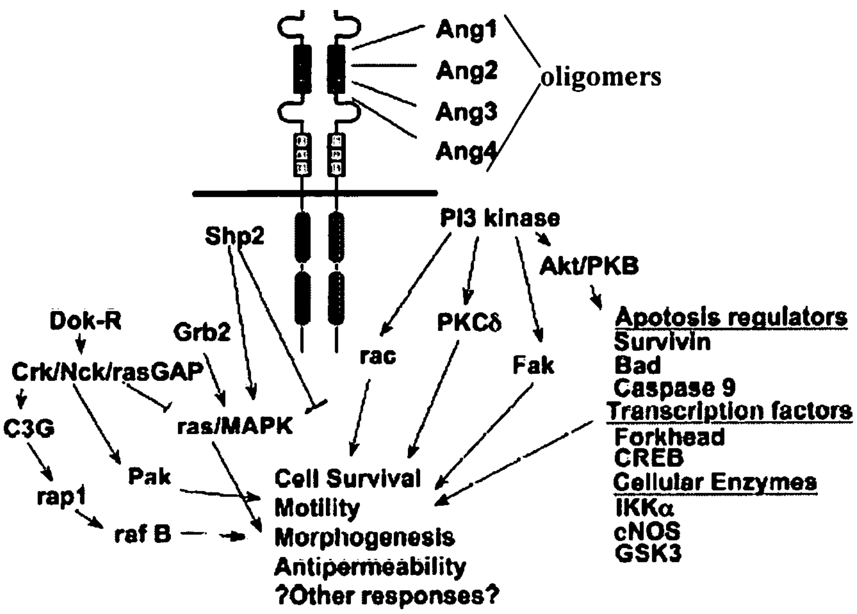
Ang1
Ang2
Ang3
Ang4
oligomers
Shp2
PI3 kinase
Akt/PKB
Dok-R
Grb2
PKCδ
rac
Fak
Crk/Nck/rasGAP
C3G
ras/MAPK
Pak
rap1
raf B
Cell Survival
Motility
Morphogenesis
Antipermeability
?Other responses?
Apotosis regulators
Survivin
Bad
Caspase 9
Transcription factors
Forkhead
CREB
Cellular Enzymes
IKKα
cNOS
GSK3

Fig. 1

## Description

The present invention relates to pyrazolopyrimidine compounds of general formula (I) and salts thereof, to pharmaceutical compositions comprising said pyrazolopyrimidine compounds, to methods of preparing said pyrazolopyrimidines as well as to the use thereof.

In order to defeat diseases with dysregulated vascular growth such as cancer different strategies were developed. One possible strategy is the blockade of angiogenesis to the tumour tissue. A key process in tumourigenesis is the formation of new blood vessels to supply nutrients and oxygen to the growing tumour. This multistep process called angiogenesis is characterised by endothelial cell (EC) proliferation and migration to form capillary sprouts that progressively recruit pericytes and vascular smooth muscle cells for vessel stabilisation.

Angiogenesis represents besides vasculogenesis one of two basic processes during the genesis of vasculature. Vasculogenesis describes the neoplasm of vascular tissue during embryo development, whereas angiogenesis describes the neoplasm of vasculature by sprouts or division of present vasculature. It has been found that specific receptors expressed on endothelial cells, e.g. VEGF- (vascular endothelial growth factor) and ALK1 (activin receptor-like kinase, synonym ACVRL1) receptors, are essential for normal development of blood vessels (Urness et al.: "Arteriovenous malformations in mice lacking activin receptor-like kinase-1". Nat Genet. 2000, 26(3):328-31.)

The mechanism of ALK1 signalling was characterised by different researchers. ALK1 is nominated as a type I receptor for the Transforming Growth Factor beta (TGFB) family proteins. It is a transmembrane receptor with serine/threonine (ser/thr) kinase activity. Morphogens of the TGFB superfamily bind to heterodimers of type I and type II receptors of transmembrane ser/thr kinases and mediate intracellular signals via SMAD proteins. These ALK1 containing heteromeric receptor complexes are accomplished by an accessory type III receptor called endoglin (Heldin, C.H. et al.: "TGF-beta signalling from cell membrane to nucleus through SMAD proteins". Nature, 1997. 390(6659): 465-71).

The ligand for ALK1 is not yet known exactly. Activin A, TGFB1 /3, bone morphogenic protein-9 (BMP-9) and another, yet not known protein, have been postulated as ALK1 modulators (Lux, A., et al:. "Assignment of transforming growth factor beta1 and beta3 and a third new ligand to the type I receptor ALK1". J Biol Chem, 1999. 274(15): 9984-92 ; Brown, M.A., et at.: "Crystal structure of BMP-9 and functional interactions with pro-region and receptors". J Biol Chem, 2005. 280(26): 25111-8.)

Upon ligand binding, a heteromeric receptor complex consisting of two type 11 and two type I receptors is formed. In the tetrameric complex the type 11 receptor phosphorylates and thereby activates the intracellular so-called GS (SGSGSG) domain of ALK1 which is located between the transmembrane and the kinase domain (Carcamo, J., et al.: "Type I receptors specify growth-inhibitory and transcriptional responses to transforming growth factor beta and activin". Mol Cell Biol, 1994. 14(6): 3810-21.

Sequentially, ALK1 phosphorylates the receptor-regulated SMADs (R-SMADs) SMAD-1 and -5. R-SMADs form heterodimeric complexes with the common SMAD (Co-SMAD) SMAD4. Afterwards, these proteins translocate into the nucleus where they activate target genes. It has been demonstrated that the ID1, ID2 and ID3 promoters are activated by SMAD 1/4/5 transcription factors The SMAD-signalling is blocked by the inhibitory SMADS (I-SMADs) SMAD6 and 7 (ten Dijke P, M.K., Heldin CH.: "Signalling inputs converge on nuclear effectors in TGF-beta signalling". Trends Biochem Sci, 2000. (25(2)): 64-70; Chen, Y.G. and J. Massague: "SMAD1 recognition and activation by the ALK1 group of transforming growth factor-beta family receptors". J Biol Chem, 1999. 274(6): 3672-7).

ALK1 is involved in endothelial cell (EC) proliferation and migration. Adenoviral infection of constitutively active ALK1 kinase in bovine aortic endothelial cells (BAECs) demonstrated an increase of the cellular proliferation rate as determined by the cell number. Furthermore, ALK1 antisense oligonucleotides lead to inhibition of TGFB3 induced migration of microvascular EC (Goumans, M.J., et al.: "Balancing the activation state of the endothelium via two distinct TGF-beta type I receptors". Embo J, 2002. 21(7):1743-53).

Knock-out of the ALK1 gene in transgenic mice leads to an embryonic lethal phenotype due to defective angiogenesis. These embryos display arteriovenous capillary fusions and dilated blood vessels due to delayed recruitment and differentiation of perivascular cells. As tumour- and embryonic angiogenesis are considered to work mechanistically very similar, inhibition of ALK1 activity should interfere with tumour vascularisation. This phenotype is redundant to endoglin and SMAD5 gene knock-outs, suggesting them acting via homologous (or the same) signal transduction pathways (Li, D.Y., et al.: "Defective angiogenesis in mice lacking endoglin". Science, 1999. 284(5419):1534-7; Yang, X., et al.: "Angiogenesis defects and mesenchymal apoptosis in mice lacking SMAD5". Development, 1999. 126(8): 1571-80).

Disruption of ALK1 in zebrafish leads to the mutant phenotype called violet beauregarde (vbg). The name depicts the violet (purple) colour of the zebrafish which have an abnormal circulation pattern in which most blood cells flow through a limited number of dilated cranial vessels and fail to perfuse the trunk and tail leading to a lethal phenotype (Roman B.L. et al.: "Disruption of acvrl1 increases endothelial cell number in zebrafish cranial vessels". Development 2002. 129: 3009-3019).

In humans, mutations in Endoglin and ALK1 are responsible for the autosomal dominant vascular dysplasias,

hereditary hemorrhagic telangiectasia (HHT) type 1 and type 2, respectively, which together occur with a frequency of 1 in 10,000 (McDonald et al.: "Clinical manifestations in a large hereditary hemorrhagic telangiectasia (HHT) type 2 kindred". Am. J. Med. Genet., 2000. 93: 320-327; van den Driesche S, M.C., Westermann CJ.: "Hereditary hemorrhagic telangiectasia: an update on transforming growth factor beta signalling in vasculogenesis and angiogenesis". Cardiovasc Res, 2003. 58((1)): 20-31.).

These diseases present themselves clinically in a similar manner, with symptoms including epistaxis (recurrent nosebleeds), mucocutaneous telangiectases (superficial vascular dilations that present as small red spots), and arteriovenous malformations (AVMs). Large AVMs, particularly in the brain and lung, can lead to stroke if severe shunting or rupture occurs. The basis for the localised nature of these defects is not known, although it has been suggested that the appearance of pathological lesions is precipitated by some independent, site-specific event. The age of onset and expressivity of these diseases are highly variable and seem to depend on both genetic and epigenetic factors (Guttmacher et al.: "Hereditary hemorrhagic telangiectasia". N. Engl. J. Med. 1995. 333:918-924.).

Together with the mouse knock-out and the zebrafish mutant, these findings strongly impose ALK1 as a key regulating molecule for the stabilisation of blood vessels, the recruitment of perivascular cells and the differentiation of arteries and veins.

Recent data indicate an important role of ALK1 for tumour angiogenesis. It has been demonstrated that ALK1 expression is greatly diminished in the adult organism but again induced in pre-existing feeding arteries and newly formed arterial vessels during tumour angiogenesis. Therefore a heterozygous ALK1 lacZ knock-in mouse was used for a teratoma tumour model. These mice express ß-Galactosidase under the control of the native ALK1 gene promoter and therefore were used to study ALK1 expression during tumour angiogenesis. The authors have experimentally proven that ß-Gal expression (represents ALK1 expression) essentially occurred in the main arteries feeding the tumour (Seki, T. et al.: "Arterial endothelium-specific activin receptor-like kinase 1 expression suggests its role in arterialisation and vascular remodelling". Circ Res 2003. 93(7):682-9.).

The inhibition of tumour angiogenesis is a key therapeutic strategy that holds great promise for the advancement of metastatic cancer therapy. For example, the therapeutic value of inhibiting the vascular endothelial growth factor (VEGF) pathway has been demonstrated by using drugs that prevent vascular endothelial growth factor receptor binding and by using drugs that inhibit receptor activation. A specific example is bevacizumab (Avastin; Genentech, South San Francisco, California, USA), a humanised monoclonal antibody that acts by binding and neutralizing vascular endothelial growth factor. Avastin is a clinically effective antibody that functions as tumour growth inhibitor in colon carcinoma. Thus, interference with angiogenesis is a proven clinical principle.

BACKGROUND to the INVENTION

Despite the advances in cancer research, there is still a high demand for more effective compounds which can be used in the treatment or prophylaxis of diseases of dysregulated vascular growth or diseases which are accompanied with dysregulated vascular growth, in particular solid tumours and metastases thereof. Particularly, it would be desirable to have compounds at one's disposal which can be used in the treatment or prophylaxis of diseases of dysregulated vascular growth or diseases which are accompanied with dysregulated vascular growth, in particular solid tumours and metastases thereof. More particularly, it would be desirable to have compounds at one's disposal which can be used in the treatment or prophylaxis of diseases of dysregulated vascular growth or diseases which are accompanied with dysregulated vascular growth, in particular solid tumours and metastases thereof, wherein their mechanism of action is the inhibition of ALK1 kinase.

Surprisingly, compounds of the present invention display a potent inhibition of ALK1 kinase. Thus, the solution to the above-mentioned novel technical problem is achieved by providing compounds derived, in accordance with the present invention, from a class of pyrazolopyrimidines and salts thereof, methods of preparing pyrazolopyrimidines, a pharmaceutical composition containing said pyrazolopyrimidines, use of said pyrazolopyrimidines and a method for treating diseases with said pyrazolopyrimidines, all in accordance with the description, as defined in the claims of the present Application. Such a pharmacological profile is highly desirable for treating diseases of dysregulated vascular growth or diseases which are accompanied with dysregulated vascular growth, in particular solid tumours and metastases thereof, as well as retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel, diseases such as coronary and peripheral artery disease.

The present invention thus relates to compounds of general formula (I) :

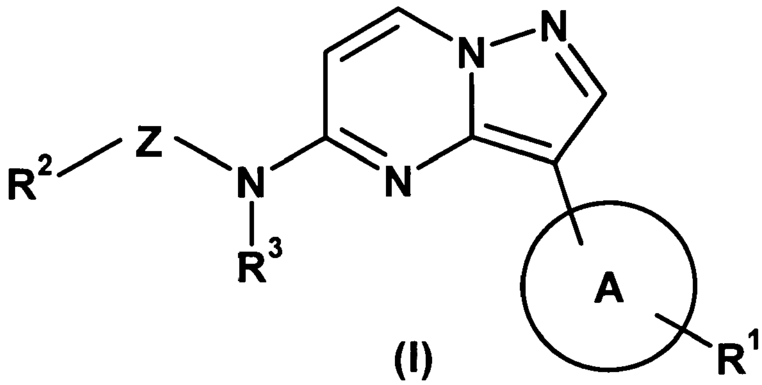

(I)

wherein :

A represents aryl or heteroaryl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, -$(CH_2)_n$heteroaryl, -O$(CH_2)_p$aryl, -O$(CH_2)_q$heteroaryl,C(O)$R^5$, -C$(O)_2R^5$, -$NR^4$C(O)$R^5$, -$NR^4$S$(O)_2R^5$, -C(O)$NR^6R^7$,OC(O)$NR^6R^7$, -$NR^4$C$(O)_2R^5$, -$NR^4$C(O)$NR^6R^7$, -S(O)$R^5$, -S$(O)_2R^5$,S$(O)_2NR^6R^7$, wherein $C_1$-$C_6$-alkyl, $C_1C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,O$(CH_2)_p$aryl, -O$(CH_2)_q$heteroaryl is optionally substituted one or more times, in the same way or differently with halogen, hydroxyl, cyano, nitro, -C$(O)_2R^5$, or a -$NR^6R^7$ group, or
the moiety:

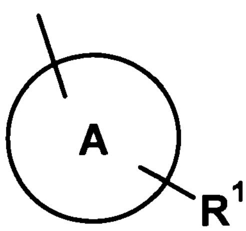

represents :

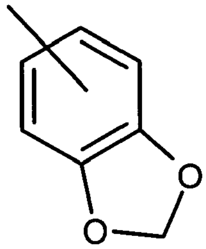

, or

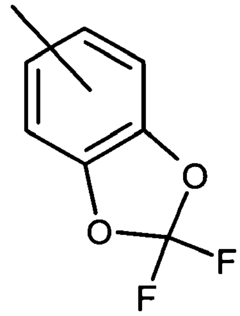

,

Z represents a linker group which is a bond, $C_1$-$C_6$-alkyl, -C(O)-,C(O)$NR^8$-, or -$S(O)_2$ group, in which $C_1$-$C_6$-alkyl is optionally substituted one or more times, in the same way or differently with halogen, hydroxyl, $C_1$-$C_6$-haloalkyl, $C_1$-$C_6$-alkoxy, $C_1$-$C_6$-haloalkyloxy, or a -$NR^6R^7$ group,

$R^2$ represents a substituent selected from the group comprising, preferably consisting of, hydrogen, $C_3$-$C_{10}$-cycloalkyl, $C_3$-$C_{10}$-heterocycloalkyl, aryl, heteroaryl, wherein $C_3$-$C_{10}$-cycloalkyl, $C_3$-$C_{10}$-heterocycloalkyl, aryl, heteroaryl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -C(O)$R^5$, -$C(O)_2R^5$, -$NR^4$C(O)$R^5$,- $NR^4S(O)_2R^5$, -C(O)$NR^6R^7$, -OC(O)$NR^6R^7$, -$NR^4C(O)_2R^5$, -$NR^4$C(O)$NR^6R^7$, -S(O)$R^5$, -$S(O)_2R^5$, or -$S(O)_2NR^6R^7$;

or represents the moiety :

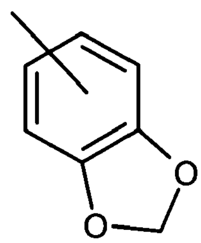

, or

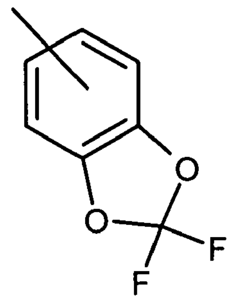

,

with the proviso that when Z is a bond, $R^2$ is not hydrogen,

$R^3$ represents a substituent selected from the group comprising, preferably consisting of, hydrogen, $C_1$-$C_6$-alkyl, $C_1$-$C_6$-haloalkyl, $C_1C_6$-alkoxy, $C_1$-$C_6$-alkoxy-$C_1$-$C_6$-alkyl, or halo-$C_1$-$C_6$-alkoxy-$C_1$-$C_6$-alkyl,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, heteroaryl, $C_1$-$C_6$-alkoxy-$C_1$-$C_6$-alkyl, or halo-$C_1$-$C_6$-alkoxy-$C_1$-$C_6$-alkyl, wherein aryl or heteroaryl is optionally further substituted with the group $C_1$-$C_6$-alkyl, or$NR^6R^7$,

$R^6$ and $R^7$ independently from one another represent a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, $C_1$-$C_6$- haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$ -$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -$S(O)_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C(O)$R^5$, -$C(O)_2R^5$, -$NR^4$C(O)$R^5$,$NR^4S(O)_2R^5$, -C(O)$NR^8R^9$, -OC(O)$NR^8R^9$, -$NR^4C(O)_2R^5$, -$NR^4$C(O)$NR^8R^9$, -S(O)$R^5$, -$S(O)_2R^5$, or -$S(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

The terms as mentioned herein below and in the claims have preferably the following meanings :

The term "alkyl" is to be understood as preferably meaning branched and unbranched alkyl, meaning e.g. methyl, ethyl, *n*-propyl, *iso*-propyl, *n*-butyl, *iso*-butyl, *tert*-butyl, *sec*-butyl, pentyl, *iso*-pentyl, hexyl, heptyl, octyl, nonyl and decyl and the isomers thereof.

The term "alkoxy" is to be understood as preferably meaning branched and unbranched alkoxy, meaning e.g. methoxy, ethoxy, propyloxy, *iso*-propyloxy, butyloxy, *iso*-butyloxy, tert-butyloxy, *sec*-butyloxy, pentyloxy, *iso*-pentyloxy, hexyloxy, heptyloxy, octyloxy, nonyloxy, decyloxy, undecyloxy and dodecyloxy and the isomers thereof.

The term "haloalkyl" is to be understood as preferably meaning branched and unbranched alkyl, meaning *e.g.* methyl, ethyl, *n-propyl, iso*-propyl, *n-butyl, iso-butyl, tert*-butyl, *sec*-butyl, pentyl, *iso*-pentyl, hexyl, heptyl, octyl, nonyl and decyl and the isomers thereof, in which one or more of the hydrogen substituents is replaced in the same way or differently by halogen. More preferably the halogen is fluorine. Particularly preferably haloalkyl is selected from $-CF_3$, $-CHF_2$, $-CH_2F$, $-CF_2CF_3$, or $-CH_2CF_3$.

The term "haloalkyloxy" is to be understood as preferably meaning branched and unbranched alkoxy, meaning e.g. methoxy, ethoxy, propyloxy, *iso*-propyloxy, butyloxy, *iso*-butyloxy, *tert-butyloxy,* sec-butyloxy, pentyloxy, i*so*-pentyloxy, hexyloxy, heptyloxy, octyloxy, nonyloxy, decyloxy, undecyloxy and dodecyloxy and the isomers thereof, in which one or more of the hydrogen substituents is replaced in the same way or differently by halogen. More preferably the halogen is fluorine. Particularly preferably haloalkyloxy is selected from $-OCF_3$, $-OCHF_2$, $-OCH_2F$, $-OCF_2CF_3$, or $-OCH_2CF_3$.

The term "alkoxyalkyl" is to be understood as preferably meaning branched and unbranched alkoxyalkyl, meaning e.g. methoxyalkyl, ethoxyalkyl, propyloxyalkyl, *iso*-propyloxyalkyl, butyloxyalkyl, *iso*-butyloxyalkyl, tert-butyloxyalkyl, sec-butyloxyalkyl, pentyloxyalkyl, iso-pentyloxyalkyl, hexyloxyalkyl, heptyloxyalkyl, octyloxyalkyl, nonyloxyalkyl, decyloxyalkyl, undecyloxyalkyl and dodecyloxyalkyl, wherein the term "alkyl" is defined *supra,* and the isomers thereof.

The term "haloalkoxyalkyl" is to be understood as preferably meaning branched and unbranched alkoxyalkyl, meaning e.g. methoxyalkyl, ethoxyalkyl, propyloxyalkyl, iso-propyloxyalkyl, butyloxyalkyl, *iso*-butyloxyalkyl, tert-butyloxyalkyl, sec-butyloxyalkyl, pentyloxyalkyl, *iso*-pentyloxyalkyl, hexyloxyalkyl, heptyloxyalkyl, octyloxyalkyl, nonyloxyalkyl, decyloxyalkyl, undecyloxyalkyl and dodecyloxyalkyl, wherein the term "alkyl" is defined *supra* , and the isomers thereof, in which one or more of the hydrogen substituents is replaced in the same way or differently by halogen. More preferably the halogen is fluorine. Particularly preferably haloalkoxyalkyl is selected from $-CH_2CH_2OCF_3$, $-CH_2CH_2OCHF_2$, $-CH_2CH_2OCH_2F$, $-CH_2CH_2OCF_2CF_3$, or $-CH_2CH_2OCH_2CF_3$.

The term "halogen" or "hal" is to be understood as preferably meaning fluorine, chlorine, bromine, or iodine.

The term "alkenyl" is to be understood as preferably meaning branched and unbranched alkenyl, e.g. vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl and 2-methyl-prop-1-en-1-yl.

The term "alkynyl" is to be understood as preferably meaning branched and unbranched alkynyl, e.g. ethynyl, prop-1-yn-1-yl, but-1-yn-1-yl, but-2-yn-1-yl and but-3-yn-1-yl.

As used herein, the term "aryl" is defined in each case as having 3-12 carbon atoms, preferably 6-12 carbon atoms, such as, for example, cyclopropenyl, cyclopentadienyl, phenyl, tropyl, cyclooctadienyl, indenyl, naphthyl, azulenyl, biphenyl, fluorenyl, anthracenyl etc, phenyl being preferred.

As used herein, the term "heteroaryl" is understood as meaning an aromatic ring system which comprises 3-16 ring atoms, preferably 5 or 6 or 9 or 10 atoms, and which contains at least one heteroatom which may be identical or different, said heteroatom being such as oxygen, nitrogen or sulfur, and can be monocyclic, bicyclic, or tricyclic, and in addition in each case can be benzocondensed. Preferably, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl etc., and benzo derivatives thereof, such as, e.g., benzofuranyl, benzothienyl, benzoxazolyl, benzimidazolyl, benzotriazolyl, indazolyl, indolyl, isoindolyl, etc.; or pyridyl, pyridazinyl, pyrimidinyl, pyrazinyl, triazinyl, etc., and benzo derivatives thereof, such as, for example, quinolinyl, isoquinolinyl, etc.; or azocinyl, indolizinyl, purinyl, etc., and benzo derivatives thereof; or cinnolinyl, phthalazinyl, quinazolinyl, quinoxalinyl, naphthpyridinyl, pteridinyl, carbazolyl, acridinyl, phenazinyl, phenothiazinyl, phenoxazinyl, xanthenyl, or oxepinyl, etc. More preferably heteroaryl is selected from pyridyl, benzofuranyl,

benzothiophenyl, quinolinyl, thiophenyl, pyrazolyl, or furanyl.

As used herein, the term "$C_1$-$C_6$", as used throughout this text, e.g. in the context of the definition of "$C_1$-$C_6$-alkyl", "$C_1$-$C_6$-haloalkyl", "$C_1$-$C_6$-alkoxy", or "$C_1C_6$-haloalkoxy" is to be understood as meaning an alkyl group having a finite number of carbon atoms of 1 to 6, *i.e.* 1, 2, 3, 4, 5, or 6 carbon atoms. It is to be understood further that said term "$C_1$-$C_6$" is to be interpreted as any sub-range comprised therein, e.g. $C_1$-$C_6$ $C_2$-$C_5$ , $C_3$-$C_4$, $C_1$-$C_2$, $C_1$-$C_3$ $C_1$-$C_4$, $C_1$-$C_5$ $C_1$-$C_6$; preferably $C_1$-$C_2$, $C_1$-$C_3$, $C_1$-$C_4$, $C_1$-$C_5$, $C_1$-$C_6$; more preferably $C_1$-$C_4$; in the case of "$C_1C_6$-haloalkyl" or "$C_1$-$C_6$-haloalkyloxy" even more preferably $C_1$-$C_2$.

Similarly, as used herein, the term "$C_2$-$C_6$", as used throughout this text, e.g. in the context of the definitions of "$C_2$-$C_6$-alkenyl" and "$C_2$-$C_6$-alkynyl", is to be understood as meaning an alkenyl group or an alkynyl group having a finite number of carbon atoms of 2 to 6, *i.e.* 2, 3, 4, 5, or 6 carbon atoms. It is to be understood further that said term "$C_2$-$C_6$" is to be interpreted as any sub-range comprised therein, e.g. $C_2$-$C_6$ , $C_3$-$C_5$ , $C_3$-$C_4$ , $C_2$-$C_3$ , $C_2$-$C_4$, $C_2$-$C_5$; preferably $C_2$-$C_3$.

Further, as used herein, the term "$C_3$-$C_{10}$", as used throughout this text, e.g. in the context of the definitions of "$C_3$-$C_{10}$-cycloalkyl" or "$C_3$-$C_{10}$-heterocycloalkyl", is to be understood as meaning a cycloalkyl group having a finite number of carbon atoms of 3 to 10, *i.e.* 3, 4, 5, 6, 7, 8, 9 or 10 carbon atoms, preferably 3, 4, 5 or 6 carbon atoms. It is to be understood further that said term "$C_3$-$C_{10}$" is to be interpreted as any sub-range comprised therein, e.g. $C_3$-$C_{10}$, $C_4$-$C_9$, $C_5$-$C_8$, $C_6$-$C_7$; Preferably $C_3$-$C_6$.

The term "$C_3$-$C_{10}$-cycloalkyl" is to be understood as preferably meaning cycloalkyl, meaning e.g. cyclopropyl, cyclobutyl, cyclopentyl, cyclohexyl, cycloheptyl, cyclooctyl, cyclononyl and cyclodecyl. $C_3$-$C_{10}$-cycloalkyl ring can optionally be interrupted one or more times, the same or differently with a group -C(O)-, -S(O)- or -$S(O)_2$- and can optionally contain one or more double bonds e.g. cycloalkenyl, such as cyclopropenyl, cyclobutenyl, cyclopentenyl, cyclohexenyl and cycloheptenyl, cyclooctenyl, cyclononenyl, cyclodecenyl, wherein the linkage can be provided to the double or single bond.

The term "$C_3$-$C_{10}$-heterocycloalkyl" preferably is a $C_3$-$C_{10}$-cycloalkyl group which is at least once interrupted by an atom, the same or different, selected from the group comprising, preferably consisting of, nitrogen, oxygen and/or sulfur *e.g.* oxyranyl, oxetanyl, aziridinyl, azetidinyl, tetrahydrofuranyl, pyrrolidinyl, morpholinyl, dithianyl, thiomorpholinyl, piperazinyl, trithianyl and chinuclidinyl. $C_3$-$C_{10}$-heterocycloalkyl ring can optionally be interrupted one or more times, the same or differently with a group -C(O)-, -S(O)- or - $S(O)_2$- and $C_3$-$C_{10}$-heterocycloalkyl ring can optionally contain one or more double bonds, e.g. 4H-pyran, 2H-pyran, 3H-diazirine, 2,5-dihydro-1 H-pyrrole, [1,3]dioxole; 4H-[1,3,4] thiadiazine, 2,5-dihydrofuran, 2,3-dihydrofuran, 2,5-dihydrothiophene, 2,3-dihydrothiophene, 4,5-dihydrooxazote or 4H-[1,4]thiazine.

The term "isomers" is to be understood as meaning chemical compounds with the same number and types of atoms as another chemical species. There are two main classes of isomers, constitutional isomers and stereoisomers.

The term "constitutional isomers" is to be understood as meaning chemical compounds with the same number and types of atoms, but they are connected in differing sequences. There are functional isomers, structural isomers, tautomers or valence isomers.

In stereoisomers, the atoms are connected sequentially in the same way, such that condensed formulae for two isomeric molecules are identical. The isomers differ, however, in the way the atoms are arranged in space. There are two major sub-classes of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centres, as long as each centre is the exact mirror image of the corresponding centre in the other molecule. If one or more of these centres differs in configuration, the two molecules are no longer mirror images. Stereoisomers which are not enantiomers are called diastereomers. Diastereomers which still have a different constitution, are another sub-class of diastereomers, the best known of which are simple *cis* - trans isomers.

In order to limit different types of isomers from each other reference is made to IUPAC Rules Section E *(*Pure Appl Chem 45, 11-30, 1976).

The compound according to Fomula (I) can exist in free form or in a salt form. A suitably pharmaceutically acceptable salt of the pyrazolopyrimidines of the present invention may be, for example, an acid-addition salt of a pyrazolopyrimidine of the invention which is sufficiently basic, for example, an acid-addition salt with, for example, an inorganic or organic acid, for example hydrochloric, hydrobromic, sulfuric, phosphoric, trifluoroacetic, para-toluenesulfonic, methylsulfonic, citric, tartaric, succinic or maleic acid. In addition, another suitably pharmaceutically acceptable salt of a pyrazolopyrimidine of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glucamine, dimethylglucamine, ethylglucamine, lysine, 1,6-hexadiamine, ethanolamine, glucosamine, sarcosine, serinol, tris-hydroxy-methyl-aminomethane, aminopropandiol, sovak-base, 1-amino-2,3,4-butantriol.

The compound according to Formula (I) can exist as N-oxides which are defined in that at least one nitrogen of the compounds of the general Formula (I) may be oxidised.

The compound according to Formula (I) can exist as solvates, in particular as hydrate, wherein the compound according to Formula (I) may contain polar solvents, in particular water, as structural element of the crystal lattice of the compounds. The amount of polar solvents, in particular water, may exist in a stoichiometric or unstoichiometric ratio. In case of stoichiometric solvates, *e.g.* hydrate, are possible hemi-, (semi-), mono-, sesqui-, di-, tri-, tetra-, penta- etc. solvates or hydrates, respectively.

As used herein, the term "*in vivo* hydrolysable ester" is understood as meaning an *in vivo* hydrolysable ester of a compound of formula (I) containing a carboxy or hydroxy group, for example, a pharmaceutically acceptable ester which is hydrolysed in the human or animal body to produce the parent acid or alcohol. Suitable pharmaceutically acceptable esters for carboxy include for example alkyl, cycloalkyl and optionally substituted phenylalkyl, in particular benzyl esters, $C_1$-$C_6$ alkoxymethyl esters, e.g. methoxymethyl, $C_1$-$C_6$ alkanoyloxymethyl esters, e.g. pivaloyloxymethyl, phthalidyl esters, $C_3$-$C_8$ cycloalkoxy-carbonyloxy-$C_1$-$C_6$ alkyl esters, e.g. 1-cyclohexylcarbonyloxyethyl ; 1,3-dioxolen-2-onylmethyl esters, e.g. 5-methyl-1,3-dioxolen-2-onylmethyl ; and $C_1$-$C_6$-alkoxycarbonyloxyethyl esters, e.g. 1-methoxycarbonyloxyethyl, and may be formed at any carboxy group in the compounds of this invention. An *in vivo* hydrolysable ester of a compound of formula (I) containing a hydroxy group includes inorganic esters such as phosphate esters and [alpha]-acyloxyalkyl ethers and related compounds which as a result of the *in vivo* hydrolysis of the ester breakdown to give the parent hydroxy group. Examples of [alpha]-acyloxyalkyl ethers include acetoxymethoxy and 2,2-dimethylpropionyloxymethoxy. A selection of *in vivo* hydrolysable ester forming groups for hydroxy include alkanoyl, benzoyl, phenylacetyl and substituted benzoyl and phenylacetyl, alkoxycarbonyl (to give alkyl carbonate esters), dialkylcarbamoyl and N-(dialkylaminoethyl)-N-alkylcarbamoyl (to give carbamates), dialkylaminoacetyl and carboxyacetyl.

Compounds of general formula (I), *supra,* of the present invention are preferred wherein :

A represents aryl or heteroaryl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycLoalkyl, aryl,$(CH_2)_m$aryl, -$(CH_2)_n$heteroaryl, -O$(CH_2)_p$aryl, -O$(CH_2)_q$heteroaryl,C(O)$R^5$, -C$(O)_2R^5$, -$NR^4$C(O)$R^5$, -$NR^4$S$(O)_2R^5$, -C(O)$NR^6R^7$,OC(O)$NR^6R^7$, -$NR^4$C$(O)_2R^5$, -$NR^4$C(O)$NR^6R^7$, -S(O)$R^5$, -S$(O)_2R^5$,S$(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,O$(CH_2)_p$aryl, -O$(CH_2)_q$heteroaryl is optionally substituted one or more times, in the same way or differently with halogen, hydroxyl, cyano, nitro, -C$(O)_2R^5$, or a -$NR^6R^7$ group, or

the moiety:

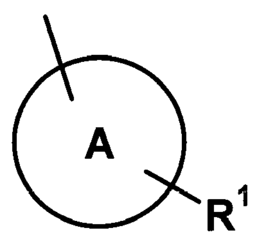

represents :

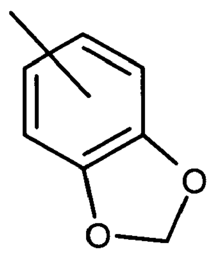

, or

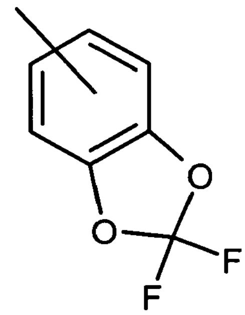

;

Z represents a linker group which is a bond, or $C_1C_6$-alkyl, in which $C_1$-$C_6$-alkyl is optionally substituted one or more times, in the same way or differently with halogen, hydroxyl, $C_1$-$C_6$-haloalkyl, $C_1$-$C_6$-alkoxy, $C_1$-$C_6$-haloalkyloxy, or a -$NR^6R^7$ group,

$R^2$ represents a substituent selected from the group comprising, preferably consisting of, hydrogen, $C_3$-$C_{10}$-cycloalkyl, $C_3$-$C_{10}$-heterocycloalkyl, aryl, heteroaryl, wherein $C_3$-$C_{10}$-cycloalkyl, $C_3$-$C_{10}$-heterocycloalkyl, aryl, heteroaryl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -C(O)$R^5$, -C(O)$_2R^5$, -$NR^4C(O)R^5$,$NR^4S(O)_2R^5$, -C(O)$NR^6R^7$, -OC(O)$NR^6R^7$, -$NR^4C(O)_2R^5$, -$NR^4C(O)NR^6R^7$, -S(O)$R^5$, -S(O)$_2R^5$, or -S(O)$_2NR^6R^7$;

or represents the moiety :

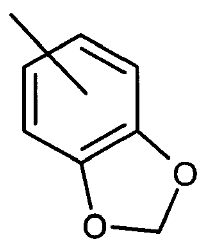

, or

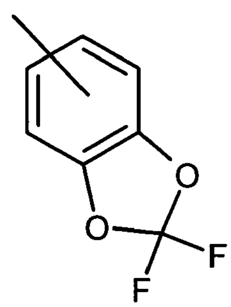

,

with the proviso that when Z is a bond, $R^2$ is not hydrogen,

$R^3$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, heteroaryl, $C_1$-$C_6$-alkoxy-$C_1$-$C_6$-alkyl, or halo-$C_1$-$C_6$-alkoxy-$C_1$-$C_6$-alkyl, wherein aryl or heteroaryl is optionally further substituted with the group $C_1$-$C_6$-alkyl, or$NR^6R^7$,

$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -S(O)$_2$-group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C(O)$R^5$, -C(O)$_2R^5$, -NR $^4$C(O)$R^5$,NR$^4$S(O)$_2R^5$, -C(O)NR$^8R^9$, -OC(O)NR$^8R^9$, -NR$^4$C(O)$_2R^5$, -NR$^4$C(O)NR$^8R^9$, -S(O)$R^5$, -S(O)$_2R^5$, or -S(O)$_2$NR$^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

Compounds of general formula (I), *supra,* of the present invention are more preferred wherein :

A represents aryl or heteroaryl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyL, halo-$C_1$-$C_6$-alkoxy-$C_1$-$C_6$-alkyL, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, -$(CH_2)_n$heteroaryl, -O$(CH_2)$paryl, -O$(CH_2)_q$heteroaryl,C(O)$R^5$, -C(O)$_2R^5$, -NR$^4$C(O)$R^5$, -NR$^4$S(O)$_2R^5$, -C(O)NR$^6R^7$,OC(O)NR $^6R^7$, -NR$^4$C(O)$_2R^5$, -NR$^4$C(O)NR$^6R^7$, -S(O)$R^5$, -S(O)$_2R^5$,S(O)$_2$NR$^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,O$(CH_2)$paryl, -O$(CH_2)_q$heteroaryl is optionally substituted one or more times, in the same way or differently with halogen, hydroxyl, cyano, nitro, -C(O)$_2R^5$, or a -NR$^6R^7$ group, or the moiety:

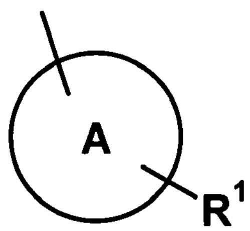

represents :

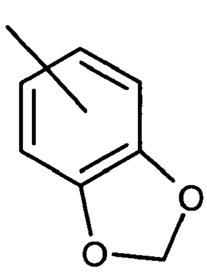

, or

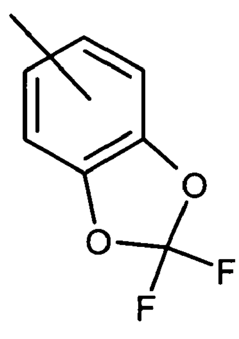

;

Z represents a linker group which is a bond, or $C_1$-$C_6$-alkyl,

$R^2$ represents a substituent selected from the group comprising, preferably consisting of $C_3$-$C_{10}$-cycloalkyl, $C_3$-$C_{10}$-heterocycloalkyl, aryl, heteroaryl, wherein $C_3$-$C_{10}$-cycloalkyl, $C_3$-$C_{10}$-heterocycloalkyl, aryl, heteroaryl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), $-NR^6R^7$, $-C(O)R^5$, $-C(O)_2R^5$, $-NR^4C(O)R^5$,$NR^4S(O)_2R^5$, $-C(O)NR^6R^7$, $-OC(O)NR^6R^7$, $-NR^4C(O)_2R^5$, $-NR^4C(O)NR^6R^7$, $-S(O)R^5$, $-S(O)_2R^5$, or $-S(O)_2NR^6R^7$;
or represents the moiety :

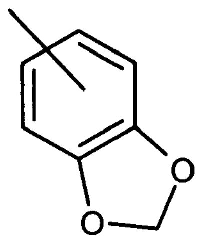

, or

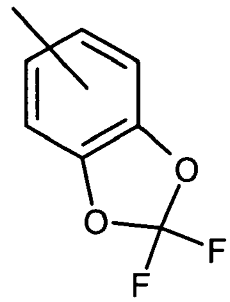

,

$R^3$ is hydrogen,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, $C_1$-$C_6$-alkoxy-$C_1$-$C_6$-alkyl, or halo-$C_1$-$C_6$-alkoxy-$C_1$-$C_6$-alkyl, wherein aryl is optionally further substituted with the group $C_1$-$C_6$-alkyl, or $-NR^6R^7$,

$R^6$ and $R^7$ independently from one another represent a substituent selected from the group comprising, preferably consisting of, hydrogen, $C_1$-$C_6$-alkyl , $C_3$-$C_{10}$-cycloalyl, $C_1$-$C_6$-haloalkyl, $C_1$-$C_6$-alkoxy, $C_1$-$C_6$- haloalkoxy-alkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or $-NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or $-S(O)_2-$ group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, $-C(O)R^5$, $-C(O)_2R^5$, $-NR^4C(O)R^5$,$NR^4S(O)_2R^5$, $-C(O)NR^8R^9$, $-OC(O)NR^8R^9$, $-NR^4C(O)_2R^5$, $-NR^4C(O)NR^8R^9$, $-S(O)R^5$, $-S(O)_2R^5$, or $-S(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

Compounds of general formula (I), *supra,* of the present invention are more particularly preferred wherein :

A represents aryl or heteroaryl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, -$(CH_2)_n$heteroaryl, -$O(CH_2)_p$aryl, -$O(CH_2)_q$heteroaryl,$C(O)R^5$,- $C(O)_2R^5$, -$NR^4C(O)R^5$, -$NR^4S(O)_2R^5$, -$C(O)NR^6R^7$,$OC(O)NR^6R^7$, -$NR^4C(O)_2R^5$, -$NR^4C(O)NR^6R^7$, -$S(O)R^5$, -S $(0)_2R^5$,$S(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,O $(CH_2)_p$aryL, -$O(CH_2)_q$heteroaryl is optionally substituted one or more times, in the same way or differently with halogen, hydroxyl, cyano, nitro, -$C(O)_2R^5$, or a -$NR^6R^7$ group, or
the moiety :

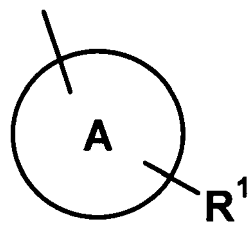

represents :

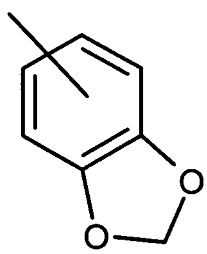

, or

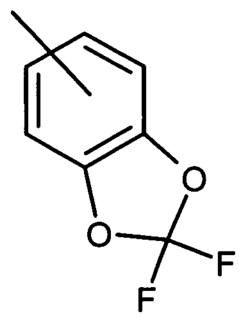

;

Z represents a linker group which is a bond, or $C_1$-$C_6$-alkyl,

$R^2$ represents a substituent selected from the group comprising, preferably consisting of $C_3$-$C_{10}$-cycloalkyl, aryl, heteroaryl, wherein $C_3$-$C_{10}$-cycloalkyl, aryl, heteroaryl is optionally substituted one or more times, in the same way or differently with halogen, hydroxy, cyano, nitro, $C_1$-$C_6$-alkyl, $C_1$-$C_6$-haloalkyl, $C_1C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl,- O(phenyl), -$NR^6R^7$, -C(O) $R^5$, -$C(O)_2R^5$, -$NR^4C(O)R^5$,$NR^4S(O)_2R^5$, -$C(O)NR^6R^7$, -$OC(O)NR^6R^7$, -$NR^4C(O)_2R^5$, -$NR^4C(O)NR^6R^7$, -$S(O)R^5$, -$S(O)_2R^5$, or -$S(O)_2NR^6R^7$;
or represents the moiety :

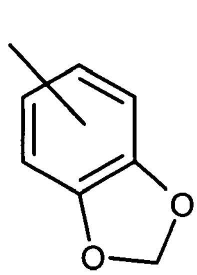

, or

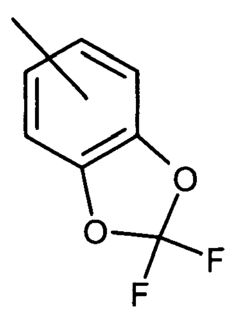

,

$R^3$ is hydrogen,

$R^4$ represents a substituent selected from the group comprising, preferably consisting of, hydrogen, $C_1$-$C_6$-alkyl, $C_1$-$C_6$-haloalkyl, $C_1$-$C_6$-aLkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, $C_1$-$C_6$-alkoxy-$C_1$-$C_6$-alkyl, or halo-$C_1$-$C_6$-alkoxy-$C_1$-$C_6$-alkyl, wherein aryl is optionally further substituted with the group $C_1$-$C_6$-alkyl, or -$NR^6R^7$,

$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -$S(O)_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C(O)$R^5$, -$C(O)_2R^5$, -$NR^4C(O)R^5$,$NR^4S(O)_2R^5$, -C(O)$NR^8R^9$, -OC(O)$NR^8R^9$, -$NR^4C(O)_2R^5$, -$NR^4C(O)NR^8R^9$, -S(O)$R^5$, -$S(O)_2R^5$, or -$S(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

Compounds of general formula (I), *supra,* of the present invention are yet more particularly preferred wherein :

A represents phenyl, naphthyl, pyridyl, benzofuranyl, benzothiophenyl, quinolinyl, thiophenyl, pyrazolyl, furanyl, wherein A is optionally substituted in the same way or differently with one or more $R^1$ groups,

$R^1$ represents a substituent selected from the group comprising, preferably consisting of, hydrogen, halogen, hydroxy, cyano, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_3$-$C_{10}$-heterocycloalkyl, -($CH_2$)phenyl, -O$(CH_2)_p$phenyl, -C(O)$R^5$,$C(O)_2R^5$, -$NR^4C(O)R^5$, -$NR^4S(O)_2R^5$, -C(O)$NR^6R^7$, -$NR^4C(O)_2R^5$, -S(O)$R^5$, -$S(O)_2R^5$, -$S(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-aLkenyl, $C_3$-$C_{10}$-heterocycloalkyl is optionally substituted one or more times, in the same way or differently with halogen, hydroxyl, cyano, nitro, -$C(O)_2R^5$, or a$NR^6R^7$ group, or

the moiety:

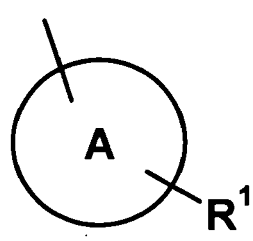

represents :

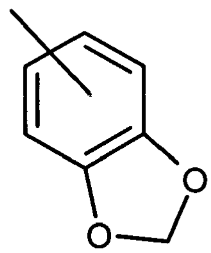

, or

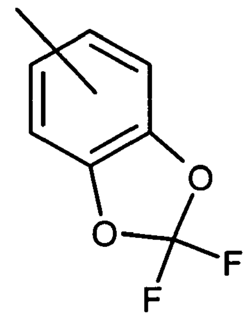

;

Z represents a linker group which is a bond, or $C_1$-$C_6$-alkyl,

$R^2$ represents a substituent selected from the group comprising, preferably consisting of $C_3$-$C_{10}$-cycloalkyl, phenyl, or pyridyl, wherein $C_3$-$C_{10}$-cycloalkyl, phenyl, or pyridyl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -C(O)$R^5$, -C(O)$_2R^5$,$NR^4$C(O)$R^5$, -$NR^4$S(O)$_2R^5$, -C(O)$NR^6R^7$, -OC(O)$NR^6R^7$, -$NR^4$C(O)$_2R^5$,$NR^4$C(O)$NR^6R^7$, -S(O)$R^5$, -S(O)$_2R^5$, or -S(O)$_2NR^6R^7$;
or represents the moiety :

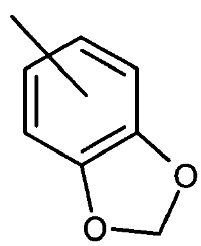

, or

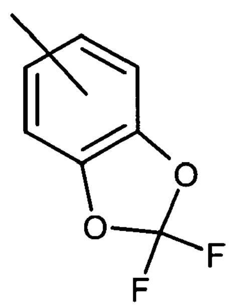

,

$R^3$ is hydrogen,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, phenyl, $C_1$-$C_6$-alkoxy-$C_1$-$C_6$-alkyl, or halo-$C_1$-$C_6$-alkoxy-$C_1$-$C_6$-alkyl, wherein phenyl is optionally further substituted with the group $C_1$-$C_6$-alkyl, or -$NR^6R^7$,

$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -S(O)$_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-aLkoxy-$C_1$-$C_6$-alkyl, halo-$C_1$-$C_6$-alkoxy-$C_1$-$C_6$-alkyl, -C(O)$R^5$, -C(O)$_2R^5$, -$NR^4$C(O)$R^5$,$NR^4$S(O)$_2R^5$, -C(O)$NR^8R^9$, -OC(O)$NR^8R^9$, -$NR^4$C(O)$_2R^5$, -$NR^4$C(O)$NR^8R^9$, -S(O)$R^5$, -S(O)$_2R^5$, or -S(O)$_2NR^8R^9$, wherein $C_1$-$C_6$-alkyl may be further optionally substituted with hydroxy,

$R^8$ and $R^9$ independently from one another, represent a substituent selected from the group comprising, preferably

consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy and

p represents an integer of 0, or 1,

as well as :

N-oxides, solvates, hydrates, isomers, diastereomers, enantiomers and salts thereof.

In accordance with a variant, compounds of general formula (I), *supra,* of the present invention are preferred wherein :

A phenyl, naphthyl, pyridyl, benzofuranyl, benzothiophenyl, quinolinyl, thiophenyl, pyrazolyl, furanyl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, -$(CH_2)_n$heteroaryl, -O$(CH_2)_p$aryl, -O$(CH_2)_q$heteroaryl,C(O)$R^5$, -C$(O)_2R^5$, -$NR^4$C(O)$R^5$, -$NR^4S(O)_2R^5$, -C(O)$NR^6R^7$,OC(O)NR $^6R^7$, -$NR^4C(O)_2R^5$, -$NR^4$C(O)$NR^6R^7$, -S(O)$R^5$, -S$(O)_2R^5$,S$(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,O$(CH_2)_p$aryl, -O$(CH_2)_q$heteroaryl is optionally substituted one or more times, in the same way or differently with halogen, hydroxyl, cyano, nitro, -C$(O)_2R^5$, or a -$NR^6R^7$ group, or

the moiety :

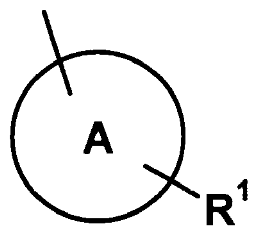

represents :

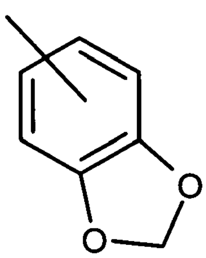

, or

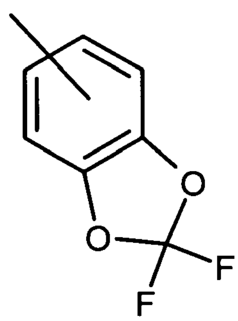

;

Z represents $C_1$-$C_6$-alkyl, in which $C_1$-$C_6$-alkyl is optionally substituted one or more times, in the same way or differently with halogen, hydroxyl, $C_1$-$C_6$-haloalkyl, $C_1C_6$-alkoxy, $C_1$-$C_6$-haloalkyloxy, or a -$NR^6R^7$ group,

$R^2$ is hydrogen,

$R^3$ is hydrogen,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, heteroaryl, $C_1$-$C_6$-alkoxy-$C_1$-$C_6$-alkyl,

or halo-$C_1$-$C_6$-alkoxy-$C_1$-$C_6$-alkyl, wherein aryl or heteroaryl is optionally further substituted with the group $C_1$-$C_6$-alkyl, or$NR^6R^7$

$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-aLkoxy, $C_1$-$C_6$-haloalkoxyalkyl, $C_1$-$C_6$-alkoxy-$C_1$-$C_6$-alky, or halo-$C_1$-$C_6$-alkoxy-$C_1$-$C_6$-alkyl, wherein $C_1C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S (O)- and/or -$S(O)_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C (O)$R^5$, -$C(O)_2R^5$, -$NR^4C(O)R^5$,$NR^4S(O)_2R^5$, -$C(O)NR^8R^9$, -$OC(O)NR^8R^9$, -$NR^4C(O)_2R^5$, -$NR^4C(O)NR^8R^9$, -$S(O)R^5$, -$S(O)_2R^5$, or -$S(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

More particularly preferred still, are compounds according to general formula (I) which are selected from the group consisting of :

*N*-(2-Dimethylamino-ethyl)-3-[5-(4-isopropyl-phenylamino)-pyrazolo[1,5-a]pyrimidin-3-yl]-benzamide;
Phenyl-[3-(3,4,5-trimethoxy-phenyl)-pyrazolo[1,5-a]pyrimidin-5-yl]-amine;
(4-Fluoro-phenyl)-[3-(3,4, 5-trimethoxy-phenyl)-pyrazolo[1, 5-a]pyrimidin-5-yl]-amine;
(3-Pyridin-4-yl-pyrazolo[1,5-a] pyrimidin-5-yl)-(3,4,5-trimethoxyphenyl)-amine;
[3-(2,4-Dimethoxy-pyrimidin-5-yl)-pyrazolo[1,5-a]pyrimidin-5-yl]-(3,4,5-trimethoxy-phenyl)-amine;
(3-Benzofuran-2-yl-pyrazolo[1,5-a]pyrimidin-5-yl)-(3,4,5-trimethoxyphenyl)-amine;
(3-Benzo[b]thiophen-2-yl-pyrazolo[1,5-a]pyrimidin-5-yl)-(3,4,5-trimethoxy-phenyl)-amine;
N-{3-[5-(3,4,5-Trimethoxy-phenylamino)-pyrazolo[1,5-a]pyrimidin-3-yl] - phenyl}-acetamide;
N-[4-(3-Benzo[1, 3]dioxol-5-yl-pyrazolo[1, 5-a]pyrimidin-5-ylamino)-phenyl]-acetamide;
(3-Benzo[1,3]dioxol-5-yl-pyrazolo[1,5-a]pyrimidin-5-yl)-(6-methoxy-pyridin-3-yl)-amine;
(6-Methoxy-pyridin-3-yl)-(3-quinolin-8-yl-pyrazolo[1,5-a]pyrimidin-5-yl)-amine;
4-[5-(6-Methoxy-pyridin-3-ylamino)-pyrazolo[1,5-a]pyrimidin-3-yl]-phenol;
(3-Benzo[1,3]dioxol-5-yl-pyrazolo[1,5-a]pyrimidin-5-yl)-(1H-indol-5-yl)-amine;
(3-Benzofuran-2-yl-pyrazolo[1,5-a]pyrimidin-5-yl)-(1H-indol-5-yl)-amine; (1H-Indol-5-yl)-(3-thiophen-3-yl-pyrazolo [1,5-a]pyrimidin-5-yl)-amine;
[3-(3,4-Dimethoxy-phenyl)-pyrazolo[1,5-a]pyrimidin-5-yl]-(1H-indol-5-yl)-amine;
4-[5-(1H-Indol-5-ylamino)-pyrazolo[1,5-a]pyrimidin-3-yl]-phenol;
N-{3-[5-(1H-Indol-5-ylamino)-pyrazolo[1,5-a]pyrimidin-3-yl]-phenyl}-acetamide;
Benzo[1,3]dioxol-5-ylmethyl-[3-(2,4-dimethoxy-pyrimidin-5-yl)-pyrazolo[1,5-a]pyrimidin-5-yl]-amine;
Benzo[1,3]dioxol-5-ylmethyl-(3-pyridin-3-yl-pyrazolo[1,5-a]pyrimidin-5-yl)-amine;
Benzo[1,3]dioxol-5-ylmethyl-(3-benzofuran- 2-yl-pyrazolo[1,5-a]pyrimidin-5-yl)-amine;
Benzo[1,3]dioxol-5-ylmethyl-(3-quinolin-8-yl-pyrazolo[1,5-a]pyrimidin-5-yl)-amine;
N-(3-{5-[(Benzo[1,3]dioxol-5-ylmethyl)-amino]-pyrazolo[1,5-a]pyrimidin-3-yl}-phenyl)-acetamide;
[3-(3,4-Dimethoxy-phenyl)-pyrazolo[1,5-a]pyrimidin-5-yl]-(4-morpholin-4-yl-phenyl)-amine;
4-[5-(4-Morpholin-4-yl-phenylamino)-pyrazolo[1,5-a]pyrimidin-3-yl]-phenol;
N-{3-[5-(4-Morpholin-4-yl-phenylamino)-pyrazolo[1,5-a]pyrimidin-3-yl]-phenyl}-acetamide;
1-{5-[5-(4-Phenoxy-phenylamino)-pyrazolo[1,5-a]pyrimidin-3-yl]-thiophen-2-yl}-ethanone;
(4-Phenoxy-phenyl)- (3-quinolin-8-yl-pyrazolo[1 5-a]pyrimidin-5-yl)-amine;

[3- (3 ,4-Dimethoxy-phenyl)-pyrazolo[1 5-a] pyrimidin-5-yl]-(4-phenoxyphenyl)-amine;
1-{5-[5-(4-Isopropyl-phenylamino)-pyrazolo[1,5-a]pyrimidin-3-yl]-thiophen-2-yl}-ethanone;
(4-Isopropyl-phenyl)-(3-pyridin-4-yl-pyrazolo[1,5-a]pyrimidin-5-yl)-amine;
[3-(3,4-Dimethoxy-phenyl)-pyrazolo[1,5-a] pyrimidin-5-yl]-(4-isopropylphenyl)-amine;
3-[5-(4-Phenoxy-phenylamino)-pyrazolo[1,5-a]pyrimidin-3-yl]-benzamide;
4-[5-(4-Phenoxy-phenylamino)-pyrazolo[1,5-a] pyrimidin-3-yl]-benzoic acid methyl ester;
[3-(3-Methoxy-phenyl)-pyrazolo[1,5-a]pyrimidin-5-yl]-(4-phenoxyphenyl)-amine;
[3-(2,4-Dimethoxy-phenyl)-pyrazolo[1, 5-a] pyrimidin-5-yl]-(4-phenoxyphenyl)-amine;
N-{3-[5-(4-Phenoxy-phenylamino)-pyrazolo[1, 5-a]pyrimidin-3-yl]-phenyl}-methanesulfonamide;
[3-(1-Benzyl-1 H-pyrazol-4-yl)-pyrazolo[1,5-a]pyrimidin-5-yl]-(4-phenoxyphenyl)-amine;
4-(3-Benzo[1,3]dioxol-5-yl-pyrazolo[1,5-a]pyrimidin-5-ylamino)-phenol;
4-[3-(2,4-Dimethoxy-pyrimidin-5-yl)-pyrazolo[1, 5-a] pyrimidin-5-ylamino]-phenol;
4-(3-Benzofuran-2-yl-pyrazolo[1,5-a]pyrimidin-5-ylamino)-phenol;
4-(3-Thiophen-3-yl-pyrazolo[1,5-a]pyrimidin-5-ylamino)-phenol;
4-[5-[(4-hydroxyphenyl)amino]pyrazolo[1,5-a]pyrimidin-3-yl]-phenol;
N-{3-[5-(4-Hydroxy-phenylamino)-pyrazolo[1,5-a]pyrimidin-3-yl]-phenyl}-acetamide;
4-Methyl-N-[4-(3-pyridin-4-yl-pyrazolo[1,5-a]pyrimidin-5-ylamino)-phenyl]-benzenesulfonamide;
4-Methyl-N-[4-(3-pyridin-3-yl-pyrazolo[1, 5-a] pyrimidin-5-ylamino)-phenyl]-benzenesulfonamide;
N-[4-(3-Benzofuran-2-yl-pyrazolo[1,5-a]pyrimidin-5-ylamino)-phenyl]-4-methyl-benzenesulfonamide;
4-Methyl-N-[4-(3-thiophen-3-yl-pyrazolo[1,5-a]pyrimidin-5-ylamino)-phenyl]-benzenesulfonamide;
4-Methyl-N-[4-(3-quinolin-8-yl-pyrazolo[1,5-a]pyrimidin-5-ylamino)-phenyl]-benzenesulfonamide;
N-{4-[3-(3,4-Dimethoxy-phenyl)-pyrazolo[1,5-a]pyrimidin-5-ylamino]-phenyl}-4-methyl-benzenesulfonamide;
N-[5-(3-Benzofuran-2-yl-pyrazolo[1,5-a]pyrimidin-5-ylamino)-2-methylphenyl]-methanesulfonamide;
N-[5-(3-Benzo[b]thiophen-2-yl-pyrazolo[1,5-a]pyrimidin-5-ylamino)-2-methyl-phenyl]-methanesulfonamide;
N-[2-Methyl-5-(3-quinolin-8-yl-pyrazolo[1,5-a]pyrimidin-5-ylamino)-phenyl]-methanesulfonamide;
N-{5- [3-(4-Hydroxy-phenyl)-pyrazolo[1,5-a]pyrimidin-5-ylamino]-2-methyl-phenyl}-methanesulfonamide;
(3-Benzo[1,3]dioxol-5-yl-pyrazolo[1,5-a]pyrimidin-5-yl)-pyridin-4-ylmethyl-amine;
(3-Benzofuran-2-yl-pyrazolo[1,5-a]pyrimidin-5-yl)-pyridin-4-ylmethyl-amine;
Pyridin-4-ylmethyl- (3-thiophen- 3-yl-pyrazolo[1 5-a]pyrimidin-5-yl)-amine;
(3-Benzo[b]thiophen-2-yl-pyrazolo[1,5-a]pyrimidin-5-yl)-pyridin-4-ylmethyl-amine;
[3-(3,4-Dimethoxy-phenyl)-pyrazoto[1,5-a]pyrimidin-5-yl]-pyridin-4-ylmethyl-amine;
4-{5-[(Pyridin-4-ylmethyl)-amino]-pyrazolo[1,5-a]pyrimidin-3-yl}-phenol;
4-(3-Pyridin-3-yl-pyrazolo[1,5-a]pyrimidin-5-ylamino)-cyclohexanol;
4-(3-Quinolin-8-yl-pyrazolo[1,5-a]pyrimidin-5-ylamino)-cyclohexanol;
4-[3-(3,4-Dimethoxy-phenyl)-pyrazolo[1,5-a]pyrimidin-5-ylamino]-cyclohexanol;
2-(3-Benzo[1,3]dioxol-5-yl-pyrazolo[1,5-a]pyrimidin-5-ylamino)-ethanol;
2-(3-Benzofuran-2-yl-pyrazolo[1,5-a]pyrimidin-5-ylamino)-ethanol;
2-(3-Quinolin-8-yl-pyrazolo[1,5-a]pyrimidin-5-ytamino)-ethanol;
2-[3-(3,4-Dimethoxy-phenyl)-pyrazolo[1,5-a]pyrimidin-5-ylamino]-ethanol;
4-[5-(2-Hydroxy-ethylamino)-pyrazolo[1,5-a]pyrimidin-3-yl]-phenol;
3-{4-[5-(3 ,4, 5- Trimethoxy-phenylamino)-pyrazolo[1,5-a]pyrimidin-3-yl]-phenyl}-propionic acid;
[3-(4-Trifluoromethoxy-phenyl)-pyrazolo[1,5-a]pyrimidin-5-yl]-(3,4,5-trimethoxy-phenyl)-amine;
(3,4,5-Trimethoxy-phenyl)-[3-(3,4,5-trimethoxy-phenyl)-pyrazolo[1,5-a]pyrimidin-5-yl]-amine;
[3-(4-Chloro-phenyl)-pyrazolo[1,5-a]pyrimidin-5-yl]-(3,4,5-trimethoxyphenyl)-amine;
N-(2-Hydroxy-ethyl)-3-[5-(3,4,5-trimethoxy-phenylamino)-pyrazolo[1,5-a]pyrimidin-3-yl]-benzamide;
[3-(5-Methoxy-pyridin-3-yl)-pyrazolo[1,5-a]pyrimidin-5-yl]-(3,4,5-trimethoxy-phenyl)-amine;
3-{4-[5-(4-Acetylamino-phenylamino)-pyrazolo[1,5-a]pyrimidin-3-yl]-phenyl}-propionic acid;
3-[5-(4-Acetylamino-phenylamino)-pyrazolo[1,5-a]pyrimidin-3-yl]-benzoic acid;
3-{4-[5-(4-Hydroxy-phenylamino)-pyrazolo[1,5-a] pyrimidin-3-yl]-phenyl}-propionic acid;
4-[3-(2-Methoxy-phenyl)-pyrazolo[1,5-a]pyrimidin-5-ylamino]-phenol;
4-[3-(4-Trifluoromethoxy-phenyl)-pyrazolo[1,5-a]pyrimidin-5-ylamino]-phenol;
4-[5-(4-Hydroxy-phenylamino)-pyrazolo[1, 5-a]pyrimidin-3-yl]-benzonitrile;
4-[3-(5-Methoxy-pyridin-3-yl)-pyrazolo[1,5-a]pyrimidin-5-ylamino]-phenol;
[3-(2-Methoxy-phenyl)-pyrazolo[1,5-a]pyrimidin-5-yl]-(6-methoxy-pyridin-3-yl)-amine;
(6-Methoxy-pyridin-3-yl)-[3-(4-trifluoromethoxy-phenyl)-pyrazolo[1,5-a]pyrimidin-5-yl]-amine;
(6-Methoxy-pyridin-3-yl)-[3-(3,4, 5-trimethoxy-phenyl)-pyrazolo[1,5-a]pyrimidin-5-yl]-amine;
(6-Methoxy-pyridin-3-yl)-[3-(3-trifluoromethoxy-phenyl)-pyrazolo[1,5-a]pyrimidin-5-yl]-amine;
N-(2-Hydroxy-ethyl)-3-[5-(6-methoxy-pyridin-3-ylamino)-pyrazolo[1,5-a]pyrimidin-3-yl]-benzamide;

(6-Methoxy-pyridin-3-yl)-[3-(5-methoxy-pyridin-3-yl)-pyrazolo[1,5-a]pyrimidin-5-yl]-amine;
N-{4-[3-(5-Methoxy-pyridin-3-yl)-pyrazolo[1,5-a]pyrimidin-5-ylamino]-phenyl}-4-methyl-benzenesulfonamide;
3-{4-[5-(4-Isopropyl-phenylamino)-pyrazolo[1,5-a]pyrimidin-3-yl]-phenyl}-propionic acid;
(4-Isopropyl-phenyl)-[3-(4-trifluoromethoxy-phenyl)-pyrazolo[1,5-a]pyrimidin-5-yl]-amine;
(4-Isopropyl-phenyl)-[3-(3,4,5-trimethoxy-phenyl)-pyrazolo[1,5-a]pyrimidin-5-yl]-amine;
3-[5-(4-Isopropyl-phenylamino)-pyrazolo[1,5-a]pyrimidin-3-yl]-benzoic acid;
[3-(4-Chloro-phenyl)-pyrazolo[1,5-a]pyrimidin-5-yl]-(4-isopropylphenyl)-amine;
(4-Isopropyl-phenyl)-[3-(3-trifluoromethoxy-phenyl)-pyrazolo[1,5-a]pyrimidin-5-yl]-amine;
N-(2-Hydroxy-ethyl)-3-[5-(4-isopropyl-phenylamino)-pyrazolo[1,5-a]pyrimidin-3-yl]-benzamide;
(4-isopropyl-phenyl)-[3-(5-methoxy-pyridin-3-yl)-pyrazolo[1,5-a]pyrimidin-5-yl]-amine;
N-{5-[3-(2-Methoxy-phenyl)-pyrazolo[1,5-a]pyrimidin-5-ylamino]-2-methyl-phenyl}-methanesulfonamide;
N-{2-Methyt-5-[3-(4-triftuoromethoxy-phenyl)-pyrazoto[1,5-a]pyrimidin-5-ylamino]-phenyl}-methanesulfonamide;
N-{2-Methyl-5-[3-(3,4, 5-trimethoxy-phenyl)-pyrazolo[1, 5-a] pyrimidin-5-ylamino]-phenyl}-methanesulfonamide;
N-{2-Methyl-5-[3-(3-trifluoromethoxy-phenyl)-pyrazolo[1,5-a]pyrimidin-5-ylamino]-phenyl}-methanesulfonamide;
N-{5-[3-(5-Methoxy-pyridin-3-yl)-pyrazolo[1,5-a]pyrimidin-5-ylamino]-2-methyl-phenyl}-methanesulfonamide;
Benzo[1, 3] dioxol-5-ylmethyl-[3-(2-methoxy-phenyl)-pyrazolo[1, 5-a]pyrimidin-5-yl]-amine;
Benzo[1,3]dioxol-5-ylmethyl-[3-(4-trifluoromethoxy-phenyl)-pyrazolo[1,5-a]pyrimidin-5-yt]-amine;
Benzo[1,3]dioxol-5-ylmethyl-[3-(5-methoxy-pyridin-3-yl)-pyrazolo[1,5-a]pyrimidin-5-yl]-amine;
[3-(2-Methoxy-phenyl)-pyrazolo[1,5-a] pyrimidin-5-yl]-pyridin-4-ylmethyl-amine;
Pyridin-4-ylmethyl-[3-(4-trifluoromethoxy-phenyl)-pyrazolo[1,5-a]pyrimidin-5-yl]-amine;
Pyridin-4-ylmethyl-[3-(3-trifluoromethoxy-phenyl)-pyrazolo[1, 5-a]pyrimidin-5-yl]-amine;
3-{4-[5-(4-Hydroxy-cyclohexylamino)-pyrazolo[1,5-a]pyrimidin-3-yl]-phenyl}-propionic acid;
3-[5-(4-Hydroxy-cyclohexylamino)-pyrazolo[1,5-a]pyrimidin-3-yl]-benzoic acid;
4-[3-(5-Methoxy-pyridin-3-yl)-pyrazolo[1,5-a]pyrimidin-5-ylamino]-cyclohexanol;
3-{4-[5-(2-Hydroxy-ethylamino)-pyrazolo[1,5-a]pyrimidin-3-yl]-phenyl}-propionic acid;
2-[3-(4-Trifluoromethoxy-phenyl)-pyrazolo[1,5-a]pyrimidin-5-ylamino]-ethanol;
4-[5-(2-Hydroxy-ethylamino)-pyrazolo[1,5-a]pyrimidin-3-yl]-benzonitrite;
2-[3-(3,4, 5-Trimethoxy-phenyl)-pyrazolo[1,5-a]pyrimidin-5-ylamino]-ethanol;
2-[3-(4-Chloro-phenyl)-pyrazolo[1,5-a]pyrimidin-5-ylamino]-ethanol;
2-[3-(3-Trifluoromethoxy-phenyl)-pyrazolo[1,5-a]pyrimidin-5-ylamino]-ethanol;
2-[3-(5-Methoxy-pyridin-3-yl)-pyrazolo[1,5-a]pyrimidin-5-ylamino]-ethanol;
3-(4-{5-[(Pyridin-3-ylmethyl)-amino]-pyrazolo[1,5-a]pyrimidin-3-yl}-phenyl)-propionic acid;
[3-(2-Methoxy-phenyl)-pyrazolo[1,5-a]pyrimidin-5-yl]-pyridin-3-ylmethyl-amine;
Pyridin-3-ylmethyl-[3-(3,4,5-trimethoxy-phenyl)-pyrazolo[1,5-a]pyrimidin-5-yl]-amine;
[3-(3,5-Dimethoxy-phenyl)-pyrazolo[1,5-a]pyrimidin-5-yl]-(4-morpholin-4-yl-phenyl)-amine;
Pyridin-3-ylmethyl-[3-(3-trifluoromethoxy-phenyl)-pyrazolo[1,5-a]pyrimidin-5-yl]-amine;
(4-Morpholin-4-yl-phenyl)-[3-(3-trifluoromethoxy-phenyl)-pyrazolo[1,5-a]pyrimidin-5-yl]-amine;
N-(2-Hydroxy-ethyl)-3-[5-(4-morpholin-4-yl-phenylamino)-pyrazolo[1,5-a]pyrimidin-3-yl]-benzamide;
N'-[3- (2 -Methoxy-phenyl)-pyrazolo[1,5-a]pyrimidin-5-yl] -N, N-dimethylethane-1,2-diamine;
N, N-Dimethyl-N'-[3-(4-trifluoromethoxy-phenyl)-pyrazolo[1, 5-a]pyrimidin-5-yl]-ethane-1,2-diamine;
N,N-Dimethyl-N'-[3-(3,4, 5-trimethoxy-phenyl)-pyrazolo[1,5-a] pyrimidin-5-yl]-ethane-1,2-diamine;
3-[5-(2-Dimethylamino-ethylamino)-pyrazolo[1,5-a]pyrimidin- 3-yl]-benzoic acid;
4-[5-(2-Dimethylamino-ethylamino)-pyrazolo[1,5-a]pyrimidin-3-yl]-benzoic acid;
N'-[3-(4-Chloro-phenyl)-pyrazolo[1, 5-a]pyrimidin-5-yl]-N, N-dimethylethane-1,2-diamine;
3-[5-(3,4,5-Trimethoxy-phenylamino)-pyrazolo[1,5-a]pyrimidin-3-yl]-phenol;
(E)-3-{3-[5-(3,4,5-Trimethoxy-phenylamino)-pyrazolo[1,5-a]pyrimidin-3-yl]-phenyl}-acrylic acid;
{2-[5-(3,4, 5-Trimethoxy-phenylamino)-pyrazolo[1,5-a]pyrimidin-3-yl]-phenyl}-methanol;
[3-(6-Methoxy-pyridin-3-yl)-pyrazolo[1,5-a] pyrimidin-5-yl]-(3,4,5-trimethoxy-phenyl)-amine;
(3-Furan-3-yl-pyrazolo[1,5-a]pyrimidin-5-yl)-(3,4,5-trimethoxy-phenyl)-amine;
(3-Chloro-4-fluoro-phenyl)-[3-(3-methoxy-phenyl)-pyrazolo[1, 5-a]pyrimidin-5-yl]-amine;
(3-Chloro-4-fluoro-phenyl)-[3-(2,4-dimethoxy-phenyl)-pyrazolo[1,5-a]pyrimidin-5-yl]-amine;
(3-Chloro-4-fluoro-phenyl)-(3-thiophen-2-yl-pyrazolo[1,5-a] pyrimidin-5-yl)-amine;
4-[3-(4-Methoxy-phenyl)-pyrazolo[1,5-a]pyrimidin-5-ylamino]-phenol;
4-[3-(6-Methoxy-pyridin-3-yl)-pyrazolo[1, 5-a]pyrimidin-5-ylamino]-phenol;
3-[5-(6-Methoxy-pyridin-3-ylamino)-pyrazolo[1,5-a]pyrimidin-3-yl]-phenol;
(6-Methoxy-pyridin-3-yl)-[3-(6-methoxy-pyridin-3-yl)-pyrazolo[1,5-a]pyrimidin-5-yl]-amine;
N-{5-[3-(6-Methoxy-pyridin-3-yl)-pyrazolo[1,5-a]pyrimidin-5-ylamino]-2-methyl-phenyl}-methanesulfonamide;
N-{5-[3-(4-Hydroxy-3-methoxy-phenyl)-pyrazolo[1,5-a] pyrimidin-5-ylamino]-2-methyl-phenyl}-methanesulfonamide;
N-{2-Methyl-5-[3-(4-methyl-thiophen-2-yl)-pyrazolo[1,5-a] pyrimidin-5-ylamino]-phenyl}-methanesulfonamide;
(2-{5-[(Benzo[1,3]dioxol-5-ylmethyl)-amino]-pyrazolo[1,5-a]pyrimidin-3-yl}-phenyl)-methanol;
[3-(4-Methoxy-phenyl)-pyrazolo[1, 5-a]pyrimidin-5-yl]-pyridin-4-ylmethyl-amine;
4-[3-(4-Methoxy-phenyl)-pyrazolo[1,5-a]pyrimidin-5-ylamino]-cyclohexanol;
3-[5-(4-Hydroxy-cyclohexylamino)-pyrazolo[1,5-a]pyrimidin-3-yl]-phenol;
(E)-3-{3-[5-(4-Hydroxy-cyclohexylamino)-pyrazolo[1,5 -a]pyrimidin-3-yl]-phenyl}-acrylic acid;
4-[3-(3-Hydroxymethyl-phenyl)-pyrazolo[1,5-a]pyrimidin-5-ylamino]-cyclohexanol;
4-[3-(2-Hydroxymethyl-phenyl)-pyrazolo[1,5-a]pyrimidin-5-ylamino]-cyclohexanol;
4-[5-(4-Hydroxy-cyclohexylamino)-pyrazolo[1,5-a]pyrimidin-3-yl]-2-methoxy-phenol;
2-[3-(4-Methoxy-phenyl)-pyrazolo[1,5-a]pyrimidin-5-ylamino]-ethanol;
(E)-3-{3-[5-(2-Hydroxy-ethylamino)-pyrazolo[1,5-a] pyrimidin-3-yl]-phenyl}-acrylic acid;
2-[3-(3-Hydroxymethyl-phenyl)-pyrazolo[1,5-a]pyrimidin-5-ylamino]-ethanol;
2-[3-(6-Methoxy-pyridin-3-yl)-pyrazolo[1,5-a]pyrimidin-5-ylamino]-ethanol;
4-[5-(2-Hydroxy-ethylamino)-pyrazolo[1,5-a]pyrimidin-3-yl]-2-methoxyphenol;
N-(4-Methoxy-phenyl)-4-{5-[(pyridin-3-ylmethyl)-amino]-pyrazolo[1,5-a]pyrimidin-3-yl}-benzamide;
[3-(4-Methoxy-phenyt)-pyrazoLo[1,5-a]pyrimidin-5-yt]-pyridin-3-ylmethyl-amine;
3-{5-[(Pyridin-3-ylmethyl)-amino]-pyrazoLo[1,5-a]pyrimidin-3-yl}-phenol;
(E)-3-(3-{5-[(Pyridin-3-ylmethyl)-amino]-pyrazolo[1,5-a]pyrimidin-3-yl}-phenyl)-acrylic acid;
(3-{5-[(Pyridin-3-ylmethyl)-amino]-pyrazolo[1,5-a]pyrimidin-3-yl}-phenyl)-methanol;
[3-(6-Methoxy-pyridin-3-yl)-pyrazolo[1,5-a]pyrimidin-5-yl]-pyridin-3-ylmethyl-amine;
[3-(4-Methyl-thiophen-2-yl)-pyrazolo[1,5-a]pyrimidin-5-yl]-pyridin-3-ylmethyl-amine;
[3-(4-Methoxy-phenyl)-pyrazolo[1,5-a]pyrimidin-5-yl]-(4-morpholin-4-yl-phenyl)-amine;
(E)-3-{3-[5-(4-Morpholin-4-yl-phenylamino)-pyrazolo[1,5-a] pyrimidin-3-yl]-phenyl}-acrylic acid;
N'-[3-(4-Methoxy-phenyl)-pyrazolo[1,5-a]pyrimidin-5-yl]-N, N-dimethylethane-1,2-diamine;
3-[5-(3,4,5-Trimethoxy-phenylamino)-pyrazolo[1,5-a]pyrimidin-3-yl]-benzamide;
4-[5-(3,4,5-Trimethoxy-phenylamino)-pyrazolo[1,5-a]pyrimidin-3-yl]-benzoic acid methyl ester;
[3-(3-Methoxy-phenyl)-pyrazolo[1,5-a]pyrimidin-5-yl]-(3,4,5-trimethoxyphenyl)-amine;
1-{3-[5-(3,4,5-Trimethoxy-phenylamino)-pyrazolo[1,5-a]pyrimidin-3-yl]-phenyl}-ethanone;
(3-Thiophen-2-yl-pyrazolo[1,5-a]pyrimidin-5-yl)-(3,4,5-trimethoxyphenyl)-amine;
N-{3-[5-(3,4,5-Trimethoxy-phenylamino)-pyrazolo[1,5-a]pyrimidin-3-yl]-phenyl}-methanesulfonamide;
[3-(1-Methyl-1H-pyrazol-4-yl)-pyrazolo[1,5-a]pyrimidin-5-yl]-(3,4,5-trimethoxy-phenyl)-amine;
N-Cyclopropyl-4-[5-(3-methanesulfonylamino-4-methyl-phenylamino)-pyrazolo[1,5-a]pyrimidin-3-yl]-benzamide;
N-{2-Methyl-5-[3-(4-morpholin-4-yl-phenyl)-pyrazolo[1,5-a]pyrimidin-5-ylamino]-phenyl}-methanesulfonamide;
N-{5-[3-(4-Dimethylamino-phenyl)-pyrazolo[1,5-a]pyrimidin-5-ylamino]-2-methyl-phenyl}-methanesulfonamide;
N-{5-[3-(3-Amino-phenyl)-pyrazolo[1,5-a]pyrimidin-5-ylamino]-2-methylphenyl}-methanesulfonamide;
[3-(4-Morpholin-4-yl-phenyl)-pyrazolo[1,5-a]pyrimidin-5-yl]-phenylamine;
[3-(4-Dimethylamino-phenyl)-pyrazolo[1,5-a]pyrimidin-5-yl]-phenylamine;
[3- (4-Amino-phenyl)-pyrazolo[1 5-a]pyrimidin-5-yl]-(4-phenoxy-phenyl)-amine;
[3-(4-Amino-phenyl)-pyrazolo[1,5-a] pyrimidin-5-yl]-(4-isopropyl-phenyl)-amine;
Benzo[1, 3]dioxol-5-ylmethyl-[3-(5-isopropyl-2-methoxy-phenyl)-pyrazolo[1,5-a]pyrimidin-5-yl]-amine;
4-[3-(3-Methoxy-phenyl)-pyrazolo[1,5-a]pyrimidin-5-ylamino]-phenol;
1-{3-[5-(4-Hydroxy-phenylamino)-pyrazolo[1, 5-a]pyrimidin-3-yl]-phenyl}-ethanone;
4-[3-(1-Methyl-1H-pyrazol-4-yl)-pyrazolo[1,5-a]pyrimidin-5-ylamino]-phenol;
4-[5-(6-Methoxy-pyridin-3-ylamino)-pyrazolo[1,5-a]pyrimidin-3-yl]-benzoic acid methyl ester;
[3-(3-Methoxy-phenyl)-pyrazolo[1,5-a]pyrimidin-5-yl]-(6-methoxy-pyridin-3-yl)-amine;
N-{3-[5-(6-Methoxy-pyridin-3-ylamino)-pyrazolo[1, 5-a]pyrimidin-3-yl]-phenyl}-methanesulfonamide;
(6-Methoxy-pyridin-3-yl)-[3-(1-methyl-1H-pyrazol-4-yl)-pyrazolo[1,5-a]pyrimidin-5-yl]-amine;
[3-(1-Benzyl-1H-pyrazol-4-yl)-pyrazolo[1,5-a]pyrimidin-5-yl]-(6-methoxy-pyridin-3-yl)-amine;
N-{4-[3-(3-Methoxy-phenyl)-pyrazolo[1,5-a]pyrimidin-5-ylamino]-phenyl}-4-methyl-benzenesulfonamide;
N-{4-[3-(3-Acetyl-phenyl)-pyrazolo[1,5-a]pyrimidin-5-ylamino]-phenyl}-4-methyl-benzenesulfonamide;
4-Methyl-N-[4-(3-thiophen-2-yl-pyrazolo[1,5-a]pyrimidin-5-ylamino)-phenyl]-benzenesulfonamide;
4-{5-[4-(4-Amino-benzoylamino)-phenylamino]-pyrazolo[1,5-a]pyrimidin-3-yl}-benzoic acid methyl ester;
4-Amino-N-{4-[3-(1-benzyl-1H-pyrazol-4-yl)-pyrazolo[1,5-a]pyrimidin-5-ylamino]-phenyl}-benzamide;
N-[2-Methyl-5-(3-pyrimidin-5-yl-pyrazolo[1,5-a]pyrimidin-5-ylamino)-phenyl] -methanesulfonamide;
N-{5-[3-(3-Methoxy-phenyl)-pyrazolo[1,5-a]pyrimidin-5-ylamino]-2-methyl-phenyl}-methanesulfonamide;
N-{5-[3-(2,4-Dimethoxy-phenyl)-pyrazolo[1,5-a]pyrimidin-5-ylamino]-2-methyl-phenyl}-methanesulfonamide;
N-[2-Methyl-5-(3-thiophen-2-yl-pyrazolo[1,5-a]pyrimidin-5-ylamino)-phenyl]-methanesulfonamide;

N-{5-[3-(3-Methanesulfonylamino-phenyl)-pyrazolo[1,5-a]pyrimidin-5-ylamino]-2-methyl-phenyl}-methanesulfonamide;
N-{2-Methyl-5-[3-(1-methyl-1H-pyrazol-4-yl)-pyrazolo[1,5-a]pyrimidin-5-ylamino]-phenyl}-methanesulfonamide;
N-(3-{5-[(Benzo[1,3]dioxol-5-ylmethyl)-amino]-pyrazolo[1,5-a]pyrimidin-3-yl}-phenyl)-methanesulfonamide;
Benzo[1,3]dioxol-5-ylmethyl-[3-(1-benzyl-1H-pyrazol-4-yl)-pyrazolo[1,5-aJpyrimidin-5-yl]-amine;
3-{5-[(Pyridin-4-ylmethyl)-amino]-pyrazolo[1,5-a]pyrimidin-3-yl}-benzamide;
4-{5-[(Pyridin-4-ylmethyl)-amino]-pyrazolo[1,5-a]pyrimidin-3-yl}-benzoic acid methyl ester;
[3-(3-Methoxy-phenyl)-pyrazolo[1,5-a]pyrimidin-5-yl]-pyridin-4-ylmethyl-amine;
1-(3-{5-[(Pyridin-4-ylmethyl)-amino]-pyrazolo[1,5-a]pyrimidin-3-yl}-phenyl)-ethanone;
Pyridin-4-ylmethyl-(3-thiophen-2-yl-pyrazolo[1,5-a]pyrimidin-5-yl)-amine;
[3-(1H-Pyrazol-4-yl)-pyrazolo[1,5-a]pyrimidin-5-yl]-pyridin-4-ylmethyl-amine;
[3-(1-Benzyl-1H-pyrazol-4-yl)-pyrazolo[1,5-a]pyrimidin-5-yl]-pyridin-4-ylmethyl-amine;
3-[5-(4-Hydroxy-cyclohexylamino)-pyrazolo[1,5-a]pyrimidin-3-yl]-benzamide;
4-[3-(1-Methyl-1 H-pyrazol-4-yl)-pyrazolo[1,5-a]pyrimidin-5-ylamino]-cyclohexanol;
4-[3-(1-Benzyl-1 H-pyrazol-4-yl)-pyrazolo[1,5-a]pyrimidin-5-ylamino]-cyclohexanol;
3-{5-[(Pyridin-3-ylmethyl)-amino]-pyrazolo[1,5-a]pyrimidin-3-yl}-benzamide;
4-{5-[(Pyridin-3-ylmethyl)-amino]-pyrazolo[1,5-a] pyrimidin-3-yl}-benzoic acid methyl ester;
[3-(2,6-Dimethoxy-phenyl)-pyrazolo[1,5-a]pyrimidin-5-yl]-pyridin-3-ylmethyl-amine;
[3-(3-Methoxy-phenyl)-pyrazolo[1,5-a]pyrimidin-5-yl]-pyridin-3-ylmethyl-amine;
1-(3-{5-[(Pyridin-3-ylmethyl)-amino]-pyrazolo[1,5-a]pyrimidin-3-yl}-phenyl)-ethanone;
Pyridin-3-ylmethyl-(3-thiophen-2-yl-pyrazolo[1,5-a]pyrimidin-5-yl)-amine;
N-(3-{5-[(Pyridin-3-ylmethyl)-amino]-pyrazolo[1,5-a]pyrimidin-3-yl}-phenyl)-methanesulfonamide;
[3-(1-Methyl-1 H-pyrazol-4-yl)-pyrazolo[1,5-a]pyrimidin-5-yl]-pyridin-3-ylmethyl-amine;
[3-(1-Benzyl-1 H-pyrazol-4-yl)-pyrazolo[1,5-a]pyrimidin-5-yl]-pyridin-3-ylmethyl-amine;
3-[5-(4-Morpholin-4-yl-phenylamino)-pyrazolo[1,5-a]pyrimidin-3-yl]-benzamide;
[3-(3-Methoxy-phenyl)-pyrazolo[1,5-a]pyrimidin-5-yl]-(4-morpholin-4-ylphenyl)-amine;
(4-Morpholin-4-yl-phenyl)-(3-thiophen-2-yl-pyrazolo[1,5-a]pyrimidin-5-yl)-amine;
[3-(1-Methyl-1H-pyrazot-4-yl)-pyrazolo[1,5-a]pyrimidin-5-yl]-(4-morpholin-4-yl-phenyl)-amine;
4-[5-(3-Chloro-phenylamino)-pyrazolo[1,5-a]pyrimidin-3-yi]-benzoic acid methyl ester;
(3-Chloro-phenyl)-(3-thiophen-2-yl-pyrazolo[1,5-a]pyrimidin-5-yl)-amine;
N-{3-[5-(3-Chloro-phenylamino)-pyrazolo[1,5-a]pyrimidin-3-yl]-phenyl}-methanesulfonamide;
[3-(1-Benzyl-1H-pyrazol-4-yl)-pyrazolo[1,5-a]pyrimidin-5-yl]-(3-chlorophenyl)-amine;
4-(5-Phenylamino-pyrazolo[1,5-a]pyrimidin-3-yl)-benzoic acid methyl ester;
[3-(3-Methoxy-phenyl)-pyrazolo[1,5-a]pyrimidin-5-yl]-phenyl-amine;
Phenyl-(3-thiaphen-2-yl-pyrazolo[1,5-a]pyrimidin-5-yl)-amine;
N-[3-(5-Phenylamino-pyrazolo[1,5-a]pyrimidin-3-yl)-phenyl]-methanesulfonamide;
[3-(1-Methyl-1H-pyrazol-4-yl)-pyrazolo[1,5-a]pyrimidin-5-yl]-phenylamine;
3-[4-(5-Phenytamino-pyrazolo[1,5-a]pyrimidin-3-yl)-phenyl]-propionic acid;
[3-(2-Methoxy-phenyl)-pyrazolo[1,5-a]pyrimidin-5-yl]-phenyl-amine;
Phenyl-[3-(4-trifluoromethoxy-phenyl)-pyrazolo[1,5-a]pyrimidin-5-yl]-amine;
4-(5-Phenylamino-pyrazolo[1,5-a]pyrimidin-3-yl)-benzoic acid;
[3-(4-Chloro-phenyl)-pyrazolo[1,5-a]pyrimidin-5-yl]-phenyl-amine;
Phenyl-[3-(3-trifluoromethoxy-phenyl)-pyrazolo[1, 5-a]pyrimidin-5-yl]-amine;
[3-(5-Methoxy-pyridin-3-yl)-pyrazolo[1,5-a] pyrimidin-5-yl]-phenylamine;
[3-(2-Methoxy-phenyl)-pyrazolo[1,5-a]pyrimidin-5-yl]-(4-phenoxyphenyl)-amine;
(4-Phenoxy-phenyl)-[3-(4-trifluoromethoxy-phenyl)-pyrazolo[1,5-a]pyrimidin-5-yl]-amine;
(4-Phenoxy-phenyl)-[3-(3 ,4,5-trimethoxy-phenyl)-pyrazolo[1,5-a]pyrimidin-5-yl]-amine;
4-[5-(4-Phenoxy-phenylamino)-pyrazolo[1,5-a]pyrimidin-3-yl]-benzoic acid;
[3-(4-Chloro-phenyl)-pyrazolo[1,5-a]pyrimidin-5-yl]-(4-phenoxy-phenyl)-amine;
(4-Phenoxy-phenyl)-[3-(3-trifluoromethoxy-phenyl)-pyrazolo[1,5-a]pyrimidin-5-yl]-amine;
[3-(5-Methoxy-pyridin-3-yl)-pyrazolo[1,5-a] pyrimidin-5-yl]-(4-phenoxyphenyl)-amine;
(3-Chloro-4-fluoro-phenyl)-[3-(4-trifluoromethoxy-phenyl)-pyrazolo[1,5-a]pyrimidin-5-yl]-amine;
(3-Chloro-4-fluoro-phenyl)- [3-(3,4,5-trimethoxy-phenyl)-pyrazolo[1,5-a]pyrimidin-5-yl)-amine;
(3-Chloro-4-fluoro-phenyl)-[3-(3-trifluoromethoxy-phenyl)-pyrazolo[1,5-a]pyrimidin-5-yl)-amine;
3- [5-(3-Chloro-4-fluoro-phenylamino)-pyrazolo[1,5-a] pyrimidin-3-yl]-N-(2-hydroxy-ethyl)-benzamide;
(3-Chloro-4-fluoro-phenyl)-[3-(5-methoxy-pyridin-3-yl)-pyrazolo[1,5-a]pyrimidin-5-yl]-amine;
4-[5-(4-Isopropyl-phenylamino)-pyrazolo[1,5-a]pyrimidin-3-yl]-benzoic acid methyl ester;
(4-isopropyl-phenyl)-[3-(3-methoxy-phenyl)-pyrazolo[1,5-a]pyrimidin-5-yl]-amine;

[3-(2,4-Dimethoxy-phenyl)-pyrazolo[1,5-a]pyrimidin-5-yl]-(4-isopropylphenyl)-amine;
1-{3-[5-(4-Isopropyl-phenylamino)-pyrazolo[1,5-a] pyrimidin-3-yl]-phenyl}-ethanone;
(4-Isopropyl-phenyl)-(3-thiophen-2-yl-pyrazolo[1,5-a]pyrimidin-5-yl)-amine;
N-{3-[5-(4-Isopropyl-phenylamino)-pyrazolo[1,5-a]pyrimidin-3-yl]-phenyl}-methanesulfonamide;
[3-(1-Benzyl-1 H-pyrazol-4-yl)-pyrazolo[1,5-a]pyrimidin-5-yl]-(4-isopropyl-phenyl)-amine;
[3-(4-Methoxy-phenyl)-pyrazolo[1,5-a]pyrimidin-5-yl]-phenyl-amine;
3-(5-Phenylamino-pyrazolo[1,5-a]pyrimidin-3-yl)-phenol;
[3-(5-Phenylamino-pyrazolo[1,5-a]pyrimidin-3-yl)-phenyl]-methanol;
(4-lsopropyl-phenyl)- [3- (4-methoxy-phenyl)-pyrazolo[1 ,5-a]pyrimidin-5-yl]-amine;
3-[5-(4-Isopropyl-phenylamino)-pyrazolo[1, 5-a]pyrimidin-3-yl]-phenol;
{3-[5-(4-Isopropyl-phenylamino)-pyrazolo[1,5-a]pyrimidin-3-yl]-phenyl}-methanol;
{2-[5-(4-Isopropyl-phenylamino)-pyrazolo[1,5-a]pyrimidin-3-yl]-phenyl}-methanol;
4-[5-(4-Isopropyl-phenylamino)-pyrazolo[1,5-a]pyrimidin-3-yl]-2-methoxy-phenol;
Phenyl-(3-pyridin-3-yl-pyrazolo[1,5-a]pyrimidin-5-yl)-amine;
(3-Benzofuran-2-yl-pyrazolo[1,5-a]pyrimidin-5-yl)-phenyl-amine;
Phenyl-(3-thiophen-3-yl-pyrazolo[1,5-a]pyrimidin-5-yl)-amine;
Phenyl-(3-quinolin-8-yl-pyrazolo[1,5-a]pyrimidin-5-yl)-amine;
4-(5-Phenylamino-pyrazolo[1,5-a]pyrimidin-3-yl)-phenol;
N-[3-(5-Phenylamino-pyrazolo[1,5-a]pyrimidin-3-yl)-phenyl]-acetamide;
(3-Chloro-4-fluoro-phenyl)-(3-pyridin-3-yl-pyrazolo[1,5-a]pyrimidin-5-yl)-amine;
(3-Benzofuran-2-yl-pyrazolo[1,5-a]pyrimidin-5-yl)-(3-chloro-4-fluorophenyl)-amine;
(3-Chloro-4-fluoro-phenyl)-(3-thiophen-3-yl-pyrazolo[1,5-a] pyrimidin-5-yl)-amine;
(3-Chloro-4-fluoro-phenyl)-(3-quinolin-8-yl-pyrazolo[1,5-a]pyrimidin-5-yl)-amine;
4-[5-(3-Chloro-4-fluoro-phenylamino)-pyrazolo[1, 5-a]pyrimidin-3-yl]-phenol;
1-(5-{5-[(Pyridin-3-ylmethyl)-amino]-pyrazolo[1,5-a]pyrimidin-3-yl}-thiophen-2-yl)-ethanone;
(3-Benzo[1,3]dioxol-5-yl-pyrazolo[1,5-a]pyrimidin-5-yl)-pyridin-3-ylmethyl-amine;
Pyridin-3-ylmethyl-(3-thiophen-3-yl-pyrazolo[1,5-a]pyrimidin-5-yl)-amine;
[3-(3,4-Dimethoxy-phenyl)-pyrazolo[1, 5-a]pyrimidin-5-yl]-pyridin-3-ylmethyl-amine;
4-{5-[(Pyridin-3-ylmethyl)-amino]-pyrazolo[1,5-a]pyrimidin-3-yl}-phenol;
N-(3-{5-[(Pyridin-3-ylmethyl)-amino]-pyrazolo[1,5-a]pyrimidin-3-yl}-phenyl)-acetamide;
1-{5-[5-(2-Dimethylamino-ethylamino)-pyrazolo[1,5-a]pyrimidin-3-yl]-thiophen- 2 -yl}-ethanone;
N'-(3-Benzo[1,3]dioxol-5-yl-pyrazolo[1,5-a]pyrimidin-5-yl)-N,N-dimethylethane-1,2-diamine;
N,N-Dimethyl-N'-(3-thiophen-3-yl-pyrazolo[1,5-a]pyrimidin-5-yl)-ethane-1,2-diamine;
N'-[3-(3,4-Dimethoxy-phenyl)-pyrazolo[1, 5-a] pyrimidin-5-yl]-N, N-dimethyl-ethane-1,2-diamine;
1-{5-[5-(3-Chloro-phenylamino)-pyrazolo[1,5-a]pyrimidin-3-yl]-thiophen-2-yl}-ethanone;
(3-Benzo[1,3]dioxol-5-yl-pyrazolo[1,5-a]pyrimidin-5-yl)-(3-chlorophenyl)-amine;
(3-Chloro-phenyl)-(3-pyridin-3-yl-pyrazolo[1,5-a]pyrimidin-5-yl)-amine;
(3-Chloro-phenyl)-(3-thiophen-3-yl-pyrazolo[1, 5-a]pyrimidin-5-yl)-amine;
(3-Chloro-phenyl)-(3-quinolin-8-yl-pyrazolo[1,5-a]pyrimidin-5-yl)-amine;
(3-Chloro-phenyl)-[3-(2-methoxy-phenyl)-pyrazolo[1,5-a]pyrimidin-5-yl]-amine;
(3-Chloro-phenyl)-[3-(4-trifluoromethoxy-phenyl)-pyrazolo[1,5-a]pyrimidin-5-yl]-amine;
(3-Chloro-phenyl)-[3- (3,4,5-trimethoxy-phenyl)-pyrazolo[1,5-a]pyrimidin-5-yl]-amine;
(3-Chloro-phenyl)-[3-(4-chloro-phenyl)-pyrazolo[1,5-a]pyrimidin-5-yl]-amine;
(3-Chloro-phenyl)-[3-(3-trifluoromethoxy-phenyl)-pyrazolo[1,5-a]pyrimidin-5-yl]-amine;
(3-Chloro-phenyl)-[3-(5-methoxy-pyridin-3-yl)-pyrazolo[1,5-a]pyrimidin-5-yl]-amine;
(3-Chloro-phenyl)-[3-(4-methoxy-phenyl)-pyrazolo[1,5-a]pyrimidin-5-yl]-amine;
3-[5-(3-Chloro-phenylamino)-pyrazolo[1,5-a]pyrimidin-3-yl]-phenol;
{3-[5-(3-Chloro-phenylamino)-pyrazolo[1,5-a]pyrimidin-3-yl]-phenyl}-methanol;
{2-[5-(3-Chloro-phenylamino)-pyrazolo)[1,5-a]pyrimidin-3-yl]-phenyl}-methanol;
4-[5-(3-Chloro-phenylamino)-pyrazolo[1,5-a]pyrimidin-3-yl]-2-methoxyphenol;
[3-(3-Amino-phenyl)-pyrazolo[1,5-a]pyrimidin-5-yl]-(3-chloro-phenyl)-amine;
(3-Chloro-phenyl)-[3-(3-dimethylamino-phenyl)-pyrazolo[1,5-a]pyrimidin-5-yl]-amine;
3-[5-(3-Chloro-phenylamino)-pyrazolo[1,5-a]pyrimidin-3-yl]-N-(2-dimethylamino-ethyl)-benzamide;
4-[5-(3-Chloro-phenylamino)-pyrazolo[1,5-a]pyrimidin-3-yl]-N-(2-dimethylamino-ethyl)-benzamide;
2-[3-(4-Methyl-thiophen-2-yl)-pyrazolo[1,5-a]pyrimidin-5-ylamino]-ethanol;
N-(2-Dimethylamino-ethyl)-4-{5-[4-(toluene-4-sulfonylamino)-phenylamino]-pyrazolo[1,5-a]pyrimidin-3-yl}-benzamide;
N-(2-Dimethylamino-ethyl)-4-{5-[(pyridin-3-ylmethyl)-amino]-pyrazolo[1,5-a]pyrimjdin-3-yl}-benzamide;

[3-(3-Aminomethyl-phenyl)-pyrazolo[1,5-a]pyrimidin-5-yl]-(3,4,5-trimethoxy-phenyl)-amine;
[3-(3-Aminomethyl-phenyl)-pyrazolo[1,5-a]pyrimidin-5-yl]-(4-phenoxyphenyl)-amine;
N-(2-Dimethylamino-ethyl)-3-[5-(3,4, 5-trimethoxy-phenylamino)-pyrazolo[1,5-a]pyrimidin-3-yl]-benzamide;
N-(2-Dimethylamino-ethyl)-3-[5-(6-methoxy-pyridin-3-ylamino)-pyrazolo[1,5-a]pyrimidin-3-yl]-benzamide;
N-(2-Dimethylamino-ethyl)-3-[5-(4-hydroxy-phenylamino)-pyrazolo[1,5-a]pyrimidin-3-yl]-benzamide;
3-{5-[(Benzo[1,3]dioxol-5-ylmethyl)-amino]-pyrazolo[1,5-a]pyrimidin-3-yl}-N-(2-dimethylamino-ethyl)-benzamide;
N-(2-Dimethylamino-ethyl)-3-{5-[(pyridin-3-ylmethyl)-amino]-pyrazolo[1,5-a]pyrimidin-3-yl}-benzamide;
N-(2-Dimethylamino-ethyl)-3-[5-(4-phenoxy-phenylamino)-pyrazolo[1,5-a]pyrimidin-3-yl]-benzamide;
[3-(3-Dimethylamino-phenyl)-pyrazolo[1,5-a]pyrimidin-5-yl]-(3,4,5-trimethoxy-phenyl)-amine;
N-{4-[3-(3-Dimethylamino-phenyl)-pyrazolo[1,5-a]pyrimidin-5-ylamino]-phenyl}-acetamide;
[3-(3-Dimethylamino-phenyl)-pyrazolo[1,5-a]pyrimidin-5-yl]-(6-methoxy-pyridin-3-yl)-amine;
4-[3-(3-Dimethylamino-phenyl)-pyrazolo[1,5-a]pyrimidin-5-ylamino]-phenol;
[3-(3-Dimethytamino-phenyL)-pyrazolo[1,5-a]pyrimidin-5-yl]-pyridin-3-ylmethyl-amine;
[3-(3-Dimethylamino-phenyl)-pyrazolo[1, 5-a]pyrimidin-5-yl] - (4-phenoxyphenyl)-amine;
[3- (3 -Dimethylamino-phenyl)-pyrazolo[1 5-a]pyrimidin-5-yl] - (4-isopropyl-phenyl)-amine;
[3-(3-Amino-phenyl)-pyrazolo[1,5-a]pyrimidin-5-yl]-(6-methoxy-pyridin-3-yl)-amine;
4-[3-(3-Amino-phenyl)-pyrazolo[1,5-a]pyrimidin-5-ylamino]-phenol;
N-{4-[3-(3-Amino-phenyl)-pyrazolo[1,5-a]pyrimidin-5-ylamino]-phenyl}-4-methyl-benzenesulfonamide;
[3-(3-Amino-phenyl)-pyrazolo[1,5-a]pyrimidin-5-yl]-pyridin-3-ylmethyl-amine;
[3-(3-Amino-phenyl)-pyrazolo[1,5-a]pyrimidin-5-yl]-(4-phenoxy-phenyl)-amine;
[3-(3-Amino-phenyl)-pyrazolo[1,5-a]pyrimidin-5-yl]-(4-isopropyl-phenyl)-amine;
[3-(4-Dimethylamino-phenyl)-pyrazolo[1,5-a]pyrimidin-5-yl]-(3,4,5-trimethoxy-phenyl)-amine;
[3-(4-Dimethylamino-phenyl)-pyrazolo[1,5-a]pyrimidin-5-yl]-6-methoxy-pyridin-3-yl)-amine;
4-[3-(4-Dimethylamino-phenyl)-pyrazolo[1,5-a]pyrimidin-5-ylamino]-phenol;
N-{4-[3-(4-Dimethylamino-phenyl)-pyrazolo[1,5-a]pyrimidin-5-ylmino]-phenyl}-4-methyl-benzenesulfonamide;
4-[3-(4-Dimethylamino-phenyl)-pyrazolo[1,5-a]pyrimidin-5-ylamino]-cyclohexanol;
[3-(4-Dimethylamino-phenyl)-pyrazolo[1,5-a]pyrimidin-5-yl]-(4-isopropyl-phenyl)-amine;
[3-(4-Morpholin-4-yl-phenyl)-pyrazolo[1,5-a]pyrimidin-5-yl]-(3,4,5-trimethoxy-phenyl)-amine;
(6-Methoxy-pyridin-3-yl)-[3-(4-morpholin-4-yl-phenyl)-pyrazolo[1, 5-a]pyrimidin-5-yl]-amine;
(4-Isopropyl-phenyl)-[3-(4-morpholin-4-yl-phenyl)-pyrazolo[1,5-a]pyrimidin-5-yl]-amine;
[3-(4-Amino-phenyl)-pyrazolo[1,5-alpyrimidin-5-yl]-(3,4,5-trimethoxyphenyl)-amine;
(3-Naphthalen-2-yl-pyrazolo[1,5-a]pyrimidin-5-yl)-(3,4,5-trimethoxyphenyl)-amine;
[3-(3,5-Bis-trifluoromethyl-phenyl)-pyrazolo[1,5-a]pyrimidin-5-yl]-(3,4,5-trimethoxy-phenyl)-amine;
(3-Phenyl-pyrazolo[1,5-a] pyrimidin-5-yl)-(3 ,4, 5-trimethoxy-phenyl)-amine;
[3-(2-Phenoxy-phenyl)-pyrazolo[1,5-a]pyrimidin-5-yl]-(3,4,5-trimethoxyphenyl)-amine;
[3-(3-Chloro-4-fluoro-phenyl)-pyrazolo[1,5-a] pyrimidin-5-yl]-(3,4,5-trimethoxy-phenyl)-amine;
[3-(2,3-Dichloro-phenyl)-pyrazolo[1,5-a]pyrimidin-5-yl]-(3,4,5-trimethoxy-phenyl)-amine;
[3-(4-Amino-phenyl)-pyrazolo[1,5-a]pyrimidin-5-yl]-(6-methoxy-pyridin-3-yl)-amine;
(6-Methoxy-pyridin-3-yl)-(3-naphthalen-2-yl-pyrazolo[1,5-a]pyrimidin-5-yl)-amine;
[3-(3,5-Bis-trifluoromethyl-phenyl)-pyrazolo[1,5-a]pyrimidin-5-yl]-(6-methoxy-pyridin-3-yl)-amine;
(6-Methoxy-pyridin-3-yl)-(3-phenyl-pyrazolo[1,5-a]pyrimidin-5-yl)-amine;
(6-Methoxy-pyridin-3-yl)-(3-naphthalen-1-yl-pyrazolo[1,5-a]pyrimidin-5-yl)-amine;
(6-Methoxy-pyridin-3-yl)-[3-(2-phenoxy-phenyl)-pyrazolo[1,5-a]pyrimidin-5-yl]-amine;
[3-(3-Chloro-4-fluoro-phenyl)-pyrazolo[1,5-a]pyrimidin-5-yl]-(6-methoxy-pyridin-3-yl)-amine;
[3-(3,4-Dimethyl-phenyl)-pyrazolo[1, 5-a]pyrimidin-5-yl]-(6-methoxy-pyridin-3-yl)-amine;
[3-(2,3-Dichloro-phenyl)-pyrazolo[1,5-a]pyrimidin-5-yl]-(6-methoxy-pyridin-3-yl)-amine;
Benzo[1,3]dioxol-5-ylmethyl-(3-naphthalen-1-yl-pyrazolo[1,5-a]pyrimidin-5-yl)-amine;
Benzo[1,3]dioxol-5-ylmethyl-[3-(2-phenoxy-phenyl)-pyrazolo[1,5-a]pyrimidin-5-yl]-amine;
4-[3-(4-Amino-phenyl)-pyrazolo[1,5-a]pyrimidin-5-ylamino]-cyclohexanol;
4-(3-Naphthalen-2-yl-pyrazolo[1,5-a]pyrimidin-5-ylamino)-cyclohexanol;
4-[3-(2-Phenoxy-phenyl)-pyrazolo[1,5-a]pyrimidin-5-ylamino]-cyclohexanol;
4-[3-(3-Chloro-4-fluoro-phenyl)-pyrazolo[1,5-a]pyrimidin-5-ylamino]-cyclohexanol;
(3-Naphthalen-2-yl-pyrazolo[1,5-a]pyrimidin-5-yl)-pyridin-3-ylmethyl-amine;
[3-(3,5-Bis-trifluoromethyl-phenyl)-pyrazolo[1,5-a]pyrimidin-5-yl]-pyridin-3-ylmethyl-amine;
(3-Phenyl-pyrazolo[1,5-a]pyrimidin-5-yl)-pyridin-3-ylmethyl-amine;
(3-Naphthalen-1-yl-pyrazolo[1,5-a]pyrimidin-5-yl)-pyridin-3-ylmethyl-amine;
[3-(2-Phenoxy-phenyl)-pyrazolo[1,5-a]pyrimidin-5-yl]-pyridin-3-ylmethyl-amine;
[3-(3-Chloro-4-fluoro-phenyl)-pyrazolo[1,5-a]pyrimidin-5-yl]-pyridin-3-ylmethyl-amine;

[3-(3,4-Dimethyl-phenyl)-pyrazolo[1,5-a]pyrimidin-5-yl]-pyridin-3-ylmethyl-amine;
[3-(2,3-Dichloro-phenyl)-pyrazolo[1,5-a] pyrimidin-5-yl]-pyridin-3 - ylmethyl-amine;
[3-(3-Chloro-phenyl)-pyrazolo[1,5-a]pyrimidin-5-yl]-(3,4,5-trimethoxyphenyl)-amine;
[3-(5-Isopropyl-2-methoxy-phenyl)-pyrazolo[1,5-a]pyrimidin-5-yl]-(3,4,5-trimethoxy-phenyl)-amine;
[3-(3-Trifluoromethyl-phenyl)-pyrazolo[1,5-a]pyrimidin-5-yl]-(3,4,5-trimethoxy-phenyl)-amine;
[3-(3-Fluoro-phenyl)-pyrazolo[1,5-a]pyrimidin-5-yl]-(3,4,5-trimethoxyphenyl)-amine;
[3-(4-Fluoro-phenyl)-pyrazolo[1,5-a]pyrimidin-5-yl]-(3,4,5-trimethoxyphenyl)-amine;
[3-(4-Phenoxy-phenyl)-pyrazolo[1,5-a]pyrimidin-5-yl]-(3,4,5-trimethoxyphenyl)-amine;
[3-(2-Chloro-phenyl)-pyrazolo[1,5-a]pyrimidin-5-yl]-(3,4,5-trimethoxyphenyl)-amine;
[3-(2-Fluoro-phenyl)-pyrazolo[1,5-a]pyrimidin-5-yl]-(3,4,5-trimethoxyphenyl)-amine;
(3-p-Tolyl-pyrazolo[1,5-a]pyrimidin-5-yl)-(3,4,5-trimethoxy-phenyl)-amine;
N-{5-[3-(3-Chloro-phenyl)-pyrazolo[1,5-a]pyrimidin-5-ylamino]-2-methyl-phenyl}-methanesulfonamide;
N-{5-[3-(5-Isopropyl-2-methoxy-phenyl)-pyrazolo[1,5-a]pyrimidin-5-ylamino]-2-methyl-phenyl}-methanesulfonamide;
N-{2-Methyl-5-[3 (3-trifluoromethyl-phenyl)-pyrazolo[1,5-a]pyrimidin-5-ylamino]-phenyl}-methanesulfonamide;
N-{5-[3-(3-Fluoro-phenyl)-pyrazolo[1,5-a]pyrimidin-5-ylamino]-2-methylphenyl}-methanesulfonamide;
[3-(3-Chloro-phenyt)-pyrazolo[1,5-a]pyrimidin-5-yt]-pyridin-4-ytmethylamine;
[3- (5-lsopropyl- 2 -methoxy-phenyl)-pyrazolo[1 ,5-a] pyrimidin- 5-yl]-pyridin-4-ylmethyl-amine;
Pyridin-4-ylmethyl- [3- (3-trifluoromethyl-phenyl)-pyrazolo[1 5-a]pyrimidin-5-yl]-amine;
[3-(4-Fluoro-phenyl)-pyrazolo[1, 5-a] pyrimidin-5-yl]-pyridi n-4-ylmethyl-amine;
4-Amino-N-{4-[3-(5-isopropyl-2-methoxy-phenyl)-pyrazolo[1,5-a]pyrimidin-5-ylamino]-phenyl}-benzamide;
4-Amino-N-{ 4- [3- (4-fluoro-phenyl)-pyrazolo[1 5-a] pyrimidin-5-ylamino]-phenyl}-benzamide;
4-[3-(4-Fluoro-phenyl)-pyrazolo[1,5-a]pyrimidin-5-ylamino]-cyclohexanol;
[3-(3-Chloro-phenyl)-pyrazolo[1,5-a]pyrimidin-5-yl]-pyridin-3-ylmethyl-amine;
[3-(5-IsopropyL-2-methoxy-phenyt)-pyrazoLo[1, 5-a]pyrimidin-5-yL]-pyridin-3-ylmethyl-amine;
Pyridin-3-ylmethyl-[3-(3-trifluoromethyl-phenyl)-pyrazolo[1,5-a]pyrimidin-5-yl]-amine;
[3-(3-Fluoro-phenyl)-pyrazolo[1,5-a]pyrimidin-5-yl]-pyridin-3-ylmethyl-amine;
[3-(4-Phenoxy-phenyl)-pyrazolo[1,5-a]pyrimidin-5-yl]-pyridin-3-ylmethyl-amine;
[3-(2-Chloro-phenyl)-pyrazolo[1,5-a]pyrimidin-5-yl]-pyridin-3-ylmethyl-amine;
[3-(2-Fluoro-phenyl)-pyrazolo[1,5-a]pyrimidin-5-yl]-pyridin-3-ylmethyl-amine;
Pyridin-3-ylmethyl-(3-p-tolyl-pyrazolo[1,5-a]pyrimidin-5-yl)-amine;
(4-Morpholin-4-yl-phenyl)-(3-naphthalen-2-yl-pyrazolo[1,5-a]pyrimidin-5-yl)-amine;
[3-(3,5-Bis-trifluoromethyl-phenyl)-pyrazolo[1,5-a]pyrimidin-5-yl]-(4-morpholin-4-yl-phenyl)-amine;
(4-Morpholin-4-yl-phenyl)-(3-naphthalen-1-yl-pyrazolo[1,5-a]pyrimidin-5-yl)-amine;
[3-(3-Chloro-4-fluoro-phenyl)-pyrazolo[1,5-a]pyrimidin-5-yl]-(4-morpholin-4-yl-phenyl)-amine;
[3-(2,3-Dichloro-phenyl)-pyrazolo[1,5-a]pyrimidin-5-yl]-(4-morpholin-4-yl-phenyl)-amine;
[3-(3-Chloro-phenyl)-pyrazolo[1,5-a]pyrimidin-5-yl]-(4-morpholin-4-ylphenyl)-amine;
[3-(5-Isopropyl-2-methoxy-phenyl)-pyrazolo[1,5-a]pyrimidin-5-yl]-(4-morpholin-4-yl-phenyl)-amine;
3-(4-{5-[(Pyridin-4-ylmethyl)-amino]-pyrazolo[1,5-a]pyrimidin-3-yl}- phenyl)-propionic acid;
Pyridin-4-ylmethyl-[3-(3,4,5-trimethoxy-phenyl)-pyrazolo[1,5-a]pyrimidin-5-yl]-amine;
2-[3-(2-Methoxy-phenyl)-pyrazolo[1,5-a]pyrimidin-5-ylamino]-ethanol;
(4-Morpholin-4-yl-phenyl)-[3-(4-trifluoromethoxy-phenyl)-pyrazolo[1,5-a]pyrimidin-5-yl]-amine;
(4-Morpholin-4-yl-phenyl)-[3-(3,4,5-trimethoxy-phenyl)-pyrazolo[1,5-a]pyrimidin-5-yl]-amine;
[3-(5-Methoxy-pyridin-3-yl)-pyrazolo[1,5-a]pyrimidin-5-yl]-(4-morpholin-4-yl-phenyl)-amine;
3-{4-[5-(2-Dimethylamino-ethylamino)-pyrazolo[1,5-a]pyrimidin-3-yl]-phenyl}-propionic acid;
2-Methoxy-4-[5-(3,4,5-trimethoxy-phenylamino)-pyrazolo[1,5-a]pyrimidin-3-yl]-phenol;
[3-(4-Methoxy-phenyl)-pyrazolo[1,5-a]pyrimidin-5-yl]-(6-methoxy-pyridin-3-yl)-amine;
N-{5-[3-(4-Methoxy-phenyl)-pyrazolo[1,5-a]pyrimidin-5-ylamino]-2-methyl-phenyl}-methanesulfonamide;
N-{5-[3-(3-Hydroxy-phenyl)-pyrazolo[1,5-a]pyrimidin-5-ylamino]-2-methyl-phenyl}-methanesulfonamide;
(E)-3-(3-{5-[(Pyridin-4-ylmethyl)-amino]-pyrazolo[1,5-a]pyrimidin-3-yl}-phenyl)-acrylic acid;
N'-[3-(1 H-Indol-6-yl)-pyrazolo[1,5-a]pyrimidin-5-yl]-N,N-dimethylethane-1,2-diamine;
3-[5-(2-Dimethylamino-ethylamino)-pyrazolo[1,5-a]pyrimidin-3-yl]-phenol;
N-{5-[3-(3-Hydroxymethyl-phenyl)-pyrazolo[1,5-a]pyrimidin-5-ylamino]-2-methyl-phenyl}-methanesulfonamide;
N-{5-[3-(2-Hydroxymethyl-phenyl)-pyrazolo[1,5-a]pyrimidin-5-ylamino]-2-methyl-phenyl}-methanesulfonamide;
[3-(2,4-Dimethoxy-phenyl)-pyrazolo[1,5-a]pyrimidin-5-yl]-(3,4,5-trimethoxy-phenyl)-amine;
[3-(1-Benzyl-1H-pyrazol-4-yl)-pyrazolo[1,5-a]pyrimidin-5-yl]-(3,4,5-trimethoxy-phenyl)-amine;
4-[3-(2,4-Dimethoxy-phenyl)-pyrazolo[1,5-a]pyrimidin-5-ylamino]-phenol;
[3-(2,4-Dimethoxy-phenyl)-pyrazolo[1,5-a]pyrimidin-5-yl]-(6-methoxy-pyridin-3-yl)-amine;

1-{3-[5-(6-Methoxy-pyridin-3-ylamino)-pyrazolo[1,5-a]pyrimidin-3-yl]-phenyl}-ethanone;
N-{4-[3-(2,6-Dimethoxy-phenyl)-pyrazolo[1,5-a]pyrimidin-5-ylamino]-phenyl}-4-methyl-benzenesulfonamide;
N-{4-[3-(2,4-Dimethoxy-phenyl)-pyrazolo[1,5-a]pyrimidin-5-ylamino]-phenyl}-4-methyl-benzenesulfonamide;
N-{5-[3-(1-Benzyl-1H-pyrazol-4-yl)-pyrazolo[1,5-a]pyrimidin-5-ylamino]-2-methyl-phenyl}-methanesulfonamide;
[3-(2,4-Dimethoxy-phenyl)-pyrazolo[1,5-a]pyrimidin-5-yl]-pyridin-4-ylmethyl-amine;
N-(3-{5-[(Pyridin-4-ylmethyl)-amino]-pyrazolo[1,5-a]pyrimidin-3-yl}-phenyl)-methanesulfonamide;
[3-(2,4-Dimethoxy-phenyl)-pyrazolo[1,5-a]pyrimidin-5-yl]-pyridin-3-ylmethyl-amine;
(3-Chloro-phenyl)- [3- (3-methoxy-phenyl)-pyrazolo[1 5-a]pyrimidin-5-yl]-amine;
(3-Chloro-phenyl)-[3-(2,4-dimethoxy-phenyl)-pyrazolo[1,5-a]pyrimidin-5-yl]-amine;
[3-(2,4-Dimethoxy-phenyl)-pyrazolo[1,5-a]pyrimidin-5-yl]-phenyl-amine;
N-{3-[5-(4-Morpholin-4-yl-phenylamino)-pyrazolo[1,5-a] pyrimidin-3-yl]-phenyl}-methanesulfonamide;
(3-Chloro-4-fluoro-phenyl)-[3-(4-chloro-phenyl)-pyrazolo[1,5-a]pyrimidin-5-yl]-amine;
3-[5-(4-Isopropyl-phenylamino)-pyrazolo[1,5-a]pyrimidin-3-yl]-benzamide;
3-{4-[5-(3-Chloro-phenylamino)-pyrazolo[1,5-a]pyrimidin-3-yt]-phenyl}-propionic acid;
Pyridin-3-ylmethyl-(3-quinolin-8-yl-pyrazolo[1, 5-a]pyrimidin-5-yl)-amine;
N-{3-[5-(2-Dimethylamino-ethylamino)-pyrazolo[1,5-a]pyrimidin-3-yl]-phenyt}-acetamide;
N-{3-[5-(3-Chloro-phenylamino)-pyrazolo[1,5-a]pyrimidin-3-yl]-phenyl}-acetamide;
(3-Chloro-phenyl)-[3-(6-methoxy-pyridin-3-yl)-pyrazolo[1, 5-a]pyrimidin-5-yl]-amine;
N-{4-[3-(3-Dimethylamino- phenyl)-pyrazolo[1,5-a]pyrimidin-5-ylamino]-phenyl}-4-methyl-benzenesulfonamide;
[3-(3-Amino-phenyl)-pyrazolo[1,5-a]pyrimidin-5-yl]-(3,4,5-trimethoxyphenyl)-amine;
(3-Naphthalen-1-yl-pyrazolo[1,5-a]pyrimidin-5-yl)-(3,4,5-trimethoxyphenyl)-amine;
(3-Benzo[1, 3]dioxol-5-yl-pyrazolo[1, 5-a]pyrimidin-5-yl)-(3,4, 5-trimethoxy-phenyl)-amine;
(3-Pyridin-3-yl-pyrazolo[1,5-a]pyrimidin-5-yl)-(3,4,5-trimethoxyphenyl)-amine;
(3-Thiophen-3-yl-pyrazolo[1,5-a]pyrimidin-5-yl)-(3,4,5-trimethoxyphenyl)-amine;
(3-Quinolin-8-yl-pyrazolo[1, 5-a]pyrimidin-5-yl)-(3,4, 5-trimethoxyphenyl)-amine;
[3-(3,4-Dimethoxy-phenyl)-pyrazolo[1,5-a]pyrimidin-5-yl]-(3,4,5-trimethoxy-phenyl)-amine;
4-[5-(3,4,5-Trimethoxy-phenylamino)-pyrazolo[1,5-a]pyrimidin-3-yl]-phenol;
(6-Methoxy-pyridin-3-yl)-(3-thiophen-3-yl-pyrazolo[1,5-a]pyrimidin-5-yl)-amine;
[3-(3,4-Dimethoxy-phenyl)-pyrazolo[1,5-a]pyrimidin-5-yl]-(6-methoxy-pyridin-3-yl)-amine;
Benzo[1,3]dioxol-5-ylmethyl-(3-benzo[1,3] dioxol-5-yl-pyrazolo[1,5-a]pyrimidin-5-yl)-amine;
(4-Morpholin-4-yl-phenyl)-(3-thiophen-3-yl-pyrazolo[1,5-a]pyrimidin-5-yl)-amine;
(4-Phenoxy-phenyl)-(3-pyridin-4-yl-pyrazolo[1,5-a]pyrimidin-5-yl)-amine;
(4-Phenoxy-phenyl)-(3-pyridin-3-yl-pyrazolo[1,5-a]pyrimidin-5-yl)-amine;
(4-Isopropyl-phenyl)-(3-thiophen-3-yl-pyrazolo[1,5-a]pyrimidin-5-yl)-amine;
[3-(3-Trifluoromethyl-phenyl)-pyrazolo[1,5-a]pyrimidin-5-yl]-(3,4,5-trimethoxy-phenyl)-amine;
(3-Benzo[1,3]dioxol-5-yl-pyrazolo[1,5-a]pyrimidin-5-yl)-(4-morpholin-4-yl-phenyl)-amine;
(4-Phenoxy-phenyl)-(3-thiophen-3-yl-pyrazolo[1,5-a]pyrimidin-5-yl)-amine;
4-[3-(3,4,5-Trimethoxy-phenyl)-pyrazolo[1,5-a]pyrimidin-5-ylamino]-cyclohexanol;
Pyridin-3-ylmethyl-[3-(4-trifluoromethoxy-phenyl)-pyrazolo[1,5-a]pyrimidin-5-yl]-amine;
N-(4-Methoxy-phenyl)-4-[5-(4-morpholin-4-yl-phenylamino)-pyrazolo[1,5-a]pyrimidin-3-yl]-benzamide;
(3-Chloro-4-fluoro-phenyl)-[3-(2-methoxy-phenyl)-pyrazolo[1,5-a]pyrimidin-5-yl]-amine;
[3-(3,4-Dimethoxy-phenyl)-pyrazolo[1,5-a]pyrimidin-5-yl]-phenyl-amine;
N'-(3-Benzofuran-2-yl-pyrazolo[1, 5-a]pyrimidin-5-yl)-N, N-dimethylethane-1,2-diamine;
[3-(4-Amino-phenyl)-pyrazolo[1,5-a]pyrimidin-5-yl]-(3-chloro-phenyl)-amine;
[3-(4-Dimethylamino-phenyl)-pyrazolo[1,5-a]pyrimidin-5-yl]-(4-phenoxyphenyl)-amine;
[3-(3,4-Dimethyl-phenyl)-pyrazolo[1,5-a]pyrimidin-5-yl]- (3,4,5-trimethoxy-phenyl)-amine;
4-Amino-N-{4-[3-(3-trifluoromethyl-phenyl)-pyrazolo[1,5-a] pyrimidin-5-ylamino]-phenyl}-benzamide;
[3-(3,4-Dimethyl-phenyl)-pyrazolo[1,5-a] pyrimidin-5-yl]-(4-morpholin-4-yl-phenyl)-amine;
N*4*-[3-(3-chloro-phenyl)-pyrazolo[1,5-a]pyrimidin-5-yl]-N*1*,N*1*-diethyl-pentane-1,4-diamine;
N'-[3-(3-chloro-phenyl)-pyrazolo[1,5-a]pyrimidin-5-yl]-N,N-diethyl-propane-1,3-diamine;
N*4*-[3-(3-Chloro-4-methyl-phenyl)-pyrazolo[1,5-a] pyrimidin-5-yl]-N*1*,N*1*-diethyl-pentane-1,4-diamine;
N'-[3-(3-Chloro-4-methyl-phenyl)-pyrazolo[1, 5-a]pyrimidin-5-yl]-N, N-diethyl-propane-1,3-diamine.

Another aspect of the invention is a method of preparing pyrazolopyrimidines of general formula (I) described *supra,* the method comprising the following method steps :

A) an intermediate of general formula 1 is allowed to react with an intermediate of general formula 2 to give an intermediate of general formula 3:

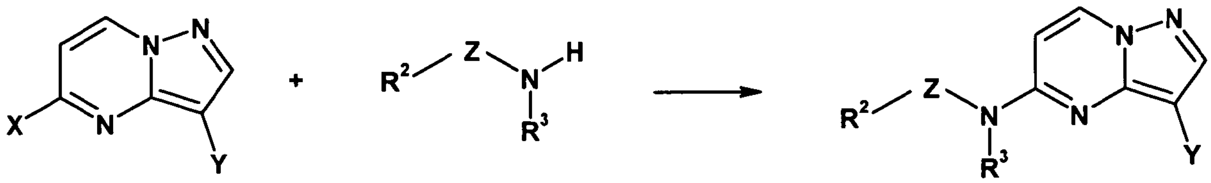

1 2 3

B) an intermediate of general formula 3 is allowed to react with an intermediate of general formula 4 to give a compound of general formula (I):

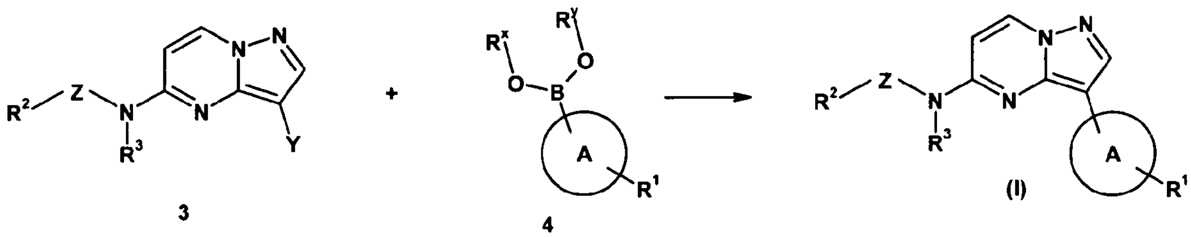

3 4 (I)

wherein X represents halogen or perfluor-$C_1$-$C_4$-alkyl sulfonyl, Y represents halogen, $R^x$ and $R^Y$ represent hydrogen, or, $R^x$ and $R^y$ are $C_1$-$C_6$-alkyl, chosen in such a way that, together with the oxygen atom to which they are attached, a 5 to 6 membered cyclic boronic acid ester is formed, and A, Z, $R^1$, $R^2$ and $R^3$ have the meaning as given for general formula (I), *supra,* it being understood that $R^1$, $R^2$ and $R^3$ may also incorporate one or more protecting groups, such as, for example $-C(O)OC(CH_3)_3$, wherein said protecting group is not incorporated in the final compound of general formula (I) and may be cleaved to provide compounds of general formula (I),

as well as N-oxides, solvates, hydrates, isomers, diastereomers, enantiomers and salts thereof.

A further method of preparing the compound of general formula (I) described *supra,* is the method comprising the following method steps :

A) an intermediate of general formula 2 is allowed to react with an intermediate of general formula 5 to give an intermediate of general formula 6:

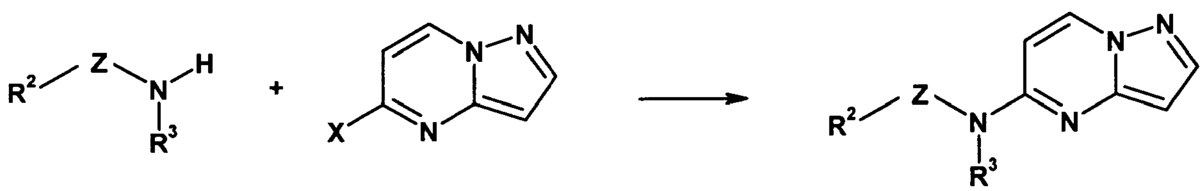

2 5 6

B) an intermediate of general formula 6 is converted to an intermediate of general formula 3:

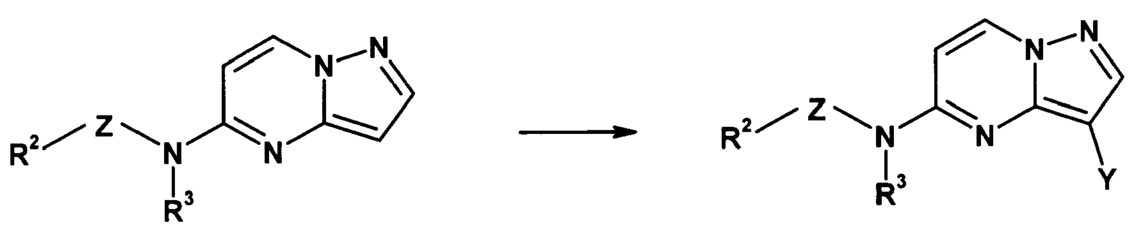

6 3

C) an intermediate of general formula 3 is allowed to react with an intermediate of general formula 4 to give a compound of general formula (I):

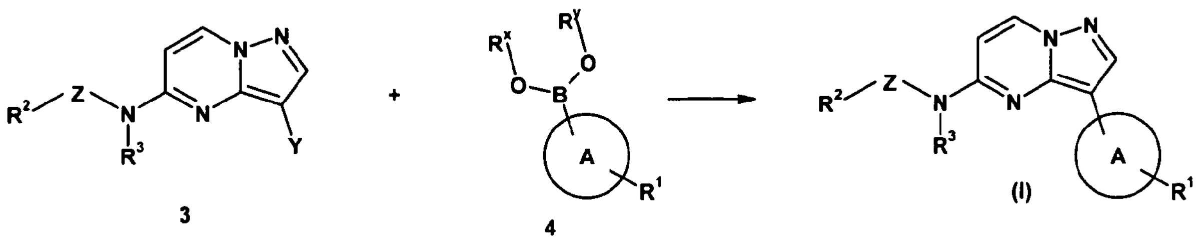

3 4 (I)

wherein X represents halogen or perfluor-$C_1$-$C_4$-alkyl sulfonyl, Y represents halogen, $R^x$ and $R^y$ represent hydrogen, or, $R^x$ and $R^y$ are $C_1$-$C_6$-alkyl, chosen in such a way that, together with the oxygen atom to which they are attached, a 5 to 6 membered cyclic boronic acid ester is formed, and A, Z, $R^1$, $R^2$ and $R^3$ have the meaning as given for general formula (I), *supra,* it being understood that $R^1$, $R^2$ and $R^3$ may also incorporate one or more protecting groups, such as, for example -C(O)OC($CH_3$)$_3$, wherein said protecting group is not incorporated in the final compound of general formula (I) and may be cleaved to provide compounds of general formula (I),

as well as N-oxides, solvates, hydrates, isomers, diastereomers, enantiomers and salts thereof.

A further method of preparing pyrazolopyri midines of general formula (I) described *supra,* is the method comprising the following method steps :

A) an intermediate compound of general formula 1 is allowed to react with an intermediate compound of general formula 4, thus providing an intermediate compound of general formula 7:

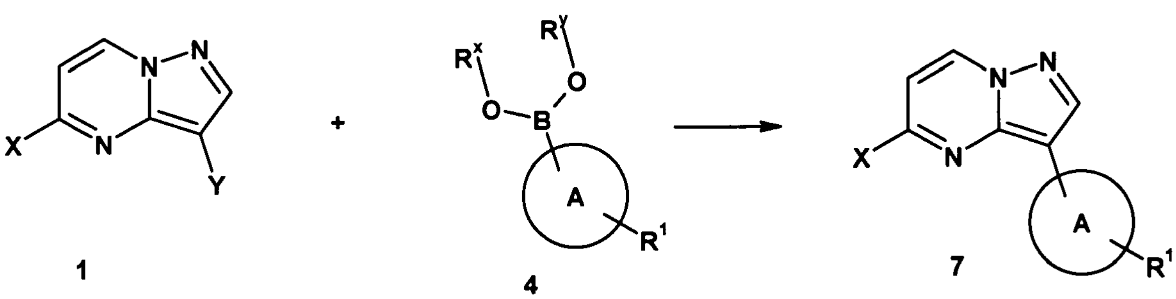

1 4 7

B) an intermediate compound of formula 7 is allowed to react with an intermediate of general formula 2 to give a compound of general formula (I):

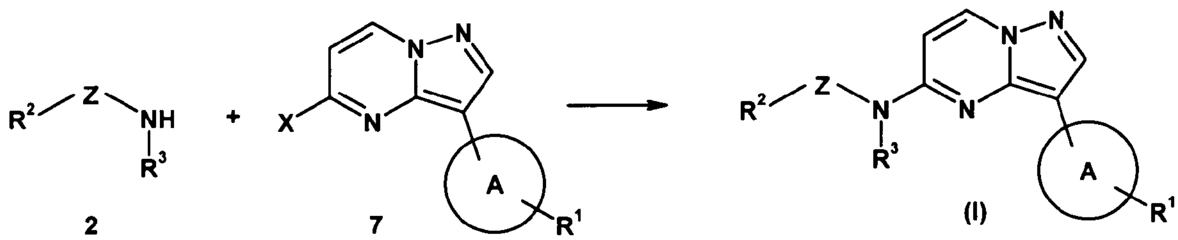

wherein X represents halogen or perfluor-$C_1$-$C_4$-alkyl sulfonyl, Y represents halogen, $R^x$ and $R^y$ represent hydrogen, or, $R^x$ and $R^y$ are $C_1$-$C_6$-alkyl, chosen in such a way that, together with the oxygen atom to which they are attached, a 5 to 6 membered cyclic boronic acid ester is formed, and A, Z, $R^1$, $R^2$ and $R^3$ have the meaning as given for general formula (I), *supra,* it being understood that $R^1$, $R^2$ and $R^3$ may also incorporate one or more protecting groups, such as for example $-C(O)OC(CH_3)_3$, wherein said protecting group is not incorporated in the final compound of general formula (I) and may be cleaved to provide compounds of general formula (I),

as well as N-oxides, solvates, hydrates, isomers, diastereomers, enantiomers and salts thereof.

The compounds of the present invention can be used in treating diseases of dysregulated vascular growth or diseases which are accompanied with dysregulated vascular growth. Especially, the compounds effectively interfere with ALK1 signalling.

Therefore, another aspect of the present invention is a use of the compound of general formula (I) described *supra* for manufacturing a pharmaceutical composition for the treatment or prophylaxis of diseases of dysregulated vascular growth or of diseases which are accompanied with dysregulated vascular growth.

Preferably, the use is in the treatment or prophylaxis of diseases, wherein the diseases are tumours and/or metastases thereof.

Another use is in the treatment or prophylaxis of diseases, wherein the diseases are retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

A further use is in the treatment or prophylaxis of diseases, wherein the diseases are coronary and peripheral artery disease.

Another use is in the treatment or prophylaxis of diseases, wherein the diseases are ascites, oedema such as brain tumour associated oedema, high altitude trauma, hypoxia induced cerebral oedema pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

A further use is in the treatment or prophylaxis of diseases, wherein the diseases are retinopathy, other angiogenesis dependent diseases of the eye, rheumatoid arthritis, and other inflammatory diseases associated with angiogenesis.

Yet another aspect of the invention is a method of treating or prophylaxis of a disease of dysregulated vascular growth or diseases which are accompanied with dysregulated vascular growth, by administering an effective amount of a compound of general formula (I) described *supra.*

Preferably, the diseases of said method is tumour and/or metastases thereof.

Also, the diseases of said method are retinopathy, other angiogenesis dependent diseases of the eye, in particular cornea transplant rejection or age-related macular degeneration, e.g. rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

Further, the disease of the method are coronary and peripheral artery disease.

Other diseases of the method are ascites, oedema such as brain tumour associated oedema, high altitude trauma, hypoxia induced cerebral oedema pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar

formation scar formation during regeneration of damaged nerves, endometriosis, pre-eclampsia, postmenopausal bleeding and ovarian hyperstimulation.

The compounds of the present invention can thus be applied for the treatment or prophylaxis of diseases accompanied by neoangiogenesis. This holds principally for all solid tumours, e.g. breast, colon, renal, lung and/or brain tumours or metastases thereof and can be extended to a broad range of diseases, where pathologic angiogenesis is persistent. This applies for diseases with inflammatory association, diseases associated with oedema of various forms and diseases associated with stromal proliferation and pathologic stromal reactions broadly. Particularly suited is the treatment for gynaecological diseases where inhibition of angiogenic, inflammatory and stromal processes with pathologic character can be inhibited. At the same time the toxic side effects on normal proliferating tissue are low. The treatment is therefore an addition to the existing armament to treat diseases associated with neoangiogenesis.

The compounds of the present invention can be used in particular in therapy and prevention, *i.e.* prophylaxis, of tumour growth and metastases, especially in solid tumours of all indications and stages with or without pre-treatment if the tumour growth is accompanied with persistent angiogenesis. However, it is not restricted to tumour therapy but is also of great value for the treatment or prophylaxis of other diseases with dysregulated vascular growth. This includes retinopathy and other angiogenesis dependent diseases of the eye (e.g. cornea transplant rejection, age-related macular degeneration), rheumatoid arthritis, and other inflammatory diseases associated with angiogenesis such as psoriasis, delayed type hypersensitivity, contact dermatitis, asthma, multiple sclerosis, restenosis, pulmonary hypertension, stroke and inflammatory diseases of the bowel, such as Crohn's disease. It includes coronary and peripheral artery disease. It can be applied for disease states such as ascites, oedema, such as brain tumour associated oedema, high altitude trauma, hypoxia induced cerebral oedema, pulmonary oedema and macular oedema or oedema following burns and trauma. Furthermore, it is useful for chronic lung disease, adult respiratory distress syndrome. Also for bone resorption and for benign proliferating diseases such as myoma, benign prostate hyperplasia and wound healing for the reduction of scar formation. It is therapeutically valuable for the treatment or prophylaxis of diseases, where deposition of fibrin or extracellular matrix is an issue and stroma proliferation is accelerated (e.g. fibrosis, cirrhosis, carpal tunnel syndrome etc). In addition it can be used for the reduction of scar formation during regeneration of damaged nerves, permitting the reconnection of axons. Further uses are endometriosis, pre-eclampsia, postmenopausal bleeding and ovarian hyperstimulation.

Another aspect of the present invention is a pharmaceutical composition which contains a compound of Formula (I) or pharmaceutically acceptable salts thereof, N-oxides, solvates, hydrates, isomers or mixtures of isomers thereof, in admixture with one or more suitable excipients. This composition is particularly suited for the treatment or prophylaxis of diseases of dysregulated vascular growth or of diseases which are accompanied with dysregulated vascular growth as explained above.

In order that the compounds of the present invention be used as pharmaceutical products, the compounds or mixtures thereof may be provided in a pharmaceutical composition, which, as well as the compounds of the present invention for enteral, oral or parenteral application contain suitably pharmaceutically acceptable organic or inorganic inert base material, e.g. purified water, gelatin, gum Arabic, lactate, starch, magnesium stearate, talcum, vegetable oils, polyalkylenglycol, etc.

The pharmaceutical compositions of the present invention may be provided in a solid form, e.g. as tablets, dragées, suppositories, capsules or in liquid form, e.g. as a solution, suspension or emulsion. The pharmaceutical composition may additionally contain auxiliary substances, e.g. preservatives, stabilisers, wetting agents or emulsifiers, salts for adjusting the osmotic pressure or buffers.

For parenteral applications, (including intravenous, subcutaneous, intramuscular, intravascular or infusion), sterile injection solutions or suspensions are preferred, especially aqueous solutions of the compounds in polyhydroxyethoxy containing castor oil.

The pharmaceutical compositions of the present invention may further contain surface active agents, e.g. salts of gallenic acid, phospholipids of animal or vegetable origin, mixtures thereof and liposomes and parts thereof.

For oral application tablets, dragées or capsules with talcum and/or hydrocarbon-containing carriers and binders, e.g. lactose, maise and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. The daily dose is in the range of 0.5 to 1,500 mg. A dose can be administered as unit dose or in part thereof and distributed over the day. Accordingly the optimum dosage may be determined by the practitioner who is treating any particular patient.

Another aspect of the present invention is a method which may be used for preparing the compounds according to the present invention.

The following Table lists the abbreviations used in this paragraph, and in the Examples section. NMR peak forms are stated as they appear in the spectra, possible higher order effects have not been considered.

| Abbreviation | Meaning |
|---|---|
| Ac | acetyl |
| ALK | activin receptor-like kinase (synonym: activin-like kinase). It does NOT refer to anaplastic lymphoma kinase NOR to anaplastic large cell lymphoma kinase. |
| BINAP | 2,2'-bis(diphenylphosphino)-1,1'-binaphthyl |
| Boc | tert-butyloxycarbonyl |
| br | broad |
| c- | cyclo- |
| d | doublet |
| dd | doublet of doublets |
| DCM | dichloromethane |
| DME | 1,2-dimethoxyethane |
| DIPEA | N,N-diisopropylethylamine |
| DMF | N,N-dimethylformamide |
| DMSO | dimethyl sulfoxide |
| dppf | 1,1'-bis(di-phenylphosphino)ferrocene |
| eq | equivalent |
| ESI | electrospray ionisation |
| m | multiplet |
| Mp. | melting point in ˚C |
| MS | mass spectrometry |
| MW | molecular weight |
| NMP | N-methylpyrrolidinone |
| NMR | nuclear magnetic resonance spectroscopy: chemical shifts (δ) are given in ppm. |
| $Pddba_2$ | bis-(dibenzylideneacetone)-palladium(0) complex |
| $Pd_2dba_3$ | tris-(dibenzylideneacetone)-dipalladium(0)-chloroform complex |
| $P(oTol)_3$ | tri-o-tolylphosphine |
| q | quartet |
| rt | room temperature |
| RT | retention time in minutes |
| s | singlet |
| sept | septet |
| t | triplet |
| TEA | triethylamine |
| TFA | trifluoroacetic acid |
| THF | tetrahydrofuran |
| xantphos | 9,9-dimethyl-4,5-bis(diphenylphosphino)xanthene |

The following schemes and general procedures illustrate general synthetic routes to the compounds of the invention and are not intended to be limiting. Specific examples are described in the subsequent paragraphs.

A first reaction scheme is outlined *infra :*

## Synthesis of compounds of general formula (I)

## Scheme 1

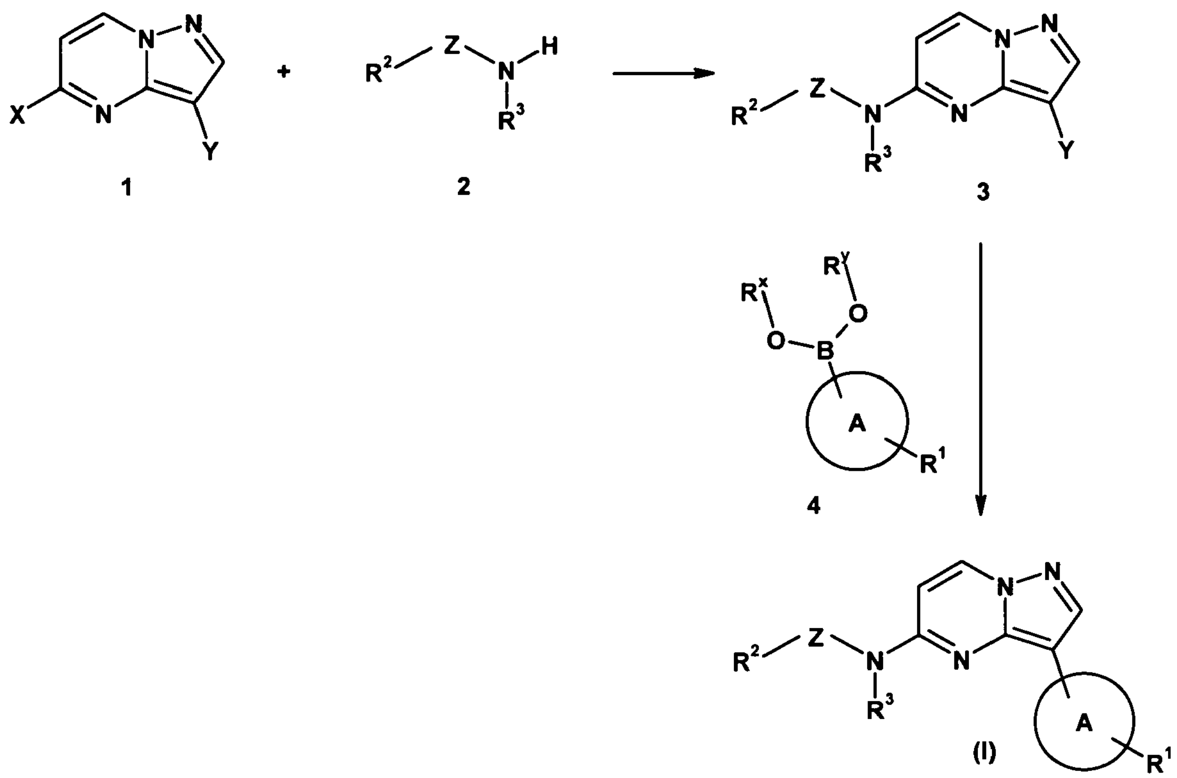

1 2 3 4 (I)

wherein X represents halogen or perftuor-$C_1$-$C_4$-alkyl sulfonyl, Y represents halogen, $R^x$ and $R^y$ represent hydrogen, or, $R^x$ and $R^y$ are $C_1$-$C_6$-alkyl, chosen in such a way that, together with the oxygen atom to which they are attached, a 5 to 6 membered cyclic boronic acid ester is formed, and A, Z, $R^1$, $R^2$ and $R^3$ have the meaning as given for general formula (I), *supra,* it being understood that $R^1$, $R^2$ and $R^3$ may also incorporate one or more protecting groups, such as, for example -C(O)OC$(CH_3)_3$, wherein said protecting group is not incorporated in the final compound of general formula (I) and may be cleaved to provide compounds of general formula (I).

Compounds of general formula (I) can be synthesised according to the procedure depicted in Scheme 1. Reaction of an intermediate of general formula 1 with an intermediate of general formula 2, in the presence of a suitable additive such as a tertiary amine base, such as, for example, DIPEA or TEA or an inorganic base such as, for example, potassium carbonate, in a suitable solvent, such as for example n-butanol, ethanol, NMP, DMF or acetonitrile, at temperatures up to the boiling point of the solvent, yields intermediate compounds of general formula 3. Reaction of an intermediate of general formula 3 with an intermediate of general formula 4 to give compounds of general formula (I) may be achieved using suitable variations of the well known *Suzuki* cross-coupling reaction. For example, intermediates of general formula 3 and 4 may be reacted together with a suitable palladium salt, such as for example Pd$(OAc)_2$, $Pddba_2$ or $Pd_2dba_3$, in the presence of a suitable ligand such as for example $PPh_3$ or P$(oTol)_3$, a suitable base such as for example sodium hydrogencarbonate, sodium carbonate, sodium hydroxide, potassium carbonate, potassium hydroxide or cesium carbonate, wherein the base is optionally used as an aqueous solution, in a suitable solvent such as for example toluene, EtOH, NMP, DME, DMF, THF, dioxane or mixtures thereof, at suitable temperatures, whereby heating between 80 °C and 110 °C is preferred, to give compounds of general formula (I). In the case that $R^3$ is a protecting group, such as for example -C(O)OC$(CH_3)_3$, cleavage under appropriate conditions, such as for example in the case of -C(O)OC$(CH_3)_3$ treatment with TFA, optionally in the presence of DCM, or aqueous hydrochloric acid in dioxane, at suitable temperatures, whereby room temperature is preferred, gives the compounds of general formula (I).

A second reaction scheme is outlined below :

## Synthesis of compounds of general formula (I)

## Scheme 2

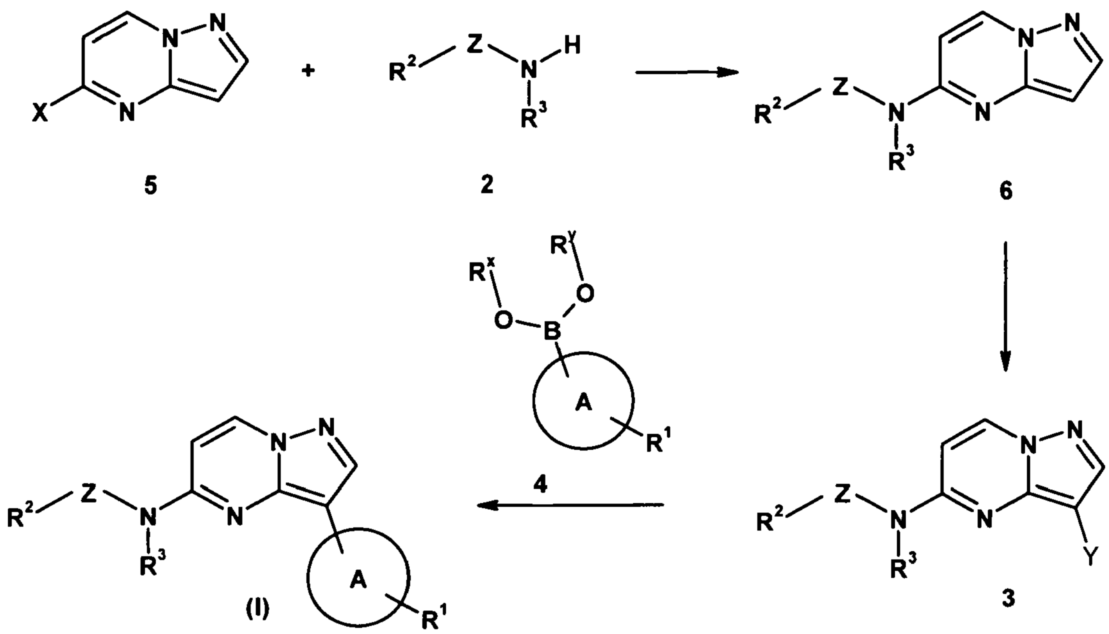

5 2 6 4 3 (I)

wherein X represents halogen or perfluor-$C_1$-$C_4$-alkyl sulfonyl, Y represents halogen, $R^x$ and $R^y$ represent hydrogen, or, $R^x$ and $R^Y$ are $C_1$-$C_6$-alkyl, chosen in such a way that, together with the oxygen atom to which they are attached, a 5 to 6 membered cyclic boronic acid ester is formed, and A, Z, $R^1$, $R^2$ and $R^3$ have the meaning as given for general formula (I), *supra,* it being understood that $R^1$, $R^2$ and $R^3$ may also incorporate one or more protecting groups, such as, for example -C(O)OC$(CH_3)_3$, wherein said protecting group is not incorporated in the final compound of general formula (I) and may be cleaved to provide compounds of general formula (I).

Scheme 2 illustrates yet another strategy for the synthesis of compounds of general formula (I). In this strategy, an intermediate of general formula 2 is reacted with an intermediate of general formula 5 to give an intermediate of general formula 6. In addition to the methods described above, the transformation may also be carried out under the promotion of a suitable metal complex. The metal complex may be used catalytically or stoichiometrically. Suitable metal complexes for this conversion are well known to the person skilled in the art. For example, suitable copper salts for the reaction are copper (I) or copper (II) salts whereby copper (I) salts such as, for example, copper (I) oxide or copper (I) iodide, are preferred. In the case of copper (I) iodide the addition of an additive such as, for example, ethylenediamine is necessary. Suitable solvents for this copper promoted coupling are, for example, dioxane or dimethylformamide, at temperatures upto the boiling point of the solvents, whereby 120 ˚C is preferred. Addition of a base is also necessary, such as for example potassium phosphate or cesium carbonate. In the case of palladium catalysis, palladium complexes such as, for example, $Pddba_2$ or $Pd_2dba_3$ maybe employed. Suitable solvents for the reaction are, for example, toluene, dioxane, THF, NMP or dimethylformamide, whereby mixtures of solvents may also be advantageous for the reaction, at temperatures from room temperature to the boiling points of the solvents, whereby 110˚C is preferred. A co-ligand such as, for example, BINAP, DPPF or xantphos is also employed. A base is also required, suitable bases for the reaction are for, example, cesium carbonate, potassium phosphate or sodium tertbutoxide. Intermediates of general formula 6 may be converted to intermediates of general formula 3 by a variety of standard halogenation transformations that are well known to those skilled in the art. Finally conversion of an intermediate of general formula 3 to a compound of general formula (I) may be performed as described above.

A third reaction scheme is outlined below :

## Synthesis of compounds of general formula (I)

## Scheme 3

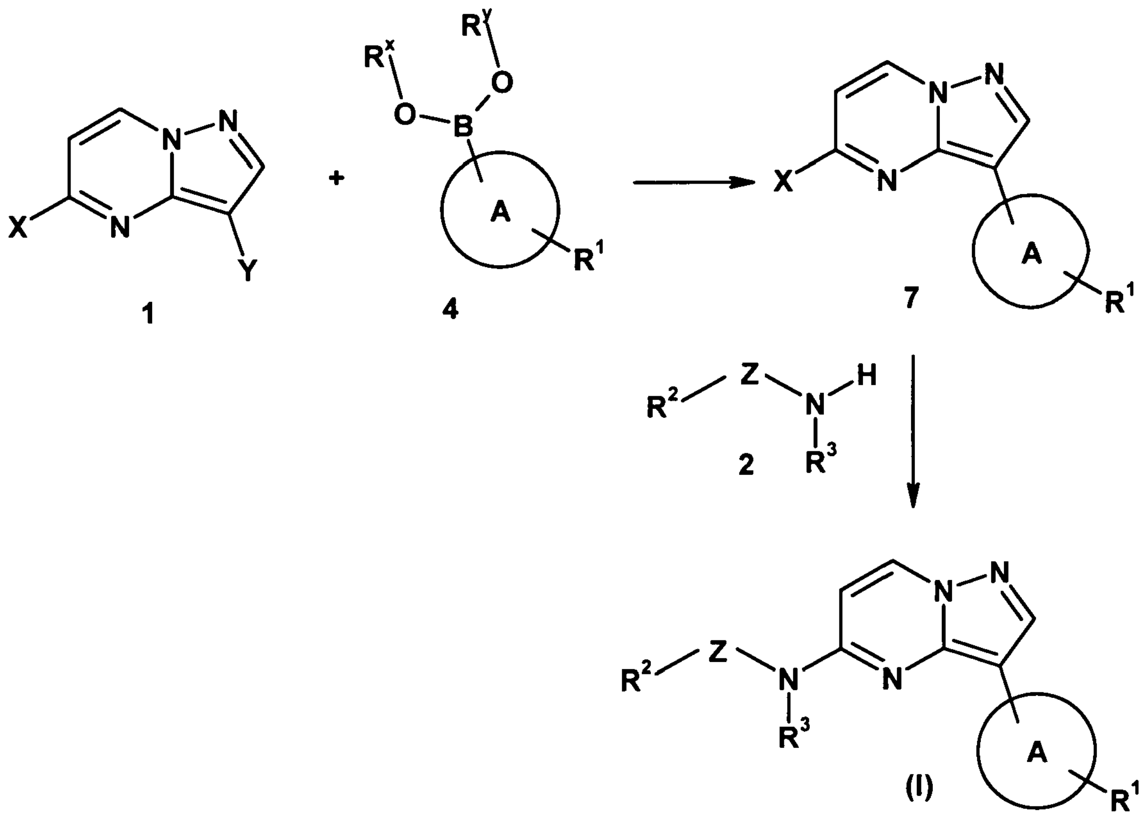

wherein X represents halogen or perfluor-$C_1$-$C_4$-alkyl sulfonyl, Y represents halogen, $R^x$ and $R^y$ represent hydrogen, or, $R^x$ and $R^y$ are $C_1$-$C_6$-alkyl, chosen in such a way that, together with the oxygen atom to which they are attached, a 5 to 6 membered cyclic boronic acid ester is formed, and A, Z, $R^1$, $R^2$ and $R^3$ have the meaning as given for general formula (I), *supra*, it being understood that $R^1$, $R^2$ and $R^3$ may also incorporate one or more protecting groups, such as for example $-C(O)OC(CH_3)_3$, wherein said protecting group is not incorporated in the final compound of general formula (I) and may be cleaved to provide compounds of general formula (I).

Scheme 3 illustrates yet another strategy for the synthesis of compounds of general formula (I). In this strategy, an intermediate of general formula 1 is reacted with an intermediate of general formula 4 to give an intermediate of general formula 7, accomplished by analogous use of the methods described above. Subsequently, reaction of an intermediate of general formula 7 with an intermediate of general formula 2 to give a compound of general formula (I) may be accomplished by analogous use of the methods described above.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallisation. In some cases, impurities may be stirred out using a suitable solvent. In some cases, the compounds may be purified by chromatography, particularly flash chromatography, using for example prepacked silica gel cartridges, e.g. from Separtis such as Isolute® Flash silica gel or Isolute® Flash NH2 silica gel in combination with a Flashmaster II autopurifier (Biotage) and eluants such as, for example, gradients of hexane/EtOAc or DCM/ethanol. In some cases, the compounds may be purified by preparative HPLC using for example a Waters autopurifier equipped with a diode array detector and/or on-line electrospray ionisation mass spectrometer in combination with a suitable prepacked reverse phase column and eluants such as, for example, gradients of water and acetonitrile which may contain additives such as trifluoroacetic acid, formic acid or aqueous ammonia.

Analytical LC-MS was performed as follows:

LC-MS Method A

Data was acquired on an Acquity HPLC (Waters), coupled to a Micromass / Waters ZQ 4000 mass spectrometer. An X-Bridge (Waters) column (dimensions 2.1 x 50 mm, filled with 1.7 μM BEH packing material) was employed. The mobile phase was water or acetonitrile (in each case containing 0.05% formic acid), using a flow rate of 1.3 mL / minute. A run consisted of a gradient of 10-90% acetonitrile over 1.7 minutes, 0.2 minutes at 90% acetonitrile, followed by a gradient back to 10% acetonitrile (total run time 2.5 minutes). UV data (200-400 nm) and mass data (mass range 160-800 Daltons; cone voltage 20 V) were collected.

LC-MS Method B

In analogy to LC-MS Method A except that data was collected on a HP1100 series HPLC (Agilent) coupled to a Micromass LCZ mass spectrometer. A YMC (Eprogen) column (dimensions 4.6 x 33 mm, filled with 1.5 μM ODS II packing material) was employed. The mobile phase was water or acetonitrile (in each case containing 0.1% formic acid), using a flow rate of 0.8 mL / minute. A run consisted of a gradient of 0-90% acetonitrile over 4.5 minutes. UV data were collected at 254 nm.

5-Chloro-pyrazolo[1,5-a]pyrimidine is commercially available from Butt Park Ltd (UK); Catalog. Nr. 51\09-77. In general, intermediates of general formula 1, 2, 4, and 5 are either commercially available, known to the person skilled in the art, or if their preparation is not detailed below, can be prepared using standard procedures known to the person skilled in the art, or can be prepared in analogy to procedures detailed below.

Names of compounds were generated using the Autonom 2000 add-in of ISIS/Draw [MDL Information Systems Inc. (Elsevier MDL)].

**Intermediate Example A Preparation of 3-bromo-5-chloro-pyrazolo[1,5-a]pyrimidine [Intermediate A]**

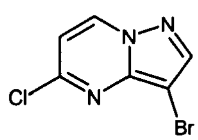

To a stirred solution of 5-Chloro-pyrazolo[1,5-a]pyrimidine (46.4 g, 0.3 mol), in glacial acetic acid (700 mL), at room temperature, was added bromine (42 mL, 0.81 mol) dropwise. On completion of addition, the mixture was stirred for 1 hour. The precipitate was filtered off, washed with glacial acetic acid and diethyl ether and dried. The filtrate was retained. The residue was suspended in water (500 mL) and the mixture neutralised with concentrated aqueous ammonia. The crude product was filtered, washed with water, isopropanol and hexane and dried to give 3-bromo-5-chloro-pyrazolo [1,5-a]pyrimidine [Intermediate A] (34.6 g, 49%). The retained filtrate was diluted with ice water, neutralised with concentrated aqueous ammonia and the resulting crude product filtered, washed with isopropanol and hexane and dried to give further 3-bromo-5-chloro-pyrazolo[1,5-a]pyrimidine [Intermediate A] (23.6 g, 33%).
$^1$H-NMR (400 MHz, $d_6$-DMSO): $\delta$ = 9.18 (2H, d), 8.41 (1H, s), 7.19 (2H, d) ppm.

**Example 1 Preparation of N-(2-dimethylamino-ethyl)-3-[5-(4-isopropyl-phenylamino)-pyrazolo[1,5-a]pyrimidin-3-yl]-benzamide**

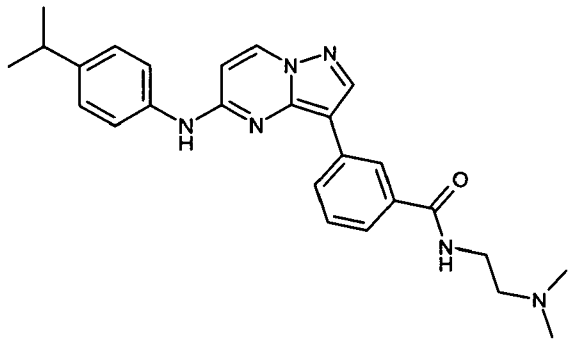

Example 1a Preparation of (3-bromo-pyrazolo[1,5-a]pyrimidin-5-yl)-(4-isopropyl-phenyl)-amine

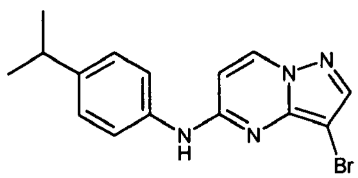

A mixture of Intermediate A (1 g, 4.3 mmol), DIPEA (0.9 mL, 5.16 mmol) and 4-isopropyl-aniline (0.87 g, 6.45 mmol) in n-BuOH (1 mL) was heated at 125 ˚C for 16 h. On cooling, the volatiles were removed in vacuo. The residue was taken up in EtOAc, washed with brine, dried and concentrated in vacuo. The crude product was purified by chromatography on silica gel to give (3-bromopyrazolo[1,5-a]pyrimidin-5-yl)-phenyl-amine (0.76 g, 53%).
$^1$H-NMR (400 MHz, $d_6$-DMSO): δ = 9.77 (1 H, s), 8.61 (1 H, d), 7.97 (1 H, s), 7.76 - 7.79 (2H, m), 7.18 - 7.21 (2H, m), 6.49 (1 H, d), 2.83 (1 H, sept), 1.16 (6H, d) ppm.

Example 1b Preparation of (3-bromo-pyrazolo[1,5-a]pyrimidin-5-yl)-(4-isopropyl-phenyl)-carbamic acid tert-butyl ester

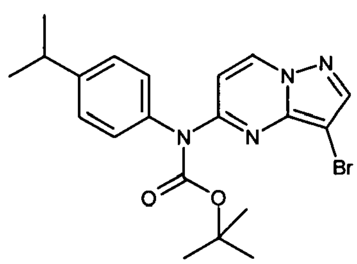

To a stirred solution of (3-bromo-pyrazolo[1,5-a]pyrimidin-5-yl)-phenyl-amine (0.75 g, 2.26 mmol) in THF (2.3 mL), under nitrogen, was added DMAP (69 mg, 0.57 mmol), followed by di-*tert.*-butyldicarbonate (1.48 g, 6.79 mmol). The reaction was stirred for 15 min before the volatiles were removed *in vacuo.* The residue was taken up in EtOAc and washed with 10% aq. citric acid solution and brine, dried and concentrated *in vacuo.* The crude product was purified by chromatography on silica gel to give (3-bromo-pyrazolo[1,5-a]pyrimidin-5-yl)-(4-isopropyl-phenyl)-carbamic acid tert-butyl ester (0.98 g, 100%).
$^1$H-NMR (300 MHz, $d_6$-DMSO): δ = 9.00 (1H, d), 8.23 (1H, s), 7.24 (2H, d), 7.14 (2H, d), 7.10 - 7.15 (3H, m), 2.89 (1H, sept), 1.39 (9H, s), 1.18 (6H, d) ppm.

Example 1c Preparation of *N*-(2-dimethylamino-ethyl)-3-[5-(4-isopropyl-phenylamino)-pyrazolo[1,5-a]pyrimidin-3-yl]-benzamide

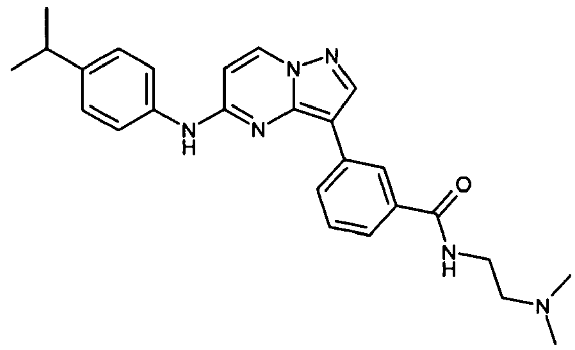

To a stirred solution of (3-bromo-pyrazolo[1,5-a]pyrimidin-5-yl)-(4-isopropylphenyl)-carbamic acid tert-butyl ester (1.67 g, 3.87 mmol) in DMF (6.7 mL) was added 3-(2-*N*,*N*-dimethylaminoethylaminocarbonyl)benzene boronic acid [Combi-Blocks (USA)] (1.04 g, 4.41 mmol) in DMF (8 mL) and aqueous sodium carbonate solution (1.5 M, 6.7 mL, 10 mmol). The mixture was purged with argon before the addition of $Pd(OAc)_2$ (60 mg, 0.27 mmol) and triphenylphosphine (0.21 g, 0.81 mmol) in dioxane (4 mL), repurged with argon and heated at 85°C for 16 h. On cooling, the volatiles were removed in *vacuo,* the residue taken up in DCM (6.7 mL), treated with TFA (10. 7 mL) and the mixture stirred for 16 h. The volatiles were removed *in vacuo,* the residue taken up in DCM and washed with saturated aq. sodium hydrogencarbonate solution and brine, dried and concentrated in vacuo. The crude product was purified by chromatography on silica gel to give N-(2-dimethylamino-ethyl)-3-[5-(4-isopropyl-phenylamino)-pyrazolo[1,5-a]pyrimidin-3-yl]-benzamide (0.14 g, 9%).
$^1$H-NMR (300 MHz, $d_6$-DMSO): $\delta$ = 9.75 (1 H, s), 8.65 (1H, d), 8.57 (1H, s), 8.44 (1 H, s), 8.36 (1 H, t), 8.10 (1 H, d), 7.83 (2H, d), 7.57 (1 H, d), 7.45 (1 H, t), 7.27 (2H, d), 6.53 (1 H, d), 3.31 - 3.42 (2H, m, partially obscured by residual water), 2.84 (1H, sept), 2.41 (2H, t), 2.16 (6H, s), 1.18 (6H, d) ppm.

**Example 2 Preparation of phenyl-[3-(3,4,5-trimethoxy-phenyl)-pyrazolo[1,5-a]pyrimidin-5-yl]-amine**

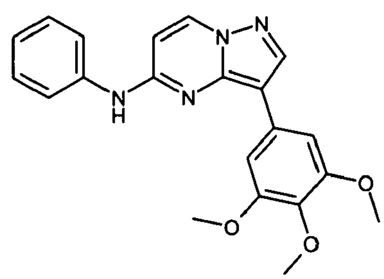

Example 2a Preparation of (3-bromo-pyrazolo[1,5-a]pyrimidin-5-yl)-phenylamine

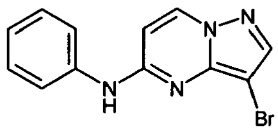

(3-bromo-pyrazolo[1,5-a]pyrimidin-5-yl)-phenyl-amine was prepared from Intermediate A (5 g, 21.5 mmol) and aniline in analogy to the procedure given in Example 1a. The yield was (4.66 g, 75%).
$^1$H-NMR (300 MHz, $d_6$-DMSO): δ = 9.83 (1H, s), 8.62 (1H, d), 7.99 (1H, s), 7.87 (2H, d), 7.33 (2H, apparent t), 7.01 (1 H, apparent t), 6.52 (1 H, d) ppm.

Example 2b Preparation of (3-bromo-pyrazolo[1,5-a]pyrimidin-5-yl)-phenylcarbamic acid tert-butyl ester

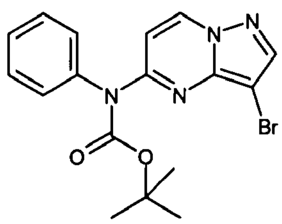

(3-Bromo-pyrazolo[1,5-a]pyrimidin-5-yl)-phenyl-carbamic acid tert-butyl ester was prepared from (3-bromo-pyrazolo[1,5-a]pyrimidin-5-yl)-phenyl-amine (4.66 g, 16.1 mmol) in analogy to the procedure given in Example 1b. The yield was (5.99 g, 95%).
$^1$H-NMR (300 MHz, $d_6$-DMSO): δ = 9.01 (2 H, d), 8.21 (1H, s), 7.37 - 7.42 (2H, m), 7.22 - 7.32 (4H, m) ppm.

Example 2c Preparation of phenyl-[3-(3,4,5-trimethoxy-phenyl)-pyrazolo[1,5-a]pyrimidin-5-yl]-amine

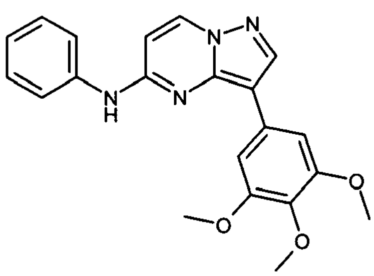

Phenyl-[3-(3,4,5-trimethoxy-phenyl)-pyrazolo[1,5-a]pyrimidin-5-yl]-amine was prepared from (3-bromo-pyrazolo[1,5-a]pyrimidin-5-yt)-phenyt-carbamic acid tert-butyl ester (5.99 g, 15.4 mmol) and 3,4, 5-trimethoxyphenyl boronic acid in analogy to the procedure given in Example 1c. The yield was (1.88 g, 32%).
$^1$H-NMR (300 MHz, $d_6$-DMSO): δ = 9.75 (1H, s), 8.65 (1H, d) 8.44 (1H, s), 7.84 (2H, d), 7.27 - 7.32 (4H, m), 7.01 (1 H, t), 6.52 (1 H, s) ppm.

**Example 3 Preparation of (4-fluoro-phenyl)-[3-(3,4,5-trimethoxyphenyl)-pyrazolo[1,5-a]pyrimidin-5-yl]-amine**

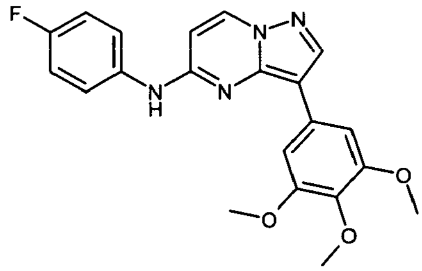

Example 3a Preparation of 5-chloro-3-(3,4,5-trimethoxy-phenyl)-pyrazolo[1,5-a]pyrimidine

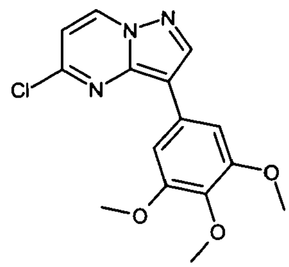

A mixture of Intermediate A (1 g, 4.3 mmol), 3,4,5-trimethoxyphenyl boronic acid (1.62 g, 7.66 mmol), $Pddba_2$ (99 mg, 0.17 mmol), $P(oTol)_3$ (157 mg, 0.12 mmol), saturated aqueous sodium hydrogen carbonate solution (6.7 mL) and DME (31 mL) was purged with argon and heated at 85 ˚C over night. On cooling, the mixture was poured onto saturated aqueous ammonium chloride solution and extracted with EtOAc. The combined organic layers were washed with brine, dried, *concentrated in vacuo* and the residue purified by chromatography to give a mixture of 5-chloro-3-(3,4,5-trimethoxy-phenyl)-pyrazolo[1,5-a]pyrimidine and 3-bromo-5-(3,4,5-trimethoxy-phenyl)-pyrazolo[1,5-a]pyrimidine, which was used in the next step without further purification.

Example 3b Preparation of (4-fluoro-phenyl)-[3-(3,4,5-trimethoxy-phenyl)-pyrazolo[1,5-a]pyrimidin-5-yl]-amine

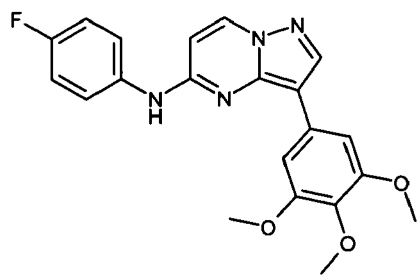

Method A

370 mg of the crude mixture of 5-chloro-3-(3,4,5-trimethoxy-phenyt)-pyrazolo[1,5-a]pyrimidine and 3-bromo-5-(3,4,5-trimethoxy-phenyl)-pyrazolo[1,5-a]pyrimidine from Example 3a was dissolved in acetonitrile (14 mL) and treated with 4-fluoroaniline (257 mg, 2.31 mmol) and potassium carbonate (320 mg, 2.31 mmol). The mixture was purged with argon and heated at reflux over night. On cooling, the mixture was diluted with saturated aqueous ammonium chloride

solution, extracted with EtOAc and the combined organic layers were washed with brine, dried and concentrated in vacuo to give 770 mg of the crude (4-fluoro-phenyl)-[3-(3,4,5-trimethoxyphenyl)-pyrazolo[1, 5-a] pyrimidin-5-yl]-amine.

Method B

264 mg of the crude mixture of 5-chtoro-3-(3,4,5-trimethoxy-phenyl)-pyrazolo[1,5-a]pyrimidine and 3-bromo-5-(3,4,5-trimethoxy-phenyl)-pyrazolo[1,5-a]pyrimidine from Example 3a was dissolved in n-butanol (0.2 mL) and treated with 4-fluoroaniline (138 mg, 1.24 mmol) and DIPEA (0.17 mL, 0.99 mmol). The mixture was purged with argon and heated at 85 °C for 8 hours. A further portion of 4-fluoroaniline (80 mg) was added and heating was continued for a further 22 hours. On cooling, the mixture was diluted with saturated aqueous ammonium chloride solution, extracted with EtOAc and the combined organic layers were washed with brine, dried. The mixture was concentrated *in vacuo* to *ca.* one-third the original volume, filtered and the filtrate concentrated *in vacuo* to give 260 mg of the crude (4-fluoro-phenyl)-[3-(3,4,5-trimethoxy-phenyl)-pyrazolo[1,5-a]pyrimidin-5-yl]-amine.

The combined crude products from Method A and Method B were purified by chromatography followed by preparative HPLC to give (4-fluoro-phenyl)-[3-(3,4,5-trimethoxy-phenyl)-pyrazolo[1,5-a]pyrimidin-5-yl]-amine (66 mg). $^1$H-NMR (300 MHz, $d_6$-DMSO): $\delta$ = 9.77 (1H, broad s), 8.65 (1H, d), 8.44 (1H, s), 7.79 - 7.84 (2H, m), 7.26 (2H, s), 7.11 - 7.16 (2H, m), 6.48 (1H, d), 3.78 (6H, s), 3.63 (3H, s) ppm.

The following examples were prepared in analogy [LC-MS data such as retention time (RT) or observed mass peak were collected using LC-MS Method A unless explicitly stated]:

| Example No. | Structure | Name | RT | MW | Observed Mass Peak [M-H] |
|---|---|---|---|---|---|
| 4 | 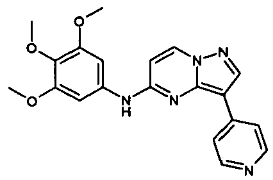 | (3-Pyridin-4-yl-pyrazolo[1,5-a]pyrimidin-5-yl)-(3,4, 5-trimethoxy-phenyl)-amine | | 377.40 | |
| 5 | 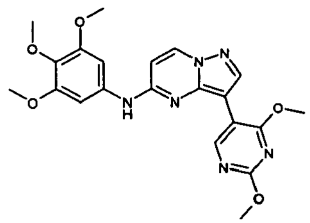 | [3-(2,4-Dimethoxy-pyrimidin-5-yl)-pyrazolo[1,5-a]pyrimidin-5-yl]-(3,4, 5-trimethoxy-phenyl)-amine | | 438.44 | |
| 6 | 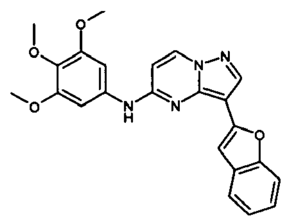 | (3-Benzofuran-2-yl-pyrazolo[1,5-a]pyrimidin-5-yl)-(3,4, 5-trimethoxy-phenyl)-amine | 1.37 | 416.43 | 415.22 |
| 7 | 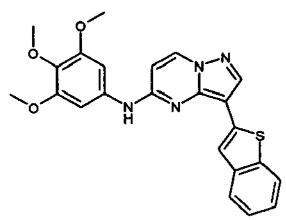 | (3-Benzo[b]thiophen-2-yl-pyrazolo[1,5-a]pyrimidin-5-yl)-(3,4, 5-trimethoxy-phenyl)-amine | 1.39 | 432.5 | 431.25 |

(continued)

| Example No. | Structure | Name | RT | MW | Observed Mass Peak [M-H] |
|---|---|---|---|---|---|
| 8 | 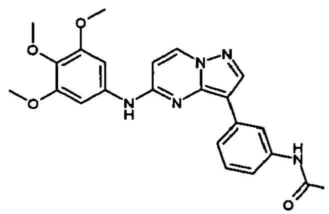 | N-{3-[5-(3,4,5-Trimethoxy-phenylamino)-pyrazolo[1,5-a]pyrimidin-3-yl]-phenyl}-acetamide | 1.02 | 433.47 | 432.34 |
| 9 | 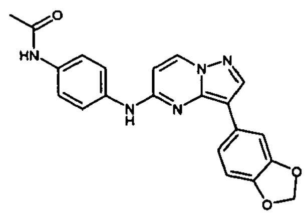 | N-[4-(3-Benzo[1,3]dioxol-5-yl-pyrazolo[1,5-a]pyrimidin-5-ylamino)-phenyl]-acetamide | 1.04 | 387.4 | 386.28 |
| 10 | 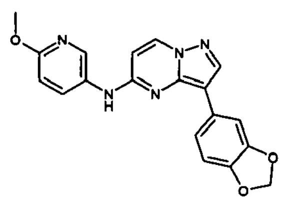 | (3-Benzo[1,3]dioxol-5-yl-pyrazolo[1,5-a]pyrimidin-5-yl)-(6-methoxy-pyridin-3-yl)-amine | 1.17 | 361.36 | 360.28 |
| 11 | 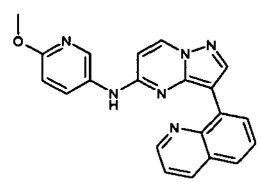 | (6-Methoxy-pyridin-3-yl)-(3-quinolin-8-yl-pyrazolo[1,5-a]pyrimidin-5-yl)-amine | 0.9 | 368.4 | 367.4 |

(continued)

| Example No. | Structure | Name | RT | MW | Observed Mass Peak [M-H] |
|---|---|---|---|---|---|
| 12 | 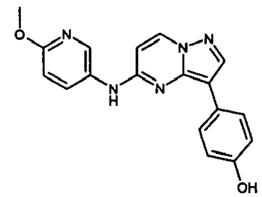 | 4-[5-(6-Methoxy-pyridin-3-ylamino)-pyrazolo[1, 5-a]pyrimidin-3-yl]-phenol | 0.94 | 333.35 | 332.29 |
| 13 | 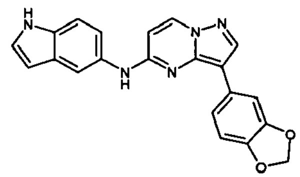 | (3-Benzo[1,3]dioxol-5-yl-pyrazolo[1,5-a]pyrimidin-5-yl)-(1H-indol-5-yl)-amine | 1.17 | 369.38 | 369.25 |
| 14 | 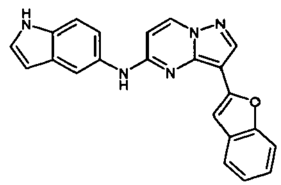 | (3-Benzofuran-2-yl-pyrazolo[1,5-a]pyrimidin-5-yl)-(1H-indol-5-yl)-amine | 1.32 | 365.39 | 365.22 |
| 15 | 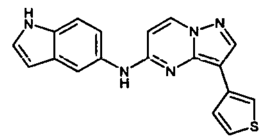 | (1H-Indol-5-yl)-(3-thiophen-3-yl-pyrazolo[1,5-a]pyrimidin-5-yl)-amine | 1.18 | 331.4 | 330.21 |
| 16 | 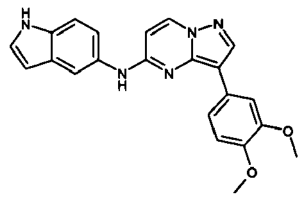 | [3-(3,4-Dimethoxy-phenyl)-pyrazolo[1,5-a]pyrimidin-5-yl]-(1H-indol-5-yl)-amine | 1.11 | 385.42 | 385.33 |

(continued)

| Example No. | Structure | Name | RT | MW | Observed Mass Peak [M-H] |
|---|---|---|---|---|---|
| 17 | 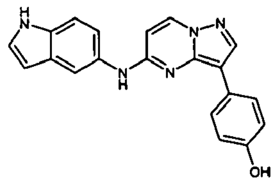 | 4-[5-(1H-Indol-5-ylamino)-pyrazolo[1,5-a]pyrimidin-3-yl]-phenol | 0.98 | 341.37 | 340.36 |
| 18 | 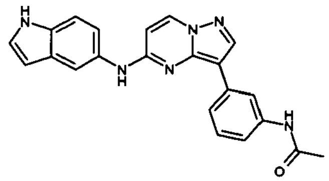 | N-{3-[5-(1H-Indol-5-ylamino)-pyrazolo[1,5-a]pyrimidin-3-yl]-phenyl}-acetamide | 0.99 | 382.42 | 381.33 |
| 19 | 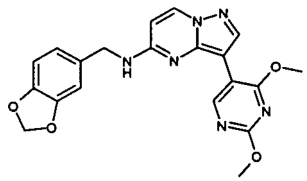 | Benzo[1,3]dioxol-5-ylmethyl-[3-(2,4-dimethoxy-pyrimidin-5-yl)-pyrazolo[1,5-a]pyrimidin-5-yl]-amine | 0.81 | 378.86 | 377.25 |
| 20 | 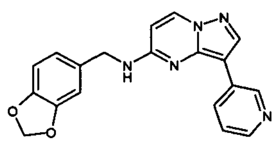 | Benzo[1,3]dioxol-5-ylmethyl-(3-pyridin-3-yl-pyrazolo[1,5-a] pyrimidin-5-yl)-amine | 0.76 | 345.36 | 344.33 |
| 21 | 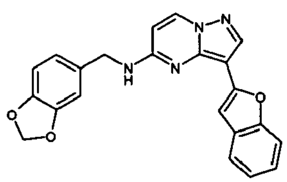 | Benzo[1,3]dioxol-5-ylmethyl-(3-benzofuran-2-yl-pyrazolo[1,5-a] pyrimidin-5-yl)-amine | 1.4 | 384.39 | 383.25 |

(continued)

| Example No. | Structure | Name | RT | MW | Observed Mass Peak [M-H] |
| --- | --- | --- | --- | --- | --- |
| 22 | 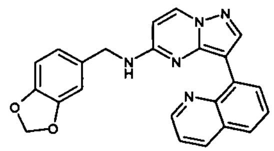 | Benzo[1,3]dioxol-5-ylmethyl-(3-quinolin-8-yl-pyrazolo[1,5-a] pyrimidin-5-yl)-amine | 0.95 | 395.42 | 394.26 |
| 23 | 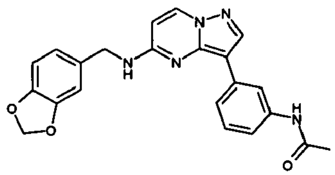 | N-(3-{5-[(Benzo[1,3]dioxol-5-ylmethyl)-amino]-pyrazolo[1,5-a] pyrimidin-3-yl}-phenyl)-acetamide | 1.03 | 401.42 | 401.34 |
| 24 | 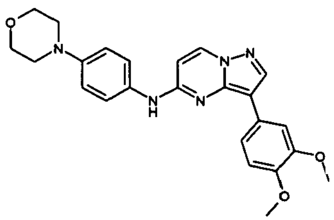 | [3-(3,4-Dimethoxy-phenyl)-pyrazolo[1,5-a]pyrimidin-5-yl]-(4-morpholin-4-yl-phenyl)-amine | 1.1 | 431.49 | 430.35 |
| 25 | 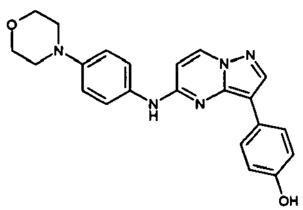 | 4-[5-(4-Morphotin-4-yl-phenylamino)-pyrazolo[1,5-a]pyrimidin-3-yl]-phenol | 0.95 | 387.44 | 386.32 |

(continued)

| Example No. | Structure | Name | RT | MW | Observed Mass Peak [M-H] |
|---|---|---|---|---|---|
| 26 | 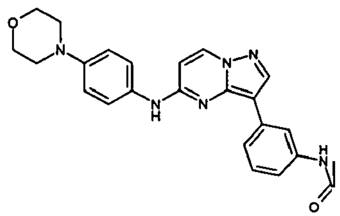 | N-{3-[5-(4-Morpholin-4-yl-phenylamino)-pyrazolo[1,5-a]pyrimidin-3-yl]-phenyl}-acetamide | 0.97 | 428.49 | 427.28 |
| 27 | 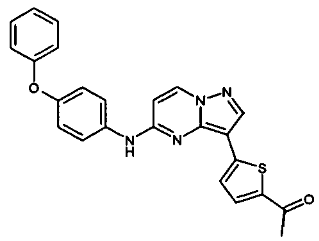 | 1-{5-[5-(4-Phenoxy-phenylamino)-pyrazolo[1,5-a]pyrimidin-3-yl]-thiophen-2-yl}-ethanone | 1.39 | 426.5 | 425.28 |
| 28 | 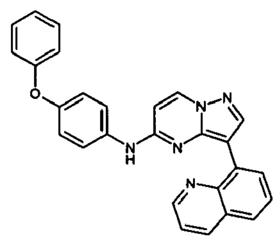 | (4-Phenoxy-phenyl)-(3-quinolin-8-yl-pyrazolo[1,5-a]pyrimidin-5-yl)-amine | 1.22 | 429.48 | 428.25 |
| 29 | 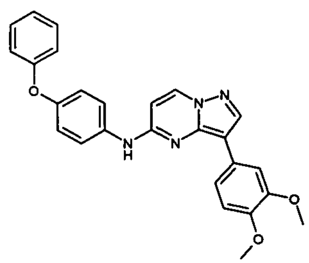 | [3-(3,4-Dimethoxy-phenyl)-pyrazolo[1,5-a]pyrimidin-5-yl]-(4-phenoxy-phenyl)-amine | 1.39 | 438.48 | 437.26 |

(continued)

| Example No. | Structure | Name | RT | MW | Observed Mass Peak [M-H] |
|---|---|---|---|---|---|
| 30 | 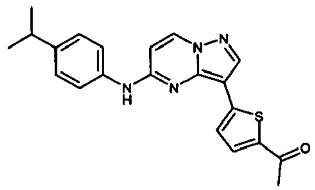 | 1-{5-[5-(4-Isopropyl-phenylamino)-pyrazolo[1,5-a]pyrimidin-3-yl]-thiophen-2-yl}-ethanone | 1.38 | 376.48 | 375.26 |
| 31 | 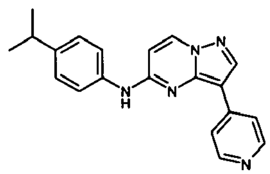 | (4-Isopropyl-phenyl)-(3-pyridin-4-yl-pyrazolo[1,5-a]pyrimidin-5-yl)-amine | 0.91 | 329.4 | 329.4 |
| 32 | 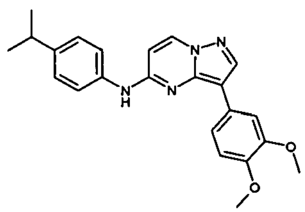 | [3-(3,4-Dimethoxy-phenyl)-pyrazolo[1,5-a]pyrimidin-5-yl]-(4-isopropyl-phenyl)-amine | 1.39 | 388.47 | 387.32 |
| 33 | 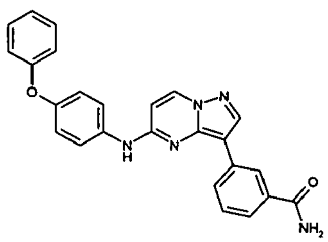 | 3-[5-(4-Phenoxy-phenylamino)-pyrazolo[1,5-a]pyrimidin-3-yl]-benzamide | 1.19 | 421.46 | 420.22 |

(continued)

| Example No. | Structure | Name | RT | MW | Observed Mass Peak [M-H] |
|---|---|---|---|---|---|
| 34 | 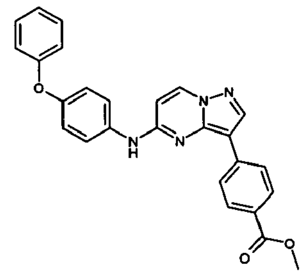 | 4-[5-(4-Phenoxy-phenylamino)-pyrazolo[1,5-a]pyrimidin-3-yl]-benzoic acid methyl ester | 1.47 | 436.47 | 435.26 |
| 35 | 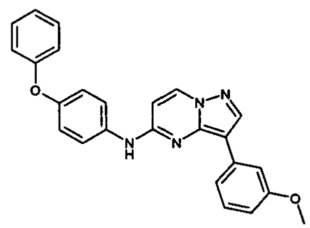 | [3-(3-Methoxy-phenyl)-pyrazolo[1,5-a]pyrimidin-5-yl]-(4-phenoxy-phenyl)-amine | 1.5 | 408.46 | 407.29 |
| 36 | 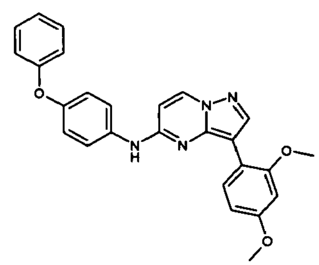 | [3-(2,4-Dimethoxy-phenyl)-pyrazolo[1,5-a]pyrimidin-5-yl]-(4-phenoxy-phenyl)-amine | 1.47 | 438.48 | 437.38 |

(continued)

| Example No. | Structure | Name | RT | MW | Observed Mass Peak [M-H] |
|---|---|---|---|---|---|
| 37 | 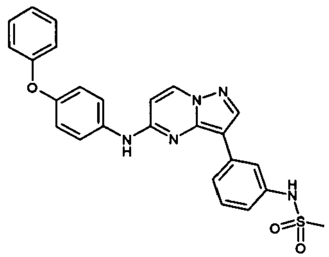 | N-{3-[5-(4-Phenoxy-phenylamino)-pyrazolo[1,5-a]pyrimidin-3-yl]-phenyl}-methanesulfonamide | 1.28 | 471.54 | 470.26 |
| 38 | 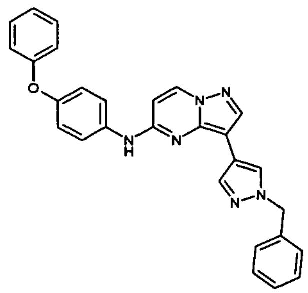 | [3-(1-Benzyl-1H-pyrazol-4-yl)-pyrazolo[1,5-a]pyrimidin-5-yl]-(4-phenoxy-phenyl)-amine | 1.4 | 458.52 | 457.32 |
| 39 | 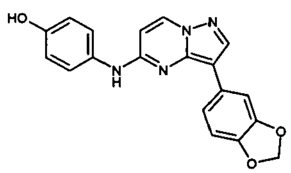 | 4-(3-Benzo[1,3]dioxol-5-yl-pyrazolo[1,5-a]pyrimidin-5-ylamino)-phenol | 1.06 | 346.35 | 345.23 |
| 40 | 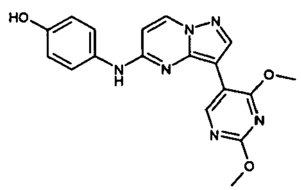 | 4-[3-(2,4-Dimethoxy-pyrimidin-5-yl)-pyrazolo[1,5-a]pyrimidin-5-ylamino]-phenol | 0.97 | 364.36 | 363.29 |

(continued)

| Example No. | Structure | Name | RT | MW | Observed Mass Peak [M-H] |
|---|---|---|---|---|---|
| 41 | 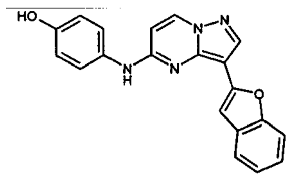 | 4-(3-Benzofuran-2-yl-pyrazolo[1,5-a]pyrimidin-5-ylamino)-phenol | 1.2 | 342.36 | 342.15 |
| 42 | 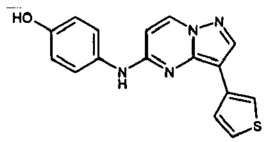 | 4-(3-Thiophen-3-yl-pyrazolo[1,5-a]pyrimidin-5-ylamino)-phenol | 1.05 | 308.36 | 307.26 |
| 43 | 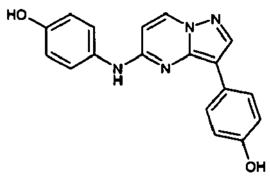 | 4-[5-[(4-hydroxyphenyl)amino]pyr azolo[1,5-a]pyrimidin-3-yl]-phenol (named using ACD) | 0.88 | 318.33 | 317.32 |
| 44 | 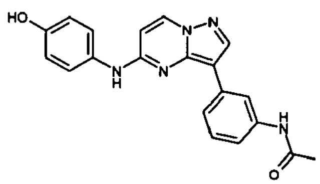 | N-{3-[5-(4-Hydroxy-phenylamino)-pyrazolo[1,5-a]pyrimidin-3-yl]-phenyl}-acetamide | 0.88 | 359.39 | 358.48 |
| 45 | 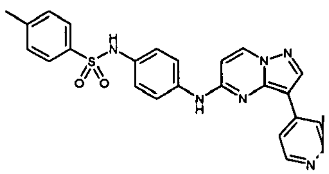 | 4-Methyl-N-[4-(3-pyridin-4-yl-pyrazolo[1,5-a]pyrimidin-5-ylamino)-phenyl]-benzenesulfonamide | 0.85 | 456.53 | 456.51 |

(continued)

| Example No. | Structure | Name | RT | MW | Observed Mass Peak [M-H] |
|---|---|---|---|---|---|
| 46 | 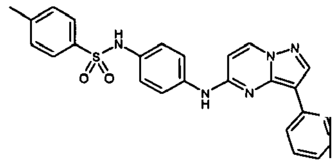 | 4-Methyl-N-[4-(3-pyridin-3-yl-pyrazolo[1,5-a]pyrimidin-5-ylamino)-phenyl]-benzenesulfonamide | 0.89 | 456.53 | 455.99 |
| 47 | 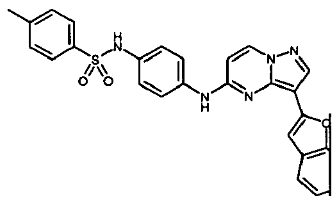 | N-[4-(3-Benzofuran-2-yl-pyrazolo[1,5-a]pyrimidin-5-ylamino)-phenyl]-4-methyl-benzenesulfonamide | | 495.56 | |
| 48 | 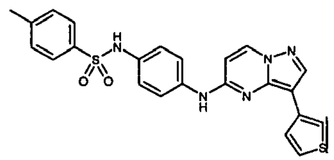 | 4-Methyl-N-[4-(3-thiophen-3-yl-pyrazolo[1,5-a]pyrimidin-5-ylamino)-phenyl]-benzenesulfonamide | 1.27 | 461.57 | 460.2 |
| 49 | 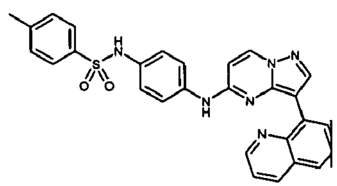 | 4-Methyl-N-[4-(3-quinolin-8-yl-pyrazolo[1,5-a]pyrimidin-5-ylamino)-phenyl]-benzenesulfonamide | 1.05 | 506.59 | 505.19 |

(continued)

| Example No. | Structure | Name | RT | MW | Observed Mass Peak [M-H] |
|---|---|---|---|---|---|
| 50 | 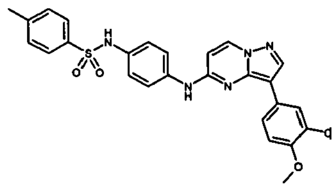 | N-{4-[3-(3,4-Dimethoxy-phenyl)-pyrazolo[1,5-a]pyrimidin-5-ylamino]-phenyl}-4-methyl-benzenesulfonamide | | 515.59 | |
| 51 | 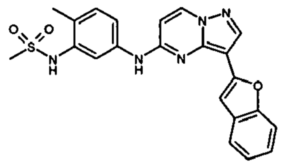 | N-[5-(3-Benzofuran-2-yl-pyrazolo[1,5-a]pyrimidin-5-ylamino)-2-methyl-phenyl]-methanesulfonamide | 1.27 | 433.49 | 432.21 |
| 52 | 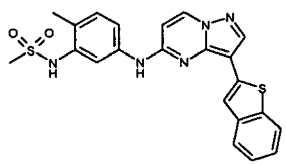 | N-[5-(3-Benzo[b]thiophen-2-yl-pyrazolo[1,5-a]pyrimidin-5-ylamino)-2-methyl-phenyl]-methanesulfonamide | 1.3 | 449.56 | 448.19 |
| 53 | 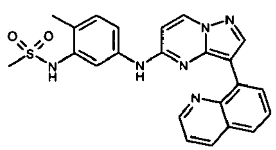 | N-[2-Methyl-5-(3-quinolin-8-yl-pyrazolo[1,5-a]pyrimidin-5-ylamino)-phenyl]-methanesulfonamide | 0.85 | 444.52 | 443.26 |
| 54 | 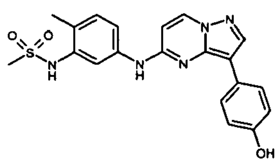 | N-{5-[3-(4-Hydroxy-phenyl)-pyrazolo[1,5-a]pyrimidin-5-ylamino]-2-methyl-phenyl}-methanesulfonamide | 0.94 | 409.47 | 409.26 |

(continued)

| Example No. | Structure | Name | RT | MW | Observed Mass Peak [M-H] |
|---|---|---|---|---|---|
| 55 | 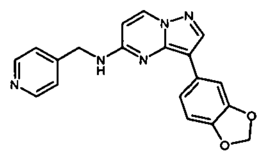 | (3-Benzo[1,3]dioxol-5-yl-pyrazolo[1,5-a]pyrimidin-5-yl)-pyridin-4-ylmethyl-amine | 0.76 | 345.36 | 344.33 |
| 56 | 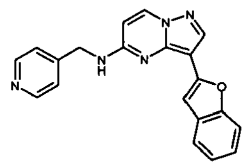 | (3-Benzofuran-2-yl-pyrazolo[1,5-a]pyrimidin-5-yl)-pyridin-4-ylmethyl-amine | 0.91 | 341.37 | 340.59 |
| 57 | 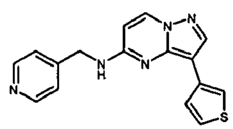 | Pyridin-4-ylmethyl-(3-thiophen-3-yl-pyrazolo[1,5-a]pyrimidin-5-yl)-amine | 0.75 | 307.38 | 306.24 |
| 58 | 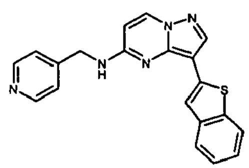 | (3-Benzo[b]thiophen-2-yl-pyrazolo[1,5-a]pyrimidin-5-yl)-pyridin-4-ylmethyl-amine | 0.93 | 357.44 | 356.3 |
| 59 | 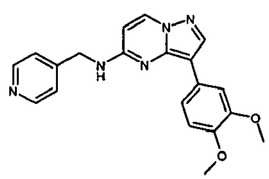 | [3-(3,4-Dimethoxy-phenyl)-pyrazolo[1,5-a]pyrimidin-5-yl]-pyridin-4-ylmethyl-amine | 0.75 | 361.4 | 360.35 |

(continued)

| Example No. | Structure | Name | RT | MW | Observed Mass Peak [M-H] |
|---|---|---|---|---|---|
| 60 | 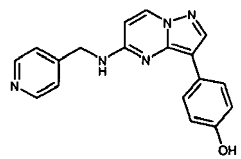 | 4-{5-[(Pyridin-4-ylmethyl)-amino]-pyrazolo[1,5-a]pyrimidin-3-yl}-phenol | 0.63 | 317.35 | 316.32 |
| 61 | 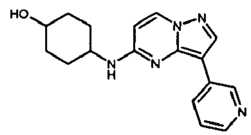 | 4-(3-Pyridin-3-yl-pyrazolo[1,5-a]pyrimidin-5-ylamino)-cyclohexanol | 0.63 | 309.37 | 308.34 |
| 62 | 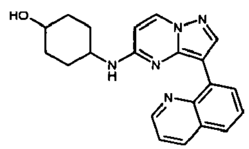 | 4-(3-Quinolin-8-yl-pyrazolo[1,5-a]pyrimidin-5-ylamino)-cyclohexanol | 0.73 | 359.43 | 358.26 |
| 63 | 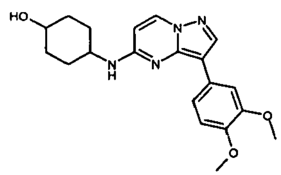 | 4-[3-(3,4-Dimethoxy-phenyl)-pyrazolo[1,5-a]pyrimidin-5-ylamino]-cyclohexanol | 0.95 | 368.43 | 367.59 |
| 64 | 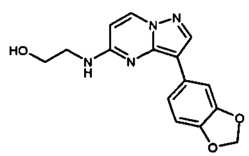 | 2-(3-Benzo[1,3]dioxol-5-yl-pyrazolo[1,5-a]pyrimidin-5-ylamino)-ethanol | 0.92 | 298.3 | 296.98 |

(continued)

| Example No. | Structure | Name | RT | MW | Observed Mass Peak [M-H] |
|---|---|---|---|---|---|
| 65 | 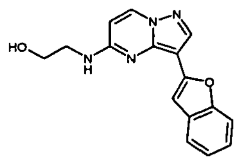 | 2-(3-Benzofuran-2-yl-pyrazolo[1,5-a]pyrimidin-5-ylamino)-ethanol | 1.09 | 294.31 | 293.56 |
| 66 | 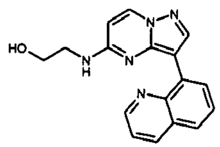 | 2-(3-Quinolin-8-yl-pyrazolo[1,5-a]pyrimidin-5-ylamino)-ethanol | 0.66 | 305.34 | 304.35 |
| 67 | 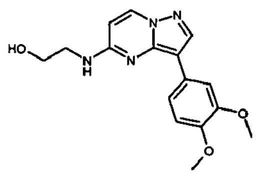 | 2-[3-(3,4-Dimethoxy-phenyl)-pyrazolo[1,5-a]pyrimidin-5-ylamino]-ethanol | 0.87 | 314.34 | 313.3 |
| 68 | 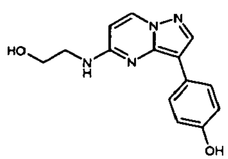 | 4-[5-(2-Hydroxy-ethylamino)-pyrazolo[1,5-a]pyrimidin-3-yl]-phenol | 0.72 | 270.29 | 269.29 |
| 69 | 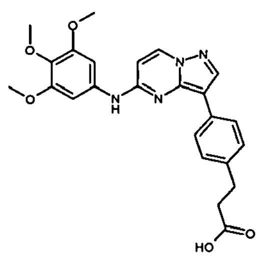 | 3-{4-[5-(3,4,5-Trimethoxy-phenylamino)-pyrazolo[1,5-a]pyrimidin-3-yl]-phenyl}-propionic acid | 1.08 | 448.48 | 447.3 |

(continued)

| Example No. | Structure | Name | RT | MW | Observed Mass Peak [M-H] |
|---|---|---|---|---|---|
| 70 | 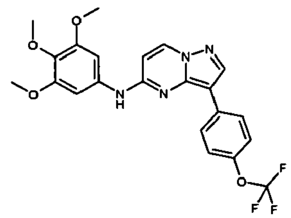 | [3-(4-Trifluoromethoxy-phenyl)-pyrazolo[1,5-a]pyrimidin-5-yl]-(3,4,5-trimethoxy-phenyl)-amine | 1.42 | 460.41 | 459.17 |
| 71 | 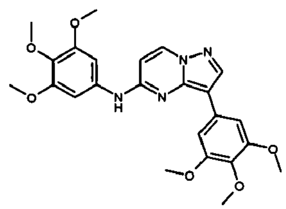 | (3,4,5-Trimethoxy-phenyl)-[3-(3,4,5-trimethoxy-phenyl)-pyrazolo[1,5-a]pyrimidin-5-yl]-amine | 1.14 | 466.49 | 464.5 |
| 72 | 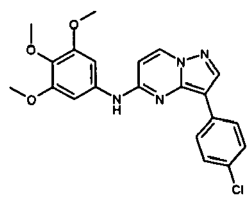 | [3-(4-Chloro-phenyl)-pyrazolo[1,5-a]pyrimidin-5-yl]-(3,4,5-trimethoxy-phenyl)-amine | 1.36 | 410.86 | 409.26 |
| 73 | 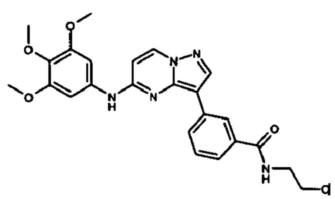 | N-(2-Hydroxy-ethyl)-3-[5-(3,4,5-trimethoxy-phenylamino)-pyrazolo[1,5-a]pyrimidin-3-yl]-benzamide | 0.94 | 463.49 | 462.2 |

(continued)

| Example No. | Structure | Name | RT | MW | Observed Mass Peak [M-H] |
|---|---|---|---|---|---|
| 74 | 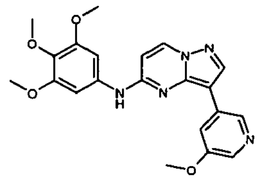 | [3-(5-Methoxy-pyridin-3-yl)-pyrazolo[1,5-a]pyrimidin-5-yl]-(3,4, 5-trimethoxy-phenyl)-amine | 0.88 | 407.43 | 407.02 |
| 75 | 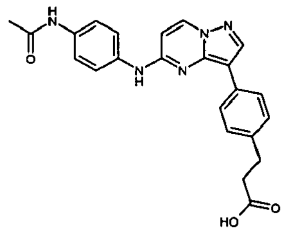 | 3-{4-[5-(4-Acetylamino-phenylamino)-pyrazolo[1,5-a]pyrimidin-3-yl]-phenyl}-propionic acid | 0.96 | 415.45 | 414.38 |
| 76 | 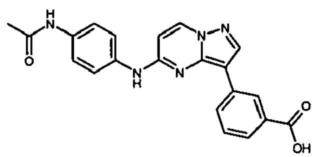 | 3-[5-(4-Acetylamino-phenylamino)-pyrazolo[1,5-a]pyrimidin-3-yl]-benzoic acid | 0.95 | 387.4 | 385.93 |
| 77 | 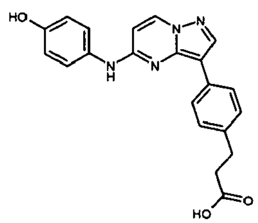 | 3-{4-[5-(4-Hydroxy-phenylamino)-pyrazolo[1,5-a]pyrimidin-3-yl]-phenyl}-propionic acid | 0.96 | 374.4 | 373.29 |

(continued)

| Example No. | Structure | Name | RT | MW | Observed Mass Peak [M-H] |
|---|---|---|---|---|---|
| 78 | 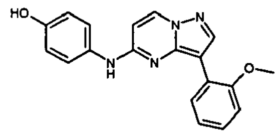 | 4-[3-(2-Methoxy-phenyl)-pyrazolo[1,5-a]pyrimidin-5-ylamino]-phenol | 1.08 | 332.36 | 331.3 |
| 79 | 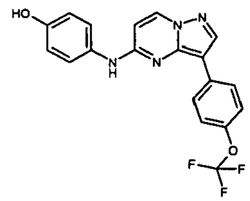 | 4-[3-(4-Trifluoromethoxy-phenyl)-pyrazolo[1,5-a]pyrimidin-5-ylamino]-phenol | 1.27 | 386.33 | 385.18 |
| 80 | 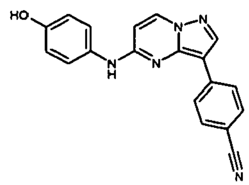 | 4-[5-(4-Hydroxy-phenylamino)-pyrazolo[1,5-a]pyrimidin-3-yl]-benzonitrile | 1.06 | 327.35 | 327.2 |
| 81 | 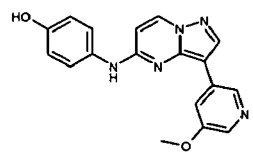 | 4-[3-(5-Methoxy-pyridin-3-yl)-pyrazolo[1,5-a]pyrimidin-5-ylamino]-phenol | 0.73 | 333.35 | 332.26 |
| 82 | 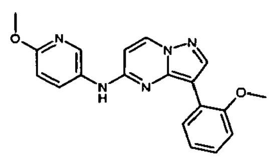 | [3-(2-Methoxy-phenyl)-pyrazolo[1,5-a]pyrimidin-5-yl]-(6-methoxy-pyridin-3-yl)-amine | 1.22 | 347.38 | 346.25 |

(continued)

| Example No. | Structure | Name | RT | MW | Observed Mass Peak [M-H] |
|---|---|---|---|---|---|
| 83 | 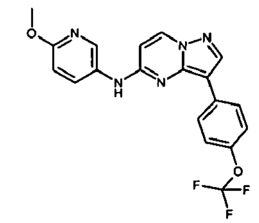 | (6-Methoxy-pyridin-3-yl)-[3-(4-trifluoromethoxy-phenyl)-pyrazolo [1,5-a]pyrimidin-5-yl]-amine | 1.4 | 401.35 | 400.22 |
| 84 | 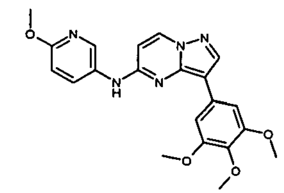 | (6-Methoxy-pyridin-3-yl)-[3-(3,4,5-trimethoxy-phenyl)-pyrazolo[1,5-a]pyrimidin-5-yl]-amine | 1.14 | 407.43 | 406.41 |
| 85 | 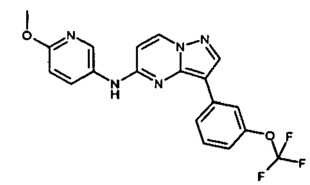 | (6-Methoxy-pyridin-3-yl)-[3-(3-trifluoromethoxy-phenyl)-pyrazolo [1,5-a]pyrimidin-5-yl]-amine | 1.39 | 401.35 | 400.23 |
| 86 | 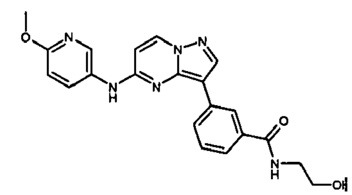 | N-(2-Hydroxy-ethyl)-3-[5-(6-methoxy-pyridin-3-ylamino)-pyrazolo [1,5-a]pyrimidin-3-yl]-benzamide | 0.88 | 404.43 | 402.93 |

(continued)

| Example No. | Structure | Name | RT | MW | Observed Mass Peak [M-H] |
|---|---|---|---|---|---|
| 87 | 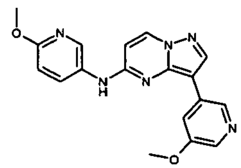 | (6-Methoxy-pyridin-3-yl)-[3-(5-methoxy-pyridin-3-yl)-pyrazolo[1,5-a]pyrimidin-5-yl]-amine | 0.81 | 348.36 | 347.3 |
| 88 | 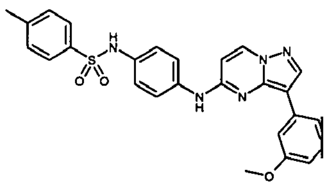 | N-{4-[3-(5-Methoxy-pyridin-3-yl)-pyrazolo[1,5-a]pyrimidin-5-ylamino]-phenyl}-4-methyl-benzenesulfonamide | 0.97 | 486.55 | 485.22 |
| 89 | 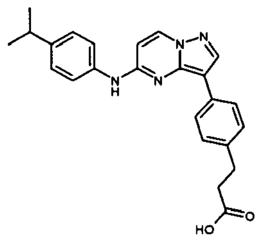 | 3-{4-[5-(4-Isopropyl-phenylamino)-pyrazolo[1,5-a]pyrimidin-3-yl]-phenyl}-propionic acid | 1.28 | 400.48 | 399.29 |
| 90 | 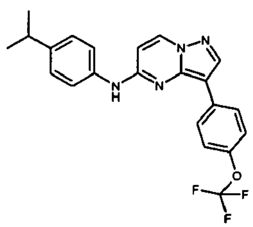 | (4-Isopropyl-phenyl)-[3-(4-trifluoromethoxy-phenyl)-pyrazolo[1,5-a] pyrimidin-5-yl]-amine | 1.66 | 412.41 | 411.24 |

(continued)

| Example No. | Structure | Name | RT | MW | Observed Mass Peak [M-H] |
|---|---|---|---|---|---|
| 91 | 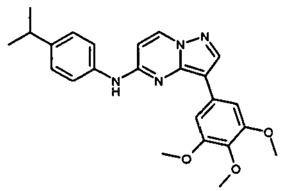 | (4-Isopropyl-phenyl)-[3-(3,4,5-trimethoxy-phenyl)-pyrazolo[1,5-a] pyrimidin-5-yl]-amine | 1.4 | 418.49 | 417.25 |
| 92 | 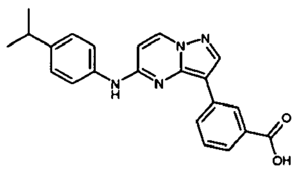 | 3-[5- (4-Isopropyl-phenylamino)-pyrazolo[1,5-a]pyrimidin-3-yl]-benzoic acid | 1.27 | 372.43 | 370.94 |
| 93 | 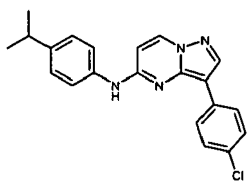 | [3-(4-Chloro-phenyl)-pyrazolo[1,5-a]pyrimidin-5-yl]-(4-isopropyl-phenyl)-amine | 1.65 | 362.86 | 361.31 |
| 94 | 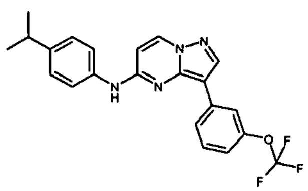 | (4-Isopropyl-phenyl)-[3-(3-trifluoromethoxy-phenyl)-pyrazolo[1,5-a] pyrimidin-5-yl]-amine | 1.66 | 412.41 | 411.26 |
| 95 | 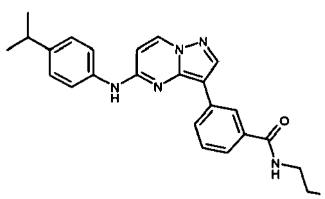 | N-(2-Hydroxy-ethyl)-3-[5-(4-isopropyl-phenylamino)-pyrazolo[1,5-a]pyrimidin-3-yl]-benzamide | 1.16 | 415.49 | 414.47 |

(continued)

| Example No. | Structure | Name | RT | MW | Observed Mass Peak [M-H] |
|---|---|---|---|---|---|
| 96 | 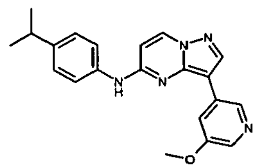 | (4-Isopropyl-phenyl)-[3-(5-methoxy-pyridin-3-yl)-pyrazolo[1,5-a] pyrimidin-5-yl]-amine | 1.1 | 359.43 | 359.15 |
| 97 | 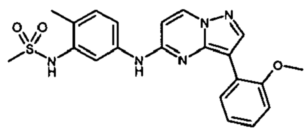 | N-{5-[3-(2-Methoxy-phenyl)-pyrazolo[1,5-a]pyrimidin-5-ylamino]-2-methyl-phenyl}-methanesulfonamide | 1.16 | 423.49 | 422.22 |
| 98 | 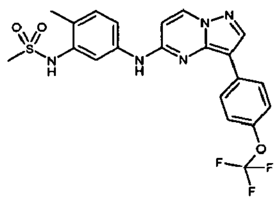 | N-{2-Methyl-5-[3-(4-trifluoromethoxy-phenyl)-pyrazolo[1,5-a] pyrimidin-5-ylamino]-phenyl}-methanesulfonamide | 1.33 | 477.46 | 476.16 |
| 99 | 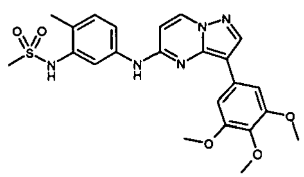 | N-{2-Methyl-5-[3-(3,4,5-trimethoxy-phenyl)-pyrazolo[1,5-a] pyrimidin-5-ylamino]-phenyl}-methanesulfonamide | 1.09 | 483.55 | 482.19 |

(continued)

| Example No. | Structure | Name | RT | MW | Observed Mass Peak [M-H] |
|---|---|---|---|---|---|
| 100 | 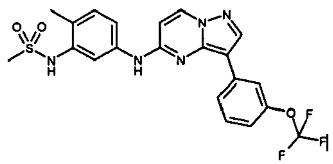 | N-{2-Methyl-5-[3-(3-trifluoromethoxy-phenyl)-pyrazolo[1,5-a] pyrimidin-5-ylamino]-phenyl}-methanesulfonamide | 1.34 | 477.46 | 476.16 |
| 101 | 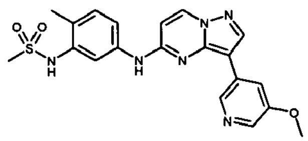 | N-{5-[3-(5-Methoxy-pyridin-3-yl)-pyrazolo[1, 5-a]pyrimidin-5-ylamino]-2-methyl-phenyl}-methanesulfonamide | 0.81 | 424.48 | 424.24 |
| 102 | 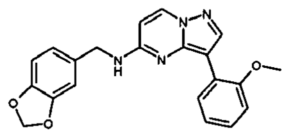 | Benzo[1,3]dioxol-5-ylmethyl-[3-(2-methoxy-phenyl)-pyrazolo[1,5-a] pyrimidin-5-yl]-amine | 1.28 | 374.4 | 373.34 |
| 103 | 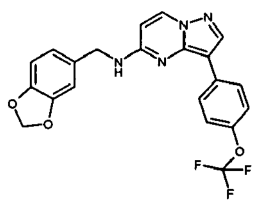 | Benzo[1,3]dioxol-5-ylmethyl-[3-(4-trifluoromethoxy-phenyl)-pyrazolo[1,5-a]pyrimidin-5-yl]-amine | 1.45 | 428.37 | 427.19 |
| 104 | 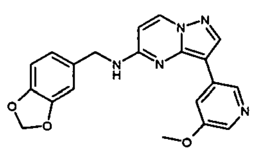 | Benzo[1,3]dioxol-5-ylmethyl-[3-(5-methoxy-pyridin-3-yl)-pyrazolo [1,5-a]pyrimidin-5-yl]-amine | 0.86 | 375.39 | 374.43 |

(continued)

| Example No. | Structure | Name | RT | MW | Observed Mass Peak [M-H] |
|---|---|---|---|---|---|
| 105 | 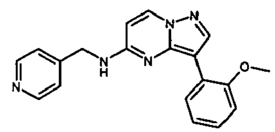 | [3-(2-Methoxy-phenyl)-pyrazolo[1,5-a]pyrimidin-5-yl]-pyridin-4-ylmethyl-amine | 0.78 | 331.38 | 330.27 |
| 106 | 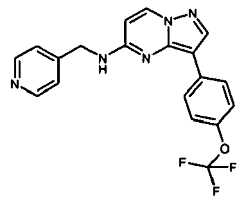 | Pyridin-4-ylmethyl-[3-(4-trifluoromethoxy-phenyl)-pyrazolo[1,5-a] pyrimidin-5-yl]-amine | 0.97 | 385.35 | 384.08 |
| 107 | 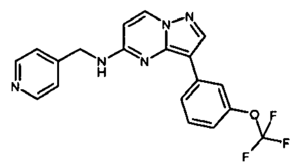 | Pyridin-4-ylmethyl-[3-(3-trifluoromethoxy-phenyl)-pyrazolo[1,5-a] pyrimidin-5-yl]-amine | 0.96 | 385.35 | 384.27 |
| 108 | 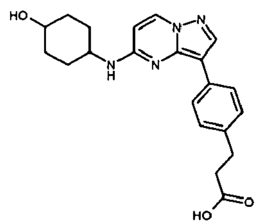 | 3-{4-[5-(4-Hydroxy-cyclohexylamino)-pyrazolo[1,5-a]pyrimidin-3-yl]-phenyl}-propionic acid | 0.92 | 380.45 | 379.29 |
| 109 | 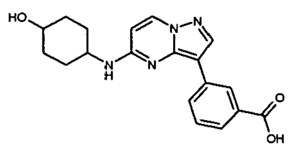 | 3-[5-(4-Hydroxy-cyclohexylamino)-pyrazolo[1,5-a]pyrimidin-3-yl]-benzoic acid | 0.91 | 352.39 | 351.29 |

(continued)

| Example No. | Structure | Name | RT | MW | Observed Mass Peak [M-H] |
|---|---|---|---|---|---|
| 110 | 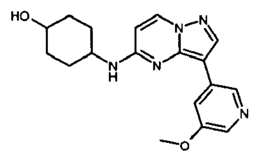 | 4-[3-(5-Methoxy-pyridin-3-yl)-pyrazolo[1,5-a]pyrimidin-5-ylamino]-cyclohexanol | 0.69 | 339.4 | 338.32 |
| 111 | 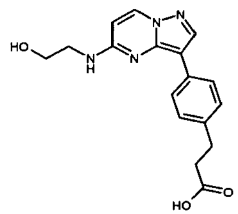 | 3-{4-[5-(2-Hydroxy-ethylamino)-pyrazolo[1,5-a]pyrimidin-3-yl]-phenyl}-propionic acid | 0.83 | 326.35 | 325.3 |
| 112 | 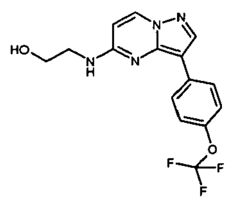 | 2-[3-(4-Trifluoromethoxy-phenyl)-pyrazolo[1,5-a]pyrimidin-5-ylamino]-ethanol | 1.17 | 338.29 | 337.23 |
| 113 | 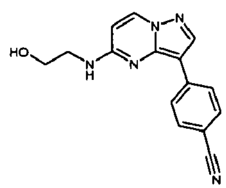 | 4-[5-(2-Hydroxy-ethylamino)-pyrazolo[1,5-a]pyrimidin-3-yl]-benzonitrile | 0.92 | 279.3 | 278.33 |
| 114 | 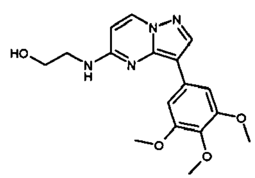 | 2-[3-(3,4,5-Trimethoxy-phenyl)-pyrazolo[1,5-a]pyrimidin-5-ylamino]-ethanol | 0.89 | 344.37 | 343.11 |

(continued)

| Example No. | Structure | Name | RT | MW | Observed Mass Peak [M-H] |
|---|---|---|---|---|---|
| 115 | 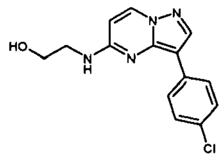 | 2-[3-(4-Chloro-phenyl)-pyrazolo[1,5-a]pyrimidin-5-ylamino]-ethanol | 1.09 | 288.74 | 287.26 |
| 116 | 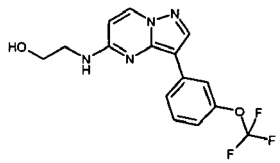 | 2-[3-(3-Trifluoromethoxy-phenyl)-pyrazolo[1,5-a]pyrimidin-5-ylamino]-ethanol | 1.16 | 338.29 | 337.21 |
| 117 | 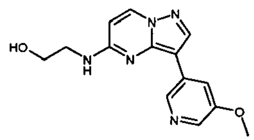 | 2-[3-(5-Methoxy-pyridin-3-yl)-pyrazolo[1,5-a]pyrimidin-5-ylamino]-ethanol | 0.61 | 285.31 | 285.09 |
| 118 | 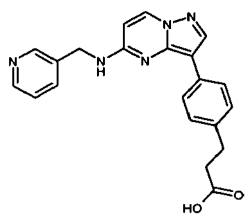 | 3-(4-{5-[(Pyridin-3-ylmethyl)-amino]-pyrazolo[1,5-a]pyrimidin-3-yl}-phenyl)-propionic acid | 0.75 | 373.41 | 372.32 |
| 119 | 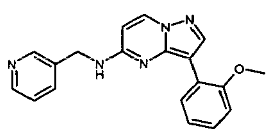 | [3-(2-Methoxy-phenyl)-pyrazolo[1,5-a]pyrimidin-5-yl]-pyridin-3-ylmethyl-amine | 0.83 | 331.38 | 330.31 |

(continued)

| Example No. | Structure | Name | RT | MW | Observed Mass Peak [M-H] |
|---|---|---|---|---|---|
| 120 | 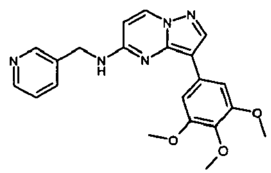 | Pyridin-3-ylmethyl-[3-(3,4,5-trimethoxy-phenyl)-pyrazolo[1,5-a] pyrimidin-5-yl]-amine | 0.81 | 391.43 | 390.27 |
| 121 | 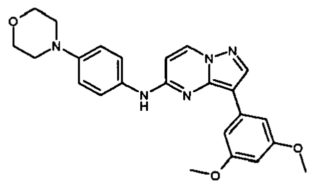 | [3-(3,5-Dimethoxy-phenyl)-pyrazolo[1,5-a]pyrimidin-5-yl]-(4-morpholin-4-yl-phenyl)-amine | | | |
| 122 | 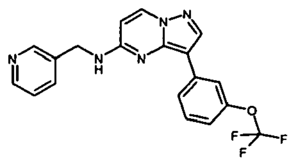 | Pyridin-3-ylmethyl-[3-(3-trifluoromethoxy-phenyl)-pyrazolo[1,5-a] pyrimidin-5-yl]-amine | 1.03 | 385.35 | 384.02 |
| 123 | 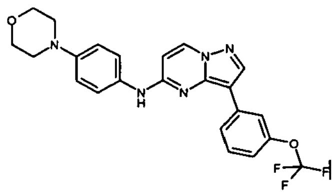 | (4-Morpholin-4-yl-phenyl)-[3-(3-trifluoromethoxy-phenyl)-pyrazolo [1,5-a]pyrimidin-5-yl]-amine | 1.39 | 455.44 | 454.26 |

(continued)

| Example No. | Structure | Name | RT | MW | Observed Mass Peak [M-H] |
|---|---|---|---|---|---|
| 124 | 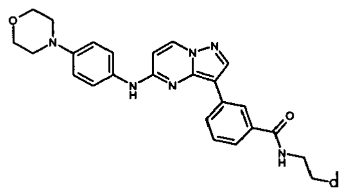 | N-(2-Hydroxy-ethyl)-3-[5-(4-morpholin-4-yl-phenylamino)-pyrazolo [1,5-a]pyrimidin-3-yl]-benzamide | 0.89 | 458.52 | 457.27 |
| 125 | 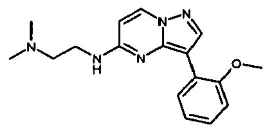 | N'-[3-(2-Methoxy-phenyl)-pyrazolo[1,5-a]pyrimidin-5-yl]-N,N-dimethyl-ethane-1,2-diamine | 0.75 | 311.39 | 309.56 |
| 126 | 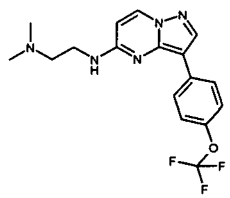 | N,N-Dimethyl-N'-[3-(4-trifluoromethoxy-phenyl)-pyrazolo[1,5-a] pyrimidin-5-yl]-ethane-1,2-diamine | 0.92 | 365.36 | 364.33 |
| 127 | 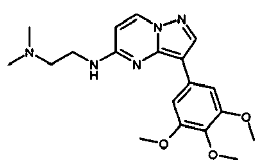 | N, N-Dimethyl-N'-[3-(3,4, 5-trimethoxy-phenyl)-pyrazolo[1,5-a] pyrimidin-5-yl]-ethane-1,2-diamine | 0.74 | 371.44 | 370.25 |
| 128 | 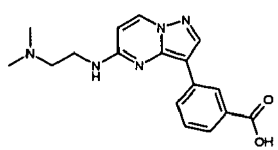 | 3-[5-(2-Dimethylamino-ethylamino)-pyrazolo[1,5-a]pyrimidin-3-yl]-benzoic acid | 0.69 | 325.37 | 324.35 |

(continued)

| Example No. | Structure | Name | RT | MW | Observed Mass Peak [M-H] |
|---|---|---|---|---|---|
| 129 | 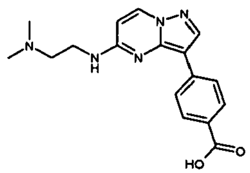 | 4-[5-(2-Dimethylamino-ethylamino)-pyrazolo[1,5-a]pyrimidin-3-yl]-benzoic acid | 0.65 | 325.37 | 324.32 |
| 130 | 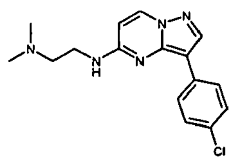 | N'-[3-(4-Chloro-phenyl)-pyrazolo[1,5-a]pyrimidin-5-yl]-N,N-dimethyl-ethane-1,2-diamine | 0.83 | 315.81 | 314.35 |
| 131 | 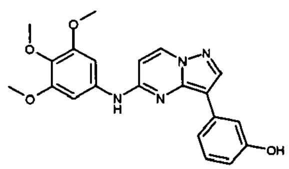 | 3-[5-(3,4,5-Trimethoxy-phenylamino)-pyrazolo[1,5-a]pyrimidin-3-yl]-phenol | 1.04 | 392.41 | 391.3 |
| 132 | 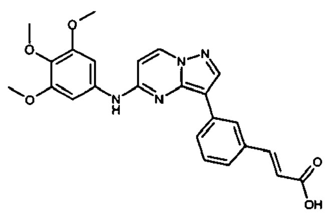 | (E)-3-{3-[5-(3,4,5-Trimethoxy-phenylamino)-pyrazolo[1,5-a] pyrimidin-3-yl]-phenyl}-acrylic acid | 1.09 | 446.46 | 445.15 |
| 133 | 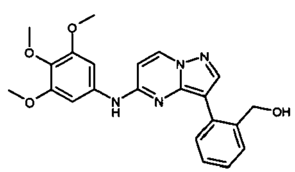 | {2-[5-(3,4,5-Trimethoxy-phenylamino)-pyrazolo[1,5-a]pyrimidin-3-yl]-phenyl}-methanol | 1.07 | 406.44 | 405.28 |

(continued)

| Example No. | Structure | Name | RT | MW | Observed Mass Peak [M-H] |
|---|---|---|---|---|---|
| 134 | 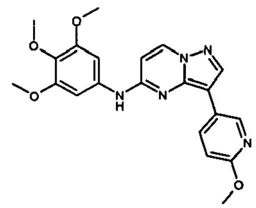 | [3-(6-Methoxy-pyridin-3-yl)-pyrazolo[1,5-a]pyrimidin-5-yl]-(3,4,5-trimethoxy-phenyl)-amine | 1.18 | 407.43 | 406.31 |
| 135 | 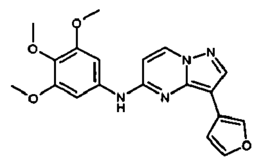 | (3-Furan-3-yl-pyrazolo[1,5-a]pyrimidin-5-yl)-(3,4,5-trimethoxy-phenyl)-amine | 1.14 | 366.38 | 365.29 |
| 136 | 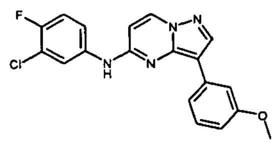 | (3-Chloro-4-fluoro-phenyl)-[3-(3-methoxy-phenyl)-pyrazolo[1,5-a]pyrimidin-5-yl]-amine | 1.41 | 368.8 | 367.2 |
| 137 | 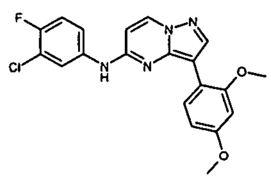 | (3-Chloro-4-fluoro-phenyl)-[3-(2,4-dimethoxy-phenyl)-pyrazolo[1,5-a]pyrimidin-5-yl]-amine | 1.4 | 398.82 | 397.16 |
| 138 | 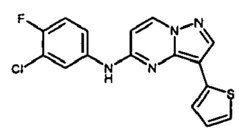 | (3-Chloro-4-fluoro-phenyl)-(3-thiophen-2-yl-pyrazolo[1,5-a]pyrimidin-5-yl)-amine | 1.41 | 344.8 | 343.32 |

(continued)

| Example No. | Structure | Name | RT | MW | Observed Mass Peak [M-H] |
|---|---|---|---|---|---|
| 139 | 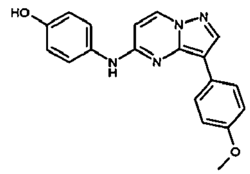 | 4-[3-(4-Methoxy-phenyl)-pyrazolo[1,5-a]pyrimidin-5-ylamino]-phenol | 1.07 | 332.36 | 331.35 |
| 140 | 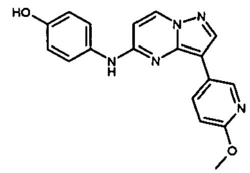 | 4-[3-(6-Methoxy-pyridin-3-yl)-pyrazolo[1,5-a]pyrimidin-5-ylamino]-phenol | 0.96 | 333.35 | 331.67 |
| 141 | 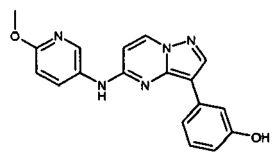 | 3-[5-(6-Methoxy-pyridin-3-ylamino)-pyrazolo[1,5-a]pyrimidin-3-yl]-phenol | 0.99 | 333.35 | 332.24 |
| 142 | 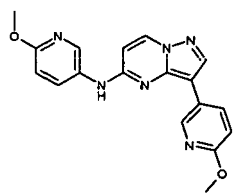 | (6-Methoxy-pyridin-3-yl)-[3-(6-methoxy-pyridin-3-yl)-pyrazolo[1,5-a]pyrimidin-5-yl]-amine | 1.08 | 348.36 | 347.3 |
| 143 | 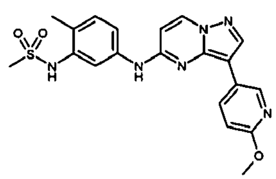 | N-{5-[3-(6-Methoxy-pyridin-3-yl)-pyrazolo[1,5-a]pyrimidin-5-ylamino]-2-methyl-phenyl}-methanesulfonamide | | 424.48 | |

(continued)

| Example No. | Structure | Name | RT | MW | Observed Mass Peak [M-H] |
|---|---|---|---|---|---|
| 144 | 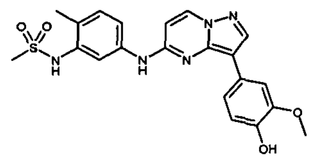 | N-{5-[3-(4-Hydroxy-3-methoxy-phenyl)-pyrazolo[1,5-a]pyrimidin-5-ylamino]-2-methyl-phenyl}-methanesulfonamide | 0.97 | 439.49 | 438.07 |
| 145 | 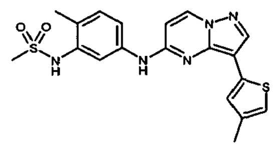 | N-{2-Methyl-5-[3-(4-methyl-thiophen-2-yl)-pyrazolo[1,5-a]pyrimidin-5-ylamino]-phenyl}-methanesulfonamide | 1.22 | 413.52 | 412.24 |
| 146 | 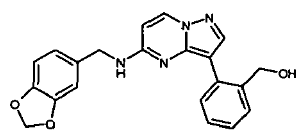 | (2-{5-[(Benzo[1,3]dioxol-5-ylmethyl)-amino]-pyrazolo[1,5-a]pyrimidin-3-yl}-phenyl)-methanol | 1.1 | 374.4 | 373.27 |
| 147 | 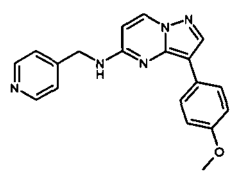 | [3-(4-Methoxy-phenyl)-pyrazolo[1,5-a]pyrimidin-5-yl]-pyridin-4-ylmethyl-amine | 0.78 | 331.38 | 330.34 |
| 148 | 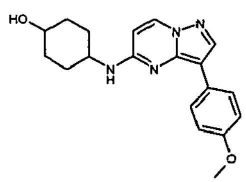 | 4-[3-(4-Methoxy-phenyl)-pyrazolo[1,5-a]pyrimidin-5-ylamino]-cyclohexanol | 1.01 | 338.41 | 337.1 |

(continued)

| Example No. | Structure | Name | RT | MW | Observed Mass Peak [M-H] |
|---|---|---|---|---|---|
| 149 | 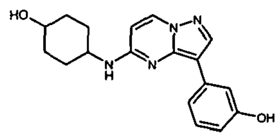 | 3-[5-(4-Hydroxy-cyclohexylamino)-pyrazolo[1,5-a]pyrimidin-3-yl]-phenol | 0.85 | 324.38 | 324.26 |
| 150 | 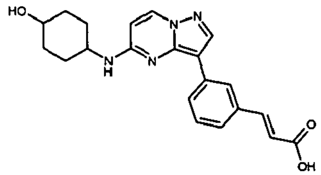 | (E)-3-{3-[5-(4-Hydroxy-cyclohexylamino)-pyrazolo[1,5-a]pyrimidin-3-yl]-phenyl}-acrytic acid | 0.98 | 378.43 | 377.38 |
| 151 | 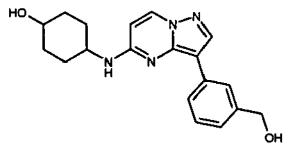 | 4-[3-(3-Hydroxymethyl-phenyl)-pyrazolo[1,5-a]pyrimidin-5-ylamino]-cyclohexanol | 0.86 | 338.41 | 337.42 |
| 152 | 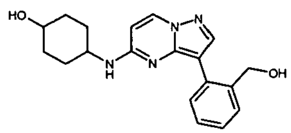 | 4-[3-(2-Hydroxymethyl-phenyl)-pyrazolo[1,5-a]pyrimidin-5-ylamino]-cyclohexanol | 0.87 | 338.41 | 337.37 |
| 153 | 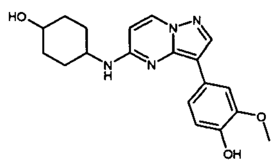 | 4-[5-(4-Hydroxy-cyclohexylamino)-pyrazolo[1,5-a]pyrimidin-3-yl]-2-methoxy-phenol | 0.86 | 354.41 | 353.08 |

(continued)

| Example No. | Structure | Name | RT | MW | Observed Mass Peak [M-H] |
|---|---|---|---|---|---|
| 154 | 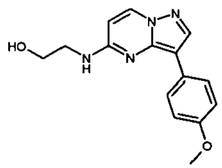 | 2-[3-(4-Methoxy-phenyl)-pyrazolo[1,5-a]pyrimidin-5-ylamino]-ethanol | 0.93 | 284.32 | 283.3 |
| 155 | 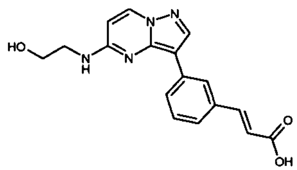 | (E)-3-{3-[5-(2-Hydroxy-ethylamino)-pyrazolo[1,5-a]pyrimidin-3-yl]-phenyl}-acrylic acid | 0.89 | 324.34 | 323.12 |
| 156 | 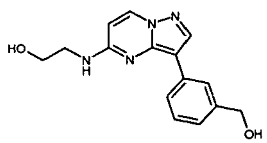 | 2-[3-(3-Hydroxymethyl-phenyl)-pyrazolo[1,5-a]pyrimidin-5-ylamino]-ethanol | 0.77 | 284.32 | 283.24 |
| 157 | 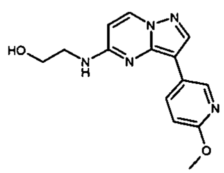 | 2-[3-(6-Methoxy-pyridin-3-yl)-pyrazolo[1,5-a]pyrimidin-5-ylamino]-ethanol | 0.81 | 285.31 | 283.62 |
| 158 | 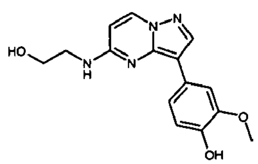 | 4-[5-(2-Hydroxy-ethylamino)-pyrazolo[1,5-a]pyrimidin-3-yl]-2-methoxy-phenol | 0.76 | 300.32 | 299.34 |

(continued)

| Example No. | Structure | Name | RT | MW | Observed Mass Peak [M-H] |
|---|---|---|---|---|---|
| 159 | 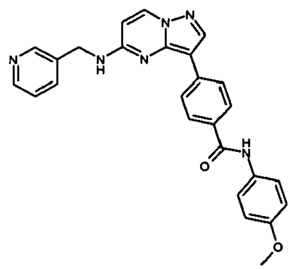 | N-(4-Methoxy-phenyl)-4-{5-[(pyridin-3-ylmethyl)-amino]-pyrazolo [1,5-a]pyrimidin-3-yl}-benzamide | 0.89 | 450.5 | 449.26 |
| 160 | 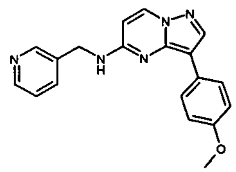 | [3-(4-Methoxy-phenyl)-pyrazolo[1,5-a]pyrimidin-5-yl]-pyridin-3-ylmethyl-amine | 0.82 | 331.38 | 329.43 |
| 161 | 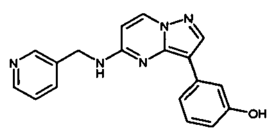 | 3-{5-[(Pyridin-3-ylmethyl)-amino]-pyrazolo[1,5-a]pyrimidin-3-yl}-phenol | 0.67 | 317.35 | 316.34 |
| 162 | 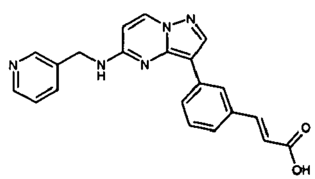 | (E)-3-(3-{5-[(Pyridin-3-ylmethyl)-amino]-pyrazolo[1,5-a]pyrimidin-3-yl}-phenyl)-acrylic acid | 0.79 | 371.4 | 370.27 |

(continued)

| Example No. | Structure | Name | RT | MW | Observed Mass Peak [M-H] |
|---|---|---|---|---|---|
| 163 | 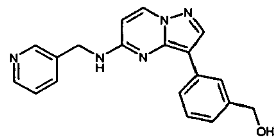 | (3-{5-[(Pyridin-3-ylmethyl)-amino]-pyrazolo[1,5-a]pyrimidin-3-yl}-phenyl)-methanol | 0.68 | 331.38 | 330.33 |
| 164 | 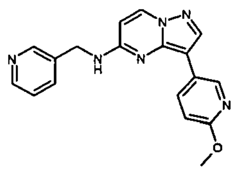 | [3-(6-Methoxy-pyridin-3-yl)-pyrazolo[1,5-a]pyrimidin-5-yl]-pyridin-3-ylmethyl-amine | 0.73 | 332.36 | 331.27 |
| 165 | 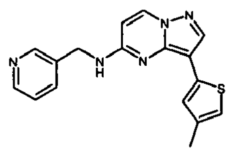 | [3-(4-Methyl-thiophen-2-yl)-pyrazolo[1,5-a]pyrimidin-5-yl]-pyridin-3-ylmethyl-amine | 0.87 | 321.41 | 320.22 |
| 166 | 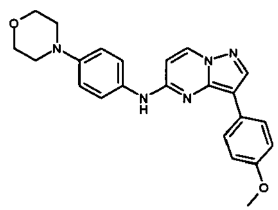 | [3-(4-Methoxy-phenyl)-pyrazolo[1,5-a]pyrimidin-5-yl]-(4-morpholin-4-yl-phenyl)-amine | 1.18 | 401.47 | 400.27 |
| 167 | 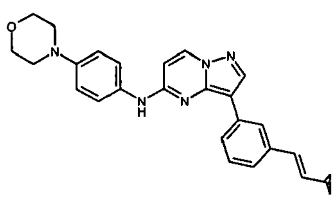 | (E)-3-{3-[5-(4-Morpholin-4-yl-phenylamino)-pyrazolo[1,5-a] pyrimidin-3-yl]-phenyl}-acrylic acid | 1.08 | 441.49 | 440.28 |

(continued)

| Example No. | Structure | Name | RT | MW | Observed Mass Peak [M-H] |
|---|---|---|---|---|---|
| 168 | 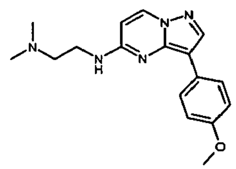 | N'-[3-(4-Methoxy-phenyl)-pyrazolo[1,5-a]pyrimidin-5-yl]-N,N-dimethyl-ethane-1,2-diamine | 0.74 | 311.39 | 311.16 |
| 169 | 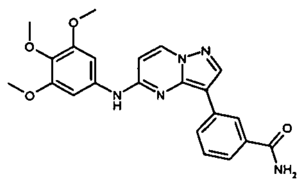 | 3-[5-(3,4,5-Trimethoxy-phenylamino)-pyrazolo[1,5-a]pyrimidin-3-yl]-benzamide | 0.95 | 419.44 | 418.28 |
| 170 | 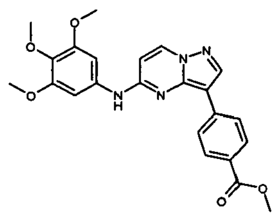 | 4-[5-(3,4,5-Trimethoxy-phenylamino)-pyrazolo[1,5-a]pyrimidin-3-yl]-benzoic acid methyl ester | 1.23 | 434.45 | 433.3 |
| 171 | 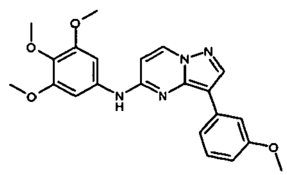 | [3-(3-Methoxy-phenyl)-pyrazolo[1,5-a]pyrimidin-5-yl]-(3,4,5-trimethoxy-phenyl)-amine | 1.23 | 406.44 | 405.29 |
| 172 | 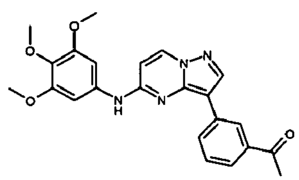 | 1-{3-[5-(3,4,5-Trimethoxy-phenylamino)-pyrazolo[1,5-a]pyrimidin-3-yl]-phenyl}-ethanone | 1.17 | 418.45 | 417.27 |

(continued)

| Example No. | Structure | Name | RT | MW | Observed Mass Peak [M-H] |
|---|---|---|---|---|---|
| 173 | 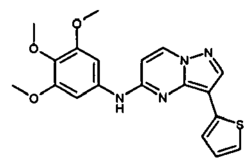 | (3-Thiophen-2-yl-pyrazolo[1,5-a]pyrimidin-5-yl)-(3,4,5-trimethoxy-phenyl)-amine | 1.22 | 382.44 | 381.22 |
| 174 | 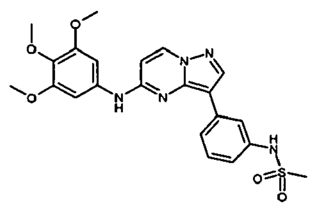 | N-{3-[5-(3,4,5-Trimethoxy-phenylamino)-pyrazolo[1,5-a]pyrimidin-3-yl]-phenyl}-methanesulfonamide | 1.05 | 469.52 | 468.19 |
| 175 | 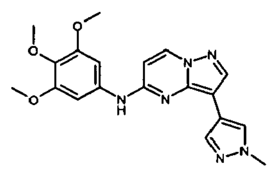 | [3-(1-Methyl-1H-pyrazol-4-yl)-pyrazolo[1,5-a]pyrimidin-5-yl]-(3,4,5-trimethoxy-phenyl)-amine | 0.95 | 380.41 | 379.22 |
| 176 | 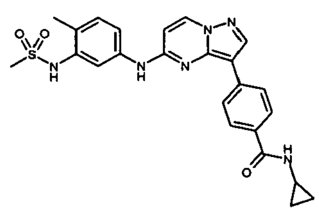 | N-Cyclopropyl-4-[5-(3-methanesulfonylamino-4-methyl-phenylamino)-pyrazolo[1,5-a]pyrimidin-3-yl]-benzamide | 0.65 | 476.56 | 475.5 |

(continued)

| Example No. | Structure | Name | RT | MW | Observed Mass Peak [M-H] |
|---|---|---|---|---|---|
| 177 | 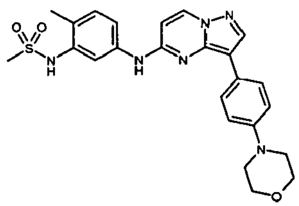 | N-{2-Methyl-5-[3-(4-morpholin-4-yl-phenyl)-pyrazolo[1,5-a] pyrimidin-5-ylamino]-phenyl}-methanesutfonamide | 1.05 | 478.57 | 477.24 |
| 178 | 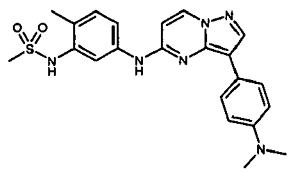 | N-{5-[3-(4-Dimethylamino-phenyl)-pyrazolo[1,5-a]pyrimidin-5-ylamino]-2-methyl-phenyl}-methanesulfonamide | 0.87 | 436.54 | 435.23 |
| 179 | 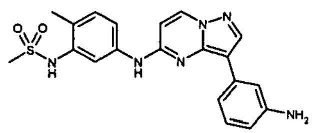 | N-{5-[3-(3-Amino-phenyl)-pyrazolo[1,5-a]pyrimidin-5-ylamino]-2-methyl-phenyl}-methanesulfonamide | 0.83 | 408.48 | 407.22 |
| 180 | 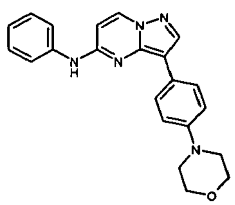 | [3-(4-Morpholin-4-yl-phenyl)-pyrazolo[1,5-a]pyrimidin-5-yl]-phenyl-amine | 1.19 | 371.44 | 370.33 |

(continued)

| Example No. | Structure | Name | RT | MW | Observed Mass Peak [M-H] |
|---|---|---|---|---|---|
| 181 | 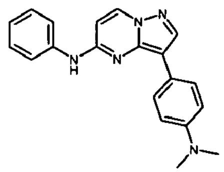 | [3-(4-Dimethylamino-phenyl)-pyrazolo[1,5-a]pyrimidin-5-yl]-phenyl-amine | 1.02 | 329.4 | 329.16 |
| 182 | 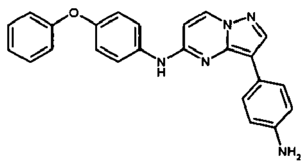 | [3-(4-Amino-phenyl)-pyrazolo[1,5-a]pyrimidin-5-yl]-(4-phenoxy-phenyl)-amine | 1.11 | 393.45 | 392.29 |
| 183 | 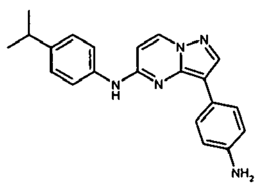 | [3-(4-Amino-phenyl)-pyrazolo[1,5-a]pyrimidin-5-yl]-(4-isopropyl-phenyl)-amine | 1.08 | 343.43 | 342.4 |
| 184 | 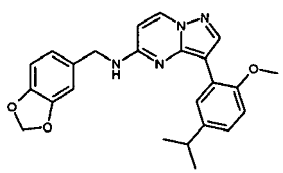 | Benzo[1,3]dioxol-5-ylmethyl-[3-(5-isopropyl-2-methoxy-phenyl)-pyrazolo[1,5-a]pyrimidin-5-yl]-amine | 1.46 | 416.48 | 415.25 |
| 185 | 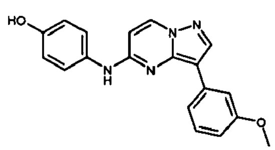 | 4-[3-(3-Methoxy-phenyl)-pyrazolo[1,5-a]pyrimidin-5-ylamino]-phenol | 1.08 | 332.36 | 331.31 |

(continued)

| Example No. | Structure | Name | RT | MW | Observed Mass Peak [M-H] |
|---|---|---|---|---|---|
| 186 | 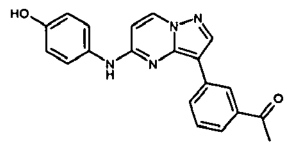 | 1-{3-[5-(4-Hydroxy-phenylamino)-pyrazolo[1,5-a]pyrimidin-3-yl]-phenyl}-ethanone | 1.03 | 344.37 | 343.34 |
| 187 | 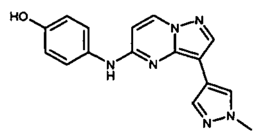 | 4-[3-(1-Methyl-1H-pyrazol-4-yl)-pyrazolo[1,5-a]pyrimidin-5-ylamino]-phenol | 0.81 | 306.33 | 305.09 |
| 188 | 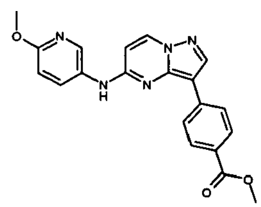 | 4-[5-(6-Methoxy-pyridin-3-ylamino)-pyrazolo[1, 5-a]pyrimidin-3-yl]-benzoic acid methyl ester | 1.19 | 375.39 | 374.26 |
| 189 | 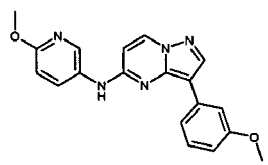 | [3-(3-Methoxy-phenyl)-pyrazolo[1,5-a]pyrimidin-5-yl]-(6-methoxy-pyridin-3-yl)-amine | 1.22 | 347.38 | 346.3 |
| 190 | 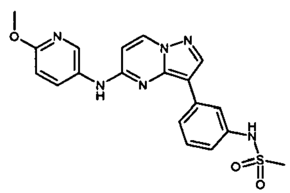 | N-{3-[5-(6-Methoxy-pyridin-3-ylamino)-pyrazolo[1,5-a]pyrimidin-3-yl]-phenyl}-methanesulfonamide | 1 | 410.46 | 409.22 |

(continued)

| Example No. | Structure | Name | RT | MW | Observed Mass Peak [M-H] |
|---|---|---|---|---|---|
| 191 | 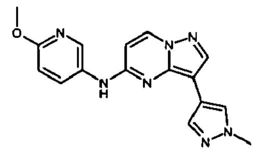 | (6-Methoxy-pyridin-3-yl)-[3-(1-methyl-1H-pyrazol-4-yl)-pyrazolo [1,5-a]pyrimidin-5-yl]-amine | 0.89 | 321.34 | 320.36 |
| 192 | 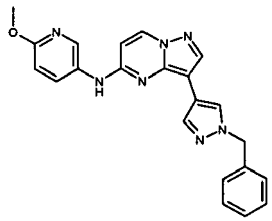 | [3-(1-Benzyl-1H-pyrazol-4-yl)-pyrazolo[1,5-a]pyrimidin-5-yl]-(6-methoxy-pyridin-3-yl)-amine | 1.13 | 397.44 | 396.22 |
| 193 | 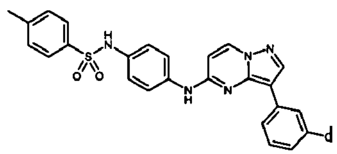 | N-{4-[3-(3-Methoxy-phenyl)-pyrazolo[1,5-a]pyrimidin-5-ylamino]-phenyl}-4-methyl-benzenesulfonamide | 1.29 | 485.57 | 484.23 |
| 194 | 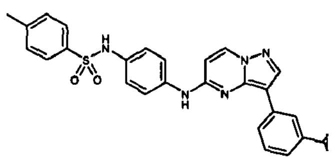 | N-{4-[3-(3-Acetyl-phenyl)-pyrazolo[1,5-a]pyrimidin-5-ylamino]-phenyl}-4-methyl-benzenesulfonamide | 1.25 | 497.58 | 496.25 |

(continued)

| Example No. | Structure | Name | RT | MW | Observed Mass Peak [M-H] |
|---|---|---|---|---|---|
| 195 | 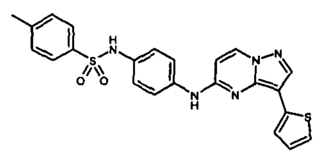 | 4-Methyl-N-[4-(3-thiophen-2-yl-pyrazolo[1,5-a]pyrimidin-5-ylamino)-phenyl]-benzenesulfonamide | 1.27 | 461.57 | 460.16 |
| 196 | 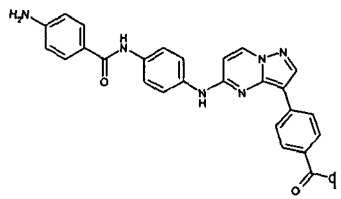 | 4-{5-[4-(4-Amino-benzoylamino)-phenylamino]-pyrazolo[1,5-a] pyrimidin-3-yl}-benzoic acid methyl ester | 1.13 | 478.51 | 477.29 |
| 197 | 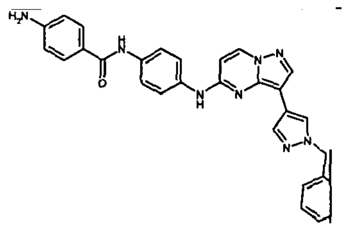 | 4-Amino-N-{4-[3-(1-benzyl-1H-pyrazol-4-yl)-pyrazolo[1,5-a] pyrimidin-5-ylamino]-phenyl}-benzamide | 1.06 | 500.56 | 499.3 |
| 198 | 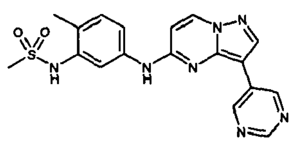 | N-[2-Methyl-5-(3-pyrimidin-5-yl-pyrazolo[1,5-a]pyrimidin-5-ylamino)-phenyl]-methanesulfonamide | 0.87 | 395.45 | 394.3 |

(continued)

| Example No. | Structure | Name | RT | MW | Observed Mass Peak [M-H] |
|---|---|---|---|---|---|
| 199 | 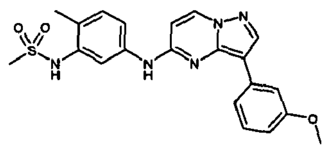 | N-{5-[3-(3-Methoxy-phenyl)-pyrazolo[1,5-a]pyrimidin-5-ylamino]-2-methyl-phenyl}-methanesulfonamide | 1.17 | 423.49 | 422.21 |
| 200 | 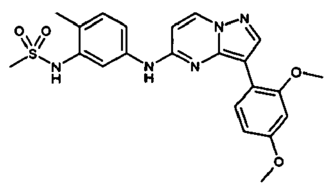 | N-{5-[3-(2,4-Dimethoxy-phenyl)-pyrazolo[1,5-a]pyrimidin-5-ylamino]-2-methyl-phenyl}-methanesulfonamide | 1.14 | 453.52 | 452.15 |
| 201 | 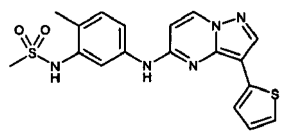 | N-[2-Methyl-5-(3-thiophen-2-yl-pyrazolo[1,5-a]pyrimidin-5-ylamino)-phenyl]-methanesulfonamide | 1.14 | 399.5 | 398.13 |
| 202 | 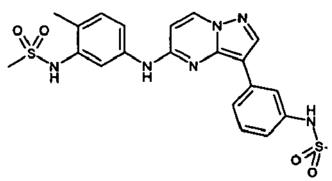 | N-{5-[3-(3-Methanesulfonylamino-phenyl)-pyrazolo[1,5-a] pyrimidin-5-ylamino]-2-methyl-phenyl}-methanesulfonamide | 0.98 | 486.58 | 485.14 |

(continued)

| Example No. | Structure | Name | RT | MW | Observed Mass Peak [M-H] |
|---|---|---|---|---|---|
| 203 | 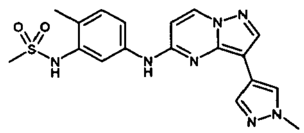 | N-{2-Methyl-5-[3-(1-methyl-1H-pyrazol-4-yl)-pyrazolo[1,5-a] pyrimidin-5-ylamino]-phenyl}-methanesulfonamide | 0.89 | 397.46 | 396.26 |
| 204 | 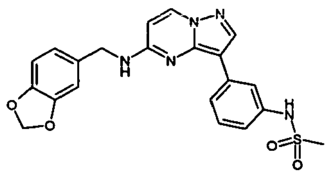 | N-(3-{5-[(Benzo[1,3]dioxol-5-ylmethyl)-amino]-pyrazolo[1,5-a] pyrimidin-3-yl}-phenyl)-methanesulfonamide | 1.07 | 437.48 | 436.26 |
| 205 | 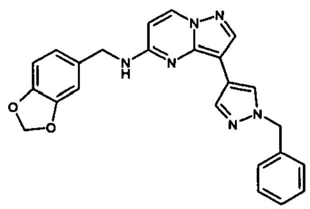 | Benzo[1,3]dioxol-5-ylmethyl-[3-(1-benzyl-1H-pyrazol-4-yl)-pyrazolo [1,5-a]pyrimidin-5-yl]-amine | 1.19 | 424.46 | 423.29 |
| 206 | 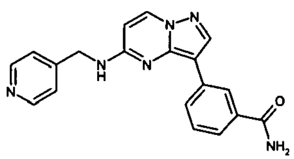 | 3-{5-[(Pyridin-4-ylmethyl)-amino]-pyrazolo[1,5-a]pyrimidin-3-yl}-benzamide | 0.62 | 344.38 | 343.31 |

(continued)

| Example No. | Structure | Name | RT | MW | Observed Mass Peak [M-H] |
|---|---|---|---|---|---|
| 207 | 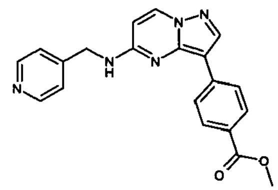 | 4-{5-[(Pyridin-4-ylmethyl)-amino]-pyrazolo[1,5-a]pyrimidin-3-yl}-benzoic acid methyl ester | 0.81 | 359.39 | 358.27 |
| 208 | 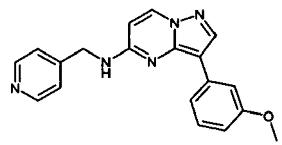 | [3-(3-Methoxy-phenyl)-pyrazolo[1,5-a]pyrimidin-5-yl]-pyridin-4-ylmethyl-amine | 0.79 | 331.38 | 330.31 |
| 209 | 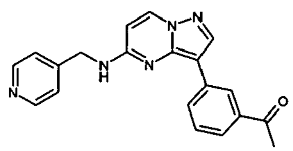 | 1-(3-{5-[(Pyridin-4-ylmethyl)-amino]-pyrazolo[1,5-a]pyrimidin-3-yl}-phenyl)-ethanone | 0.76 | 343.39 | 342.3 |
| 210 | 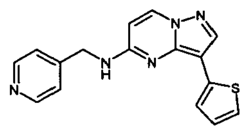 | Pyridin-4-ylmethyl- (3-thiophen-2-yl-pyrazolo[1,5-a]pyrimidin-5-yl)-amine | 0.76 | 307.38 | 306.24 |
| 211 | 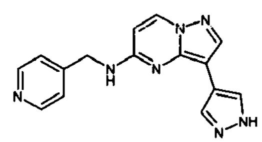 | [3-(1H-Pyrazol-4-yl)-pyrazolo[1,5-a]pyrimidin-5-yl]-pyridin-4-ylmethyl-amine | 0.53 | 291.32 | 291.17 |

(continued)

| Example No. | Structure | Name | RT | MW | Observed Mass Peak [M-H] |
|---|---|---|---|---|---|
| 212 | 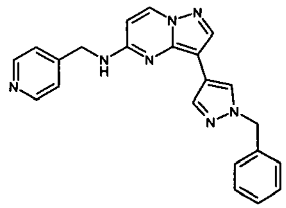 | [3-(1-Benzyl-1H-pyrazol-4-yl)-pyrazolo[1,5-a]pyrimidin-5-yl]-pyridin-4-ylmethyl-amine | 0.78 | 381.44 | 380.34 |
| 213 | 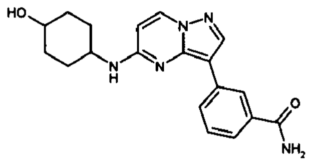 | 3-[5-(4-Hydroxy-cyclohexylamino)-pyrazolo[1,5-a]pyrimidin-3-yl]-benzamide | 0.8 | 351.41 | 350.31 |
| 214 | 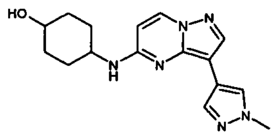 | 4-[3-(1-Methyl-1H-pyrazol-4-yl)-pyrazolo[1,5-a]pyrimidin-5-ylamino]-cyclohexanol | 0.76 | 312.38 | 311.48 |
| 215 | 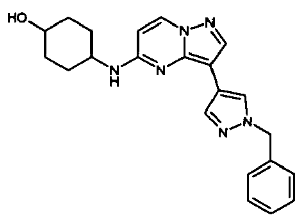 | 4-[3-(1-Benzyl-1H-pyrazol-4-yl)-pyrazolo[1,5-a]pyrimidin-5-ylamino]-cyclohexanol | 0.98 | 388.47 | 387.27 |
| 216 | 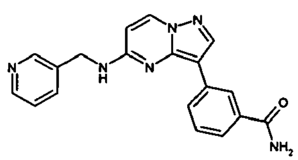 | 3-{5-[(Pyridin-3-ylmethyl)-amino]-pyrazolo[1,5-a]pyrimidin-3-yl}-benzamide | 0.63 | 344.38 | 343.05 |

(continued)

| Example No. | Structure | Name | RT | MW | Observed Mass Peak [M-H] |
|---|---|---|---|---|---|
| 217 | 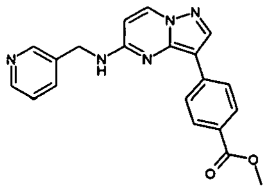 | 4-{5-[(Pyridin-3-ylmethyl)-amino]-pyrazolo[1,5-a]pyrimidin-3-yl}-benzoic acid methyl ester | 0.85 | 359.39 | 358.27 |
| 218 | 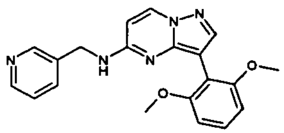 | [3-(2,6-Dimethoxy-phenyl)-pyrazolo[1,5-a]pyrimidin-5-yl]-pyridin-3-ylmethyl-amine | 0.77 | 361.4 | 360.36 |
| 219 | 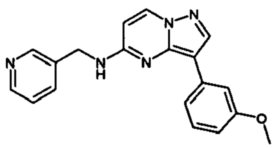 | [3-(3-Methoxy-phenyl)-pyrazolo[1,5-a]pyrimidin-5-yl]-pyridin-3-ylmethyl-amine | 0.83 | 331.38 | 330.33 |
| 220 | 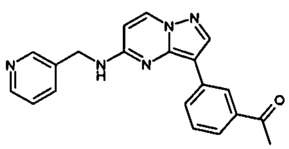 | 1-(3-{5-[(Pyridin-3-ylmethyl)-amino]-pyrazolo[1,5-a]pyrimidin-3-yl}-phenyl)-ethanone | 0.8 | 343.39 | 342.3 |
| 221 | 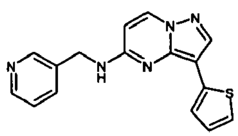 | Pyridin-3-ylmethyl-(3-thiophen-2-yl-pyrazolo[1,5-a]pyrimidin-5-yl)-amine | 0.79 | 307.38 | 307.13 |

(continued)

| Example No. | Structure | Name | RT | MW | Observed Mass Peak [M-H] |
|---|---|---|---|---|---|
| 222 | 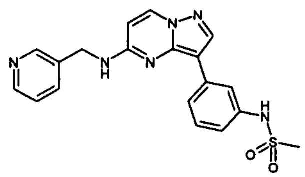 | N-(3-{5-[(Pyridin-3-ylmethyl)-amino]-pyrazolo[1,5-a]pyrimidin-3-yl}-phenyl)-methanesulfonamide | 0.7 | 394.46 | 393.28 |
| 223 | 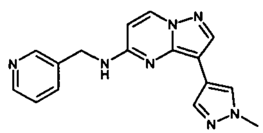 | [3-(1-Methyl-1H-pyrazol-4-yl)-pyrazolo[1,5-a]pyrimidin-5-yl]-pyridin-3-ylmethyl-amine | 0.6 | 305.34 | 304.24 |
| 224 | 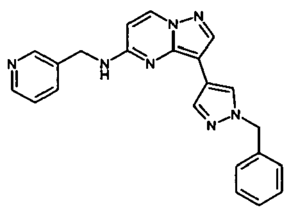 | [3-(1-Benzyl-1H-pyrazol-4-yl)-pyrazolo[1,5-a]pyrimidin-5-yl]-pyridin-3-ylmethyl-amine | 0.82 | 381.44 | 380.3 |
| 225 | 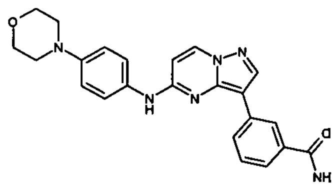 | 3-[5-(4-Morpholin-4-yl-phenylamino)-pyrazolo[1,5-a]pyrimidin-3-yl]-benzamide | 0.9 | 414.47 | 413.31 |
| 226 | 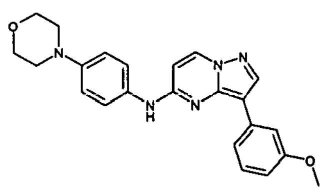 | [3-(3-Methoxy-phenyl)-pyrazolo[1,5-a]pyrimidin-5-yl]-(4-morpholin-4-yl-phenyl)-amine | 1.2 | 401.47 | 400.31 |

(continued)

| Example No. | Structure | Name | RT | MW | Observed Mass Peak [M-H] |
|---|---|---|---|---|---|
| 227 | 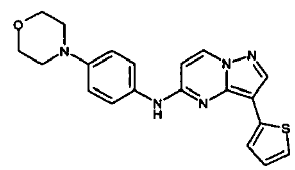 | (4-Morpholin-4-yl-phenyl)-(3-thiophen-2-yl-pyrazolo[1,5-a] pyrimidin-5-yl)-amine | 1.17 | 377.47 | 376.27 |
| 228 | 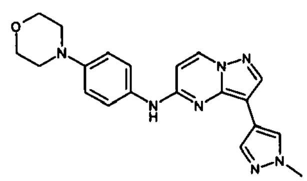 | [3-(1-Methyl-1H-pyrazol-4-yl)-pyrazolo[1,5-a]pyrimidin-5-yl]-(4-morpholin-4-yl-phenyl)-amine | 0.9 | 375.43 | 373.72 |
| 229 | 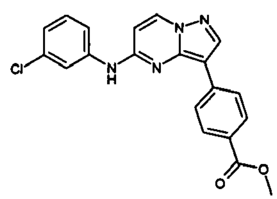 | 4-[5-(3-Chloro-phenylamino)-pyrazolo[1,5-a]pyrimidin-3-yl]-benzoic acid methyl ester | 1.38 | 378.82 | 377.2 |
| 230 | 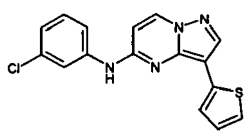 | (3-Chloro-phenyl)-(3-thiophen-2-yl-pyrazolo[1,5-a]pyrimidin-5-yl)-amine | 0.93 | 326.81 | 325.03 |
| 231 | 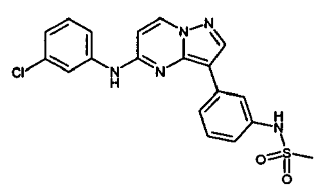 | N-{3-[5-(3-Chloro-phenylamino)-pyrazolo[1,5-a]pyrimidin-3-yl]-phenyl}-methanesulfonamide | 1.18 | 413.89 | 412.2 |

(continued)

| Example No. | Structure | Name | RT | MW | Observed Mass Peak [M-H] |
|---|---|---|---|---|---|
| 232 | 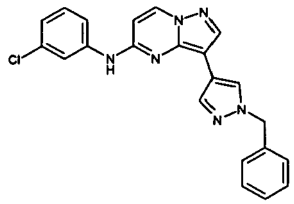 | [3-(1-Benzyt-1H-pyrazol-4-yl)-pyrazolo[1,5-a]pyrimidin-5-yl]-(3-chloro-phenyl)-amine | 1.31 | 400.87 | 399.29 |
| 233 | 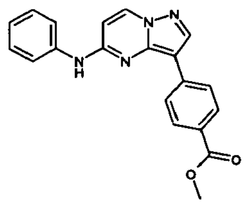 | 4-(5-Phenylamino-pyrazolo[1,5-a]pyrimidin-3-yl)-benzoic acid methyl ester | | 344.37 | |
| 234 | 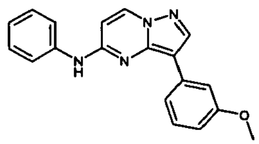 | [3-(3-Methoxy-phenyl)-pyrazolo[1,5-a]pyrimidin-5-yl]-phenyl-amine | 1.31 | 316.36 | 315.35 |
| 235 | 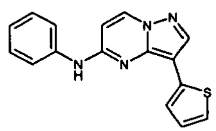 | Phenyl-(3-thiophen-2-yl-pyrazolo[1,5-a]pyrimidin-5-yl)-amine | 1.3 | 292.36 | 291.24 |
| 236 | 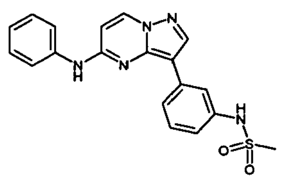 | N-[3-(5-Phenylamino-pyrazolo[1,5-a]pyrimidin-3-yl)-phenyl]-methanesulfonamide | 1.09 | 379.44 | 378.29 |

(continued)

| Example No. | Structure | Name | RT | MW | Observed Mass Peak [M-H] |
|---|---|---|---|---|---|
| 237 | 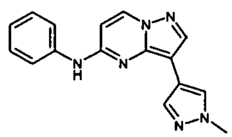 | [3-(1-Methyl-1H-pyrazol-4-yl)-pyrazolo[1,5-a]pyrimidin-5-yl]-phenyl-amine | 0.99 | 290.33 | 289.3 |
| 238 | 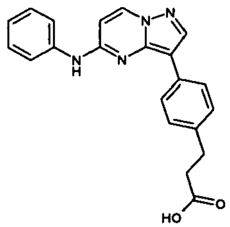 | 3-[4-(5-Phenylamino-pyrazolo[1,5-a]pyrimidin-3-yl)-phenyl]-propionic acid | 1.11 | 358.4 | 357.35 |
| 239 | 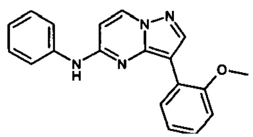 | [3-(2-Methoxy-phenyl)-pyrazolo[1,5-a]pyrimidin-5-yl]-phenyl-amine | 1.33 | 316.36 | 315.31 |
| 240 | 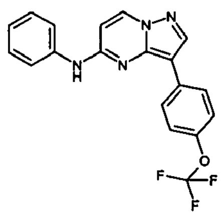 | Phenyl-[3-(4-trifluoromethoxy-phenyl)-pyrazolo[1,5-a]pyrimidin-5-yl]-amine | 1.5 | 370.33 | 369.38 |
| 241 | 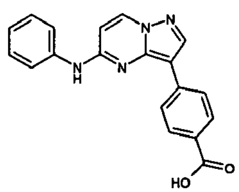 | 4-(5-Phenylamino-pyrazolo[1,5-a]pyrimidin-3-yl)-benzoic acid | 1.05 | 330.35 | 329.3 |

(continued)

| Example No. | Structure | Name | RT | MW | Observed Mass Peak [M-H] |
|---|---|---|---|---|---|
| 242 | 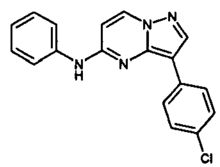 | [3-(4-Chloro-phenyl)-pyrazolo[1,5-a]pyrimidin-5-yl]-phenyl-amine | 1.47 | 320.78 | 319.23 |
| 243 | 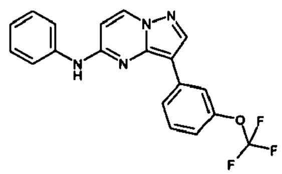 | Phenyl-[3-(3-trifluoromethoxy-phenyl)-pyrazolo[1, 5-a]pyrimidin-5-yl]-amine | 1.5 | 370.33 | 369.25 |
| 244 | 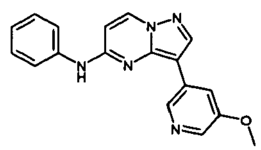 | [3-(5-Methoxy-pyridin-3-yl)-pyrazolo[1,5-a]pyrimidin-5-yl]-phenyl-amine | 0.88 | 317.35 | 316.27 |
| 245 | 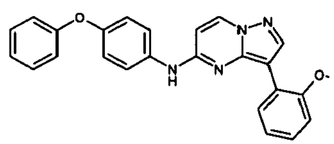 | [3-(2-Methoxy-phenyl)-pyrazolo[1,5-a]pyrimidin-5-yl]-(4-phenoxy-phenyl)-amine | 1.51 | 408.46 | 407.28 |
| 246 | 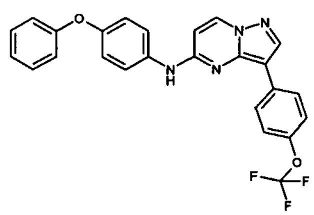 | (4-Phenoxy-phenyl)-[3-(4-trifluoromethoxy-phenyl)-pyrazolo[1,5-a]pyrimidin-5-yl]-amine | 1.64 | 462.43 | 461.2 |

(continued)

| Example No. | Structure | Name | RT | MW | Observed Mass Peak [M-H] |
|---|---|---|---|---|---|
| 247 | 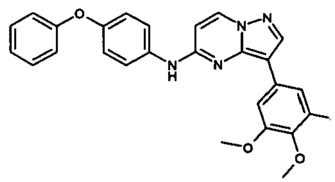 | (4-Phenoxy-phenyl)-[3-(3,4,5-trimethoxy-phenyl)-pyrazolo[1,5-a] pyrimidin-5-yl]-amine | 1.4 | 468.51 | 467.27 |
| 248 | 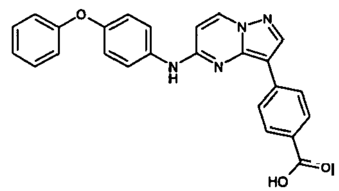 | 4-[5-(4-Phenoxy-phenylamino)-pyrazolo[1,5-a]pyrimidin-3-yl]-benzoic acid | 1.23 | 422.44 | 421.33 |
| 249 | 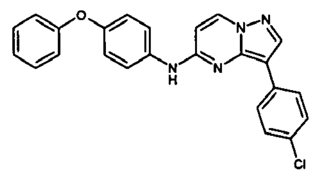 | [3-(4-Chloro-phenyl)-pyrazolo[1,5-a]pyrimidin-5-yl]-(4-phenoxy-phenyl)-amine | 1.62 | 412.88 | 411.22 |
| 250 | 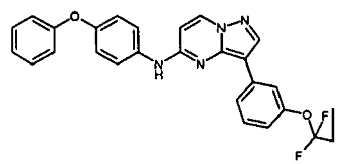 | (4-Phenoxy-phenyl)-[3-(3-trifluoromethoxy-phenyl)-pyrazolo[1,5-a] pyrimidin-5-yl]-amine | 1.63 | 462.43 | 461.23 |
| 251 | 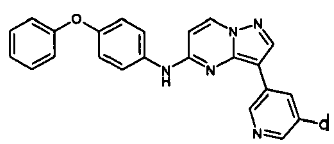 | [3-(5-Methoxy-pyridin-3-yl)-pyrazolo[1,5-a]pyrimidin-5-yl]-(4-phenoxy-phenyl)-amine | 1.12 | 409.45 | 408.28 |

(continued)

| Example No. | Structure | Name | RT | MW | Observed Mass Peak [M-H] |
|---|---|---|---|---|---|
| 252 | 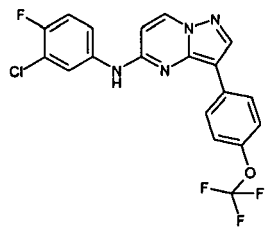 | (3-Chloro-4-fluoro-phenyl)-[3-(4-trifluoromethoxy-phenyl)-pyrazolo [1,5-a]pyrimidin-5-yl]-amine | 1.59 | 422.77 | 421.03 |
| 253 | 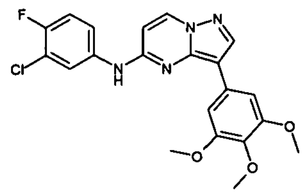 | (3-Chloro-4-fluoro-phenyl)-[3-(3,4,5-trimethoxy-phenyl)-pyrazolo [1,5-a]pyrimidin-5-yl]-amine | 1.3 | 428.85 | 427.23 |
| 254 | 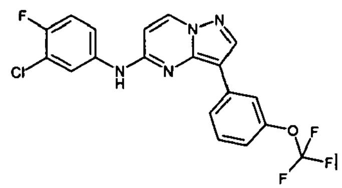 | (3-Chloro-4-fluoro-phenyl)-[3-(3-trifluoromethoxy-phenyl)-pyrazolo [1,5-a]pyrimidin-5-yl]-amine | 1.58 | 422.77 | 421.15 |
| 255 | 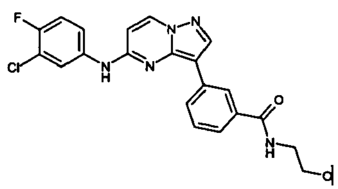 | 3-[5-(3-Chloro-4-fluoro-phenylamino)-pyrazolo[1,5-a]pyrimidin-3-yl]-N-(2-hydroxy-ethyl)-benzamide | 1.07 | 425.85 | 424.28 |

(continued)

| Example No. | Structure | Name | RT | MW | Observed Mass Peak [M-H] |
|---|---|---|---|---|---|
| 256 | 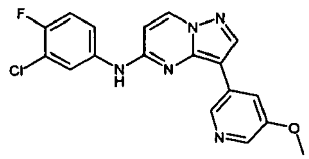 | (3-Chloro-4-fluoro-phenyl)-[3-(5-methoxy-pyridin-3-yl)-pyrazolo [1,5-a]pyrimidin-5-yl]-amine | 1.01 | 369.79 | 368.56 |
| 257 | 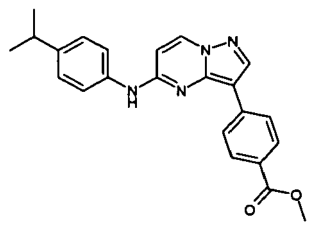 | 4-[5-(4-Isopropyl-phenylamino)-pyrazolo[1,5-a]pyrimidin-3-yl]-benzoic acid methyl ester | 1.48 | 386.45 | 385.37 |
| 258 | 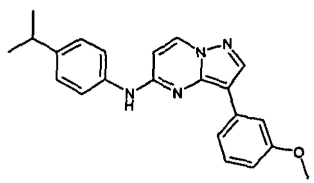 | (4-Isopropyl-phenyl)-[3-(3-methoxy-phenyl)-pyrazolo[1,5-a] pyrimidin-5-yl]-amine | 1.51 | 358.44 | 357.39 |
| 259 | 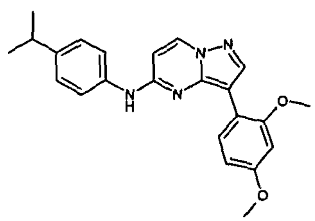 | [3-(2,4-Dimethoxy-phenyl)-pyrazolo[1,5-a]pyrimidin-5-yl]-(4-isopropyl-phenyl)-amine | 1.48 | 388.47 | 387.34 |

(continued)

| Example No. | Structure | Name | RT | MW | Observed Mass Peak [M-H] |
|---|---|---|---|---|---|
| 260 | 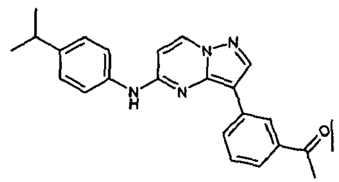 | 1-13-[5-(4-isopropyl-phenylamino)-pyrazolo[1,5-a]pyrimidin-3-yl]-phenyl}-ethanone | 1.44 | 370.45 | 369.34 |
| 261 | 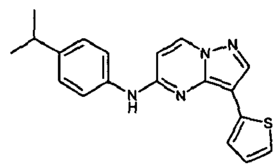 | (4-Isopropyl-phenyl)-(3-thiophen-2-yl-pyrazolo[1,5-a]pyrimidin-5-yl)-amine | 1.5 | 334.45 | 333.28 |
| 262 | 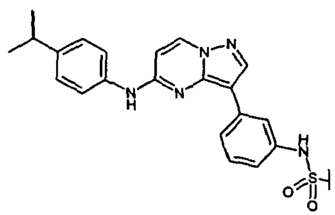 | N-{3-[5-(4-Isopropyl-phenylamino)-pyrazolo[1,5-a]pyrimidin-3-yl]-phenyl}-methanesulfonamide | 1.27 | 421.52 | 420.3 |
| 263 | 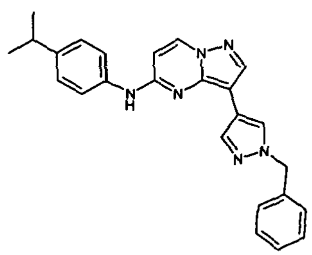 | [3-(1-Benzyl-1H-pyrazol-4-yl)-pyrazolo[1,5-a]pyrimidin-5-yl]-(4-isopropyl-phenyl)-amine | 1.4 | 408.51 | 407.35 |

(continued)

| Example No. | Structure | Name | RT | MW | Observed Mass Peak [M-H] |
|---|---|---|---|---|---|
| 264 | 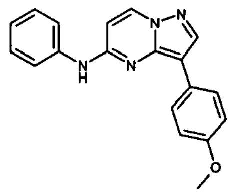 | [3-(4-Methoxyl-phenyl)-pyrazolo[1,5-a]pyrimidin-5-yl]-phenyl-amine | 1.29 | 316.36 | 315.31 |
| 265 | 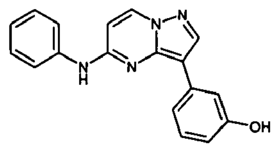 | 3-(5-Phenylamino-pyrazolo[1,5-a]pyrimidin-3-yl)-phenyl-phenol | 1.09 | 302.34 | 301.29 |
| 266 | 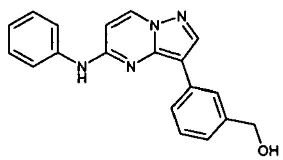 | [3-(5-Phenylamino-pyrazolo[1,5-a]pyrimidin-3-yl)-phenyl]-methanol | 1.08 | 316.36 | 315.25 |
| 267 | 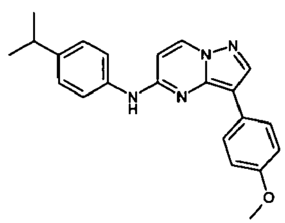 | (4-Isopropyl-phenyl)-[3-(4-methoxy-phenyl)-pyrazolo[1,5-a] pyrimidin-5-yl]-amine | 1.49 | 358.44 | 357.36 |
| 268 | 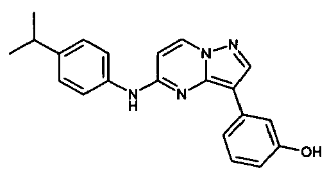 | 3-[5-(4-Isopropyl-phenylamino)-pyrazolo[1,5-a]pyrimidin-3-yl)-phenol | 1.28 | 344.42 | 343.32 |

(continued)

| Example No. | Structure | Name | RT | MW | Observed Mass Peak [M-H] |
|---|---|---|---|---|---|
| 269 | 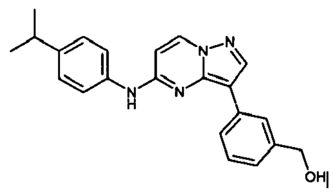 | {3-[5-(4-Isopropyl-phenylamino)-pyrazolo[1,5-a]pyrimidin-3-yl]-phenyl}-methanol | 1.28 | 358.44 | 357.4 |
| 270 | 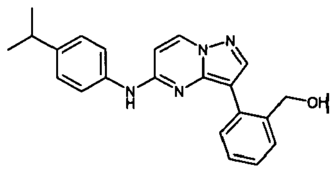 | {2-[5-(4-Isopropyl-phenylamino)-pyrazolo[1,5-a]pyrimidin-3-yl]-phenyl}-methanol | 1.32 | 358.44 | 357.37 |
| 271 | 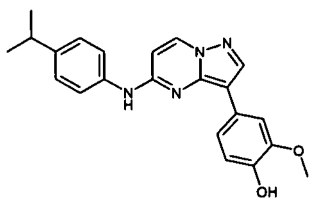 | 4-[5-(4-Isopropyl-phenylamino)-pyrazolo[1,5-a]pyrimidin-3-yl]-2-methoxy-phenol | 1.28 | 374.44 | 373.4 |
| | | | | | |
| 272 | 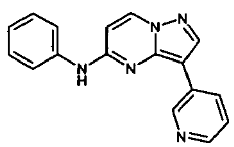 | Phenyl-(3-pyridin-3-yl-pyrazolo[1,5-a]pyrimidin-5-yl)-amine | 0.77 | 287.33 | 286.33 |
| 273 | 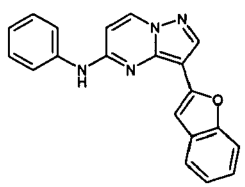 | (3-Benzofuran-2-yl-pyrazolo[1,5-a]pyrimidin-5-yl)-phenyl-amine | 1.45 | 326.36 | 325.28 |

(continued)

| Example No. | Structure | Name | RT | MW | Observed Mass Peak [M-H] |
|---|---|---|---|---|---|
| 274 | 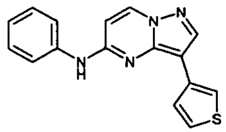 | Phenyl-(3-thiophen-3-yl-pyrazolo[1,5-a]pyrimidin-5-yl)-amine | 1.3 | 292.36 | 291.24 |
| 275 | 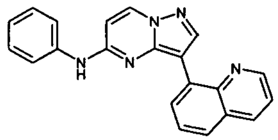 | Phenyl-(3-quinolin-8-yl-pyrazolo[1,5-a]pyrimidin-5-yl)-amine | 0.96 | 337.38 | 336.33 |
| 276 | 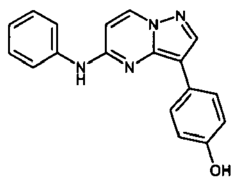 | 4-(5-Phenylamino-pyrazolo[1,5-a]pyrimidin-3-yl)-phenol | 1.04 | 302.34 | 301.32 |
| 277 | 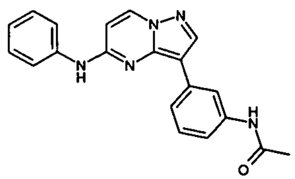 | N-[3-(5-Phenylamino-pyrazolo[1,5-a]pyrimidin-3-yl)-phenyl]-acetamide | 1.05 | 343.39 | 342.2 |
| 278 | 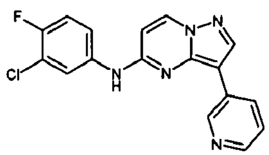 | (3-Chloro-4-fluoro-phenyl)-(3-pyridin-3-yl-pyrazolo[1,5-a]pyrimidin-5-yl)-amine | 0.91 | 339.76 | 339.06 |

(continued)

| Example No. | Structure | Name | RT | MW | Observed Mass Peak [M-H] |
|---|---|---|---|---|---|
| 279 | 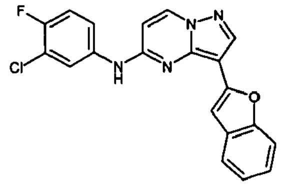 | (3-Benzofuran-2-yl-pyrazolo[1,5-a]pyrimidin-5-yl)-(3-chloro-4-fluoro-phenyl)-amine | 1.56 | 378.79 | 377.27 |
| 280 | 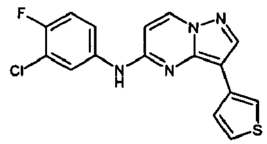 | (3-Chloro-4-fluoro-phenyl)-(3-thiophen-3-yl-pyrazolo[1,5-a] pyrimidin-5-yl)-amine | 1.42 | 344.8 | 343.17 |
| 281 | 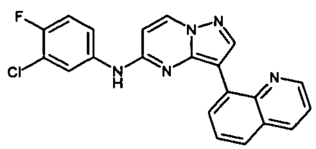 | (3-Chloro-4-fluoro-phenyl)-(3-quinolin-8-yl-pyrazolo[1,5-a] pyrimidin-5-yl)-amine | 1.23 | 389.82 | 388.23 |
| 282 | 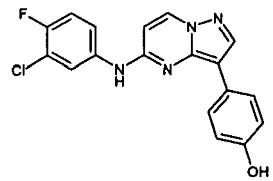 | 4-[5-(3-Chloro-4-fluoro-phenylamino)-pyrazolo[1,5-a]pyrimidin-3-yl]-phenol | 1.14 | 354.77 | 353.18 |
| 283 | 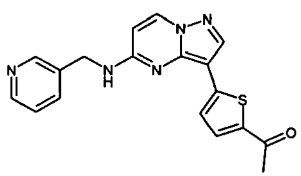 | 1-(5-{5-[(Pyridin-3-ylmethyl)-amino]-pyrazolo[1,5-a]pyrimidin-3-yl}-thiophen-2-yl)-ethanone | 0.76 | 349.42 | 348.22 |

(continued)

| Example No. | Structure | Name | RT | MW | Observed Mass Peak [M-H] |
|---|---|---|---|---|---|
| 284 | 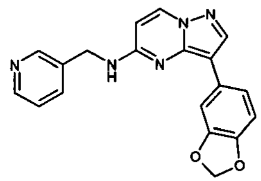 | (3-Benzo[1,3]dioxol-5-yl-pyrazolo[1,5-a]pyrimidin-5-yl)-pyridin-3-ylmethyl-amine | 0.8 | 345.36 | 344.45 |
| 285 | 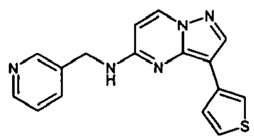 | Pyridin-3-ylmethyl-(3-thiophen-3-yl-pyrazoto[1,5-a]pyrimidin-5-yl)-amine | 0.8 | 307.38 | 306.28 |
| 286 | 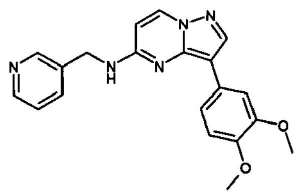 | [3-(3,4-Dimethoxy-phenyl)-pyrazolo[1,5-a]pyrimidin-5-yl]-pyridin-3-ylmethyl-amine | 0.78 | 361.4 | 360.29 |
| 287 | 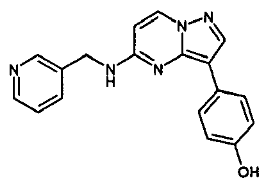 | 4-{5-[(Pyridin-3-ylmethyl)-amino]-pyrazolo[1,5-a]pyrimidin-3-yl}-phenol | 0.65 | 317.35 | 316.34 |
| 288 | 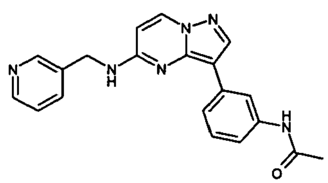 | N-(3-{5-[(Pyridin-3-ylmethyl)-amino]-pyrazolo[1,5-a]pyrimidin-3-yl}-phenyl)-acetamide | 0.68 | 358.4 | 357.36 |

(continued)

| Example No. | Structure | Name | RT | MW | Observed Mass Peak [M-H] |
|---|---|---|---|---|---|
| 289 | 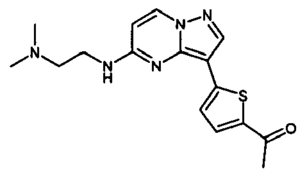 | 1-{5-[5-(2-Dimethylamino-ethylamino)-pyrazolo[1,5-a]pyrimidin-3-yl]-thiophen-2-yl}-ethanone | 0.7 | 329.43 | 328.27 |
| 290 | 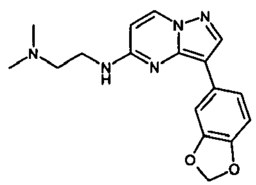 | N'-(3-Benzo[1,3]dioxol-5-yl-pyrazolo[1,5-a]pyrimidin-5-yl)-N,N-dimethyl-ethane-1,2-diamine | 0.73 | 325.37 | 325.28 |
| 291 | 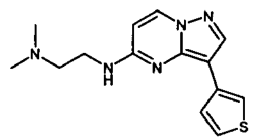 | N,N-Dimethyl-N'-(3-thiophen-3-yl-pyrazolo[1,5-a]pyrimidin-5-yl)-ethane-1,2-diamine | 0.71 | 287.39 | 286.25 |
| 292 | 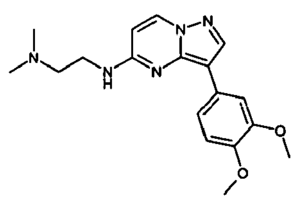 | N'-[3-(3,4-Dimethoxy-phenyl)-pyrazolo[1,5-a]pyrimidin-5-yl]-N,N-dimethyl-ethane-1,2-diamine | 0.71 | 341.41 | 340.43 |

(continued)

| Example No. | Structure | Name | RT | MW | Observed Mass Peak [M-H] |
|---|---|---|---|---|---|
| 293 | 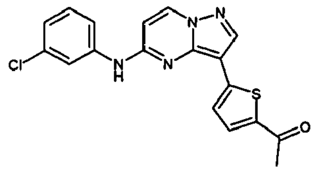 | 1-{5-[5-(3-Chloro-phenylamino)-pyrazolo[1,5-a]pyrimidin-3-yl]-thiophen-2-yl}-ethanone | 1.27 | 368.85 | 367.25 |
| 294 | 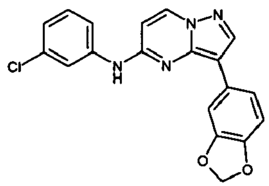 | (3-Benzo[1,3]dioxol-5-yl-pyrazolo[1,5-a]pyrimidin-5-yl)-(3-chloro-phenyl)-amine | 1.37 | 364.79 | 363.22 |
| 295 | 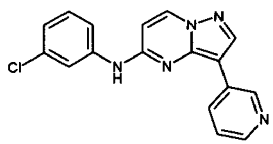 | (3-Chloro-phenyl)-(3-pyridin-3-yl-pyrazolo[1,5-a]pyrimidin-5-yl)-amine | 0.89 | 321.77 | 321.38 |
| 296 | 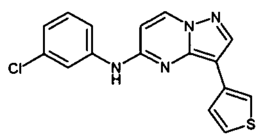 | (3-Chloro-phenyl)-(3-thiophen-3-yl-pyrazolo[1,5-a]pyrimidin-5-yl)-amine | 1.41 | 326.81 | 325.22 |
| 297 | 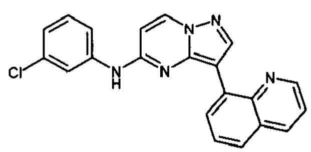 | (3-Chloro-phenyl)-(3-quinolin-8-yl-pyrazolo[1,5-a]pyrimidin-5-yl)-amine | 1.19 | 371.83 | 370.22 |

(continued)

| Example No. | Structure | Name | RT | MW | Observed Mass Peak [M-H] |
|---|---|---|---|---|---|
| 298 | 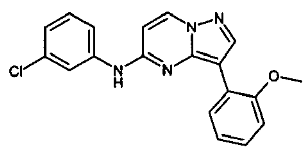 | (3-Chloro-phenyl)-[3-(2-methoxy-phenyl)-pyrazolo[1,5-a]pyrimidin-5-yl]-amine | 1.43 | 350.81 | 349.24 |
| 299 | 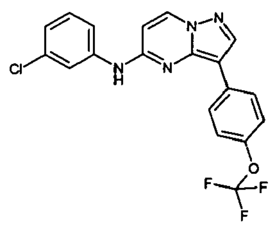 | (3-Chloro-phenyl)-[3-(4-trifluoromethoxy-phenyl)-pyrazolo[1,5-a] pyrimidin-5-yl]-amine | 1.59 | 404.78 | 403.14 |
| 300 | 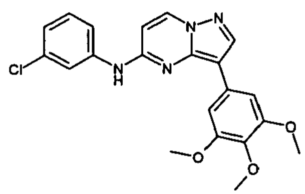 | (3-Chloro-phenyl)-[3-(3,4,5-trimethoxy-phenyl)-pyrazolo[1,5-a] pyrimidin-5-yl]-amine | 1.29 | 410.86 | 409.24 |
| 301 | 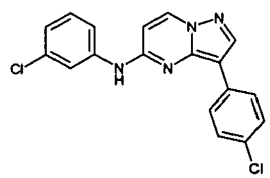 | (3-Chloro-phenyl)-[3-(4-chloro-phenyl)-pyrazolo[1,5-a]pyrimidin-5-yl]-amine | 1.56 | 355.23 | 355.2 |
| 302 | 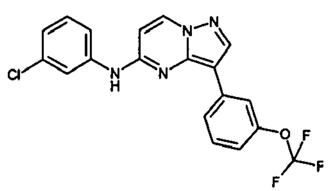 | (3-Chloro-phenyl)-[3-(3-trifluoromethoxy-phenyl)-pyrazolo[1,5-a] pyrimidin-5-yl]-amine | 1.58 | 404.78 | 403.19 |

(continued)

| Example No. | Structure | Name | RT | MW | Observed Mass Peak [M-H] |
|---|---|---|---|---|---|
| 303 | 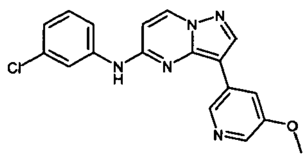 | (3-Chloro-phenyl)-[3-(5-methoxy-pyridin-3-yl)-pyrazolo[1,5-a] pyrimidin-5-yl]-amine | 0.98 | 351.8 | 351.1 |
| 304 | 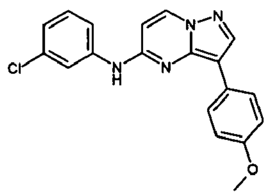 | (3-Chloro-phenyl)-[3-(4-methoxy-phenyl)-pyrazolo[1,5-a]pyrimidin-5-yl]-amine | | 350.81 | |
| 305 | 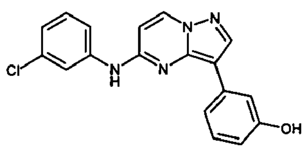 | 3-[5-(3-Chloro-phenylamino)-pyrazolo[1,5-a]pyrimidin-3-yl]-phenol | 1.18 | 336.78 | 335.31 |
| 306 | 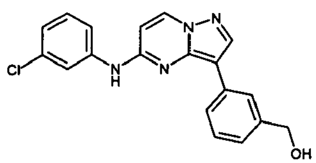 | {3-[5-(3-Chloro-phenylamino)-pyrazolo[1,5-a]pyrimidin-3-yl]-phenyl}-methanol | 1.17 | 350.81 | 349.28 |
| 307 | 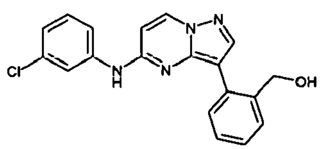 | {2-[5-(3-Chloro-phenylamino)-pyrazolo[1,5-a]pyrimidin-3-yl]-phenyl}-methanol | 1.2 | 350.81 | 349.24 |

(continued)

| Example No. | Structure | Name | RT | MW | Observed Mass Peak [M-H] |
|---|---|---|---|---|---|
| 308 | 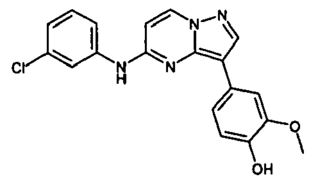 | 4-[5-(3-Chloro-phenylamino)-pyrazolo[1,5-a]pyrimidin-3-yl]-2-methoxy-phenol | 1.16 | 366.81 | 365.16 |
| 309 | 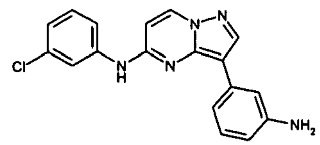 | [3-(3-Amino-phenyl)-pyrazolo[1,5-a]pyrimidin-5-yl]-(3-chloro-phenyl)-amine | 1.06 | 335.8 | 334.15 |
| 310 | 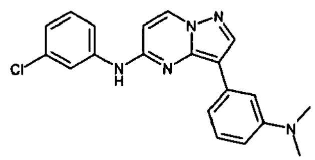 | (3-Chloro-phenyl)-[3-(3-dimethylamino-phenyl)-pyrazolo[1,5-a] pyrimidin-5-yl]-amine | 1.24 | 363.85 | 362.28 |
| 311 | 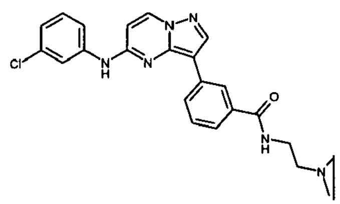 | 3-[5-(3-Chloro-phenylamino)-pyrazolo[1,5-a]pyrimidin-3-yl]-N-(2-dimethylamino-ethyl)-benzamide | 0.9 | 434.93 | 433.17 |

(continued)

| Example No. | Structure | Name | RT | MW | Observed Mass Peak [M-H] |
|---|---|---|---|---|---|
| 312 | 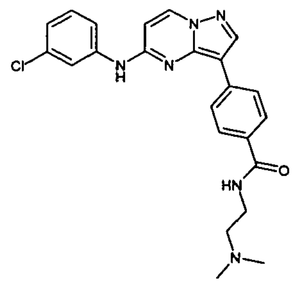 | 4-[5-(3-Chloro-phenylamino)-pyrazolo[1,5-a]pyrimidin-3-yl]-N-(2-dimethylamino-ethyl)-benzamide | 0.85 | 434.93 | 433.27 |
| 313 | 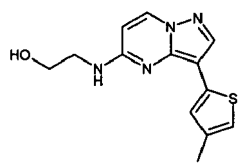 | 2-[3-(4-Methyl-thiophen-2-yl)-pyrazolo[1,5-a]pyrimidin-5-ylamino]-ethanol | 1.01 | 274.35 | 272.51 |
| 314 | 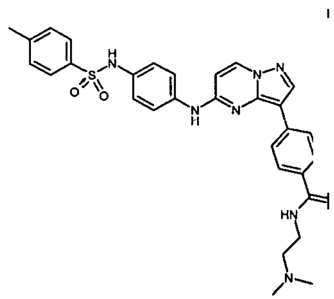 | N-(2-Dimethylamino-ethyl)-4-{5-[4-(toluene-4-sulfonylamino)-phenylamino]-pyrazolo[1,5-a]pyrimidin-3-yl}-benzamide | | 569.69 | |

(continued)

| Example No. | Structure | Name | RT | MW | Observed Mass Peak [M-H] |
|---|---|---|---|---|---|
| 315 | 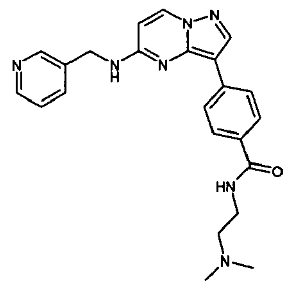 | N-(2-Dimethylamino-ethyl)-4-{5-[(pyridin-3-ylmethyl)-amino]-pyrazolo[1,5-a]pyrimidin-3-yl}-benzamide | 0.57 | 415.5 | 414.36 |
| 316 | 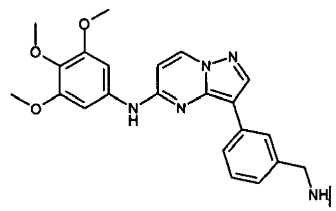 | [3-(3-Aminomethyl-phenyl)-pyrazolo[1,5-a]pyrimidin-5-yl]-(3,4,5-trimethoxy-phenyl)-amine | 0.8 | 405.46 | 404.28 |
| 317 | 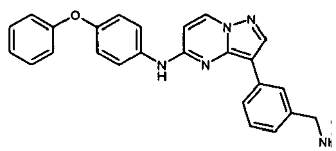 | [3-(3-Aminomethyl-phenyl)-pyrazolo[1,5-a]pyrimidin-5-yl]-(4-phenoxy-phenyl)-amine | 0.98 | 407.48 | 406.34 |
| 318 | 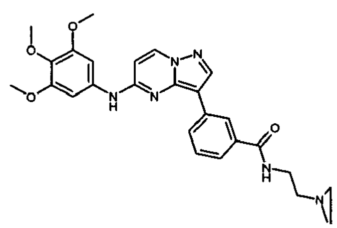 | N-(2-Dimethylamino-ethyl)-3-[5-(3,4,5-trimethoxy-phenylamino)-pyrazolo[1,5-a]pyrimidin-3-yl]-benzamide | 0.81 | 489.59 | 489.33 |

(continued)

| Example No. | Structure | Name | RT | MW | Observed Mass Peak [M-H] |
|---|---|---|---|---|---|
| 319 | 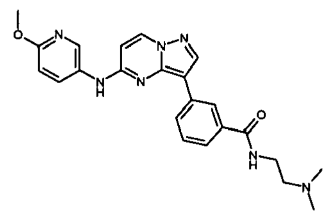 | N-(2-Dimethylamino-ethyl)-3-[5-(6-methoxy-pyridin-3-ylamino)-pyrazolo[1,5-a]pyrimidin-3-yl]-benzamide | 0.76 | 431.5 | 430.33 |
| 320 | 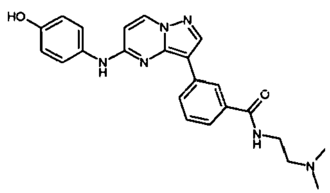 | N-(2-Dimethylamino-ethyl)-3-[5-(4-hydroxy-phenylamino)-pyrazolo [1,5-a]pyrimidin-3-yl]-benzamide | 0.72 | 416.48 | 415.21 |
| 321 | 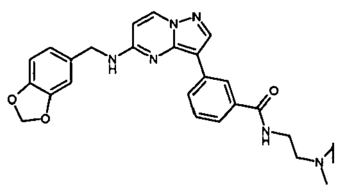 | 3-{5-[(Benzo[1,3]dioxol-5-ylmethyl)-amino]-pyrazolo[1,5-a] pyrimidin-3-yl}-N-(2-dimethylamino-ethyl)-benzamide | 0.82 | 458.52 | 457.32 |
| 322 | 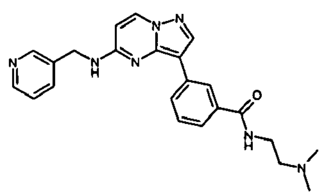 | N-(2-Dimethylamino-ethyl)-3-{5-[(pyridin-3-ylmethyl)-amino]-pyrazolo[1,5-a]pyrimidin-3-yl}-benzamide | 0.57 | 415.5 | 414.39 |

(continued)

| Example No. | Structure | Name | RT | MW | Observed Mass Peak [M-H] |
|---|---|---|---|---|---|
| 323 | 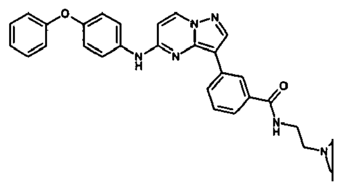 | N-(2-Dimethylamino-ethyl)-3-[5-(4-phenoxy-phenylamino)-pyrazolo [1, 5-a]pyrimidin-3-yl]-benzamide | 0.99 | 492.58 | 490.97 |
| 324 | 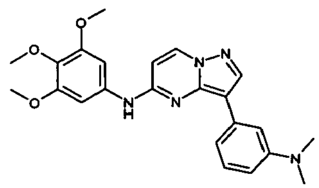 | [3-(3-Dimethylamino-phenyl)-pyrazolo[1,5-a]pyrimidin-5-yl]-(3,4,5-trimethoxy-phenyl)-amine | 1.06 | 419.48 | 419.17 |
| 325 | 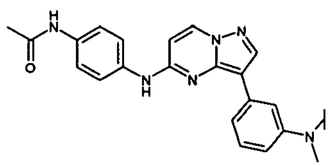 | N-{4-[3-(3-Dimethylamino-phenyl)-pyrazolo[1,5-a]pyrimidin-5-ylamino]-phenyl}-acetamide | | 386.46 | |
| 326 | 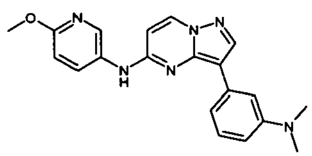 | [3-(3-Dimethylamino-phenyl)-pyrazolo[1,5-a]pyrimidin-5-yl]-(6-methoxy-pyridin-3-yl)-amine | 0.98 | 360.42 | 359.33 |

(continued)

| Example No. | Structure | Name | RT | MW | Observed Mass Peak [M-H] |
|---|---|---|---|---|---|
| 327 | 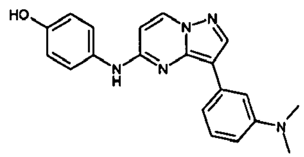 | 4-[3-(3-Dimethylamino-phenyl)-pyrazolo[1,5-a]pyrimidin-5-ylamino]-phenol | 0.83 | 345.4 | 344.32 |
| 328 | 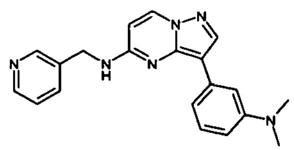 | [3-(3-Dimethylamino-phenyl)-pyrazolo[1,5-a]pyrimidin-5-yl]-pyridin-3-ylmethyl-amine | 0.68 | 344.42 | 344 |
| 329 | 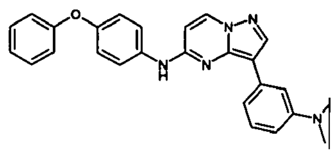 | [3-(3-Dimethylamino-phenyl)-pyrazolo[1,5-a]pyrimidin-5-yl]-(4-phenoxy-phenyl)-amine | 1.38 | 421.5 | 420.29 |
| 330 | 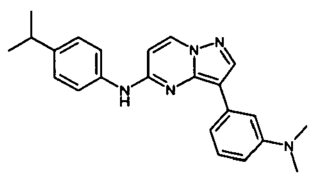 | [3-(3-Dimethylamino-phenyl)-pyrazolo[1,5-a]pyrimidin-5-yl]-(4-isopropyl-phenyl)-amine | 1.37 | 371.49 | 370.34 |
| 331 | 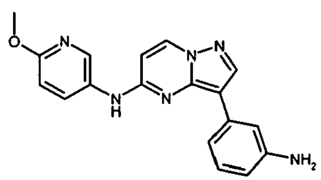 | [3-(3-Amino-phenyl)-pyrazolo[1,5-a]pyrimidin-5-yl]-(6-methoxy-pyridin-3-yl)-amine | 0.83 | 332.36 | 331.29 |

(continued)

| Example No. | Structure | Name | RT | MW | Observed Mass Peak [M-H] |
|---|---|---|---|---|---|
| 332 | 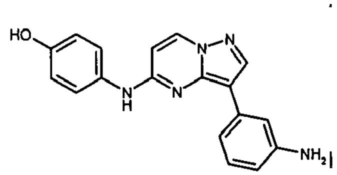 | 4-[3-(3-Amino-phenyl)-pyrazolo[1,5-a]pyrimidin-5-ylamino]-phenol | 0.74 | 317.35 | 316.32 |
| 333 | 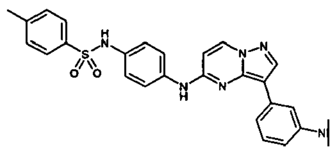 | N-{4-[3-(3-Amino-phenyl)-pyrazolo[1,5-a]pyrimidin-5-ylamino]-phenyl}-4-methyl-benzenesulfonamide | | 470.55 | |
| 334 | 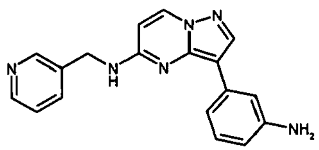 | [3-(3-Amino-phenyl)-pyrazolo[1,5-a]pyrimidin-5-yl]-pyridin-3-ylmethyl-amine | 0.57 | 316.37 | 315.31 |
| 335 | 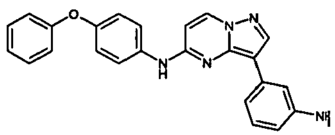 | [3-(3-Amino-phenyl)-pyrazolo[1,5-a]pyrimidin-5-yl]-(4-phenoxy-phenyl)-amine | 1.19 | 393.45 | 392.29 |
| 336 | 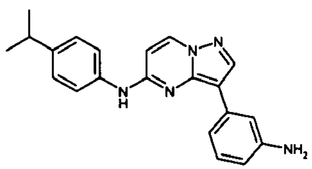 | [3-(3-Amino-phenyl)-pyrazolo[1,5-a]pyrimidin-5-yl]-(4-isopropyl-phenyl)-amine | 1.18 | 343.43 | 342.37 |

(continued)

| Example No. | Structure | Name | RT | MW | Observed Mass Peak [M-H] |
|---|---|---|---|---|---|
| 337 | 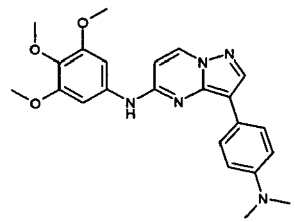 | [3-(4-Dimethylamino-phenyl)-pyrazolo[1,5-a]pyrimidin-5-yl]-(3,4,5-trimethoxy-phenyl)-amine | 1 | 419.48 | 419.44 |
| 338 | 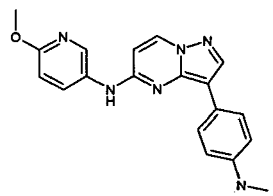 | [3-(4-Dimethylamino-phenyl)-pyrazolo[1,5-a]pyrimidin-5-yl]-(6-methoxy-pyridin-3-yl)-amine | 0.9 | 360.42 | 359.3 |
| 339 | 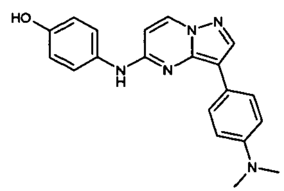 | 4-[3-(4-Dimethylamino-phenyl)-pyrazolo[1,5-a]pyrimidin-5-ylamino]-phenol | 0.79 | 345.4 | 344.28 |
| 340 | 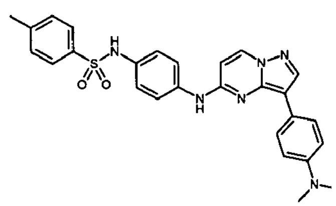 | N-{4-[3-(4-Dimethylamino-phenyl)-pyrazolo[1,5-a]pyrimidin-5-ylamino]-phenyl}-4-methyl-benzenesulfonamide | 1.09 | 498.61 | 497.29 |

(continued)

| Example No. | Structure | Name | RT | MW | Observed Mass Peak [M-H] |
|---|---|---|---|---|---|
| 341 | 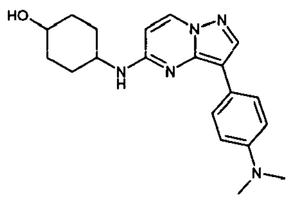 | 4-[3-(4-Dimethylamino-phenyl)-pyrazolo[1,5-a]pyrimidin-5-ylamino]-cyclohexanol | 0.73 | 351.45 | 350.45 |
| 342 | 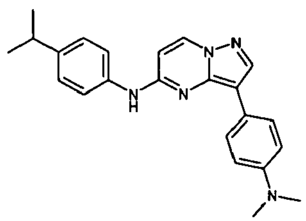 | [3-(4-Dimethylamino-phenyl)-pyrazolo[1,5-a]pyrimidin-5-yl]-(4-isopropyl-phenyl)-amine | 1.3 | 371.49 | 370.39 |
| 343 | 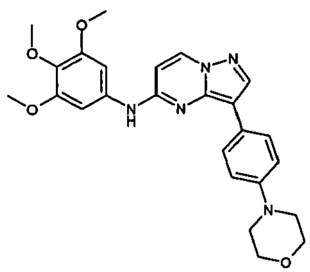 | [3-(4-Morpholin-4-yl-phenyl)-pyrazolo[1,5-a]pyrimidin-5-yl]-(3,4,5-trimethoxy-phenyl)-amine | 1.14 | 461.52 | 460.33 |
| 344 | 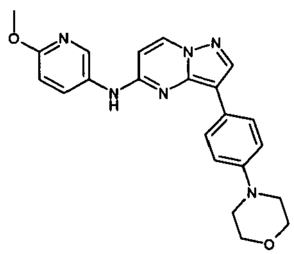 | (6-Methoxy-pyridin-3-yl)-[3-(4-morpholin-4-yl-phenyl)-pyrazolo[1,5-a]pyrimidin-5-yl]-amine | 1.09 | 402.46 | 401.35 |

(continued)

| Example No. | Structure | Name | RT | MW | Observed Mass Peak [M-H] |
|---|---|---|---|---|---|
| 345 | 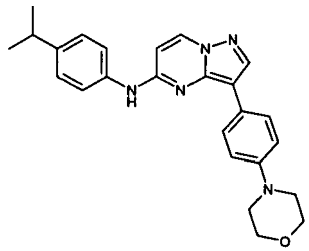 | (4-Isopropyl-phenyl)-[3-(4-morpholin-4-yl-phenyl)-pyrazolo[1,5-a] pyrimidin-5-yl]-amine | 1.4 | 413.52 | 412.33 |
| 346 | 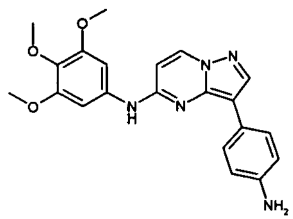 | [3-(4-Amino-phenyl)-pyrazolo[1,5-a]pyrimidin-5-yl]-(3,4,5-trimethoxy-phenyl)-amine | 0.83 | 391.43 | 390.3 |
| 347 | 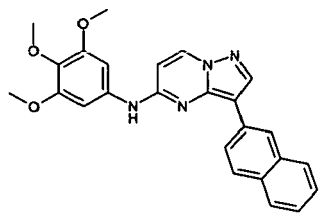 | (3-Naphthalen-2-yl-pyrazolo[1,5-a]pyrimidin-5-yl)-(3,4,5-trimethoxy-phenyl)-amine | 1.38 | 426.47 | 425.24 |
| 348 | 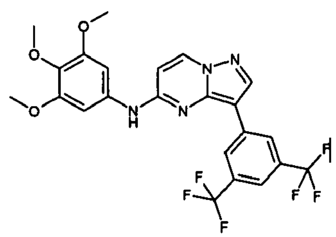 | [3-(3,5-Bis-trifluoromethyl-phenyl)-pyrazolo[1,5-a]pyrimidin-5-yl]-(3,4,5-trimethoxy-phenyl)-amine | 1.48 | 512.41 | 511.18 |

(continued)

| Example No. | Structure | Name | RT | MW | Observed Mass Peak [M-H] |
|---|---|---|---|---|---|
| 349 | 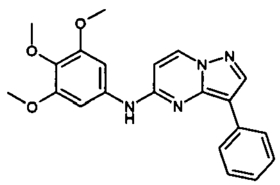 | (3-Phenyl-pyrazolo[1,5-a]pyrimidin-5-yl)-(3,4,5-trimethoxy-phenyl)-amine | 1.25 | 376.41 | 375.32 |
| 350 | 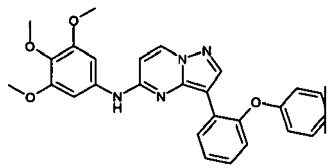 | 3-(2-Phenoxy-phenyl)-pyrazolo[1,5-a]pyrimidin-5-yl]-(3,4,5-trimethoxy-phenyl)-amine | 1.43 | 468.51 | 467.19 |
| 351 | 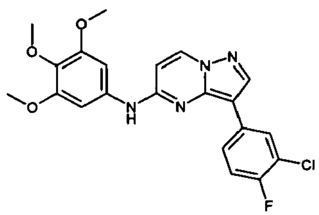 | [3-(3-Chloro-4-fluoro-phenyl)-pyrazolo[1,5-a]pyrimidin-5-yl]-(3,4,5-trimethoxy-phenyl)-amine | 1.36 | 428.85 | 427.23 |
| 352 | 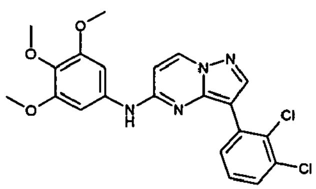 | [3-(2,3-Dichloro-phenyl)-pyrazolo[1,5-a]pyrimidin-5-yl]-(3,4,5-trimethoxy-phenyl)-amine | 1.38 | 445.3 | 445.13 |

(continued)

| Example No. | Structure | Name | RT | MW | Observed Mass Peak [M-H] |
|---|---|---|---|---|---|
| 353 | 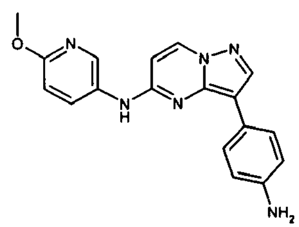 | [3-(4-Amino-phenyl)-pyrazolo[1,5-a]pyrimidin-5-yl]-(6-methoxy-pyridin-3-yl)-amine | 0.75 | 332.36 | 330.84 |
| 354 | 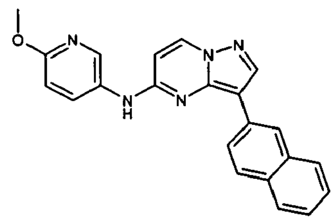 | (6-Methoxy-pyridin-3-yl)-(3-naphthalen-2-yl-pyrazolo[1,5-a] pyrimidin-5-yl)-amine | 1.38 | 367.41 | 366.25 |
| 355 | 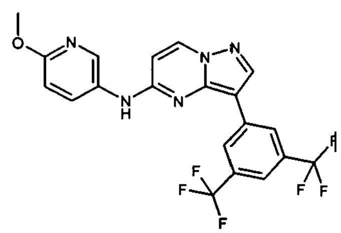 | [3-(3,5-Bis-trifluoromethyl-phenyl)-pyrazolo[1,5-a]pyrimidin-5-yl]-(6-methoxy-pyridin-3-yl)-amine | | 453.34 | |
| 356 | 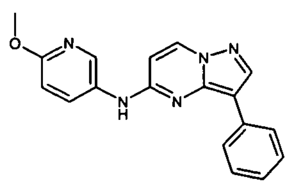 | (6-Methoxy-pyridin-3-yl)-(3-phenyl-pyrazolo[1,5-a]pyrimidin-5-yl)-amine | 1.22 | 317.35 | 316.32 |

(continued)

| Example No. | Structure | Name | RT | MW | Observed Mass Peak [M-H] |
|---|---|---|---|---|---|
| 357 | 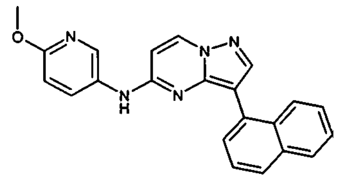 | (6-Methoxy-pyridin-3-yl)-(3-naphthalen-1-yl-pyrazolo[1,5-a] pyrimidin-5-yl)-amine | 1.31 | 367.41 | 366.31 |
| 358 | 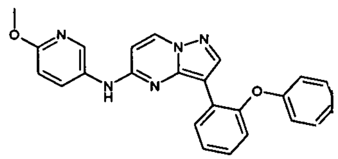 | (6-Methoxy-pyridin-3-yl)-[3-(2-phenoxy-phenyl)-pyrazolo[1,5-a] pyrimidin-5-yl]-amine | 1.43 | 409.45 | 408.33 |
| 359 | 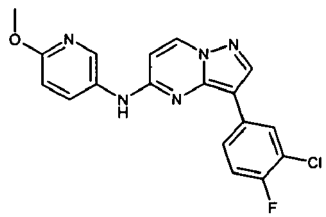 | [3-(3-Chloro-4-fluoro-phenyl)-pyrazolo[1,5-a]pyrimidin-5-yl]-(6-methoxy-pyridin-3-yl)-amine | 1.36 | 369.79 | 368.29 |
| 360 | 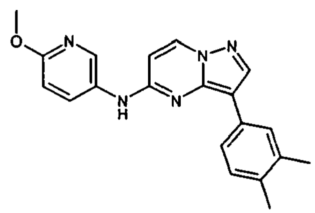 | [3-(3,4-Dimethyl-phenyl)-pyrazolo[1,5-a]pyrimidin-5-yl]-(6-methoxy-pyridin-3-yl)-amine | 1.37 | 345.4 | 344.33 |
| 361 | 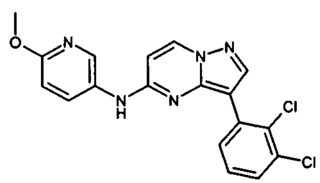 | [3-(2,3-Dichloro-phenyl)-pyrazolo[1,5-a]pyrimidin-5-yl]-(6-methoxy-pyridin-3-yl)-amine | 1.38 | 386.24 | 386.21 |

(continued)

| Example No. | Structure | Name | RT | MW | Observed Mass Peak [M-H] |
|---|---|---|---|---|---|
| 362 | 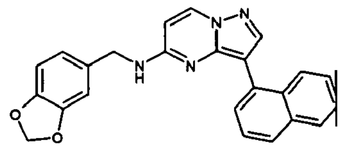 | Benzo[1,3]dioxol-5-ylmethyl-(3-naphthalen-1-yl-pyrazolo[1,5-a] pyrimidin-5-yl)-amine | 1.39 | 394.43 | 393.04 |
| 363 | 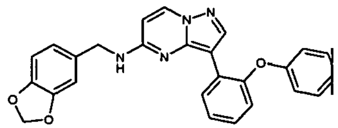 | Benzo[1,3]dioxol-5-ylmethyl-[3-(2-phenoxy-phenyl)-pyrazolo[1,5-a] pyrimidin-5-yl]-amine | 1.48 | 436.47 | 435.26 |
| 364 | 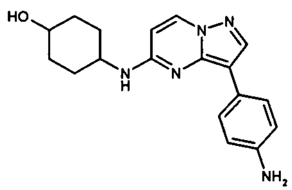 | 4-[3-(4-Amino-phenyl)-pyrazolo[1,5-a]pyrimidin-5-ylamino]-cyclohexanol | 0.65 | 323.4 | 322.32 |
| 365 | 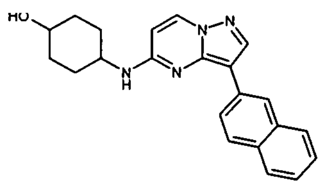 | 4-(3-Naphthalen-2-yl-pyrazolo[1,5-a]pyrimidin-5-ylamino)-cyclohexanol | 1.17 | 358.44 | 357.35 |
| 366 | 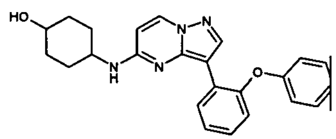 | 4-[3-(2-Phenoxy-phenyl)-pyrazolo[1,5-a]pyrimidin-5-ylamino]-cyclohexanol | 1.24 | 400.48 | 399.32 |

(continued)

| Example No. | Structure | Name | RT | MW | Observed Mass Peak [M-H] |
|---|---|---|---|---|---|
| 367 | 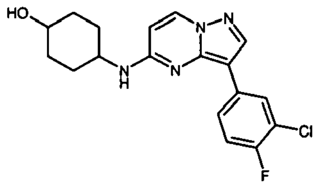 | 4-[3-(3-Chloro-4-fluoro-phenyl)-pyrazolo[1,5-a]pyrimidin-5-ylamino]-cyclohexanol | 1.16 | 360.82 | 359.36 |
| 368 | 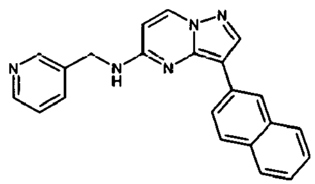 | (3-Naphthalen-2-yl-pyrazolo[1,5-a]pyrimidin-5-yl)-pyridin-3-ylmethyl-amine | 1 | 351.41 | 350.54 |
| 369 | 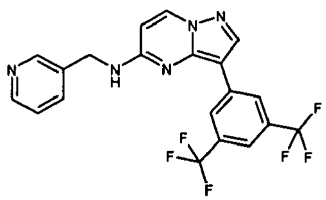 | [3-(3,5-Bis-trifluoromethyl-phenyl)-pyrazolo[1,5-a]pyrimidin-5-yl]-pyridin-3-ylmethyl-amine | 1.19 | 437.35 | 436.24 |
| 370 | 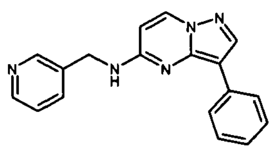 | (3-Phenyl-pyrazolo[1,5-a]pyrimidin-5-yl)-pyridin-3-ylmethyl-amine | 0.82 | 301.35 | 300.36 |
| 371 | 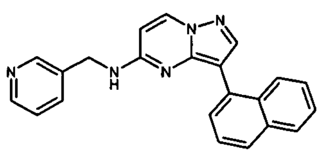 | (3-Naphthalen-1-yl-pyrazolo[1,5-a]pyrimidin-5-yl)-pyridin-3-ylmethyl-amine | 0.94 | 351.41 | 350.33 |

(continued)

| Example No. | Structure | Name | RT | MW | Observed Mass Peak [M-H] |
|---|---|---|---|---|---|
| 372 | 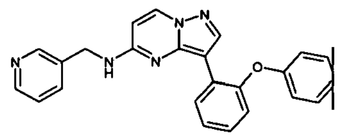 | [3-(2-Phenoxy-phenyl)-pyrazolo[1,5-a]pyrimidin-5-yl]-pyridin-3-ylmethyl-amine | 1.06 | 393.45 | 393.29 |
| 373 | 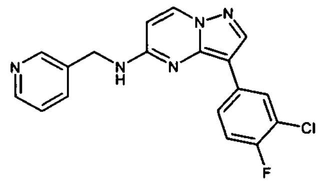 | [3-(3-Chloro-4-fluoro-phenyl)-pyrazolo[1,5-a]pyrimidin-5-yl]-pyridin-3-ylmethyl-amine | 0.97 | 353.79 | 352.26 |
| 374 | 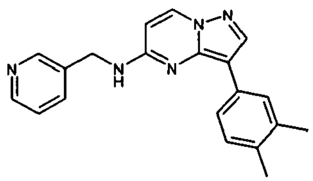 | [3-(3,4-Dimethyl-phenyl)-pyrazolo[1,5-a]pyrimidin-5-yl]-pyridin-3-ylmethyl-amine | 0.97 | 329.4 | 328.28 |
| 375 | 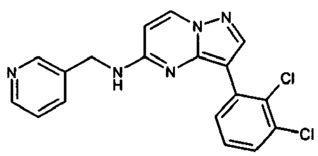 | [3-(2,3-Dichloro-phenyl)-pyrazolo[1,5-a]pyrimidin-5-yl]-pyridin-3-ylmethyl-amine | 1 | 370.24 | 370.08 |
| 376 | 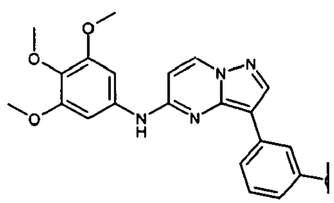 | [3-(3-Chloro-phenyl)-pyrazolo[1,5-a]pyrimidin-5-yl]-(3,4,5-trimethoxy-phenyl)-amine | 1.34 | 410.86 | 409.23 |

(continued)

| Example No. | Structure | Name | RT | MW | Observed Mass Peak [M-H] |
|---|---|---|---|---|---|
| 377 | 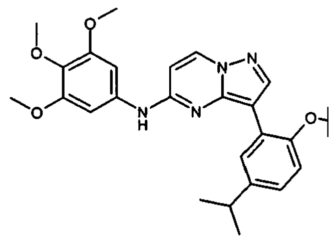 | [3-(5-Isopropyl-2-methoxy-phenyl)-pyrazolo[1,5-a]pyrimidin-5-yl]-(3,4,5-trimethoxy-phenyl)-amine | 1.39 | 448.52 | 447.36 |
| 378 | 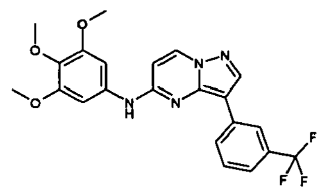 | [3-(3-Trifluoromethyl-phenyl)-pyrazolo[1,5-a]pyrimidin-5-yl]-(3,4,5-trimethoxy-phenyl)-amine | 1.37 | 444.41 | 443.27 |
| 379 | 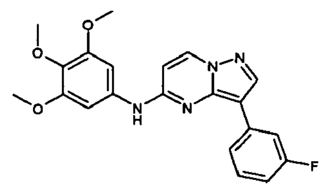 | [3-(3-Fluoro-phenyl)-pyrazolo[1,5-a]pyrimidin-5-yl]-(3,4,5-trimethoxy-phenyl)-amine | 1.28 | 394.4 | 393.28 |
| 380 | 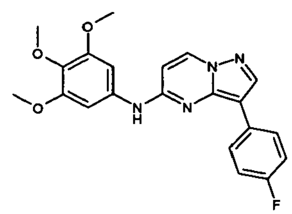 | [3-(4-Fluoro-phenyl)-pyrazolo[1,5-a]pyrimidin-5-yl]-(3,4,5-trimethoxy-phenyl)-amine | 1.27 | 394.4 | 393.28 |

(continued)

| Example No. | Structure | Name | RT | MW | Observed Mass Peak [M-H] |
|---|---|---|---|---|---|
| 381 | 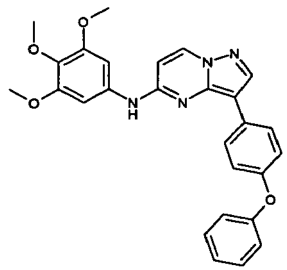 | [3-(4-Phenoxy-phenyl)-pyrazolo[1,5-a]pyrimidin-5-yl]-(3,4,5-trimethoxy-phenyl)-amine | 1.44 | 468.51 | 467.24 |
| 382 | 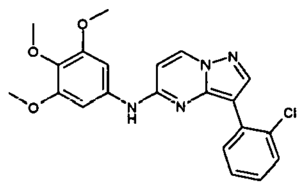 | [3-(2-Chloro-phenyl)-pyrazolo[1,5-a]pyrimidin-5-yl]-(3,4,5-trimethoxy-phenyl)-amine | 1.3 | 410.86 | 409.26 |
| 383 | 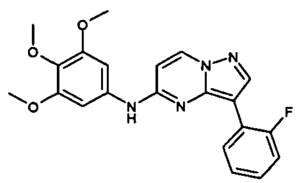 | [3-(2-Fluoro-phenyl)-pyrazolo[1,5-a]pyrimidin-5-yl]-(3,4,5-trimethoxy-phenyl)-amine | 1.27 | 394.4 | 393.28 |
| 384 | 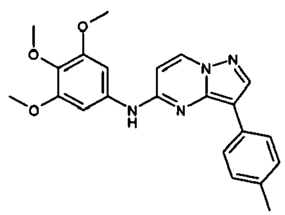 | (3-p-Tolyl-pyrazolo[1,5-a]pyrimidin-5-yl)-(3,4,5-trimethoxy-phenyl)-amine | 1.32 | 390.44 | 389.33 |

(continued)

| Example No. | Structure | Name | RT | MW | Observed Mass Peak [M-H] |
|---|---|---|---|---|---|
| 385 | 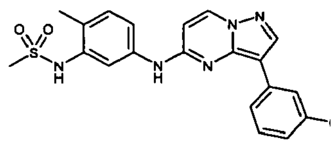 | N-{5-[3-(3-Chloro-phenyl)-pyrazolo[1,5-a]pyrimidin-5-ylamino]-2-methyl-phenyl}-methanesulfonamide | 1.28 | 427.91 | 426.25 |
| 386 | 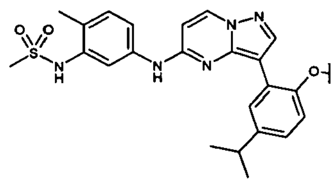 | N-{5-[3-(5-Isopropyl-2-methoxy-phenyl)-pyrazolo[1,5-a]pyrimidin-5-ylamino]-2-methyl-phenyl}-methanesulfonamide | 1.35 | 465.58 | 464.27 |
| 387 | 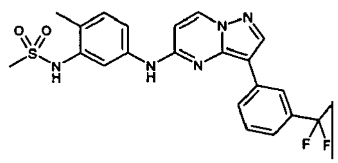 | N-{2-Methyl-5-[3-(3-trifluoromethyl-phenyl)-pyrazoto[1,5-a]pyrimidin-5-ylamino]-phenyl}-methanesulfonamide | 1.32 | 461.47 | 461.17 |
| 388 | 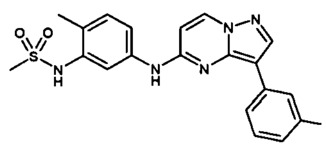 | N-{5-[3-(3-Fluoro-phenyl)-pyrazolo[1,5-a]pyrimidin-5-ylamino]-2-methyl-phenyl}-methanesulfonamide | 1.2 | 411.46 | 410.28 |

(continued)

| Example No. | Structure | Name | RT | MW | Observed Mass Peak [M-H] |
|---|---|---|---|---|---|
| 389 | 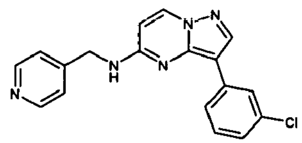 | [3-(3-Chloro-phenyl)-pyrazolo[1,5-a]pyrimidin-5-yl]-pyridin-4-ylmethyl-amine | 0.87 | 335.8 | 334.26 |
| 390 | 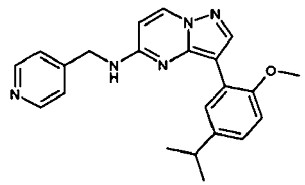 | [3-(5-isopropyl-2-methoxy-phenyl)-pyrazolo[1,5-a]pyrimidin-5-yl]-pyridin-4-ylmethyl-amine | 0.97 | 373.46 | 372.44 |
| 391 | 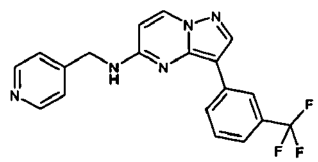 | Pyridin-4-ylmethyl-[3-(3-trifluoromethyl-phenyl)-pyrazolo[1,5-a]pyrimidin-5-yl]-amine | 0.94 | 369.35 | 368.27 |
| 392 | 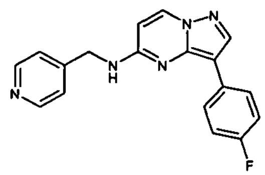 | [3-(4-Fluoro-phenyl)-pyrazolo[1,5-a]pyrimidin-5-yl]-pyridin-4-ylmethyl-amine | 0.81 | 319.34 | 318.44 |
| 393 | 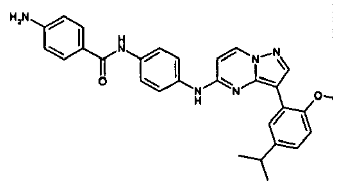 | 4-Amino-N-{4-[3-(5-isopropyl-2-methoxy-phenyl)-pyrazolo[1,5-a]pyrimidin-5-ylamino]-phenyl}-benzamide | 1.26 | 492.58 | 491.31 |

(continued)

| Example No. | Structure | Name | RT | MW | Observed Mass Peak [M-H] |
|---|---|---|---|---|---|
| 394 | 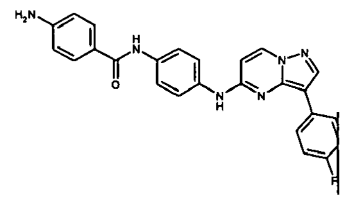 | 4-Amino-N-{4-[3-(4-fluoro-phenyl)-pyrazolo[1,5-a]pyrimidin-5-ylamino]-phenyl}-benzamide | 1.15 | 438.46 | 437.29 |
| 395 | 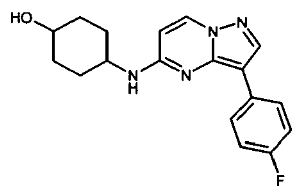 | 4-[3-(4-Fluoro-phenyl)-pyrazolo[1,5-a]pyrimidin-5-ylamino]-cyclohexanol | 1.06 | 326.37 | 325.34 |
| 396 | 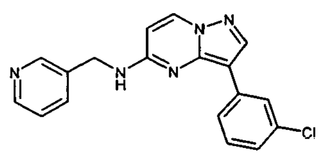 | [3-(3-Chloro-phenyl)-pyrazolo[1,5-a]pyrimidin-5-yl]-pyridin-3-ylmethyl-amine | 0.94 | 335.8 | 334.23 |
| 397 | 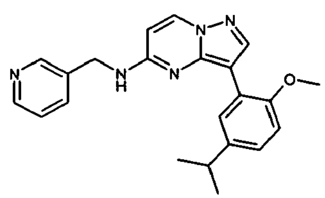 | [3-(5-Isopropyl-2-methoxy-phenyl)-pyrazolo[1,5-a]pyrimidin-5-yl]-pyridin-3-ylmethyl-amine | 1.05 | 373.46 | 373.34 |
| 398 | 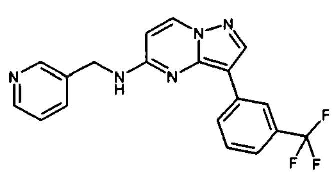 | Pyridin-3-ylmethyl-[3-(3-trifluoromethyl-phenyl)-pyrazolo[1,5-a] pyrimidin-5-yl]-amine | 1.01 | 369.35 | 369.12 |

(continued)

| Example No. | Structure | Name | RT | MW | Observed Mass Peak [M-H] |
|---|---|---|---|---|---|
| 399 | 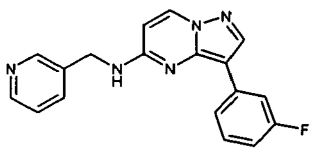 | [3-(3-Fluoro-phenyl)-pyrazolo[1,5-a]pyrimidin-5-yl]-pyridin-3-ylmethyl-amine | 0.86 | 319.34 | 317.74 |
| 400 | 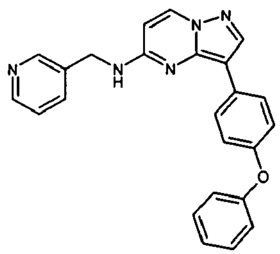 | [3-(4-Phenoxy-phenyl)-pyrazolo[1,5-a]pyrimidin-5-yl]-pyridin-3-ylmethyl-amine | 1.09 | 393.45 | 392.3 |
| 401 | 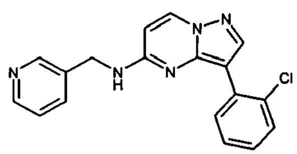 | [3-(2-Chloro-phenyl)-pyrazolo[1,5-a]pyrimidin-5-yl]-pyridin-3-ylmethyl-amine | 0.89 | 335.8 | 333.84 |
| 402 | 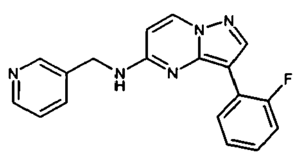 | [3-(2-Fluoro-phenyl)-pyrazolo[1,5-a]pyrimidin-5-yl]-pyridin-3-ylmethyl-amine | 0.86 | 319.34 | 318.51 |
| 403 | 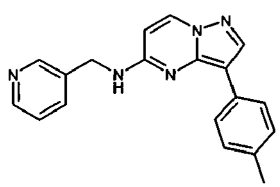 | Pyridin-3-ylmethyl-(3-p-tolyl-pyrazolo[1,5-a]pyrimidin-5-yl)-amine | 0.9 | 315.38 | 315.31 |

(continued)

| Example No. | Structure | Name | RT | MW | Observed Mass Peak [M-H] |
|---|---|---|---|---|---|
| 404 | 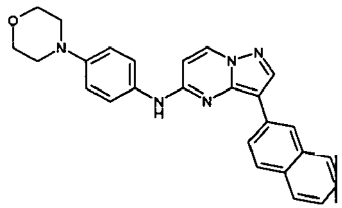 | (4-Morpholin-4-yl-phenyl)-(3-naphthalen-2-yl-pyrazolo[1,5-a] pyrimidin-5-yl)-amine | 1.36 | 421.5 | 420.36 |
| 405 | 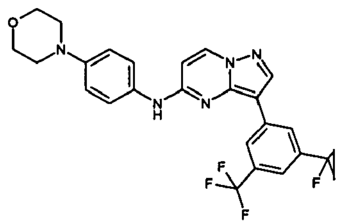 | [3-(3,5-Bis-trifluoromethyl-phenyl)-pyrazolo[1,5-a]pyrimidin-5-yl]-(4-morpholin-4-yl-phenyl)-amine | 1.52 | 507.44 | 506.23 |
| 406 | 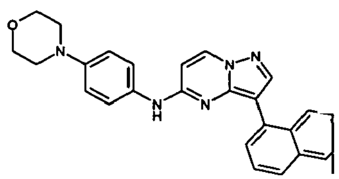 | (4-Morpholin-4-yl-phenyl)-(3-naphthalen-1-yl-pyrazolo[1,5-a] pyrimidin-5-yl)-amine | 1.3 | 421.5 | 420.36 |
| 407 | 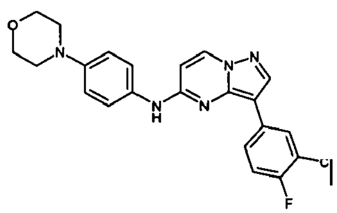 | [3-(3-Chloro-4-fluoro-phenyl)-pyrazolo[1,5-a]pyrimidin-5-yl]-(4-morpholin-4-yl-phenyl)-amine | 1.35 | 423.88 | 422.26 |

(continued)

| Example No. | Structure | Name | RT | MW | Observed Mass Peak [M-H] |
|---|---|---|---|---|---|
| 408 | 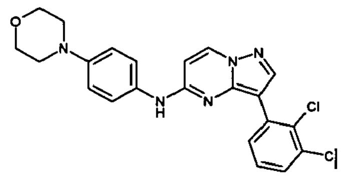 | [3-(2,-Dichloro-phenyl)-pyrazolo[1,5-a]pyrimidin-5-yl]-(4-morpholin-4-yl-phenyl)-amine | | 440.33 | |
| 409 | 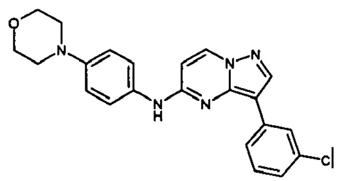 | [3-(3-Chloro-phenyl)-pyrazolo[1,5-a]pyrimidin-5-yl]-(4-morphotin-4-yl-phenyl)-amine | 1.32 | 405.89 | 404.28 |
| 410 | 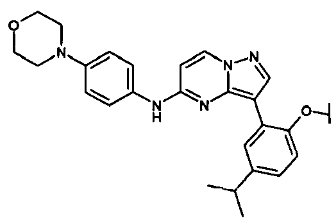 | [3-(5-Isopropyl-2-methoxy-phenyl)-pyrazolo[1,5-a]pyrimidin-5-yl]-(4-morpholin-4-yl-phenyl)-amine | 1.39 | 443.55 | 442.38 |
| 411 | 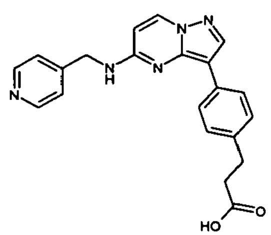 | 3-(4-{5-[(Pyridin-4-ylmethyl)-amino]-pyrazolo[1,5-a]pyrimidin-3-yl}-phenyl)-propionic acid | 0.73 | 373.41 | 372.31 |

(continued)

| Example No. | Structure | Name | RT | MW | Observed Mass Peak [M-H] |
|---|---|---|---|---|---|
| 412 | 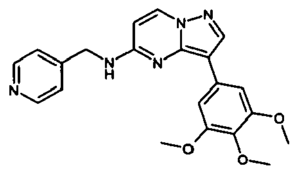 | Pyridin-4-ylmethyl-[3-(3,4,5-trimethoxy-phenyl)-pyrazolo[1,5-a] pyrimidin-5-yl]-amine | 0.77 | 391.43 | 389.57 |
| 413 | 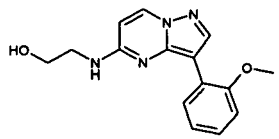 | 2-[3-(2-Methoxy-phenyl)-pyrazolo[1,5-a]pyrimidin-5-ylamino]-ethanot | 0.94 | 284.32 | 283.3 |
| 414 | 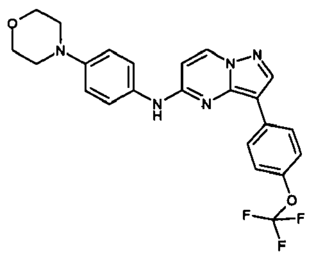 | (4-Morpholin-4-yl-phenyl)-[3-(4-trifluoromethoxy-phenyl)-pyrazolo [1,5-a]pyrimidin-5-yl]-amine | 1.39 | 455.44 | 454.19 |
| 415 | 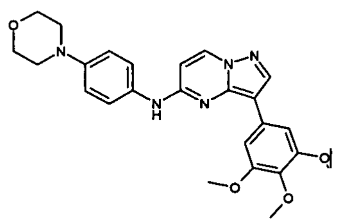 | (4-Morpholin-4-yl-phenyl)-[3-(3,4,5-trimethoxy-phenyl)-pyrazolo [1,5-a]pyrimidin-5-yl]-amine | 1.12 | 461.52 | 460.27 |

(continued)

| Example No. | Structure | Name | RT | MW | Observed Mass Peak [M-H] |
|---|---|---|---|---|---|
| 416 | 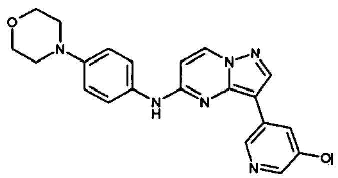 | [3-(5-Methoxy-pyridin-3-yl)-pyrazolo[1,5-a]pyrimidin-5-yl]-(4-morpholin-4-yl-phenyl)-amine | 0.83 | 402.46 | 401.28 |
| 417 | 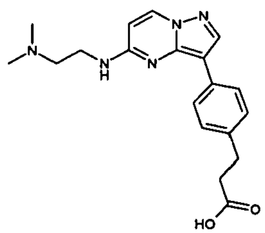 | 3-{4-[5-(2-Dimethylamino-ethylamino)-pyrazolo[1,5-a]pyrimidin-3-yl]-phenyl}-propionic acid | 0.71 | 353.42 | 352.32 |
| 418 | 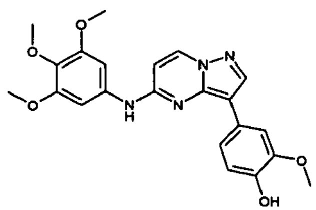 | 2-Methoxy-4-[5-(3,4,5-trimethoxy-phenylamino)-pyrazolo[1,5-a]pyrimidin-3-yl]-phenol | | 422.44 | |
| 419 | 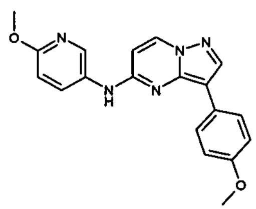 | [3-(4-Methoxy-phenyl)-pyrazolo[1,5-a]pyrimidin-5-yl]-(6-methoxy-pyridin-3-yl)-amine | | 347.38 | |

(continued)

| Example No. | Structure | Name | RT | MW | Observed Mass Peak [M-H] |
|---|---|---|---|---|---|
| 420 | 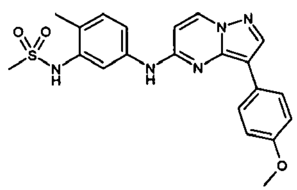 | N-{5-[3-(4-Methoxy-phenyl)-pyrazolo[1,5-a]pyrimidin-5-ylamino]-2-methyl-phenyl}-methanesulfonamide | | 423.49 | |
| 421 | 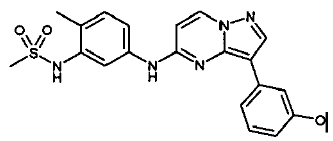 | N-{5-[3-(3-Hydroxy-phenyl)-pyrazolo[1,5-a]pyrimidin-5-ylamino]-2-methyl-phenyl}-methanesulfonamide | | 409.47 | |
| 422 | 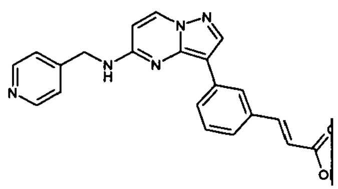 | (E)-3-(3-{5-[(Pyridin-4-ylmethyl)-amino]-pyrazolo[1,5-a]pyrimidin-3-yl}-phenyl)-acrylic acid | | 371.40 | |
| 423 | 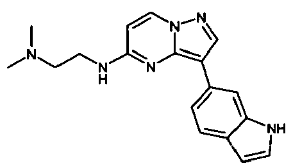 | N'-[3-(1H-Indol-6-yl)-pyrazolo[1,5-a]pyrimidin-5-yl]-N,N-dimethyl-ethane-1,2-diamine | | 320.40 | |
| 424 | 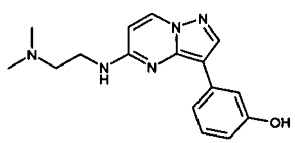 | 3-[5-(2-Dimethylamino-ethylamino)-pyrazolo[1,5-a]pyrimidin-3-yl]-phenol | | 297.36 | |

(continued)

| Example No. | Structure | Name | RT | MW | Observed Mass Peak [M-H] |
|---|---|---|---|---|---|
| 425 | 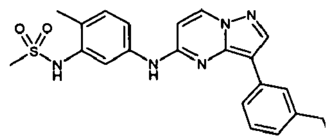 | N-{5-[3-(3-Hydroxymethyl-phenyl)-pyrazolo[1,5-a]pyrimidin-5-ylamino]-2-methyl-phenyl}-methanesulfonamide | | 423.49 | |
| 426 | 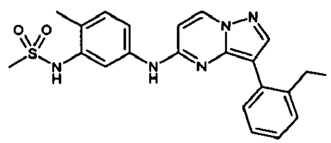 | N-{5-[3-(2-Hydroxymethyl-phenyl)-pyrazolo[1,5-a]pyrimidin-5-ylamino]-2-methyl-phenyl}-methanesulfonamide | | 423.49 | |
| 427 | 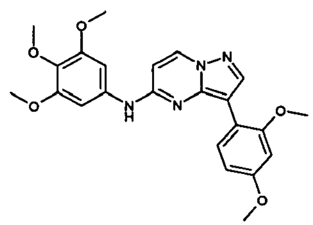 | [3-(2,4-Dimethoxy-phenyl)-pyrazolo[1,5-a]pyrimidin-5-yl]-(3,4,5-trimethoxy-phenyl)-amine | | 436.47 | |
| 428 | 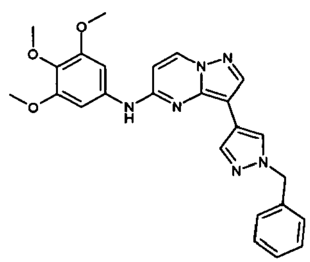 | [3-(1-Benzyl-1H-pyrazol-4-yl)-pyrazolo[1,5-a]pyrimidin-5-yl]-(3,4,5-trimethoxy-phenyl)-amine | | 456.50 | |

(continued)

| Example No. | Structure | Name | RT | MW | Observed Mass Peak [M-H] |
|---|---|---|---|---|---|
| 429 | 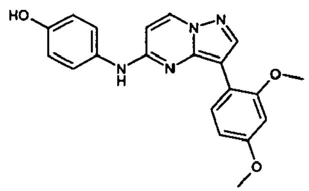 | 4-[3-(2,4-Dimethoxy-phenyl)-pyrazolo[1,5-a]pyrimidin-5-ylamino]-phenol | | 362.39 | |
| 430 | 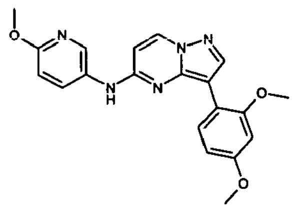 | [3-(2,4-Dimethoxy-phenyl)-pyrazolo[1,5-a]pyrimidin-5-yl]-(6-methoxy-pyridin-3-yl)-amine | | 377.40 | |
| 431 | 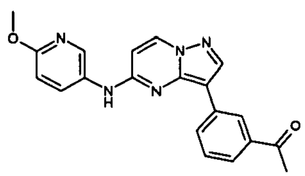 | 1-{3-[5-(6-Methoxy-pyridin-3-ylamino)-pyrazolo[1,5-a]pyrimidin-3-yl]-phenyl}-ethanone | | 359.39 | |
| 432 | 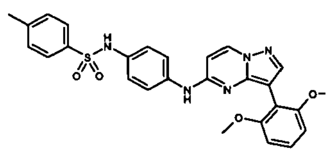 | N-{4-[3-(2,6-Dimethoxy-phenyl)-pyrazolo[1,5-a]pyrimidin-5-ylamino]-phenyl}-4-methyl-benzenesulfonamide | | 515.59 | |
| 433 | 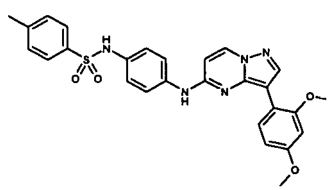 | N-{4-[3-(2,4-Dimethoxy-phenyl)-pyrazolo[1,5-a]pyrimidin-5-ylamino]-phenyl}-4-methyl-benzenesulfonamide | | 515.59 | |

(continued)

| Example No. | Structure | Name | RT | MW | Observed Mass Peak [M-H] |
|---|---|---|---|---|---|
| 434 | 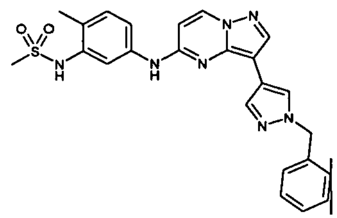 | N-{5-[3-(1-Benzyl-1H-pyrazol-4-yl)-pyrazolo[1,5-a]pyrimidin-5-ylamino]-2-methyl-phenyl}-methanesulfonamide | | 473.56 | |
| 435 | 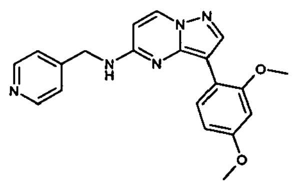 | [3-(2,4-Dimethoxy-phenyl)-pyrazolo[1,5-a]pyrimidin-5-yl]-pyridin-4-ylmethyl-amine | | 361.40 | |
| 436 | 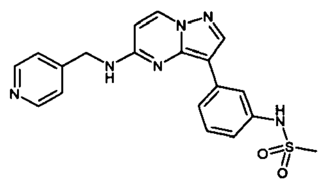 | N-(3-{5-[(Pyridin-4-ylmethyl)-amino]-pyrazolo[1,5-a]pyrimidin-3-yl}-phenyl)-methanesulfonamide | | 394.56 | |
| 437 | 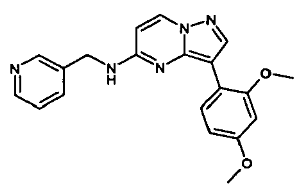 | [3-(2,4-Dimethoxy-phenyl)-pyrazolo[1,5-a]pyrimidin-5-yl]-pyridin-3-ylmethyl-amine | | 361.40 | |

(continued)

| Example No. | Structure | Name | RT | MW | Observed Mass Peak [M-H] |
|---|---|---|---|---|---|
| 438 | 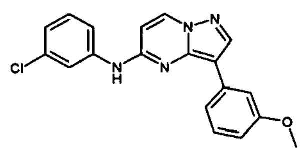 | (3-Chloro-phenyl)-[3-(3-methoxy-phenyl)-pyrazolo[1,5-a]pyrimidin-5-yl]-amine | | 350.81 | |
| 439 | 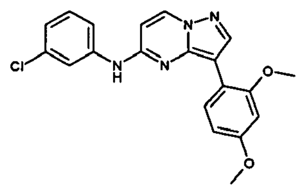 | (3-Chloro-phenyl)-[3-(2,4-dimethoxy-phenyl)-pyrazolo[1,5-a] pyrimidin-5-yl]-amine | | 380.83 | |
| 440 | 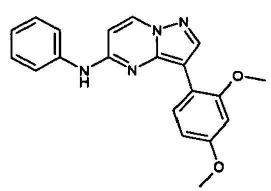 | [3-(2,4-Dimethoxy-phenyl)-pyrazolo[1,5-a]pyrimidin-5-yl]-phenyl-amine | | 346.39 | |
| 441 | 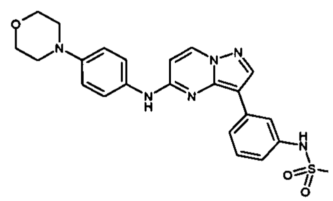 | N-{3-[5-(4-Morpholin-4-yl-phenylamino)-pyrazolo[1,5-a]pyrimidin-3-yl]-phenyl}-methanesulfonamide | | 464.55 | |
| 442 | 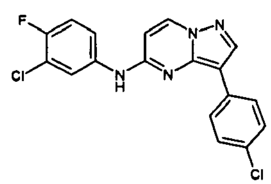 | (3-Chloro-4-fluoro-phenyl)-[3-(4-chloro-phenyl)-pyrazolo[1,5-a] pyrimidin-5-yl]-amine | | 373.22 | |

(continued)

| Example No. | Structure | Name | RT | MW | Observed Mass Peak [M-H] |
|---|---|---|---|---|---|
| 443 | 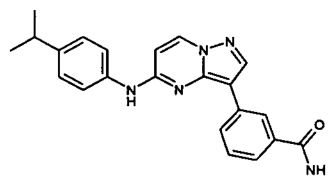 | 3-[5-(4-isopropyl-phenylamino)-pyrazolo[1,5-a]pyrimidin-3-yl]-benzamide | | 371.44 | |
| 444 | 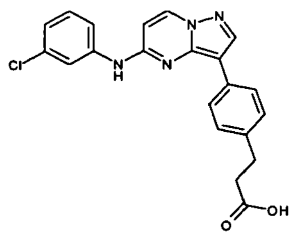 | 3-{4-[5-(3-Chloro-phenylamino)-pyrazolo[1,5-a]pyrimidin-3-yl]-phenyl}-propionic acid | | 392.84 | |
| 445 | 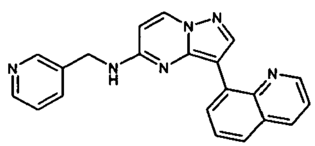 | Pyridin-3-ylmethyl-(3-quinolin-8-yl-pyrazolo[1,5-a]pyrimidin-5-yl)-amine | | 352.40 | |
| 446 | 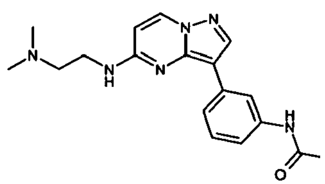 | N-{3-[5-(2-Dimethylamino-ethylamino)-pyrazolo[1,5-a]pyrimidin-3-yl]-phenyl}-acetamide | | 338.41 | |

(continued)

| Example No. | Structure | Name | RT | MW | Observed Mass Peak [M-H] |
|---|---|---|---|---|---|
| 447 | 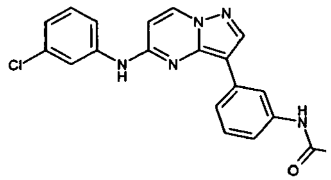 | N-{3-[5-(3-Chloro-phenylamino)-pyrazolo[1,5-a]pyrimidin-3-yl]-phenyl}-acetamide | | 377.83 | |
| 448 | 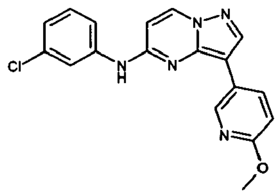 | (3-Chloro-phenyl)-[3-(6-methoxy-pyridin-3-yl)-pyrazolo[1,5-a] pyrimidin-5-yl]-amine | | 351.80 | |
| 449 | 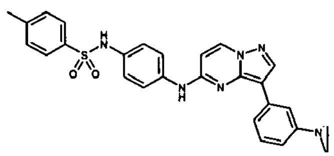 | N-{4-[3-(3-Dimethylamino-phenyl)-pyrazolo[1,5-a]pyrimidin-5-ylamino]-phenyl}-4-methyl-benzenesulfonamide | | 498.61 | |
| 450 | 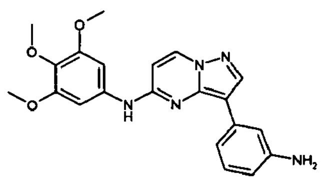 | [3-(3-Amino-phenyl)-pyrazolo[1,5-a]pyrimidin-5-yl]-(3,4, 5-trimethoxy-phenyl)-amine | | 391.43 | |

(continued)

| Example No. | Structure | Name | RT | MW | Observed Mass Peak [M-H] |
|---|---|---|---|---|---|
| 451 | 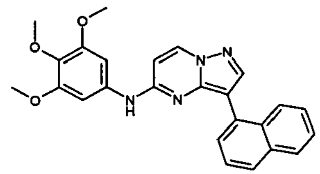 | (3-Naphthalen-1-yl-pyrazolo[1,5-a]pyrimidin-5-yl)-(3,4,5-trimethoxy-phenyl)-amine | | 426.47 | |
| 452 | 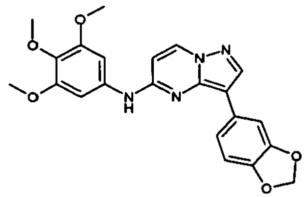 | (3-Benzo[1,3]dioxol-5-yl-pyrazolo[1,5-a]pyrimidin-5-yl)-(3,4,5-trimethoxy-phenyl)-amine | | 410.42 | |
| 453 | 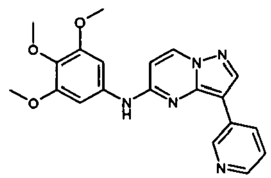 | (3-Pyridin-3-yl-pyrazolo[1,5-a]pyrimidin-5-yl)-(3,4,5-trimethoxy-phenyl)-amine | | 377.40 | |
| 454 | 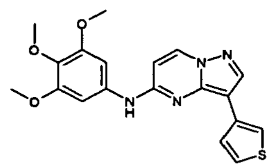 | (3-Thiophen-3-yl-pyrazolo[1,5-a]pyrimidin-5-yl)-(3,4, 5-trimethoxy-phenyl)-amine | | 382.44 | |
| 455 | 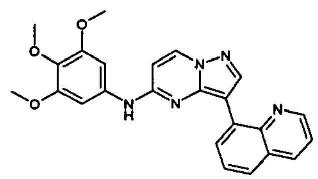 | (3-Quinolin-8-yl-pyrazolo[1,5-a]pyrimidin-5-yl)-(3,4,5-trimethoxy-phenyl)-amine | | 427.46 | |

(continued)

| Example No. | Structure | Name | RT | MW | Observed Mass Peak [M-H] |
|---|---|---|---|---|---|
| 456 | 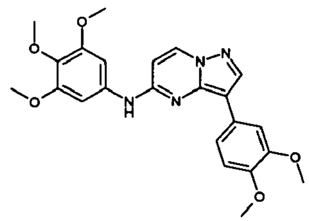 | [3-(3,4-Dimethoxy-phenyl)-pyrazolo[1,5-a]pyrimidin-5-yl]-(3,4,5-trimethoxy-phenyl)-amine | | 436.47 | |
| 457 | 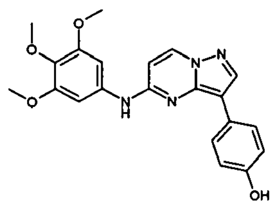 | 4-[5-(3,4,5-Trimethoxy-phenylamino)-pyrazolo[1,5-a]pyrimidin-3-yl]-phenol | | 392.41 | |
| 458 | 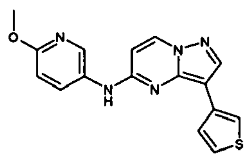 | (6-Methoxy-pyridin-3-yl)-(3-thiophen-3-yl-pyrazolo[1,5-a]pyrimidin-5-yl)-amine | | 323.38 | |
| 459 | 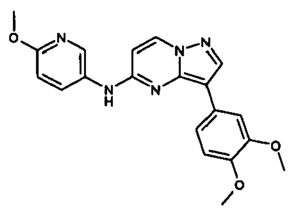 | [3-(3,4-Dimethoxy-phenyl)-pyrazolo[1,5-a]pyrimidin-5-yl]-(6-methoxy-pyridin-3-yl)-amine | | 377.40 | |

(continued)

| Example No. | Structure | Name | RT | MW | Observed Mass Peak [M-H] |
|---|---|---|---|---|---|
| 460 | 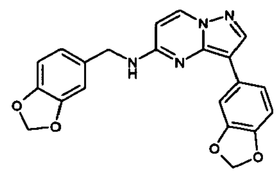 | Benzo[1,3]dioxol-5-ylmethyl-(3-benzo[1,3]dioxol-5-yl-pyrazolo[1,5-a]pyrimidin-5-yl)-amine | | 388.38 | |
| 461 | 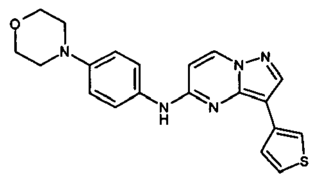 | (4-Morpholin-4-yl-phenyl)-(3-thiophen-3-yl-pyrazolo[1,5-a] pyrimidin-5-yl)-amine | | 377.47 | |
| 462 | 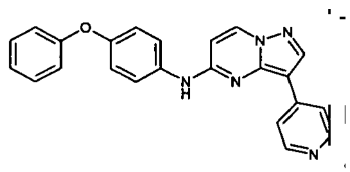 | (4-Phenoxy-phenyl)-(3-pyridin-4-yl-pyrazolo[1,5-a]pyrimidin-5-yl)-amine | | 379.42 | |
| 463 | 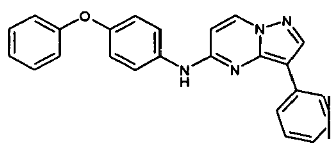 | (4-Phenoxy-phenyl)-(3-pyridin-3-yl-pyrazolo[1,5-a]pyrimidin-5-yl)-amine | | 379.42 | |
| 464 | 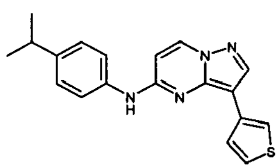 | (4-Isopropyl-phenyl)-(3-thiophen-3-yl-pyrazolo[1,5-a]pyrimidin-5-yl)-amine | | 334.44 | |

(continued)

| Example No. | Structure | Name | RT | MW | Observed Mass Peak [M-H] |
|---|---|---|---|---|---|
| 465 | 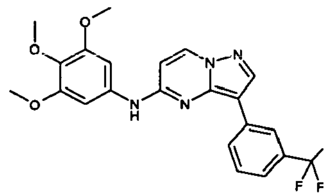 | [3-(3-Trifluoromethyl-phenyl)-pyrazolo[1, 5-a]pyrimidin-5-yl]-(3,4, 5-trimethoxy-phenyl)-amine | | 460.41 | |
| 466 | 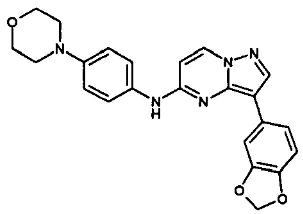 | (3-Benzo[1,3]dioxol-5-yl-pyrazolo[1,5-a]pyrimidin-5-yl)-(4-morpholin-4-yl-phenyl)-amine | 1.17 | 415.45 | 414.32 |
| 467 | 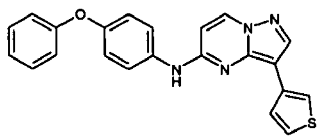 | (4-Phenoxy-phenyl)-(3-thiophen-3-yl-pyrazolo[1,5-a]pyrimidin-5-yl)-amine | 1.49 | 384.46 | 383.25 |
| 468 | 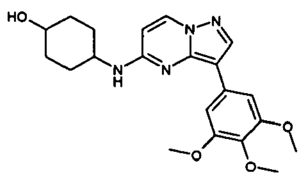 | 4-[3-(3,4,5-Trimethoxy-phenyl)-pyrazolo[1,5-a]pyrimidin-5-ylamino]-cyclohexanol | 0.96 | 398.46 | 397.34 |
| 469 | 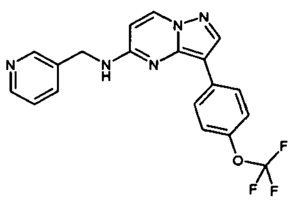 | Pyridin-3-ylmethyl-[3-(4-trifluoromethoxy-phenyl)-pyrazolo[1,5-a] pyrimidin-5-yl]-amine | 1.05 | 385.35 | 384.2 |

(continued)

| Example No. | Structure | Name | RT | MW | Observed Mass Peak [M-H] |
|---|---|---|---|---|---|
| 470 | 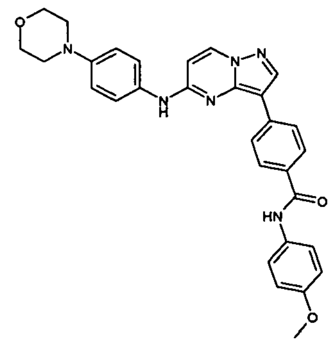 | N-(4-Methoxy-phenyl)-4-[5-(4-morpholin-4-yl-phenylamino)-pyrazolo[1,5-a]pyrimidin-3-yl]-benzamide | 1.19 | 520.59 | 519.3 |
| 471 | 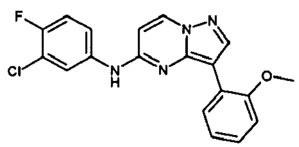 | (3-Chloro-4-fluoro-phenyl)-[3-(2-methoxy-phenyl)-pyrazolo[1,5-a] pyrimidin-5-yl]-amine | 1.44 | 368.8 | 367.23 |
| 472 | 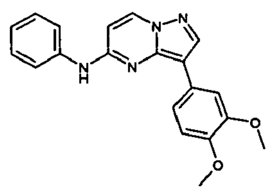 | [3-(3,4-Dimethoxy-phenyl)-pyrazolo[1,5-a]pyrimidin-5-yl]-phenyl-amine | 1.2 | 346.39 | 345.34 |
| 473 | 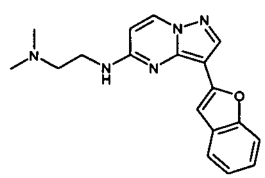 | N'-(3-Benzofuran-2-yl-pyrazolo[1,5-a]pyrimidin-5-yl)-N,N-dimethyl-ethane-1,2-diamine | 0.84 | 321.38 | 320.4 |

(continued)

| Example No. | Structure | Name | RT | MW | Observed Mass Peak [M-H] |
|---|---|---|---|---|---|
| 474 | 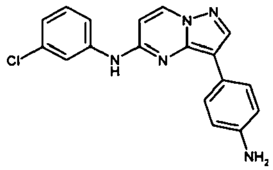 | [3-(4-Amino-phenyl)-pyrazolo[1,5-a]pyrimidin-5-yl]-(3-chloro-phenyl)-amine | 0.95 | 335.8 | 334.19 |
| 475 | 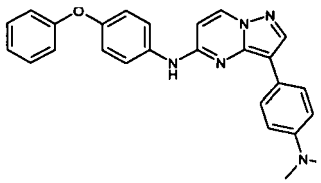 | [3-(4-Dimethylamino-phenyl)-pyrazolo[1,5-a]pyrimidin-5-yl]-(4-phenoxy-phenyl)-amine | 1.3 | 421.5 | 420.32 |
| 476 | 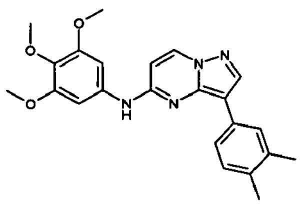 | [3-(3,4-Dimethyl-phenyl)-pyrazolo[1,5-a]pyrimidin-5-yl]-(3,4,5-trimethoxy-phenyl)-amine | 1.36 | 404.47 | 403.29 |
| 477 | 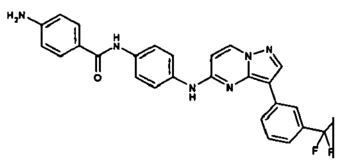 | 4-Amino-N-{4-[3-(3-trifluoromethyl-phenyl)-pyrazolo[1,5-a] pyrimidin-5-ylamino]-phenyl}-benzamide | 1.24 | 488.47 | 487.2 |

(continued)

| Example No. | Structure | Name | RT | MW | Observed Mass Peak [M-H] |
|---|---|---|---|---|---|
| 478 | 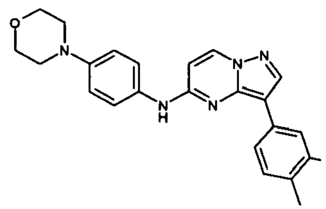 | [3-(3,4-Dimethyl-phenyl)-pyrazolo[1,5-a]pyrimidin-5-yl]-(4-morpholin-4-yl-phenyl)-amine | | 399.50 | |

**Example 479 Preparation of N*4*-[3-(3-chloro-phenyl)-pyrazolo[1,5-a]pyhmidin-5-yl]-N*1*,N*1*-diethyl-pentane-1,4-diamine**

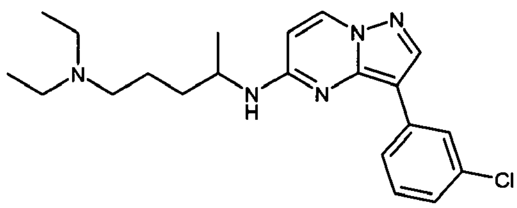

Example 479a Preparation of N*4*-(3-bromo-pyrazolo[1,5-a]pyrimidin-5-yl)-N*1*,N*1*-diethyl-pentane-1,4-diamine

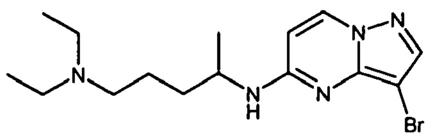

In analogy to Example 1a, Intermediate A (1 g, 4.3 mmol), was reacted with 2-amino-5-diethylaminopentane (1.29 mL, 6.45 mmol). The resulting crude product was purified by chromatography on silica gel to give N*4*-(3-bromopyrazolo[1,5-a]pyrimidin-5-yl)-N*1*,N*1*-diethyl-pentane-1,4-diamine (0.69 g, 45%).
$^1$H-NMR (300 MHz, $CDCl_3$): $\delta$ = 8.10 (1 H, d), 7.77 (1 H, s), 6.00 (1 H, d), 5.88 (1 H, m), 4.13 (1H, m), 2.43 - 2.66 (6H, m), 1.52 - 1.68 (4H, m), 1.26 (3H, d), 1.04 (6H, t) ppm.

Example 479b Preparation of N*4*-[3-(3-chloro-phenyl)-pyrazolo[1,5-a]pyrimidin-5-yl]-N*1*,N*1*-diethyl-pentane-1,4-diamine

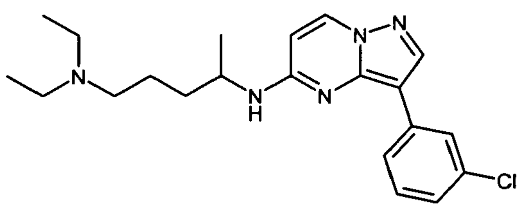

To a stirred solution of N*4*-(3-bromo-pyrazolo[1,5-a]pyrimidin-5-yl)-N*1*,N*1*-diethyl-pentane-1,4-diamine (0.3 g, 0.85 mmol) in DME (3.5 mL) under argon was added 3-chlorophenyl boronic acid (0.15 g, 0.93 mmol), $Pddba_2$ (0.024 g, 0.042 mmol), $P(oTol)_3$ (0.026 g, 0.085 mmol) and saturated aqueous sodium hydrogencarbonate solution (1.3 mL). The mixture was heated at reflux for 6 h. On cooling, the reaction was quenched by addition of saturated aqueous ammonium chloride solution (10 mL). The mixture was diluted with water and DCM, filtered through a Celite® plug and resulting filtrate extracted with DCM (3x). The combined organic layers were washed with brine, dried over $Na_2SO_4$ and concentrated in vacuo. The crude product was purified by chromatography on silica gel to give N*4*-[3-(3-chloro-phenyl)-pyrazolo[1,5-a]pyrimidin-5-yl]-N*1*,N*1*-diethyl-pentane-1,4-diamine (0.005 mg, 2%).
LC-MS (LC-MS Method B): RT = 2.08 min, MW = 385.94, observed mass peaks = 386/388 [M+H, Cl isotopes].

**Example 480 Preparation of N'-[3-(3-chloro-phenyl)-pyrazolo[1,5-a]pyrimidin-5-yl]-N,N-diethyl-propane-1,3-diamine**

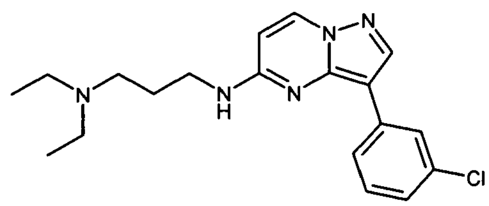

Example 480a Preparation of N'-(3-bromo-pyrazolo[1,5-a]pyrimidin-5-yl)-N,N-diethyl-propane-1,3-diamine

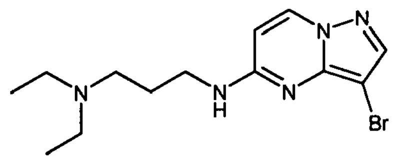

In analogy to Example 1a, Intermediate A (0.5 g, 2.15 mmol), was reacted with 3-diethylamino-propyl amine (0.51 mL, 3.23 mmol) to give N'-(3-bromopyrazolo[1,5-a]pyrimidin-5-yl)-N,N-diethyl-propane-1,3-diamine (0.65 g, 92%). $^{1}$H-NMR (400 MHz, $CDCl_3$): $\delta$ = 8.07 (1H, d), 7.78 (1H, s), 7.58 (1H, broad s), 5.96 (1H, d), 3.60 (2H, m), 2.63 (2H, m), 2.58 (4H, q), 1.80 (2H, m), 1.07 (6H, t) ppm.

Example 480b Preparation of 5-chloro-3-(3-chloro-phenyl)-pyrazolo[1,5-a]pyrimidine

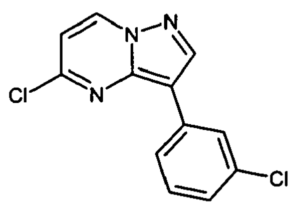

Intermediate A (0.5 g, 2.15 mmol) was dissolved in DME (9 mL), under argon and treated with $P(oTol)_3$ (0.065 g, 0.22 mmol), $Pddba_2$ (0.062 mg, 0.11 mmol), 3-chlorophenyl boronic acid (0.37 g, 2.37 mmol) and saturated aqueous sodium hydrogencarbonate (3.3 mL). The mixture was heated at reflux for 5 h. On cooling, the reaction mixture was diluted with saturated aqueous ammonium chloride solution and extracted with DCM/MeOH. The combined organic extracts were washed with brine, dried over $Na_2SO_4$ and concentrated in vacuo. The crude product was purified by chromatography on silica gel to give 5-chloro-3-(3-chloro-phenyl)-pyrazolo[1,5-a]pyrimidine (0.20 g, 35%).
LC-MS (LC-MS Method A): RT = 1.40 min, MW = 264.12, observed mass peaks = 264/266 [M+H, Cl isotopes].

Example 480c Preparation of N'-[3-(3-chloro-phenyl)-pyrazolo[1,5-a]pyrimidin-5-yl]-N,N-diethyl-propane-1,3-diamine

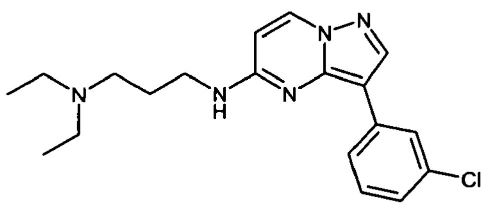

Method A

In analogy to Example 479b, N'-(3-bromo-pyrazolo[1,5-a]pyrimidin-5-yl)-N,N-diethyl-propane-1,3-diamine [Example 480a] (0.3 g, 0.92 mmol), was reacted with 3-chlorophenyl boronic acid (0.16 g, 1.01 mmol). The crude product was purified by chromatography on silica gel to give N'-[3-(3-chloro-phenyl)-pyrazolo[1,5-a]pyrimidin-5-yl]-N,N-diethyl-propane-1,3-diamine (0.017 g, 5%).
LC-MS (LC-MS Method B): RT = 2.05 min, MW = 357.89, observed mass peaks = 358/360 [M+H, Cl isotopes].

Method B

5-Chloro-3-(3-chloro-phenyl)-pyrazolo[1,5-a]pyrimidine [Example 480b] (0.19 g, 0.72 mmol) was suspended in acetonitrile (2.5 mL) under argon and treated with potassium carbonate (0.2 g, 1.44 mmol) and 3-diethylaminopropane (0.23 mL, 1.44 mmol). The mixture was heated at reflux for 25 h. On cooling the reaction mixture was filtered to give N'-[3-(3-chloro-phenyl)-pyrazolo[1,5-a]pyrimidin-5-yl]-N,N-diethyl-propane-1,3-diamine (0.15 g). The filtrate was concentrated in vacuo to give a further portion of N'-[3-(3-chloro-phenyl)-pyrazolo[1,5-a]pyrimidin-5-yl]-N,N-diethyl-propane-1,3-diamine (0.23 g, combined yield 91%).
LC-MS (LC-MS Method A): RT = 0.87 min, MW = 357.89, observed mass peaks = 356/358 [M-H, Cl isotopes].
The following examples were prepared in analogy to Example 479 and Example 480 [LC-MS data such as retention time (RT) or observed mass peak were collected using LC-MS Method B unless explicitly stated]:

| Example No. | Structure | Name | RT | MW | Observed Mass Peaks [M+H] |
|---|---|---|---|---|---|
| 481 | 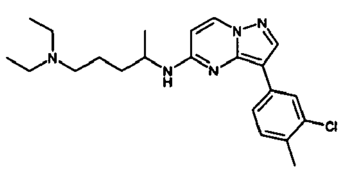 | N*4*-[3-(3-Chloro-4-methyl-phenyl)-pyrazolo[1,5-a]pyrimidin-5-yl]-N*1*,N*1*-diethyl-pentane-1,4-diamine | 2.39 | 399.97 | 400/402 (Cl isotopes) |
| 482 | 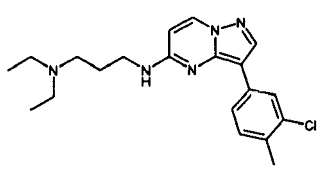 | N'-[3-(3-Chloro-4-methyl-phenyl)-pyrazolo[1,5-a]pyrimidin-5-yl]-N,N-diethyl-propane-1,3-diamine | 2.19 | 371.92 | 372/374 (Cl isotopes) |

Methods of testing for a particular pharmacological property are well known to persons skilled in the art. The example testing experiments described herein serve to illustrate the present invention and the invention is not limited to the examples given.

**Biological assay 1: ALK1-Kinase Flashplate-Assay**

To prove the effectiveness of the compounds according to the present invention an ALK1-Kinase Flashplate-Assay was established and used.
ALK1 phosphorylates serine/threonine residues of the biotinylated substrate bovine α-casein in the presence

of [$\gamma$-$^{33}$P]ATP. Detection of the radiolabeled phosphorylated product is achieved by binding to streptavidine-coated Flashplates. The biotin moieties of biotinylated casein bind with high affinity to the streptavidine. The radiolabeled biotinylated casein produced by the ALK1 kinase reaction is able to generate a chemoluminescent signal when strepatavidine-mediated binding occurs to the scintillant-containing surface of the Flashplates due to the close proximity of the radiolabel and the scintillant. Unphosphorylated substrate does not give rise to such a signal because it does not contain radiolabeled phosphate groups. Any free [$\gamma$-$^{33}$P]ATP which remains unbound in solution is washed away from the wells of the Flashplates and, therefore, does not significantly contribute to the background signal obtained. The signals obtained are therefore indicative of the ALK1 kinase activity. Measurement is performed in a Perkin-Elmer TopCount or Perkin-Elmer ViewLux instrument.

**Materials:**

Enzyme: Purified human recombinant ALK1 (GST fused to ALK1 intracellular domain [His142-Gln503]) produced in-house, aliquots stored at -80˚C; Diluted enzyme working solution: 2.5 ng/μL ALK1 (in assay buffer) freshly prepared and chilled on ice until use.
Substrate: biotinylated bovine α-casein. Unbiotinylated casein obtained from Sigma was biotinylated by standard procedures using N-hydroxysuccinimide (NHS) ester of biotin.
Substrate working solution: 0.83 μM ATP, 1.67 μM biotinylated a-casein, 7.4 nCi [$\gamma$-$^{33}$P]ATP/μl in assay buffer
Assay plates: 384-well plates, small volume, white, Greiner (# 784075)
Flashplates: Streptavidin-coated Flashplates, Perkin Elmer (384-well # SPM410A)
Assay buffer: 50 mM Tris/HCl pH 8.0, 1 mM $MnCl_2$, 1 mM DTT, 0.01% NP40, 0.5x Complete EDTA-free
Stop solution: 33.3 μM ATP, 33.3 mM EDTA, 0.07% Triton X-100 in PBS
Saturating buffer for Flashplates: 100 μM ATP, 0.2% Triton X-100 in PBS
Sealing tape: Greiner (# 676080)

**Assay steps**

Protocol for small volume 5 μl assay (all steps are performed at 20˚C, pipetted with CyBi-Well and Multidrop Micro):

1. 50 nl or 250 nl compound in 100% DMSO
2. addition of 3 μl substrate working solution with CyBi-Well pipettor
3. addition of 2 μl enzyme working solution with Multidrop Micro incubation for 60 min at room temperature
4. addition of 15 μl Stop solution with CyBi-Well pipettor
5. transfer of 18 μl assay mixture to Flashplates** with CyBi-Well pipettor incubation for at least 3 h at room temperature or over night at 4˚C to allow binding to the streptavidine-coated Flashplates
6. washing the Flashplates 3 times with 50 μl PBS without Ca++ and Mg++
7. sealing with sealing tape
8. Measurement in Topcount (60 sec/well) hour with 50 μl saturating buffer. Remove 18 μl from the 50 μl and transfer 18 μl of the assay mixture to the Flashplate.

**Saturation of Flashplates: The Flashplates are preincubated for at least 1

Final concentrations, calculated for 5 μl reaction volume: ALK1 5 ng/well; 1 μM biotinylated α-casein; 0.5 μM ATP; 22 nCi/well [$\gamma$-$^{33}$P]ATP; 1 mM $MnCl_2$; 1 mM DTT; 50 mM Tris-HCl; pH 8.0; 0.01% NP40; 0.5x Complete EDTA-free; 1% or 5% DMSO.

The data were normalised (enzyme reaction without inhibitor = 0 % inhibition, enzyme reaction in the presence of 10 mM EDTA = 100 % inhibition) and $IC_{50}$ values were calculated by a 4 parameter fit using an in-house software.

Typical $IC_{50}$ values for preferred compounds of the present invention are in the range of 10 μM to 1 nM, as determined by the above assay. Typical $IC_{50}$ values for more preferred compounds of the present invention are in the range of 1 μM to 1 nM, even more preferably 0.1 μM to 1 nM, as determined by the above assay.

**Biological experiment 2: ALK1 transactivation assay**

To prove the effectiveness of the compounds according to the present invention the following method was established and used.

Herein HepG2 cell-cultures were transiently transfected by known techniques with an ALK1 plasmid (expression

vector for wildtype full-length ALK1 receptor) and ID1 reporter plasmid containing 1.3 kB (-1370 to +86) of the ID1-promoter upstream of a luciferase reporter gene. ID1 is a known target gene of ALK1 and therefore gets transactivated by cotransfection with the ALK1 receptor. The specific transactivation is quantified via relative light units (RLU) which are detected in dependence of luciferase expression. Therefore a commercially available detection kit which contains the luciferase substrate luciferine was used.

**Materials:**

HepG2 cells, ATCC HB-8065
96well Culture Plates 96 white (Packard # 6005680)
96well plate polypropylene for compound dilution in DMSO
PBS-; PBS++, DMSO
DMEM Ham's F12 (Biochrom #F4815) with 10% FCS after dialysis, 1% PenStrep and 200 mM Glutamine
OPTI MEM (Gibco #51985-026)
Fugene (Roche #1814443 1 mL)
steadyliteHTS (Perkin Elmer# 6016981)

**Experimental procedure**

<u>day 1: seeding of cells on 96-well plates</u>

HepG2 cells are seeded on 96-well plates at a density of 7000 cells/ well in DMEM/HamsF12 +5%FCS (+ 1% P/S, +1 % Gln).

<u>day 2: transfection of cells</u>

per well:

200 ng DNA: 100 ng ID1-luc (in pGL3basic, Promega)+ 5 ng ALK1wt (in pcDNA3.1) + 95 ng pcDNA3.1 (empty vector, Invitogen)
0,4 µl Fugene
6 µl OptiMEM

Fugene and OptiMEM are incubated for 5 min at RT. This mixture is incubated with the DNA for 15 min at RT.
Afterwards, the plate is incubated under shaking conditions at room temperature (RT) for 1 h. After 4 hours at 37°C the supernatants are drawn off by suction and the wells are replaced with medium (100 µl/well) containing low serum (0,2 %FCS) and test substances. Plates are incubated for further 18 h at 37°C.

<u>day3: RLU measurement</u>

100 µl luciferase substrate (steadyliteHTS, Packard) are added per well and plates are measured after 10 minutes in a luminometer (e.g. Viktor luminometer, Perkin Elmer). Luciferase activity is quantified by relative light units (RLU).
Calculation of IC50:

ALK1wt - DMSO Control (without ALK1) = 100 %

Substance (+ALK1wt) -DMSO control (without ALK1)= x %

IC50 = 50 % inhibition of ALK1 transactivation activity

From the given results it can clearly be seen that the compounds of the present invention display inhibition of ALK1 kinase.

## Claims

1. A compound of general formula (I) :

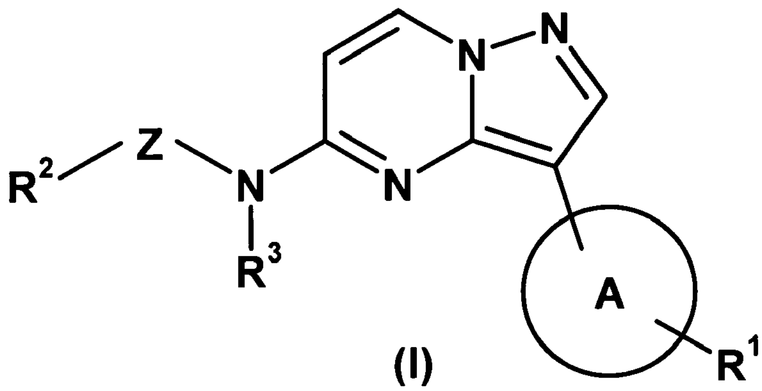

(I)

wherein :

A represents aryl or heteroaryl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, -$(CH_2)_n$heteroaryl, -O$(CH_2)$paryl, -O$(CH_2)_q$heteroaryl,C(O)$R^5$, -C$(O)_2R^5$, -$NR^4$C(O)$R^5$, -$NR^4$S$(O)_2R^5$, -C(O)$NR^6R^7$,OC(O)$NR^6R^7$, -$NR^4$C$(O)_2R^5$, -$NR^4$C(O)$NR^6R^7$, -S(O)$R^5$, -S$(O)_2R^5$,S$(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,O$(CH_2)$paryl, -O$(CH_2)_q$heteroaryl is optionally substituted one or more times, in the same way or differently with halogen, hydroxyl, cyano, nitro, -C$(O)_2R^5$, or a -$NR^6R^7$ group, or

the moiety :

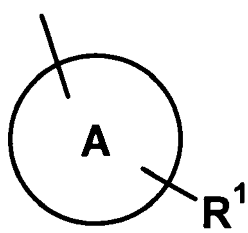

represents :

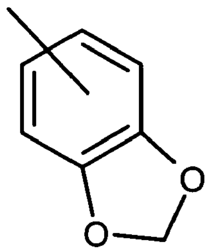

, or

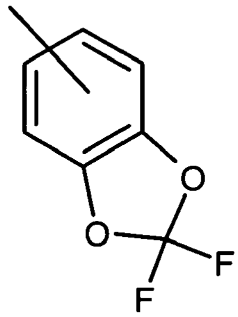

,

Z represents a linker group which is a bond, $C_1$-$C_6$-alkyl, -C(O)-,C(O)$NR^8$-, or -$S(O)_2$ group, in which $C_1$-$C_6$-alkyl is optionally substituted one or more times, in the same way or differently with halogen, hydroxyl, $C_1$-$C_6$-haloalkyl, $C_1$-$C_6$-alkoxy, $C_1$-$C_6$-haloalkyloxy, or a -$NR^6R^7$ group,

$R^2$ represents a substituent selected from the group comprising, preferably consisting of, hydrogen, $C_3$-$C_{10}$-cycloalkyl, $C_3$-$C_{10}$-heterocycloalkyl, aryl, heteroaryl, wherein $C_3$-$C_{10}$-cycloalkyl, $C_3$-$C_{10}$-heterocyctoalkyl, aryl, heteroaryl is optionally substituted one or more times, in the same way or differently with halogen, hydroxy, cyano, nitro, $C_1$-$C_6$-alkyl, $C_1C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -C(O)$R^5$, -C$(O)_2R^5$, -$NR^4$C(O)$R^5$,$NR^4S(O)_2R^5$, -C(O)$NR^6R^7$, -OC(O)$NR^6R^7$, -$NR^4C(O)_2R^5$, -$NR^4$C(O)$NR^6R^7$, -S(O)$R^5$, -$S(O)_2R^5$, or -$S(O)_2NR^6R^7$;

or represents the moiety :

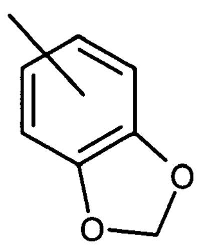

, or

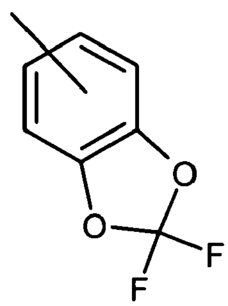

,

with the proviso that when Z is a bond, $R^2$ is not hydrogen,

$R^3$ represents a substituent selected from the group comprising, preferably consisting of, hydrogen, $C_1$-$C_6$-alkyl, $C_1$-$C_6$-haloalkyl, $C_1$-$C_6$-alkoxy, $C_1C_6$-alkoxy-$C_1C_6$-alkyl, or halo-$C_1$-$C_6$-alkoxy-$C_1$-$C_6$-alkyl,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, heteroaryl, $C_1$-$C_6$-alkoxy-$C_1$-$C_6$-alkyl, or halo-$C_1$-$C_6$-alkoxy-$C_1$-$C_6$-alkyl, wherein aryl or heteroaryl is optionally further substituted with the group $C_1$-$C_6$-alkyl, or$NR^6R^7$,

$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-haloalkoxyalkyl, $C_1$-$C_6$-alkoxy-$C_1C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -S$(O)_2$- group, and can optionally contain one or more double bonds, wherein said heterocycloalkyl ring is optionally

substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, $-C(O)R^5$, $-C(O)_2R^5$, $-NR^4C(O)R^5$, $NR^4S(O)_2R^5$, $-C(O)NR^8R^9$, $-OC(O)NR^8R^9$, $-NR^4C(O)_2R^5$, $-NR^4C(O)NR^8R^9$, $-S(O)R^5$, $-S(O)_2R^5$, or $-S(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

**2.** The compound according to claim 1, wherein :

A represents aryl or heteroaryl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, $-(CH_2)_n$heteroaryl, $-O(CH_2)_p$aryl, $-O(CH_2)_q$heteroaryl,$C(O)R^5$, $-C(O)_2R^5$, $-NR^4C(O)R^5$, $-NR^4S(O)_2R^5$, $-C(O)NR^6R^7$,$OC(O)NR^6R^7$, $-NR^4C(O)_2R^5$, $-NR^4C(O)NR^6R^7$, $-S(O)R^5$, $-S(O)_2R^5$,$S(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, $-(CH_2)_m$aryl, $-(CH_2)_n$heteroaryl,$O(CH_2)_p$aryl, $-O(CH_2)_q$heteroaryl is optionally substituted one or more times, in the same way or differently with halogen, hydroxyl, cyano, nitro, $-C(O)_2R^5$, or a $-NR^6R^7$ group, or

the moiety:

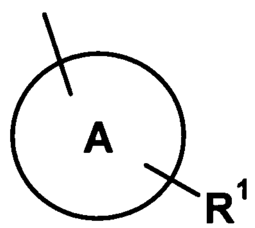

represents :

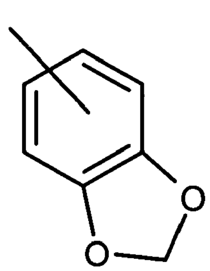

, or

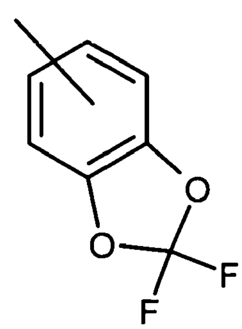

;

Z represents a linker group which is a bond, or $C_1$-$C_6$-alkyl, in which $C_1$-$C_6$-alkyl is optionally substituted one

or more times, in the same way or differently with halogen, hydroxyl, $C_1$-$C_6$-haloalkyl, $C_1$-$C_6$-alkoxy, $C_1$-$C_6$-haloalkyloxy, or a -$NR^6R^7$ group,
$R^2$ represents a substituent selected from the group comprising, preferably consisting of, hydrogen, $C_3$-$C_{10}$-cycloalkyl, $C_3$-$C_{10}$-heterocycloalkyl, aryl, heteroaryl, wherein $C_3$-$C_{10}$-cycloalkyl, $C_3$-$C_{10}$-heterocycloalkyl, aryl, heteroaryl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -$C(O)R^5$, -$C(O)_2R^5$, -$NR^4C(O)R^5$,$NR^4S(O)_2R^5$, -$C(O)NR^6R^7$, -$OC(O)NR^6R^7$, -$NR^4C(O)_2R^5$, -$NR^4C(O)NR^6R^7$, -$S(O)R^5$, -$S(O)_2R^5$, or -$S(O)_2NR^6R^7$;
or represents the moiety :

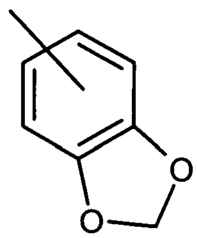

, or

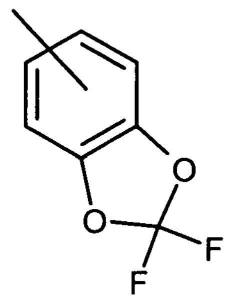

,

with the proviso that when Z is a bond, $R^2$ is not hydrogen,
$R^3$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,
$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,
$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, heteroaryl, $C_1$-$C_6$-alkoxy-$C_1$-$C_6$-alkyl, or halo-$C_1$-$C_6$-alkoxy-$C_1$-$C_6$-alkyl, wherein aryl or heteroaryl is optionally further substituted with the group $C_1$-$C_6$-alkyl, or$NR^6R^7$,
$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or
$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -S$(O)_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -$C(O)R^5$, -$C(O)_2R^5$, -$NR^4C(O)R^5$, $NR^4S(O)_2R^5$, -$C(O)NR^8R^9$, -$OC(O)NR^8R^9$, -$NR^4C(O)_2R^5$, -$NR^4C(O)NR^8R^9$, -$S(O)R^5$, -$S(O)_2R^5$, or -S$(O)_2NR^8R^9$, wherein $C_1$-$C_6$-alkyl may be further optionally substituted with hydroxy,
$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,
m represents an integer of 0, 1, 2, 3, or 4,
n represents an integer of 0, 1, 2, 3, or 4,
p represents an integer of 0, 1, 2, 3, or 4, and
q represents an integer of 0, 1, 2, 3, or 4,

as well as :
N-oxides, solvates, hydrates, isomers, diastereomers, enantiomers and salts thereof.

**3.** The compound according to claim 1 or 2, wherein :

A represents aryl or heteroaryl, wherein A is optionally substituted in the same way or differently with one or more $R^1$ groups,
$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycLoalkyl, aryl,$(CH_2)_m$aryl, -$(CH_2)_n$heteroaryl, -$O(CH_2)_p$aryl, -$O(CH_2)_q$heteroaryl,$C(O)R^5$, -$C(O)_2R^5$, -$NR^4C(O)R^5$, -$NR^4S(O)_2R^5$, -$C(O)NR^6R^7$,$OC(O)NR^6R^7$, -$NR^4C(O)_2R^5$, -$NR^4C(O)NR^6R^7$, -$S(O)R^5$, -$S(O)_2R^5$,$S(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,$O(CH_2)_p$aryl, -$O(CH_2)_q$heteroaryl is optionally substituted one or more times, in the same way or differently with halogen, hydroxyl, cyano, nitro, -$C(O)_2R^5$, or a -$NR^6R^7$ group, or
the moiety:

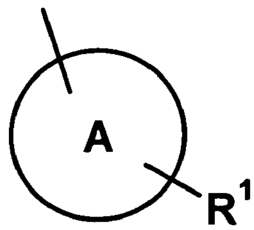

represents :

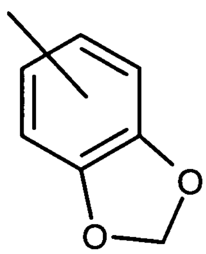

, or

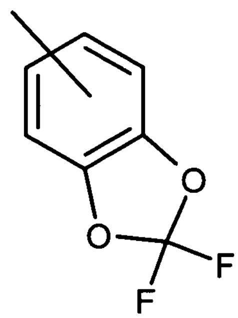

;

Z represents a linker group which is a bond, or $C_1$-$C_6$-alkyl,
$R^2$ represents a substituent selected from the group comprising, preferably consisting of $C_3$-$C_{10}$-cycloalkyl, $C_3$-$C_{10}$-heterocycloalkyl, aryl, heteroaryl, wherein $C_3$-$C_{10}$-cycloalkyl, $C_3$-$C_{10}$-heterocycloalkyl, aryl, heteroaryl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -$C(O)R^5$, -$C(O)_2R^5$, -$NR^4C(O)R^5$,$NR^4S(O)_2R^5$, -$C(O)NR^6R^7$, -$OC(O)NR^6R^7$, -$NR^4C(O)_2R^5$, -$NR^4C(O)NR^6R^7$, -$S(O)R^5$, -$S(O)_2R^5$, or -$S(O)_2NR^6R^7$;
or represents the moiety :

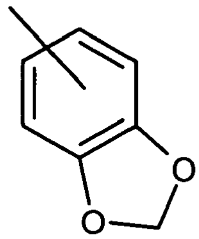

, or

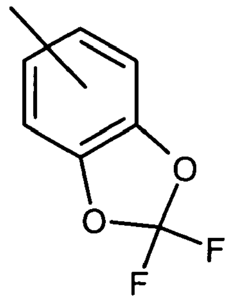

,

$R^3$ is hydrogen,

$R^4$ represents a substituent selected from the group comprising, preferably consisting of, hydrogen, $C_1C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, $C_1$-$C_6$-alkoxy-$C_1$-$C_6$-alkyl, or halo-$C_1$-$C_6$-alkoxy-$C_1$-$C_6$-alkyl, wherein aryl is optionally further substituted with the group $C_1$-$C_6$-alkyl, or -$NR^6R^7$,

$R^6$ and $R^7$ independently from one another represent a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, $C_1$-$C_6$-haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(0)-, -S(O)- and/or -S$(O)_2$. group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C(O)$R^5$, -$C(O)_2R^5$, -$NR^4C(O)R^5$, $NR^4S(O)_2R^5$, -C(O)$NR^8R^9$, -OC(O)$NR^8R^9$, -$NR^4C(O)_2R^5$, -$NR^4C(O)NR^8R^9$, -S(O)$R^5$, -$S(O)_2R^5$, or -S$(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

**4.** The compound according to any one of claims 1 to 3, wherein :

A represents aryl or heteroaryl, wherein A is optionally substituted in the same way or differently with one or more $R^1$ groups,

$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, -$(CH_2)_n$heteroaryl, -O$(CH_2)_p$aryl, -O$(CH_2)_q$heteroaryl,C(O)$R^5$, -$C(O)_2R^5$, -$NR^4C(O)R^5$, -$NR^4S(O)_2R^5$, -C(O)$NR^6R^7$,OC(O)$NR^6R^7$,- $NR^4C(O)_2R^5$, -$NR^4C(O)NR^6R^7$, -S(O)$R^5$, -$S(O)_2R^5$,$S(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,O$(CH_2)_p$aryl, -O$(CH_2)_q$heteroaryl is optionally substituted one or more times, in the same way or differently with halogen, hydroxyl, cyano, nitro, -$C(O)_2R^5$, or a -$NR^6R^7$ group, or

the moiety:

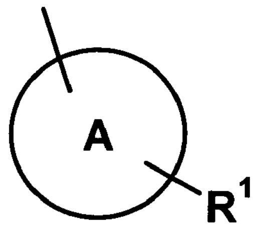

represents :

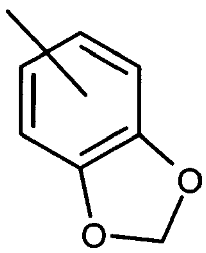

, or

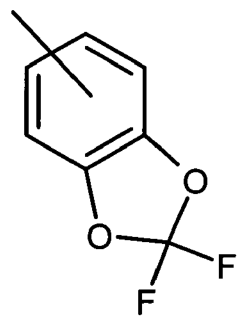

;

Z represents a linker group which is a bond, or $C_1$-$C_6$-alkyl,

$R^2$ represents a substituent selected from the group comprising, preferably consisting of $C_3$-$C_{10}$-cycloalkyl, aryl, heteroaryl, wherein $C_3$-$C_{10}$-cycloalkyl, aryl, heteroaryl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -C(0)$R^5$, -$C(O)_2R^5$, -$NR^4$C (O)$R^5$,$NR^4S(O)_2R^5$,- C(O)$NR^6R^7$, -OC(O)$NR^6R^7$, -$NR^4C(O)_2R^5$, -$NR^4$C(O)$NR^6R^7$, -S(O)$R^5$, -$S(O)_2R^5$, or -S $(O)_2NR^6R^7$;

or represents the moiety :

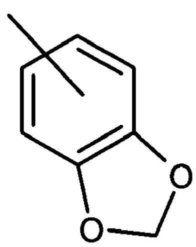

, or

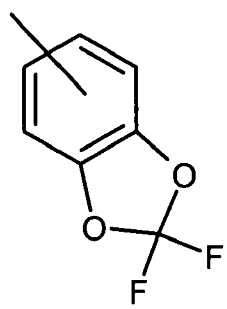

,

$R^3$ is hydrogen,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, $C_1$-$C_6$-alkoxy-$C_1$-$C_6$-alkyl, or halo-$C_1$-$C_6$-alkoxy-$C_1$-$C_6$-alkyl, wherein aryl is optionally further substituted with the group $C_1$-$C_6$-alkyl, or -$NR^6R^7$,

$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-

haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or
$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -S$(O)_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C(O)$R^5$, -C$(O)_2R^5$, -$NR^4C(O)R^5$, $NR^4S(O)_2R^5$, -C(O)$NR^8R^9$, -OC(O)$NR^8R^9$, -$NR^4C(O)_2R^5$, -$NR^4C(O)NR^8R^9$, -S(O)$R^5$, -S$(O)_2R^5$, or -S$(O)_2NR^8R^9$, wherein $C_1$-$C_6$-alkyl may be further optionally substituted with hydroxy,
$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,
m represents an integer of 0, 1, 2, 3, or 4,
n represents an integer of 0, 1, 2, 3, or 4,
p represents an integer of 0, 1, 2, 3, or 4, and
q represents an integer of 0, 1, 2, 3, or 4,

as well as :
N-oxides, solvates, hydrates, isomers, diastereomers, enantiomers and salts thereof.

**5.** The compound according to any one of claims 1 to 4, wherein :

A represents phenyl, naphthyl, pyridyl, benzofuranyl, benzothiophenyl, quinolinyl, thiophenyl, pyrazolyl, furanyl, wherein A is optionally substituted in the same way or differently with one or more $R^1$ groups,
$R^1$ represents a substituent selected from the group comprising, preferably consisting of, hydrogen, halogen, hydroxy, cyano, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_3$-$C_{10}$-heterocycloalkyl, -$(CH_2)$phenyl, -O$(CH_2)_p$phenyl, -C(O)$R^5$, C$(O)_2R^5$, -$NR^4C(O)R^5$, -$NR^4S(O)_2R^5$, -C(O)$NR^6R^7$, -NR $^4C(O)_2R^5$, -S(O)$R^5$, -S$(O)_2R^5$, -S$(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_3$-$C_{10}$-heterocycloalkyl is optionally substituted one or more times, in the same way or differently with halogen, hydroxyl, cyano, nitro, -C$(O)_2R^5$, or aNR $^6R^7$ group, or
the moiety :

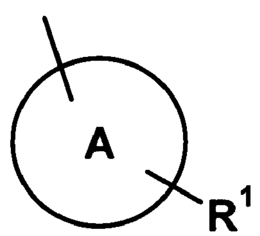

represents :

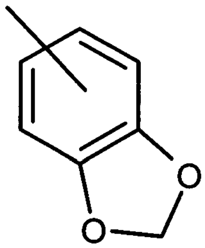

, or

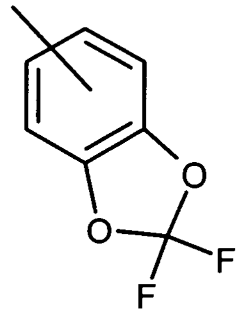

;

Z represents a linker group which is a bond, or $C_1$-$C_6$-alkyl,

$R^2$ represents a substituent selected from the group comprising, preferably consisting of $C_3$-$C_{10}$-cycloalkyl, phenyl, or pyridyl, wherein $C_3$-$C_{10}$-cycloalkyl, phenyl, or pyridyl is optionally substituted one or more times, in the same way or differently with halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, -O(phenyl), -$NR^6R^7$, -C(O)$R^5$, -$C(O)_2R^5$,$NR^4C(O)R^5$, -$NR^4S(O)_2R^5$, -C(O)$NR^6R^7$, -OC(O)$NR^6R^7$, -$NR^4C(O)_2R^5$,$NR^4C(O)NR^6R^7$, -S(O)$R^5$, -S$(O)_2R^5$, or -$S(O)_2NR^6R^7$;

or represents the moiety :

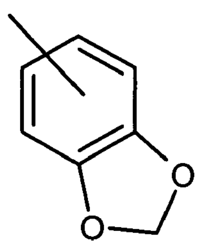

, or

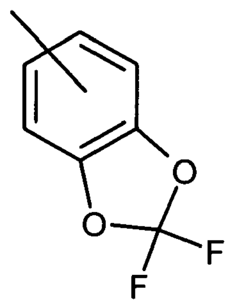

,

$R^3$ is hydrogen,

$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,

$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, phenyl, $C_1$-$C_6$-alkoxy-$C_1$-$C_6$-alkyl, or halo-$C_1$-$C_6$-alkoxy-$C_1$-$C_6$-alkyl, wherein phenyl is optionally further substituted with the group $C_1$-$C_6$-alkyl, or -$NR^6R^7$,

$R^6$ and $R^7$ independently from one another represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -S$(O)_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C(O)$R^5$, -$C(O)_2R^5$, -$NR^4C(O)R^5$, $NR^4S(O)_2R^5$, -C(O)$NR^8R^9$, -OC(O)$NR^8R^9$, -$NR^4C(O)_2R^5$, -$NR^4C(O)NR^8R^9$, -S(O)$R^5$, -$S(O)_2R^5$, or -S$(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy

and
p represents an integer of 0, or 1,

as well as :
N-oxides, solvates, hydrates, isomers, diastereomers, enantiomers and salts thereof.

**6.** The compound according to any one of claims 1 to 2, wherein :

A phenyl, naphthyl, pyridyl, benzofuranyl, benzothiophenyl, quinolinyl, thiophenyl, pyrazolyl, furanyl, wherein A is optionally substituted in the same way or differently with one or more $R^1$ groups,
$R^1$ represents a substituent selected from the group comprising, preferably consisting of, hydrogen, halogen, hydroxy, cyano, nitro, $C_1$-$C_6$-alkyl, $C_1$-$C_6$-haloalkyl, $C_1$-$C_6$-alkoxy, $C_1$-$C_6$-haloalkyloxy, $C_1$-$C_6$-alkoxy-$C_1$-$C_6$-alkyl, halo-$C_1$-$C_6$-alkoxy-$C_1$-$C_6$-alkyl, $C_2$-$C_6$-alkenyl, $C_2$-$C_6$-alkynyl, $C_3$-$C_{10}$-cycloalkyl, $C_3$-$C_{10}$-heterocycloalkyl, aryl,$(CH_2)_m$aryl, -$(CH_2)_n$heteroaryl, -$O(CH_2)_p$aryl, -$O(CH_2)_q$heteroaryl,$C(O)R^5$, -$C(O)_2R^5$, -$NR^4C(O)R^5$, -$NR^4S(O)_2R^5$, -$C(O)NR^6R^7$,$OC(O)NR^6R^7$, -$NR^4C(O)_2R^5$, -$NR^4C(O)NR^6R^7$, -$S(O)R^5$, -$S(O)_2R^5$,$S(O)_2NR^6R^7$, wherein $C_1$-$C_6$-alkyl, $C_1$-$C_6$-haloalkyl, $C_1$-$C_6$-alkoxy, $C_1$-$C_6$-haloalkyloxy, $C_2$-$C_6$-alkenyl, $C_2$-$C_6$-alkynyl, $C_3$-$C_{10}$-cycloalkyl, $C_3$-$C_{10}$-heterocycloalkyl, aryl, -$(CH_2)_m$aryl, -$(CH_2)_n$heteroaryl,$O(CH_2)_P$aryl, -$O(CH_2)_q$heteroaryl is optionally substituted one or more times, in the same way or differently with halogen, hydroxyl, cyano, nitro, -$C(O)_2R^5$, or a -$NR^6R^7$ group, or
the moiety:

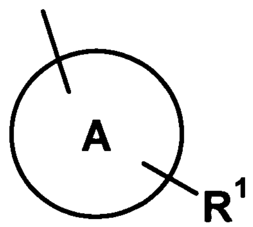

represents :

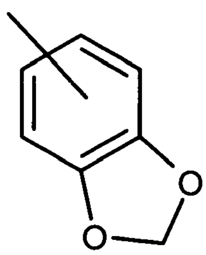

, or

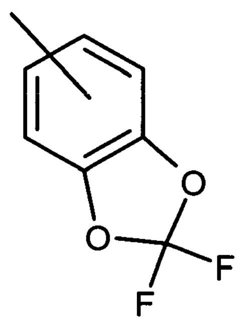

;

Z represents $C_1$-$C_6$-alkyl, in which $C_1$-$C_6$-alkyl is optionally substituted one or more times, in the same way or differently with halogen, hydroxyl, $C_1$-$C_6$-haloalkyl, $C_1$-$C_6$-alkoxy, $C_1$-$C_6$-haloalkyloxy, or a -$NR^6R^7$ group,
$R^2$ is hydrogen,
$R^3$ is hydrogen,
$R^4$ represents a substituent selected from the group comprising, preferably consisting of, hydrogen, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl,
$R^5$ represents a substituent selected from the group comprising, preferably consisting of, hydrogen, $C_1$-$C_6$-alkyl, $C_3$-$C_{10}$-cycloalkyl, $C_1$-$C_6$-haloalkyl, $C_1$-$C_6$-alkoxy, aryl, heteroaryl, $C_1$-$C_6$-alkoxy-$C_1$-$C_6$-alkyl, or halo-$C_1$-$C_6$-alkoxy-$C_1$-$C_6$-alkyl, wherein aryl or heteroaryl is optionally further substituted with the group $C_1$-$C_6$-alkyl, or$NR^6R^7$,
$R^6$ and $R^7$ independently from one another represent a substituent selected from the group comprising, pref-

erably consisting of, hydrogen, $C_1$-$C_6$-alkyl , $C_3$-$C_{10}$-cyctoalkyl, $C_1$-$C_6$-hatoatkyl, $C_1$-$C_6$-alkoxy, $C_1$-$C_6$-haloalkoxyalkyl, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with a hydroxy, or -$NR^8R^9$ group, or

$R^6$ and $R^7$ together with the nitrogen atom to which they are attached, form a 3 to 10 membered heterocycloalkyl ring, which heterocycloalkyl ring may optionally be interrupted one or more times, the same way or differently, with an atom selected from the group comprising, preferably consisting of, nitrogen, oxygen and/or sulfur and can optionally be interrupted one or more times, the same way or differently, with a -C(O)-, -S(O)- and/or -S$(O)_2$- group, and can optionally contain one or more double bonds, wherein said heterocycloalkyl ring is optionally substituted one or more times, the same way or differently with halogen, hydroxy, $C_1$-$C_6$-alkyl, $C_1$-$C_6$-haloalkyl, $C_1$-$C_6$-alkoxy, $C_1$-$C_6$-alkoxy-$C_1$-$C_6$-alkyl, halo-$C_1$-$C_6$-alkoxy-$C_1$-$C_6$-alkyl, -C(O)$R^5$, -$C(O)_2R^5$, -$NR^4C(O)R^5$, $NR^4S(O)_2R^5$,- C(O)$NR^8R^9$, -OC(O)$NR^8R^9$, -$NR^4C(O)_2R^5$, -$NR^4C(O)NR^8R^9$, -S(O)$R^5$, -$S(O)_2R^2$, or -S$(O)_2NR^8R^9$, wherein $C_1$-$C_6$-alkyl may be further optionally substituted with hydroxy,

$R^8$ and $R^9$ independently from one another, represent a substituent selected from the group comprising, preferably consisting of, hydrogen, $C_1$-$C_6$-alkyl , $C_3$-$C_{10}$-cycloalkyl, $C_1$-$C_6$-haloalkyl, $C_1$-$C_6$-alkoxy, $C_1$-$C_6$-alkoxy-$C_1$-$C_6$-alkyl, or halo-$C_1$-$C_6$-alkoxy-$C_1$-$C_6$-alkyl, wherein $C_1$-$C_6$-alkyl is optionally further substituted with hydroxy,

m represents an integer of 0, 1, 2, 3, or 4,

n represents an integer of 0, 1, 2, 3, or 4,

p represents an integer of 0, 1, 2, 3, or 4, and

q represents an integer of 0, 1, 2, 3, or 4,

as well as :

N-oxides, solvates, hydrates, isomers, diastereomers, enantiomers and salts thereof.

**7.** The compound according to any one of claims 1 to 6, which is selected from the group consisting of:

*N*-(2-Dimethylamino-ethyl)-3-[5-(4-isopropyl-phenylamino)-pyrazolo[1,5-a]pyrimidin-3-yl]-benzamide;
Phenyl-[3-(3,4,5-trimethoxy-phenyl)-pyrazolo[1,5-a]pyrimidin-5-yl]-amine;
(4-Fluoro-phenyl)-[3-(3,4,5-trimethoxy-phenyl)-pyrazolo[1,5-a]pyrimidin-5-yl]-amine;
(3-Pyridin-4-yl-pyrazolo[1,5-a]pyrimidin-5-yl)-(3,4,5-trimethoxyphenyl)-amine;
[3-(2,4-Dimethoxy-pyrimidin-5-yl)-pyrazolo[1,5-a]pyrimidin-5-yl]-(3,4,5-trimethoxy-phenyl)-amine;
(3-Benzofuran-2-yl-pyrazolo[1,5-a]pyrimidin-5-yl)-(3,4,5-trimethoxyphenyl)-amine;
(3-Benzo[b]thiophen-2-yl-pyrazolo[1,5-a]pyrimidin-5-yl)-(3,4, 5-trimethoxy-phenyl)-amine;
N-{3-[5-(3,4,5-Trimethoxy-phenylamino)-pyrazolo[1,5-a]pyrimidin-3-yl]-phenyl}-acetamide;
N-[4-(3-Benzo[1,3]dioxol-5-yl-pyrazolo[1,5-a]pyrimidin-5-ylamino)-phenyl]-acetamide;
(3-Benzo[1,3]dioxol-5-yl-pyrazolo[1,5-a]pyrimidin-5-yl)-(6-methoxy-pyridin-3-yl)-amine;
(b-Methoxy-pyridin-3-yl)-(3-quinolin-8-yl-pyrazolo[1,5-a]pyrimidin-5-yl)-amine;
4-[5-(6-Methoxy-pyridin-3-ylamino)-pyrazolo[1,5-a]pyrimidin-3-yl]-phenol;
(3-Benzo[1,3]dioxol-5-yl-pyrazolo[1,5-a]pyrimidin-5-yl)-(1H-indol-5-yl)-amine;
(3-Benzofuran-2-yl-pyrazolo[1,5-a]pyrimidin-5-yl)-(1 H-indol-5-yl)-amine;
(1 H-Indol-5-yl)- (3-thiophen- 3-yl-pyrazolo[1 ,5-a]pyrimidin-S-yl)-amine;
[3-(3,4-Dimethoxy-phenyl)-pyrazolo[1,5-a]pyrimidin-5-yl]-(1H-indol-5-yl)-amine;
4-[5-(1 H-Indol-5-ylamino)-pyrazolo[1,5-a]pyrimidin-3-yl] -phenol;
N-{3-[5-(1H-Indol-5-ylamino)-pyrazolo[1,5-a]pyrimidin-3-yl]-phenyl}-acetamide;
Benzo[1,3]dioxol-5-ylmethyl-[3-(2,4-dimethoxy-pyrimidin-5-yl)-pyrazolo[1,5-a]pyrimidin-5-yl]-amine;
Benzo[1,3]dioxol-5-ylmethyl-(3-pyridin-3-yl-pyrazolo[1,5-a]pyrimidin-5-yl)-amine;
Benzo[1, 3]dioxol-5-ylmethyl-(3-benzofuran-2-yl-pyrazolo[1, 5-a]pyrimidin-5-yl)-amine;
Benzo[1,3]dioxol- S-ylmethyl- (3-quinolin-8-yl-pyrazolo[1 , 5-a]pyrimidin-5-yl)-amine;
N-(3-{5-[(Benzo[1,3]dioxol-5-ylmethyl)-amino]-pyrazolo[1, 5-a]pyrimidin-3-yl}-phenyl)-acetamide;
[3-(3,4-Dimethoxy-phenyl)-pyrazolo[1,5-a]pyrimidin-5-yl]-(4-morpholin-4-yl-phenyl)-amine;
4-[5-(4-Morpholin-4-yl-phenylamino)-pyrazolo[1,5-a]pyrimidin-3-yl]-phenol;
N-{3-[5-(4-Morpholin-4-yl-phenylamino)-pyrazolo[1,5-a] pyrimidin-3-yl]-phenyl}-acetamide;
1-{5-[5-(4-Phenoxy-phenylamino)-pyrazolo[1,5-a]pyrimidin-3-yl]-thiophen-2-yl}-ethanone;
(4-Phenoxy-phenyl)-(3-quinolin-8-yl-pyrazolo[1,5-a]pyrimidin-5-yl)-amine;
[3-(3,4-Dimethoxy-phenyl)-pyrazolo[1,5-a]pyrimidin-5-yl]-(4-phenoxyphenyl)-amine;
1-{5-[5-(4-Isopropyl-phenylamino)-pyrazolo[1,5-a]pyrimidin-3-yl]-thiophen-2-yl}-ethanone;
(4-Isopropyl-phenyl)-(3-pyridin-4-yl-pyrazolo[1,5-a]pyrimidin-5-yl)-amine;
[3-(3,4-Dimethoxy-phenyl)-pyrazolo[1,5-a]pyrimidin-5-yl]-(4-isopropylphenyl)-amine;
3-[5-(4-Phenoxy-phenylamino)-pyrazolo[1,5-a]pyrimidin-3-yl]-benzamide;

4-[5-(4-Phenoxy-phenylamino)-pyrazolo[1,5-a]pyrimidin-3-yl]-benzoic acid methyl ester;
[3-(3-Methoxy-phenyl)-pyrazolo[1,5-a]pyrimidin-5-yl]-(4-phenoxyphenyl)-amine;
[3-(2,4-Dimethoxy-phenyl)-pyrazolo[1,5-a]pyrimidin-5-yl]-(4-phenoxyphenyl)-amine;
N-{3-[5-(4-Phenoxy-phenylamino)-pyrazolo[1,5-a]pyrimidin-3-yl]-phenyl}-methanesulfonamide;
[3-(1-Benzyl-1H-pyrazol-4-yl)-pyrazolo[1,5-a]pyrimidin-5-yl]-(4-phenoxyphenyl)-amine;
4-(3-Benzo[1,3]dioxol-5-yl-pyrazolo[1,5-a]pyrimidin-5-ylamino)-phenol;
4-[3-(2,4-Dimethoxy-pyrimidin-5-yl)-pyrazolo[1, 5-a]pyrimidin-5-ylamino]-phenol;
4-(3-Benzofuran-2-yl-pyrazolo[1,5-a]pyrimidin-5-ylamino)-phenol;
4-(3-Thiophen-3-yl-pyrazolo[1,5-a]pyrimidin-5-ylamino)-phenol;
4-[5-[(4-hydroxyphenyl)amino]pyrazolo[1,5-a]pyrimidin-3-yl]-phenol;
N-{3-[5-(4-Hydroxy-phenylamino)-pyrazolo[1,5-a]pyrimidin-3-yl]-phenyl}-acetamide;
4-Methyl-N-[4-(3-pyridin-4-yl-pyrazolo[1,5-a]pyrimidin-5-ylamino)-phenyl]-benzenesulfonamide;
4-Methyl-N-[4-(3-pyridin-3-yl-pyrazolo[1,5-a]pyrimidin-5-ylamino)-phenyl]-benzenesulfonamide;
N-[4-(3-Benzofuran-2-yl-pyrazolo[1,5-a]pyrimidin-5-ylamino)-phenyl]-4-methyl-benzenesulfonamide;
4-Methyl-N-[4-(3-thiophen-3-yl-pyrazolo[1,5-a]pyrimidin-5-ylamino)-phenyl]-benzenesulfonamide;
4-Methyl-N-[4-(3-quinolin-8-yl-pyrazolo[1,5-a]pyrimidin-5-ylamino)-phenyl]-benzenesulfonamide;
N-{4-[3-(3,4-Dimethoxy-phenyl)-pyrazolo[1,5-a]pyrimidin-5-ylamino]-phenyl}-4-methyl-benzenesulfonamide;
N-[5-(3-Benzofuran-2-yl-pyrazolo[1,5-a]pyrimidin-5-ylamino)-2-methylphenyl]-methanesulfonamide;
N-[5-(3-Benzo[b]thiophen-2-yl-pyrazolo[1,5-a]pyrimidin-5-ylamino)-2-methyl-phenyl]-methanesulfonamide;
N-[2-Methyl-5-(3-quinolin-8-yl-pyrazolo[1,5-a]pyrimidin-5-ylamino)-phenyl]-methanesulfonamide;
N-{5-[3-(4-Hydroxy-phenyl)-pyrazolo[1,5-a]pyrimidin-5-ylamino]-2-methyl-phenyl}-methanesulfonamide;
(3-Benzo[1,3]dioxol-5-yl-pyrazolo[1,5-a]pyrimidin-5-yl)-pyridin-4-ylmethyl-amine;
(3-Benzofuran-2-yl-pyrazolo[1,5-a]pyrimidin-5-yl)-pyridin-4-ylmethyl-amine;
Pyridin-4-ylmethyl-(3-thiophen-3-yl-pyrazolo[1,5-a] pyrimidin-5-yl)-amine;
(3-Benzo[b]thiophen-2-yl-pyrazolo[1, 5-a]pyrimidin-5-yl)-pyridin-4-ylmethyl-amine;
[3-(3,4-Dimethoxy-phenyl)-pyrazolo[1,5-a]pyrimidin-5-yl]-pyridin-4-ylmethyl-amine;
4-{5-[(Pyridin-4-ylmethyl)-amino]-pyrazolo[1,5-a]pyrimidin-3-yl}-phenol;
4-(3-Pyridin-3-yl-pyrazolo[1,5-a] pyrimidin-5-ylamino)-cyclohexanol;
4-(3-Quinolin-8-yl-pyrazolo[1,5-a]pyrimidin-5-ylamino)-cyclohexanol;
4-[3-(3,4-Dimethoxy-phenyl)-pyrazolo[1,5-a]pyrimidin-5-ylamino]-cyclohexanol;
2-(3-Benzo[1,3]dioxol-5-yl-pyrazolo[1,5-a]pyrimidin-5-ylamino)-ethanol;
2-(3-Benzofuran-2-yl-pyrazolo[1,5-a]pyrimidin-5-ylamino)-ethanol;
2-(3-Quinolin-8-yl-pyrazolo[1,5-a]pyrimidin-5-ylamino)-ethanol;
2-[3-(3,4-Dimethoxy-phenyl)-pyrazolo[1,5-a]pyrimidin-5-ylamino]-ethanol;
4-[5-(2-Hydroxy-ethylamino)-pyrazolo[1,5-a]pyrimidin-3-yl]-phenol;
3-{4-[5-(3,4, 5-Trimethoxy-phenylamino)-pyrazolo[1, 5-a] pyrimidin-3-yl]-phenyl}-propionic acid;
[3-(4-Trifluoromethoxy-phenyl)-pyrazolo[1,5-a]pyrimidin-5-yl]-(3,4,5-trimethoxy-phenyl)-amine;
(3,4,5-Trimethoxy-phenyl)-[3-(3,4, 5-trimethoxy-phenyl)-pyrazolo[1,5-a]pyrimidin-5-yl]-amine;
[3-(4-Chloro-phenyl)-pyrazolo[1,5-a]pyrimidin-5-yl]-(3,4,5-trimethoxyphenyl)-amine;
N-(2-Hydroxy-ethyl)-3-[5-(3,4,5-trimethoxy-phenylamino)-pyrazolo[1,5-a]pyrimidin-3-yl]-benzamide;
[3-(5-Methoxy-pyridin-3-yl)-pyrazolo[1,5-a]pyrimidin-5-yl]-(3,4, 5-trimethoxy-phenyl)-amine;
3-{4-[5-(4-Acetylamino-phenylamino)-pyrazolo[1,5-a]pyrimidin-3-yl]-phenyl}-propionic acid;
3-[5-(4-Acetylamino-phenylamino)-pyrazolo[1,5-a]pyrimidin-3-yl]-benzoic acid;
3-{4-[5-(4-Hydroxy-phenylamino)-pyrazolo[1,5-a]pyrimidin-3-yl]-phenyl}-propionic acid;
4-[3-(2-Methoxy-phenyl)-pyrazolo[1,5-a]pyrimidin-5-ylamino]-phenol;
4-[3-(4-Trifluoromethoxy-phenyl)-pyrazolo[1,5-a]pyrimidin-5-ylamino]-phenol;
4-[5-(4-Hydroxy-phenylamino)-pyrazolo[1,5-a]pyrimidin-3-yl]-benzonitrile;
40-[3-(5-Methoxy-pyridin-3-yl)-pyrazolo[1,5-a]pyrimidin-5-ylamino]-phenol;
[3-(2-Methoxy-phenyl)-pyrazolo[1,5-a]pyrimidin-5-yl]-(6-methoxy-pyridin-3-yl)-amine;
(6-Methoxy-pyridin- 3-yl)-[3-(4-trifluoromethoxy-phenyl)-pyrazolo[1,5-a]pyrimidin-5-yl]-amine;
(6-Methoxy-pyridin-3-yl)-[3-(3,4,5-trimethoxy-phenyl)-pyrazolo[1,5-a]pyrimidin-5-yl]-amine;
(6-Methoxy-pyridin-3-yl)-[3-(3-trifluoromethoxy-phenyl)-pyrazolo[1,5-a]pyrimidin-5-yl]-amine;
N-(2-Hydroxy-ethyl)-3-[5-(6-methoxy-pyridin-3-ylamino)-pyrazolo[1,5-a]pyrimidin-3-yl]-benzamide;
(6-Methoxy-pyridin-3-yl)-[3-(5-methoxy-pyridin-3-yl)-pyrazolo[1, 5-a]pyrimidin-5-yl]-amine;
N-{4-[3-(5-Methoxy-pyridin-3-yl)-pyrazolo[1,5-a]pyrimidin-5-ylamino]-phenyl}-4-methyl-benzenesulfonamide;
3-{4-[5-(4-Isopropyl-phenylamino)-pyrazolo[1,5-a]pyrimidin-3-yl]-phenyl}-propionic acid;
(4-Isopropyl-phenyl)-[3-(4-trifluoromethoxy-phenyl)-pyrazolo[1,5-a]pyrimidin-5-yl]-amine;
(4-lsopropyl-phenyl)- [3- (3 ,4, 5-trimethoxy-phenyl)-pyrazolo[1 ,5-a]pyrimidin-5-yl]-amine;

3-[5-(4-isopropyl-phenylamino)-pyrazolo[1,5-a]pyrimidin-3-yl]-benzoic acid;
[3-(4-Chloro-phenyl)-pyrazolo[1,5-a]pyrimidin-5-yl]-(4-isopropylphenyl)-amine;
(4-Isopropyl-phenyl)-[3-(3-trifluoromethoxy-phenyl)-pyrazolo[1,5-a]pyrimidin-5-yl]-amine;
N-(2-Hydroxy-ethyl)-3-[5-(4-isopropyl-phenylamino)-pyrazolo[1,5-a]pyrimidin-3-yl]-benzamide;
(4-Isopropyl-phenyl)-[3-(5-methoxy-pyridin-3-yl)-pyrazolo[1,5-a]pyrimidin-5-yl]-amine;
N-{5-[3-(2-Methoxy-phenyl)-pyrazolo[1,5-a]pyrimidin-5-ylamino]-2-methyl-phenyl}-methanesulfonamide;
N-{2- Methyl- 5-[3-(4- trifluoromethoxy- phenyl)-pyrazolo [1,5- a] pyrimidin- 5- ylamino]-phenyl}-methanesulfonamide;
N-{2-Methyl-5-[3-(3,4,5-trimethoxy-phenyl)-pyrazolo[1,5-a]pyrimidin-5-ylamino]-phenyl}-methanesulfonamide;
N-{2-Methyl-5-[3-(3-trifluoromethoxy-phenyl)-pyrazolo[1,5-a] pyrimidin-5-ylamino]-phenyl}-methanesulfonamide;
N-{5-[3-(5-Methoxy-pyridin-3-yl)-pyrazolo[1,5-a]pyrimidin-5-ylamino]-2-methyl-phenyl}-methanesulfonamide;
Benzo[1,3]dioxol-5-ylmethyl-[3-(2-methoxy-phenyl)-pyrazolo[1,5-a]pyrimidin-5-yl]-amine;
Benzo[1, 3]dioxol-5-ylmethyl-[3-(4-trifluoromethoxy-phenyl)-pyrazolo[1,5-a] pyrimidin-5-yl]-amine;
Benzo[1,3]dioxol-5-ylmethyl-[3-(5-methoxy-pyridin-3-yl)-pyrazolo[1,5-a]pyrimidin-5-yl]-amine;
[3-(2-Methoxy-phenyl)-pyrazolo[1,5-a]pyrimidin-5-yl]-pyridin-4-ylmethyl-amine;
Pyridin-4-ylmethyl-[3-(4-trifluoromethoxy-phenyl)-pyrazolo[1,5-a]pyrimidin-5-yl]-amine;
Pyridin-4-ylmethyl- [3- (3-trifluoromethoxy-phenyl)-pyrazolo[1 5-a]pyrimidin-5-yl]-amine;
3-{4-[5-(4-Hydroxy-cyclohexylamino)-pyrazolo[1,5-a]pyrimidin-3-yl]-phenyl}-propionic acid;
3-[5-(4-Hydroxy-cyclohexylamino)-pyrazolo[1, 5-a] pyrimidin-3-yl]-benzoic acid;
4-[3-(5-Methoxy-pyridin-3-yl)-pyrazolo[1,5-a]pyrimidin-5-ylamino]-cyclohexanol;
3-{4-[5-(2-Hydroxy-ethylamino)-pyrazolo[1,5-a]pyrimidin-3-yl]-phenyl}-propionic acid;
2 - [3-(4- Trifluoromethoxy-phenyl)-pyrazolo[1,5-a] pyrimidin-5-ylamino]-ethanol;
4-[5-(2-Hydroxy-ethylamino)-pyrazolo[1,5-a]pyrimidin-3-yl]-benzonitrile;
2-[3-(3,4, 5-Trimethoxy-phenyl)-pyrazolo[1,5-a] pyrimidin-5-ylamino]-ethanol;
2-[3-(4-Chloro-phenyl)-pyrazolo[1,5-a]pyrimidin-5-ylamino]-ethanol;
2-[3-(3-Trifluoromethoxy-phenyl)-pyrazolo[1,5-a]pyrimidin-5-ylamino]-ethanol;
2-[3-(5-Methoxy-pyridin-3-yl)-pyrazolo[1,5-a]pyrimidin-5-ylamino]-ethanol;
3-(4-{5-[(Pyridin-3-ylmethyl)-amino]-pyrazolo[1,5-a]pyrimidin-3-yl}-phenyl)-propionic acid;
[3-(2-Methoxy-phenyl)-pyrazolo[1,5-a]pyrimidin-5-yl]-pyridin-3-ylmethyl-amine;
Pyridin-3-ylmethyl-[3-(3,4,5-trimethoxy-phenyl)-pyrazolo[1,5-a]pyrimidin-5-yl]-amine;
[3-(3,5-Dimethoxy-phenyl)-pyrazolo[1,5-a]pyrimidin-5-yl]-(4-morpholin-4-yl-phenyl)-amine;
Pyridin-3-ylmethyl-[3-(3-trifluoromethoxy-phenyl)-pyrazolo[1,5-a]pyrimidin-5-yl]-amine;
(4-Morphol in-4-yl-phenyl)-[3-(3-trifluoromethoxy-phenyl)-pyrazolo[1 5-a]pyrimidin-5-yl]-amine;
N-(2-Hydroxy-ethyl)-3-[5-(4-morpholin-4-yl-phenylamino)-pyrazolo[1,5-a]pyrimidin-3-yl]-benzamide;
N'-[3-(2-Methoxy-phenyl)-pyrazolo[1,5-a]pyrimidin-5-yl]-N,N-dimethylethane-1,2-diamine;
N,N-Dimethyl-N'-[3-(4-trifluoromethoxy-phenyl)-pyrazolo[1,5-a]pyrimidin-5-yl]-ethane-1,2-diamine;
N,N-Dimethyl-N'-[3-(3,4,5-trimethoxy-phenyl)-pyrazolo[1,5-a]pyrimidin-5-yl]-ethane-1,2-diamine;
3-[5-(2-Dimethytamino-ethylamino)-pyrazolo[1,5-a]pyrimidin-3-yl]-benzoic acid;
4-[5-(2-Dimethylamino-ethylamino)-pyrazolo[1,5-a]pyrimidin-3-yl]-benzoic acid;
N'-[3-(4-Chloro-phenyl)-pyrazolo[1,5-a]pyrimidin-5-yl]-N,N-dimethylethane-1,2-diamine;
3-[5-(3,4,5-Trimethoxy-phenylamino)-pyrazolo[1,5-a]pyrimidin-3-yl]-phenol;
(E)-3-{3-[5-(3,4,5-Trimethoxy-phenylamino)-pyrazolo[1,5-a]pyrimidin-3-yl]-phenyl}-acrylic acid;
{2-[5-(3,4,5-Trimethoxy-phenylamino)-pyrazolo[1,5-a]pyrimidin-3-yl]-phenyl}-methanol;
[3-(6-Methoxy-pyridin-3-yl)-pyrazolo[1,5-a]pyrimidin-5-yl]-(3,4,5-trimethoxy-phenyl)-amine;
(3-Furan-3-yl-pyrazolo[1,5-a]pyrimidin-5-yl)-(3,4,5-trimethoxy-phenyl)-amine;
(3-Chloro-4-fluoro-phenyl)-[3-(3-methoxy-phenyl)-pyrazolo[1,5-a]pyrimidin-5-yl]-amine;
(3-Chloro-4-fluoro-phenyl)-[3-(2,4-dimethoxy-phenyl)-pyrazolo[1,5-a]pyrimidin-5-yl]-amine;
(3-Chloro-4-fluoro-phenyl)-(3-thiophen-2-yl-pyrazolo[1,5-a]pyrimidin-5-yl)-amine;
4-[3-(4-Methoxy-phenyl)-pyrazolo[1,5-a]pyrimidin-5-ylamino]-phenol;
4-[3-(6-Methoxy-pyridin-3-yl)-pyrazolo[1,5-a]pyrimidin-5-ylamino] - phenol;
3-[5-(6-Methoxy-pyridin-3-ylamino)-pyrazolo[1,5-a] pyrimidin-3-yl] - phenol;
(6-Methoxy-pyridin-3-yl)-[3-(6-methoxy-pyridin-3-yl)-pyrazolo[1,5-a]pyrimidin-5-yl]-amine;
N-{5- [3- (6-Methoxy-pyridin- 3-yl)-pyrazolo[1 5-a]pyrimidin-5-ytamino] -2-methyl-phenyl}-methanesulfonamide;
N-{5-[3-(4-Hydroxy-3-methoxy-phenyl)-pyrazolo[1,5-a]pyrimidin-5-ylamino]-2-methyl-phenyl}-methanesulfonamide;
N-{2-Methyl-5- [3-(4-methyl-thiophen-2-yl)-pyrazolo[l,5-a]pyrimidin-5-ylamino]-phenyl}-methanesulfonamide;
(2-{5- [(Benzo[1 ,3]dioxol-5-ylmethyl)-amino] -pyrazolo[1 5-a]pyrimidin-3-yl}-phenyl)-methanol;

[3-(4-Methoxy-phenyl)-pyrazolo[1,5-a]pyrimidin-5-yl]-pyridin-4-ylmethyl-amine;
4-[3-(4-Methoxy-phenyl)-pyrazolo[1,5-a]pyrimidin-5-ylamino]-cyclohexanol;
3-[5-(4-Hydroxy-cyclohexylamino)-pyrazolo[1,5-a]pyrimidin-3-yl]-phenol;
(E)-3-{3-[5-(4-Hydroxy-cyclohexylamino)-pyrazolo[1,5-a]pyrimidin-3-yl] - phenyl}-acrylic acid;
4-[3-(3-Hydroxymethyl-phenyl)-pyrazolo[1,5-a]pyrimidin-5-ylamino]-cyclohexanol;
4-[3-(2-Hydroxymethyl-phenyl)-pyrazolo[1,5-a]pyrimidin-5-ylamino]-cyclohexanol;
4-[5-(4-Hydroxy-cyclohexylamino)-pyrazolo[1,5-a]pyrimidin-3-yl]-2-methoxy-phenol;
2-[3-(4-Methoxy-phenyl)-pyrazolo[1,5-a]pyrimidin-5-ylamino]-ethanol;
(E)-3-{3-[5-(2-Hydroxy-ethylamino)-pyrazolo[1,5-a]pyrimidin-3-yl]-phenyl}-acrylic acid;
2-[3-(3-Hydroxymethyl-phenyl)-pyrazolo[1,5-a]pyrimidin-5-ylamino]-ethanol;
2-[3-(6-Methoxy-pyridin-3-yl)-pyrazolo[1,5-a]pyrimidin-5-ylamino]-ethanol;
4-[5-(2-Hydroxy-ethylamino)-pyrazolo[1,5-a]pyrimidin-3-yl]-2-methoxyphenol;
N-(4-Methoxy-phenyl)-4-{5-[(pyridin-3-ylmethyl)-amino]-pyrazolo[1,5-a]pyrimidin-3-yl}-benzamide;
[3-(4-Methoxy-phenyl)-pyrazolo[1,5-a]pyrimidin-5-yl]-pyridin-3-ylmethyl-amine;
3-{5-[(Pyridin-3-ylmethyl)-amino]-pyrazolo[1,5-a]pyrimidin-3-yl}-phenol;
(E)-3-(3-{5-[(Pyridin-3-ylmethyl)-amino]-pyrazolo[1,5-a]pyrimidin-3-yl}-phenyl)-acrylic acid;
(3-{5-[(Pyridin-3-ylmethyl)-amino]-pyrazolo[1,5-a]pyrimidin-3-yl}-phenyl)-methanol;
[3-(6-Methoxy-pyridin-3-yl)-pyrazolo[1,5-a]pyrimidin-5-yl]-pyridin-3-ylmethyl-amine;
[3-(4-Methyl-thiophen-2-yl)-pyrazolo[1,5-a]pyrimidin-5-yl]-pyridin-3-ylmethyl-amine;
[3-(4-Methoxy-phenyl)-pyrazolo[1,5-a]pyrimidin-5-yl]-(4-morpholin-4-yl-phenyl)-amine;
(E)-3-{3-[5-(4-Morpholin-4-yl-phenylamino)-pyrazolo[1,5-a]pyrimidin-3-yl]-phenyl}-acrylic acid;
N'-[3-(4-Methoxy-phenyl)-pyrazolo[1,5-a]pyrimidin-5-yl]-N,N-dimethylethane-1,2-diamine;
3-[5-(3,4,5-Trimethoxy-phenylamino)-pyrazolo[1, 5-a]pyrimidin-3-yl]-benzamide;
4-[5-(3,4,5-Trimethoxy-phenylamino)-pyrazolo[1,5-a]pyrimidin-3-yl]-benzoic acid methyl ester;
[3-(3-Methoxy-phenyl)-pyrazolo[1,5-a]pyrimidin-5-yl]-(3,4,5-trimethoxyphenyl)-amine;
1-{3-[5-(3,4,5-Trimethoxy-phenylamino)-pyrazolo[1,5-a]pyrimidin-3-yl]-phenyl}-ethanone;
(3-Thiophen-2-yl-pyrazolo[1,5-a]pyrimidin-5-yl)-(3,4,5-trimethoxyphenyl)-amine;
N-{3-[5-(3,4,5-Trimethoxy-phenylamino)-pyrazolo[1,5-a]pyrimidin-3-yl]-phenyl}-methanesulfonamide;
[3-(1-Methyl-1H-pyrazol-4-yl)-pyrazolo[1,5-a] pyrimidin-5-yl]-(3,4,5-trimethoxy-phenyl)-amine;
N-Cyclopropyl-4-[5-(3-methanesulfonylamino-4-methyl-phenylamino)-pyrazolo[1,5-a]pyrimidin-3-yl]-benzamide;
N-{2-Methyl-5-[3-(4-morpholin-4-yl-phenyl)-pyrazolo[1,5-a]pyrimidin-5-ylamino]-phenyl}-methanesulfonamide;
N-{5-[3-(4-Dimethylamino-phenyl)-pyrazolo[1,5-a]pyrimidin-5-ylamino]-2-methyl-phenyl}-methanesulfonamide;
N-{5-[3-(3-Amino-phenyl)-pyrazolo[1,5-a]pyrimidin-5-ytamino]-2-methylphenyl}-methanesulfonamide;
[3-(4-Morpholin-4-yl-phenyl)-pyrazolo[1,5-a]pyrimidin-5-yl]-phenylamine;
[3-(4-Dimethylamino-phenyl)-pyrazolo[1, 5-a]pyrimidin-5-yl]-phenylamine;
[3-(4-Amino-phenyl)-pyrazolo[1,5-a]pyrimidin-5-yl]-(4-phenoxy-phenyl)-amine;
[3-(4-Amino-phenyl)-pyrazolo[1,5-a]pyrimidin-5-yl]-(4-isopropyl-phenyl)-amine;
Benzo[1,3]dioxol-5-ylmethyl-[3-(5-isopropyl-2-methoxy-phenyl)-pyrazolo[1, 5-a] pyrimidin-5-yl]-amine;
4-[3-(3-Methoxy-phenyl)-pyrazolo[1,5-a]pyrimidin-5-ylamino]-phenol;
1-{3-[5-(4-Hydroxy-phenylamino)-pyrazolo[1,5-a]pyrimidin-3-yl]-phenyl} ethanone;
4-[3-(1-Methyl-1 H-pyrazol-4-yl)-pyrazolo[1,5-a]pyrimidin-5-ylamino]-phenol;
4-[5-(6-Methoxy-pyridin-3-ylamino)-pyrazolo[1,5-a]pyrimidin-3-yl]-benzoic acid methyl ester;
[3-(3-Methoxy-phenyl)-pyrazolo[1,5-a]pyrimidin-5-yl]-(6-methoxy-pyridin-3-yl)-amine;
N-{3-[5-(6-Methoxy-pyridin-3-ylamino)-pyrazolo[1,5-a]pyrimidin-3-yl]-phenyl}-methanesulfonamide;
(6-Methoxy-pyridin-3-yl)-[3-(1-methyl-1H-pyrazol-4-yl)-pyrazolo[1,5-a]pyrimidin-5-yl]-amine;
[3-(1-Benzyl-1H-pyrazol-4-yl)-pyrazolo[1,5-a]pyrimidin-5-yl]-(6-methoxy-pyridin-3-yl)-amine;
N-{4-[3-(3-Methoxy-phenyl)-pyrazolo[1,5-a]pyrimidin-5-ylamino]-phenyl}-4-methyl-benzenesulfonamide;
N-{4-[3-(3-Acetyl-phenyl)-pyrazolo[1,5-a]pyrimidin-5-ylamino]-phenyl}-4-methyl-benzenesulfonamide;
4-Methyl-N-[4-(3-thiophen-2-yl-pyrazolo[1,5-a]pyrimidin-5-ylamino)-phenyl]-benzenesulfonamide;
4-{5-[4-(4-Amino-benzoylamino)-phenylamino]-pyrazolo[1,5-a]pyrimidin-3-yl}-benzoic acid methyl ester;
4-Amino-N-{4-[3-(1-benzyl-1 H-pyrazol-4-yl)-pyrazolo[1, 5-a]pyrimidin-5-ylamino]-phenyl}-benzamide;
N-[2-Methyl-5-(3-pyrimidin-5-yl-pyrazolo[1,5-a]pyrimidin-5-ylamino)-phenyl]-methanesulfonamide;
N-{5-[3-(3-Methoxy-phenyl)-pyrazolo[1,5-a]pyrimidin-5-ylamino]-2-methyl-phenyl}-methanesulfonamide;
N-{5-[3-(2,4-Dimethoxy-phenyl)-pyrazolo[1,5-a]pyrimidin-5-ylamino]-2-methyl-phenyl}-methanesulfonamide;
N-[2-Methyl-5-(3-thiophen-2-yl-pyrazolo[1,5-a]pyrimidin-5-ylamino)-phenyl]-methanesulfonamide;

N-{5-[3-(3-Methanesulfonylamino-phenyl)-pyrazolo[1,5-a]pyrimidin-5-ylamino]-2-methyl-phenyl}-methanesulfonamide;
N-{2-Methyl-5-[3-(1-methyl-1H-pyrazol-4-yl)-pyrazolo[1,5-a]pyrimidin-5-ylamino]-phenyl}-methanesulfonamide;
N-(3-{5-[(Benzo[1,3]dioxol-5-ytmethyl)-amino]-pyrazolo[1,5-a]pyrimidin-3-yl}-phenyl)-methanesulfonamide;
Benzo[1,3]dioxol-5-ylmethyl-[3-(1-benzyl-1H-pyrazol-4-yl)-pyrazolo[1,5-a]pyrimidin-5-yl]-amine;
3-{5-[(Pyridin-4-ylmethyl)-amino]-pyrazolo[1,5-a]pyrimidin-3-yl}-benzamide;
4-{5-[(Pyridin-4-ylmethyl)-amino]-pyrazolo[1,5-a]pyrimidin-3-yl}-benzoic acid methyl ester;
[3-(3-Methoxy-phenyl)-pyrazolo[1,5-a]pyrimidin-5-yl]-pyridin-4-ylmethyl-amine;
1-(3-{5-[(Pyridin-4-ylmethyl)-amino]-pyrazolo[1,5-a]pyrimidin-3-yl}-phenyl)-ethanone;
Pyridin-4-ylmethyl-(3-thiophen-2-yl-pyrazolo[1,5-a]pyrimidin-5-yl)-amine;
[3-(1H-Pyrazol-4-yl)-pyrazolo[1,5-a]pyrimidin-5-yl]-pyridin-4-ylmethyl-amine;
[3-(1-Benzyl-1H-pyrazol-4-yl)-pyrazolo[1,5-a]pyrimidin-5-yl]-pyridin-4-ylmethyl-amine;
3-[5-(4-Hydroxy-cyclohexylamino)-pyrazolo[1,5-a]pyrimidin-3-yl]-benzamide;
4-[3-(1-Methyl-1H-pyrazol-4-yl)-pyrazolo[1,5-a]pyrimidin-5-ylamino]-cyclohexanol;
4-[3-(1-Benzyl-1H-pyrazol-4-yl)-pyrazolo[1,5-a]pyrimidin-5-ylamino]-cyclohexanol;
3-{5-[(Pyridin-3-ylmethyl)-amino]-pyrazolo[1,5-a]pyrimidin-3-yl}-benzamide;
4-{5-[(Pyridin-3-ylmethyl)-amino]-pyrazolo[1,5-a]pyrimidin-3-yl}-benzoic acid methyl ester;
[3-(2,6-Dimethoxy-phenyl)-pyrazolo[1,5-a]pyrimidin-5-yl]-pyridin-3-ylmethyl-amine;
[3-(3-Methoxy-phenyl)-pyrazolo[1, 5-a]pyrimidin-5-yl]-pyridin-3-ylmethyl-amine;
1-(3-{5-[(Pyridin-3-ylmethyl)-amino]-pyrazolo[1,5-a]pyrimidin-3-yl}-phenyl)-ethanone;
Pyridin-3-ytmethyl-(3-thiophen-2-yl-pyrazolo[1,5-a]pyrimidin-5-yl)-amine;
N-(3-{5-[(Pyridin-3-ylmethyl)-amino]-pyrazolo[1,5-a]pyrimidin-3-yl}-phenyl)-methanesulfonamide;
[3-(1-Methyl-1H-pyrazol-4-yt)-pyrazolo[1,5-a]pyrimidin-5-yl]-pyridin-3-ylmethyl-amine;
[3-(1-Benzyl-1H-pyrazol-4-yl)-pyrazolo[1,5-a]pyrimidin-5-yl]-pyridin-3-ylmethyl-amine;
3-[5-(4-Morpholin-4-yl-phenylamino)-pyrazolo[1, 5-a]pyrimidin-3-yl]-benzamide;
[3-(3-Methoxy-phenyl)-pyrazolo[1, 5-a]pyrimidin-5-yl]-(4-morpholin-4-ylphenyl)-amine;
(4-Morpholin-4-yl-phenyl)-(3-thiophen-2-yl-pyrazolo[1,5-a]pyrimidin-5-yl)-amine;
[3-(1-Methyl-1H-pyrazol-4-yl)-pyrazolo[1,5-a]pyrimidin-5-yl]-(4-morpholin-4-yl-phenyl)-amine;
4-[5-(3-Chloro-phenylamino)-pyrazolo[1,5-a]pyrimidin-3-yl]-benzoic acid methyl ester;
(3-Chloro-phenyl)-(3-thiophen-2-yl-pyrazolo[1,5-a]pyrimidin-5-yl)-amine;
N-{3-[5-(3-Chloro-phenylamino)-pyrazolo[1,5-a]pyrimidin-3-yl]-phenyl}-methanesulfonamide;
[3-(1-Benzyl-1 H-pyrazol-4-yl)-pyrazolo[1,5-a]pyrimidin-5-yl]-(3-chlorophenyl)-amine;
4-(5-Phenylamino-pyrazolo[1,5-a]pyrimidin-3-yl)-benzoic acid methyl ester;
[3-(3-Methoxy-phenyl)-pyrazolo[1,5-a]pyrimidin-5-yl]-phenyl-amine;
Phenyl-(3-thiophen-2-yl-pyrazolo[1,5-a]pyrimidin-5-yl)-amine;
N-[3-(5-Phenylamino-pyrazolo[1,5-a]pyrimidin-3-yl)-phenyl]-methanesulfonamide;
[3-(1-Methyl-1H-pyrazol-4-yl)-pyrazolo[1,5-a]pyrimidin-5-yl]-phenylamine;
3-[4-(5-Phenylamino-pyrazolo[1,5-a]pyrimidin-3-yl)-phenyl]-propionic acid;
[3-(2-Methoxy-phenyl)-pyrazolo[1,5-a]pyrimidin-5-yl]-phenyl-amine;
Phenyl-[3-(4-trifluoromethoxy-phenyl)-pyrazolo[1,5-a]pyrimidin-5-yl]-amine;
4-(5-Phenylamino-pyrazolo[1,5-a]pyrimidin-3-yl)-benzoic acid;
[3-(4-Chloro-phenyl)-pyrazolo[1, 5-a] pyrimidin-5-yl]-phenyl-amine;
Phenyl-[3-(3-trifluoromethoxy-phenyl)-pyrazolo[1,5-a]pyrimidin-5-yl]-amine;
[3-(5-Methoxy-pyridin-3-yl)-pyrazolo[1,5-a] pyrimidin-5-yl]-phenylamine;
[3-(2-Methoxy-phenyl)-pyrazolo[1,5-a]pyrimidin-5-yl]-(4-phenoxyphenyl)-amine;
(4-Phenoxy-phenyl)-[3-(4-trifluoromethoxy-phenyl)-pyrazolo[1,5-a] pyrimidin-5-yl] -amine;
(4-Phenoxy-phenyl)-[3-(3,4, 5-trimethoxy-phenyl)-pyrazolo[1, 5-a]pyrimidin-5-yl]-amine;
4-[5-(4-Phenoxy-phenylamino)-pyrazolo[1,5-a]pyrimidin-3-yl]-benzoic acid;
[3-(4-Chloro-phenyl)-pyrazolo[1,5-a]pyrimidin-5-yl]-(4-phenoxy-phenyl)-amine;
(4-Phenoxy-phenyl)- [3- (3-trifluoromethoxy-phenyl)-pyrazolo[1 5-a]pyrimidin-5-yl]-amine;
[3-(5-Methoxy-pyridin-3-yl)-pyrazolo[1,5-a]pyrimidin-5-yl]-(4-phenoxyphenyl)-amine;
(3-Chloro-4-fluoro-phenyl)-[3-(4-trifluoromethoxy-phenyl)-pyrazolo[1,5-a]pyrimidin-5-yl]-amine;
(3-Chloro-4-fluoro-phenyl)-[3-(3,4,5-trimethoxy-phenyl)-pyrazolo[1,5-a]pyrimidin-5-yl]-amine;
(3-Chloro-4-fluoro-phenyl)-[3-(3-trifluoromethoxy-phenyl)-pyrazolo[1,5-a]pyrimidin-5-yl]-amine;
3-[5-(3-Chloro-4-fluoro-phenylamino)-pyrazolo[1,5-a]pyrimidin-3-yl]-N-(2-hydroxy-ethyl)-benzamide;
(3-Chloro-4-fluoro-phenyl)-[3-(5-methoxy-pyridin-3-yl)-pyrazolo[1,5-a]pyrimidin-5-yl]-amine;
4-[5-(4-Isopropyl-phenylamino)-pyrazolo[1,5-a]pyrimidin-3-yl]-benzoic acid methyl ester;

(4-Isopropyl-phenyl)-[3-(3-methoxy-phenyl)-pyrazolo[1,5-a]pyrimidin-5-yl]-amine;
[3-(2,4-Dimethoxy-phenyl)-pyrazolo[1,5-a]pyrimidin-5-yl]-(4-isopropylphenyl)-amine;
1-{3-[5-(4-Isopropyl-phenylamino)-pyrazolo[1,5-a]pyrimidin-3-yl]-phenyl}-ethanone;
(4-isopropyl-phenyl)-(3-thiophen-2-yl-pyrazolo[1,5-a]pyrimidin-5-yl)-amine;
N-{3-[5-(4-Isopropyl-phenylamino)-pyrazolo[1,5-a]pyrimidin-3-yl]-phenyl}-methanesulfonamide;
[3-(1-Benzyl-1 H-pyrazol-4-yl)-pyrazolo[1,5-a]pyrimidin-5-yl]-(4-isopropyl-phenyl)-amine;
[3-(4-Methoxy-phenyl)-pyrazolo[1,5-a]pyrimidin-5-yl]-phenyl-amine;
3-(5-Phenylamino-pyrazolo[1,5-a]pyrimidin-3-yl)-phenol;
[3-(5-Phenylamino-pyrazolo[1,5-a]pyrimidin-3-yl)-phenyl]-methanol;
(4-Isopropyl-phenyl)-[3-(4-methoxy-phenyl)-pyrazolo[1,5-a]pyrimidin-5-yl]-amine;
3-[5-(4-Isopropyl-phenylamino)-pyrazolo[1,5-a]pyrimidin-3-yl]-phenol;
{3-[5-(4-Isopropyl-phenylamino)-pyrazolo[1,5-a]pyrimidin-3-yl]-phenyl}-methanol;
{2-[5-(4-Isopropyl-phenylamino)-pyrazolo[1,5-a]pyrimidin-3-yl]-phenyl}-methanol;
4- [5-(4-Isopropyl-phenylamino)-pyrazolo[1,5-a]pyrimidin-3-yl]-2-methoxy-phenol;
Phenyl-(3-pyridin-3-yl-pyrazolo[1,5-a]pyrimidin-5-yl)-amine;
(3-Benzofuran-2-yl-pyrazolo[1,5-a]pyrimidin-5-yl)-phenyl-amine;
Phenyl-(3-thiophen-3-yl-pyrazolo[1,5-a]pyrimidin-5-yl)-amine;
Phenyl-(3-quinolin-8-yl-pyrazolo[1,5-a]pyrimidin-5-yl)-amine;
4-(5-Phenylamino-pyrazolo[1,5-a]pyrimidin-3-yl)-phenol;
N-[3-(5-Phenylamino-pyrazolo[1,5-a]pyrimidin-3-yl)-phenyl]-acetamide;
(3-Chloro-4-fluoro-phenyl)-(3-pyridin-3-yl-pyrazolo[1,5-a]pyrimidin-5-yl)-amine;
(3-Benzofuran-2-yl-pyrazolo[1,5-a]pyrimidin-5-yl)-(3-chloro-4-fluorophenyl)-amine;
(3-Chloro-4-fluoro-phenyl)-(3-thiophen-3-yl-pyrazolo[1,5-a]pyrimidin-5-yl)-amine;
(3-Chloro-4-fluoro-phenyl)-(3-quinolin-8-yl-pyrazolo[1,5-a]pyrimidin-5-yl)-amine;
4-[5-(3-Chloro-4-fluoro-phenylamino)-pyrazolo[1,5-a]pyrimidin-3-yl]-phenol;
1-(5-{5-[(Pyridin-3-ylmethyl)-amino]-pyrazolo[1,5-a]pyrimidin-3-yl}-thiophen-2-yl)-ethanone;
(3-Benzo[1,3]dioxol-5-yl-pyrazolo[1,5-a] pyrimidin-5-yl)-pyridin-3-ylmethyl-amine;
Pyridin-3-ylmethyl-(3-thiophen-3-yl-pyrazolo[1,5-a]pyrimidin-5-yl)-amine;
[3-(3,4-Dimethoxy-phenyl)-pyrazolo[1,5-a]pyrimidin-5-yl]-pyridin-3-ylmethyl-amine;
4-{5-[(Pyridin-3-ylmethyl)-amino]-pyrazolo[1,5-a]pyrimidin-3-yl}-phenol;
N-(3-{5-[(Pyridin-3-ylmethyl)-amino]-pyrazolo[1,5-a]pyrimidin-3-yl}-phenyl)-acetamide;
1-{5-[5-(2-Dimethylamino-ethylamino)-pyrazolo[1,5-a]pyrimidin-3-yl]-thiophen-2-yl}-ethanone;
N'-(3-Benzo[1,3]dioxol-5-yl-pyrazolo[1,5-a]pyrimidin-5-yl)-N,N-dimethylethane-1,2-diamine;
N,N-Dimethyl-N'-(3-thiophen-3-yl-pyrazolo[1,5-a]pyrimidin-5-yl)-ethane-1,2-diamine;
N'-[3-(3,4-Dimethoxy-phenyl)-pyrazolo[1,5-a]pyrimidin-5-yl]-N,N-dimethyl-ethane-1,2-diamine;
1-{5-[5-(3-Chloro-phenylamino)-pyrazolo[1,5-a]pyrimidin-3-yl]-thiophen-2-yl}-ethanone;
(3-Benzo[1,3]dioxol-5-yl-pyrazolo[1,5-a]pyrimidin-5-yl)-(3-chlorophenyl)-amine;
(3-Chloro-phenyl)-(3-pyridin-3-yl-pyrazolo[1,5-a]pyrimidin-5-yl)-amine;
(3-Chloro-phenyl)-(3-thiophen-3-yl-pyrazolo[1,5-a]pyrimidin-5-yl)-amine;
(3-Chloro-phenyl)-(3-quinolin-8-yl-pyrazolo[1,5-a]pyrimidin-5-yl)-amine;
(3-Chloro-phenyl)-[3-(2-methoxy-phenyl)-pyrazolo[1,5-a]pyrimidin-5-yl]-amine;
(3-Chloro-phenyl)-[3-(4-trifluoromethoxy-phenyl)-pyrazolo[1,5-a]pyrimidin-5-yl]-amine;
(3-Chloro-phenyl)-[3-(3,4,5-trimethoxy-phenyl)-pyrazolo[1,5-a]pyrimidin-5-yl]-amine;
(3-Chloro-phenyl)-[3-(4-chloro-phenyl)-pyrazolo[1,5-a]pyrimidin-5-yl]-amine;
(3-Chloro-phenyl)-[3-(3-trifluoromethoxy-phenyl)-pyrazolo[1,5-a]pyrimidin-5-yl]-amine;
(3-Chloro-phenyl)-[3-(5-methoxy-pyridin-3-yl)-pyrazolo[1,5-a]pyrimidin-5-yl]-amine;
(3-Chloro-phenyl)-[3-(4-methoxy-phenyl)-pyrazolo[1,5-a]pyrimidin-5-yl]-amine;
3-[5-(3-Chloro-phenylamino)-pyrazolo[1, 5-a]pyrimidin-3-yl]-phenol;
{3-[5-(3-Chloro-phenylamino)-pyrazolo[1,5-a]pyrimidin-3-yl]-phenyl}-methanol;
{2-[5-(3-Chloro-phenylamino)-pyrazolo[1,5-a]pyrimidin-3-yl]-phenyl}-methanol;
4-[5-(3-Chloro-phenylamino)-pyrazolo[1,5-a]pyrimidin-3-yl]-2-methoxyphenol;
[3-(3-Amino-phenyl)-pyrazolo[1,5-a]pyrimidin-5-yl]-(3-chloro-phenyl)-amine;
(3-Chloro-phenyl)-[3-(3-dimethylamino-phenyl)-pyrazolo[1,5-a]pyrimidin-5-yl]-amine;
3-[5-(3-Chloro-phenylamino)-pyrazolo[1,5-a]pyrimidin-3-yl]-N-(2-dimethylamino-ethyl)-benzamide;
4-[5-(3-Chtoro-phenylamino)-pyrazolo[1, 5-a]pyrimidin-3-yl]-N-(2-dimethylamino-ethyl)-benzamide;
2-[3-(4-Methyl-thiophen-2-yl)-pyrazolo[1, 5-a]pyrimidin-5-ylamino]-ethanol;
N-(2-Dimethylamino-ethyl)-4-{5-[4-(toluene-4-sulfonylamino)-phenylamino] -pyrazolo[1 ,5-a] pyrimidin- 3-yl}-benzamide;

N-(2-Dimethylamino-ethyl)-4-{5-[(pyridin-3-ylmethyl)-amino]-pyrazolo[1,5-a]pyrimidin-3-yl}-benzamide;
[3-(3-Aminomethyl-phenyl)-pyrazolo[1,5-a]pyrimidin-5-yl]-(3,4,5-trimethoxy-phenyl)-amine;
[3-(3-Aminomethyl-phenyl)-pyrazolo[1,5-a]pyrimidin-5-yl]-(4-phenoxyphenyl)-amine;
N-(2-Dimethylamino-ethyl)-3-[5-(3,4,5-trimethoxy-phenylamino)-pyrazolo[1,5-a]pyrimidin-3-yl]-benzamide;
N-(2-Dimethylamino-ethyl)-3-[5-(6-methoxy-pyridin-3-ylamino)-pyrazolo[1,5-a]pyrimidin-3-yl]-benzamide;
N-(2-Dimethylamino-ethyl)-3-[5-(4-hydroxy-phenylamino)-pyrazolo[1,5-a]pyrimidin-3-yl]-benzamide;
3-{5-[(Benzo[1,3]dioxol-5-ylmethyl)-amino]-pyrazolo[1,5-a]pyrimidin-3-yl}-N-(2-dimethylamino-ethyl)-benzamide;
N-(2-Dimethylamino-ethyl)-3-{5-[(pyridin-3-ylmethyl)-amino]-pyrazolo[1,5-a]pyrimidin-3-yl}-benzamide;
N-(2-Dimethylamino-ethyl)-3-[5-(4-phenoxy-phenylamino)-pyrazolo[1,5-a]pyrimidin-3-yl]-benzamide;
[3-(3-Dimethylamino-phenyl)-pyrazolo[1,5-a]pyrimidin-5-yl]-(3,4,5-trimethoxy-phenyl)-amine;
N-{4-[3-(3-Dimethylamino-phenyl)-pyrazolo[1,5-a]pyrimidin-5-ylamino]-phenyl}-acetamide;
[3-(3-Dimethylamino-phenyl)-pyrazolo[1,5-a]pyrimidin-5-yl]-(6-methoxy-pyridin-3-yl)-amine;
4-[3-(3-Dimethylamino-phenyl)-pyrazolo[1,5-a]pyrimidin-5-ylamino]-phenol;
[3-(3-Dimethylamino-phenyl)-pyrazolo[1,5-a]pyrimidin-5-yl]-pyridin-3-ylmethyl-amine;
[3-(3-Dimethylamino-phenyl)-pyrazolo[1,5-a]pyrimidin-5-yl]-(4-phenoxyphenyl)-amine;
[3-(3-Dimethylamino-phenyl)-pyrazolo[1,5-a]pyrimidin-5-yl]-(4-isopropyl-phenyl)-amine;
[3-(3-Amino-phenyl)-pyrazolo[1,5-a]pyrimidin-5-yl]-(6-methoxy-pyridin-3-yl)-amine;
4-[3-(3-Amino-phenyt)-pyrazolo[1,5-a]pyrimidin-5-ylamino]-phenol;
N-{4-[3-(3-Amino-phenyl)-pyrazolo[1,5-a]pyrimidin-5-ylamino]-phenyl}-4-methyl-benzenesulfonamide;
[3-(3-Amino-phenyl)-pyrazolo[1,5-a]pyrimidin-5-yl]-pyridin-3-ylmethyl-amine;
[3-(3-Amino-phenyl)-pyrazolo[1,5-a]pyrimidin-5-yl]-(4-phenoxy-phenyl)-amine;
[3-(3-Amino-phenyl)-pyrazolo[1,5-a]pyrimidin-5-yl]-(4-isopropyl-phenyl)-amine;
[3-(4-Dimethylamino-phenyl)-pyrazolo[1,5-a]pyrimidin-5-yl]-(3,4,5-trimethoxy-phenyl)-amine;
[3-(4-Dimethylamino-phenyl)-pyrazolo[1,5-a]pyrimidin-5-yl]-(6-methoxy-pyridin-3-yl)-amine;
4-[3-(4-Dimethylamino-phenyl)-pyrazolo[1,5-a]pyrimidin-5-ylamino]-phenol;
N-{4-[3-(4-Dimethylamino-phenyl)-pyrazolo[1,5-a]pyrimidin-5-ylamino]-phenyl}-4-methyl-benzenesulfonamide;
4-[3-(4-Dimethylamino-phenyl)-pyrazolo[1, 5-a]pyrimidin-5-ylamino]-cyclohexanol;
[3-(4-Dimethylamino-phenyl)-pyrazolo[1,5-a]pyrimidin-5-yl]-(4-isopropyl-phenyl)-amine;
[3-(4-Morpholin-4-yl-phenyl)-pyrazolo[1,5-a]pyrimidin-5-yl]-(3,4,5-trimethoxy-phenyl)-amine;
(6-Methoxy-pyridin-3-yl)-[3-(4-morpholin-4-yl-phenyl)-pyrazolo[1,5-a]pyrimidin-5-yl]-amine;
(4-Isopropyl-phenyl)-[3-(4-morpholin-4-yl-phenyl)-pyrazolo[1,5-a]pyrimidin-5-yl]-amine;
[3-(4-Amino-phenyl)-pyrazolo[1,5-a]pyrimidin-5-yl]-(3,4,5-trimethoxyphenyl)-amine;
(3-Naphthalen-2-yl-pyrazolo[1,5-a]pyrimidin-5-yl)-(3,4,5-trimethoxyphenyl)-amine;
[3-(3,5-Bis-trifluoromethyl-phenyl)-pyrazolo[1,5-a]pyrimidin-5-yl]-(3,4,5-trimethoxy-phenyl)-amine;
(3-Phenyl-pyrazolo[1,5-a] pyrimidin-5-yl)-(3,4,5-trimethoxy-phenyl)-amine;
[3-(2-Phenoxy-phenyl)-pyrazolo[1,5-a]pyrimidin-5-yl]-(3,4,5-trimethoxyphenyl)-amine;
[3-(3-Chloro-4-fluoro-phenyl)-pyrazolo[1,5-a]pyrimidin-5-yl]-(3,4,5-trimethoxy-phenyl)-amine;
[3-(2,3-Dichloro-phenyl)-pyrazolo[1,5-a]pyrimidin-5-yl]-(3,4,5-trimethoxy-phenyl)-amine;
[3-(4-Amino-phenyl)-pyrazolo[1,5-a]pyrimidin-5-yl]-(6-methoxy-pyridin-3-yl)-amine;
(6-Methoxy-pyridin-3-yl)-(3-naphthalen-2-yl-pyrazolo[1,5-a]pyrimidin-5-yl)-amine;
[3-(3,5-Bis-trifluoromethyl-phenyl)-pyrazolo[1,5-a]pyrimidin-5-yl]-(6-methoxy-pyridin-3-yl)-amine;
(6-Methoxy-pyridin-3-yl)-(3-phenyl-pyrazolo[1,5-a]pyrimidin-5-yl)-amine;
(6-Methoxy-pyridin-3-yl)-(3-naphthalen-1-yl-pyrazolo[1,5-a]pyrimidin-5-yl)-amine;
(6-Methoxy-pyridin-3-yl)-[3-(2-phenoxy-phenyl)-pyrazolo[1,5-a]pyrimidin-5-yl]-amine;
[3-(3-Chloro-4-fluoro-phenyl)-pyrazolo[1,5-a]pyrimidin-5-yl]-(6-methoxy-pyridin-3-yl)-amine;
[3-(3,4-Dimethyl-phenyl)-pyrazolo[1,5-a] pyrimidin-5-yl]-(6-methoxy-pyridin-3-yl)-amine;
[3-(2,3-Dichloro-phenyl)-pyrazolo[1,5-a]pyrimidin-5-yl]-(6-methoxy-pyridin-3-yl)-amine;
Benzo[1,3]dioxol-5-ylmethyl-(3-naphthalen-1-yl-pyrazolo[1,5-a]pyrimidin-5-yl)-amine;
Benzo[1,3]dioxol-5-ylmethyl-[3-(2-phenoxy-phenyl)-pyrazolo[1,5-a]pyrimidin-5-yl]-amine;
4-[3-(4-Amino-phenyl)-pyrazolo[1,5-a]pyrimidin-5-ylamino]-cyclohexanol;
4-(3-Naphthalen-2-yl-pyrazolo[1, 5-a]pyrimidin-5-ylamino)-cyclohexanol;
4- [3- (2 -Phenoxy-phenyl)-pyrazolo[1 ,5-a] pyrimidin-5-ylamino]-cyclohexanol;
4-[3-(3-Chloro-4-fluoro-phenyl)-pyrazoto[1, 5-a]pyrimidin-5-ylamino]-cyclohexanol;
(3-Naphthalen-2-yl-pyrazolo[1,5-a]pyrimidin-5-yl)-pyridin-3-ylmethyl-amine;
[3-(3,5-Bis-trifluoromethyl-phenyl)-pyrazolo[1,5-a]pyrimidin-5-yl]-pyridin-3-ylmethyl-amine;
(3-Phenyl-pyrazolo[1,5-a]pyrimidin-5-yl)-pyridin-3-ylmethyl-amine;

(3-Naphthalen-1-yl-pyrazolo[1,5-a]pyrimidin-5-yl)-pyridin-3-ylmethyl-amine;
[3-(2-Phenoxy-phenyl)-pyrazolo[1,5-a]pyrimidin-5-yl]-pyridin-3-ylmethyl-amine;
[3-(3-Chloro-4-fluoro-phenyl)-pyrazolo[1,5-a]pyrimidin-5-yl]-pyridin-3-ylmethyl-amine;
[3-(3,4-Dimethyl-phenyl)-pyrazolo[1,5-a]pyrimidin-5-yl]-pyridin-3-ylmethyl-amine;
[3-(2,3-Dichloro-phenyl)-pyrazolo[1,5-a]pyrimidin-5-yl]-pyridin-3-ylmethyl-amine;
[3-(3-Chloro-phenyl)-pyrazolo[1, 5-a]pyrimidin-5-yl] -(3,4, 5-trimethoxyphenyt)-amine;
[3-(5-Isopropyl-2-methoxy-phenyl)-pyrazolo[1, 5-a]pyrimidin-5-yl]-(3,4, 5-trimethoxy-phenyl)-amine;
[3-(3-Trifluoromethyl-phenyl)-pyrazolo[1, 5-a] pyrimidin-5-yl]-(3,4, 5-trimethoxy-phenyl)-amine;
[3-(3-Fluoro-phenyl)-pyrazolo[1 ,5-a]pyrimidin- 5-yl] -(3 ,4, 5-trimethoxyphenyl)-amine;
[3-(4-Fluoro-phenyl)-pyrazolo[1,5-a]pyrimidin-5-yl]-(3,4,5-trimethoxyphenyl)-amine;
[3-(4-Phenoxy-phenyl)-pyrazolo[1,5-a]pyrimidin-5-yl]-(3,4,5-trimethoxyphenyl)-amine;
[3-(2-Chloro-phenyl)-pyrazolo[1,5-a]pyrimidin-5-yl]-(3,4,5-trimethoxyphenyl)-amine;
[3-(2-Fluoro-phenyl)-pyrazolo[1,5-a]pyrimidin-5-yl]-(3,4, 5-trimethoxyphenyl)-amine;
(3-p-Tolyl-pyrazolo[1,5-a]pyrimidin-5-yl)-(3,4,5-trimethoxy-phenyl)-amine;
N-{5-[3-(3-Chloro-phenyl)-pyrazoto[1, 5-a]pyrimidin-5-ylamino]-2-methyl-phenyl}-methanesulfonamide;
N-{5-[3-(5-Isopropyl-2-methoxy-phenyl)-pyrazolo[1,5-a]pyrimidin-5-ylamino]-2-methyl-phenyl}-methanesutfonamide;
N-{2 -Methyl- 5- [3- (3-trifluoromethyl-phenyl)-pyrazolo[1 ,5-a] pyrimidin-5-ylamino] -phenyl}-methanesulfonamide;
N-{5-[3-(3-Fluoro-phenyl)-pyrazolo[1,5-a]pyrimidin-5-ylamino]-2-methylphenyl}-methanesulfonamide;
[3-(3-Chloro-phenyl)-pyrazolo[1, 5-a]pyrimidin-5-yl]-pyridin-4-ylmethyl-amine;
[3-(5-Isopropyl-2-methoxy-phenyl)-pyrazolo[1,5-a]pyrimidin-5-yl]-pyridin-4-ylmethyl-amine;
Pyridin-4-ylmethyl-[3-(3-trifluoromethyl-phenyl)-pyrazolo[1,5-a]pyrimidin-5-yl]-amine;
[3-(4-Fluoro-phenyl)-pyrazolo[1,5-a] pyrimidin-5-yl]-pyridin-4-ylmethyl-amine;
4-Amino-N-{4-[3-(5-isopropyl-2-methoxy-phenyl)-pyrazolo[1, 5-a]pyrimidin-5-ylamino]-phenyl}-benzamide;
4-Amino-N-{4-[3-(4-fluoro-phenyl)-pyrazolo[1,5-a]pyrimidin-5-ylamino]-phenyl}-benzamide;
4-[3-(4-Fluoro-phenyl)-pyrazolo[1, 5-a]pyrimidin-5-ylamino]-cyclohexanol;
[3-(3-Chloro-phenyl)-pyrazolo[1,5-a]pyrimidin-5-yl]-pyridin-3-ylmethyl-amine;
[3-(5-Isopropyl-2-methoxy-phenyl)-pyrazolo[1, 5-a]pyrimidin-5-yl]-pyridin-3-ylmethyl-amine;
Pyridin-3-ylmethyl-[3-(3-trifluoromethyl-phenyl)-pyrazolo[1,5-a]pyrimidin-5-yl]-amine;
[3-(3-Fluoro-phenyl)-pyrazolo[1,5-a]pyrimidin-5-yl]-pyridin-3-ylmethyl-amine;
[3-(4-Phenoxy-phenyl)-pyrazoto[1, 5-a]pyrimidin-5-yl]-pyridin-3-ylmethyl-amine;
[3-(2-Chloro-phenyl)-pyrazolo[1,5-a]pyrimidin-5-yl]-pyridin-3-ylmethyl-amine;
[3-(2-Fluoro-phenyl)-pyrazolo[1, 5-a]pyrimidin-5-yl]-pyridin-3-ylmethyl-amine;
Pyridin-3-ylmethyl-(3-p-tolyl-pyrazolo[1,5-a]pyrimidin-5-yl)-amine;
(4-Morpholin-4-yl-phenyl)-(3-naphthalen-2-yl-pyrazolo[1,5-a]pyrimidin-5-yl)-amine;
[3-(3,5-Bis-trifluoromethyl-phenyl)-pyrazolo[1,5-a]pyrimidin-5-yl]-(4-morpholin-4-yl-phenyl)-amine;
(4-Morpholin-4-yl-phenyl)-(3-naphthalen-1-yl-pyrazolo[1,5-a]pyrimidin-5-yl)-amine;
[3-(3-Chloro-4-fluoro-phenyl)-pyrazolo[1,5-a]pyrimidin-5-yl]-(4-morpholin-4-yl-phenyl)-amine;
[3-(2,3-Dichloro-phenyl)-pyrazolo[1,5-a]pyrimidin-5-yl]-(4-morpholin-4-yl-phenyl)-amine;
[3-(3-Chloro-phenyl)-pyrazolo[1,5-a]pyrimidin-5-yl]-(4-morpholin-4-ylphenyl)-amine;
[3-(5-Isopropyl-2-methoxy-phenyl)-pyrazolo[1,5-a]pyrimidin-5-yl]-(4-morpholin-4-yl-phenyl)-amine;
3-(4-{5-[(Pyridin-4-ylmethyl)-amino]-pyrazolo[1,5-a] pyrimidin-3-yl}-phenyl)-propionic acid;
Pyridin-4-ylmethyl-[3-(3,4, 5-trimethoxy-phenyl)-pyrazolo[1,5-a]pyrimidin-5-yl]-amine;
2-[3-(2-Methoxy-phenyl)-pyrazolo[1,5-a]pyrimidin-5-ylamino]-ethanol;
(4-Morpholin-4-yl-phenyl)-[3-(4-trifluoromethoxy-phenyl)-pyrazolo[1,5-a]pyrimidin-5-yl]-amine;
(4-Morpholin-4-yl-phenyl)-[3-(3,4,5-trimethoxy-phenyl)-pyrazolo[1,5-a]pyrimidin-5-yl]-amine;
[3-(5-Methoxy-pyridin-3-yl)-pyrazolo[1,5-a]pyrimidin-5-yl]-(4-morpholin-4-yl-phenyl)-amine;
3-{4-[5-(2-Dimethylamino-ethylamino)-pyrazolo[1,5-a]pyrimidin-3-yl]-phenyl}-propionic acid;
2-Methoxy-4-[5-(3,4,5-trimethoxy-phenylamino)-pyrazolo[1,5-a]pyrimidin-3-yl]-phenol;
[3-(4-Methoxy-phenyl)-pyrazolo[1,5-a]pyrimidin-5-yl]-(6-methoxy-pyridin-3-yl)-amine;
N-{5-[3-(4-Methoxy-phenyl)-pyrazolo[1,5-a]pyrimidin-5-ylamino]-2-methyl-phenyl}-methanesulfonamide;
N-{5-[3-(3-Hydroxy-phenyl)-pyrazolo[1,5-a]pyrimidin-5-ylamino]-2-methyl-phenyl}-methanesulfonamide;
(E)-3-(3-{5-[(Pyridin-4-ylmethyl)-amino]-pyrazolo[1,5-a]pyrimidin-3-yl}-phenyl)-acrylic acid;
N'-[3-(1H-Indol-6-yl)-pyrazolo[1,5-a]pyrimidin-5-yl]-N,N-dimethylethane-1,2-diamine;
3-[5-(2-Dimethylamino-ethylamino)-pyrazolo[1,5-a]pyrimidin-3-yl]-phenol;
N-{5-[3-(3- Hydroxymethyl- phenyl)-pyrazolo [1,5- a] pyrimidin- 5- ylamino]- 2- methyl- phenyl}-methanesulfonamide;

N-{5-[3-(2-Hydroxymethyl-phenyl)-pyrazolo[1,5-a]pyrimidin-5-ylamino]-2-methyl-phenyl}-methanesulfonamide;
[3-(2,4-Dimethoxy-phenyl)-pyrazolo[1,5-a]pyrimidin-5-yl]-(3,4, 5-trimethoxy-phenyl)-amine;
[3-(1-Benzyl-1 H-pyrazol-4-yl)-pyrazolo[1,5-a]pyrimidin-5-yl]-(3,4,5-trimethoxy-phenyl)-amine;
4-[3-(2,4-Dimethoxy-phenyl)-pyrazolo[1,5-a]pyrimidin-5-ylamino]-phenol;
[3-(2,4-Dimethoxy-phenyl)-pyrazolo[1,5-a]pyrimidin-5-yl]-(6-methoxy-pyridin-3-yl)-amine;
1-{3-[5-(6-Methoxy-pyridin-3-ylamino)-pyrazolo[1,5-a]pyrimidin-3-yl]-phenyl}-ethanone;
N-{4-[3-(2,6-Dimethoxy-phenyl)-pyrazolo[1,5-a]pyrimidin-5-ylamino]-phenyl}-4-methyl-benzenesulfonamide;
N-{4-[3-(2,4-Dimethoxy-phenyl)-pyrazolo[1,5-a]pyrimidin-5-ylamino]-phenyl}-4-methyl-benzenesulfonamide;
N-{5-[3-(1-Benzyl-1 H-pyrazol-4-yl)-pyrazolo[1, 5-a]pyrimidin-5-ylamino]-2-methyl-phenyl}-methanesulfonamide;
[3-(2,4-Dimethoxy-phenyl)-pyrazolo[1,5-a]pyrimidin-5-yl]-pyridin-4-ylmethyl-amine;
N-(3-{5-[(Pyridin-4-ytmethyl)-amino]-pyrazolo[1,5-a]pyrimidin-3-yl}-phenyl)-methanesulfonamide;
[3-(2,4-Dimethoxy-phenyl)-pyrazolo[1,5-a]pyrimidin-5-yl]-pyridin-3-ylmethyl-amine;
(3-Chloro-phenyl)-[3-(3-methoxy-phenyl)-pyrazolo[1,5-a]pyrimidin-5-yl]-amine;
(3-Chloro-phenyl)-[3-(2,4-dimethoxy-phenyl)-pyrazolo[1, 5-a] pyrimidin-5-yl]-amine;
[3-(2,4-Dimethoxy-phenyl)-pyrazolo[1,5-a]pyrimidin-5-yl]-phenyl-amine;
N-{3-[5-(4-Morpholin-4-yl-phenylamino)-pyrazolo[1,5-a]pyrimidin-3-yl]-phenyl}-methanesulfonamide;
(3-Chloro-4-fluoro-phenyl)- [3- (4-chloro-phenyl)-pyrazolo[1,5-a]pyrimidin-5-yl]-amine;
3-[5-(4-Isopropyl-phenylamino)-pyrazolo[1,5-a]pyrimidin-3-yl]-benzamide;
3-{4-[5-(3-Chloro-phenylamino)-pyrazolo[1,5-a]pyrimidin-3-yl]-phenyl}-propionic acid;
Pyridin-3-ylmethyl-(3-quinolin-8-yl-pyrazolo[1,5-a]pyrimidin-5-yl)-amine;
N-{3-[5-(2-Dimethylamino-ethylamino)-pyrazolo[1,5-a] pyrimidin-3-yl]-phenyl}-acetamide;
N-{3-[5-(3-Chloro-phenylamino)-pyrazolo[1,5-a]pyrimidin-3-yl]-phenyl}-acetamide;
(3-Chloro-phenyl)-[3-(6-methoxy-pyridin-3-yl)-pyrazolo[1,5-a]pyrimidin-5-yl]-amine;
N-{4-[3-(3-Dimethylamino-phenyl)-pyrazolo[1,5-a]pyrimidin-5-ylamino]-phenyl}-4-methyl-benzenesulfonamide;
[3-(3-Amino-phenyl)-pyrazolo[1,5-a]pyrimidin-5-yl]-(3,4,5-trimethoxyphenyl)-amine;
(3-Naphthalen-1-yl-pyrazolo[1,5-a]pyrimidin-5-yl)-(3,4,5-trimethoxyphenyl)-amine;
(3-Benzo[1, 3]dioxol-5-yl-pyrazolo[1, 5-a] pyrimidin-5-yl)-(3,4, 5-trimethoxy-phenyl)-amine;
(3-Pyridin-3-yl-pyrazolo[1,5-a]pyrimidin-5-yl)-(3,4,5-trimethoxyphenyl)-amine;
(3-Thiophen-3-yl-pyrazolo[1, 5-a]pyrimidin-5-yl)-(3,4, 5-trimethoxyphenyl)-amine;
(3-Quinolin-8-yl-pyrazolo[1,5-a]pyrimidin-5-yl)-(3,4,5-trimethoxyphenyl)-amine;
[3-(3,4-Dimethoxy-phenyl)-pyrazolo[1,5-a]pyrimidin-5-yl]-(3,4, 5-trimethoxy-phenyl)-amine;
4-[5-(3,4,5-Trimethoxy-phenylamino)-pyrazolo[1,5-a]pyrimidin-3-yl]-phenol;
(6-Methoxy-pyridin-3-yl)-(3-thiophen-3-yl-pyrazolo[1,5-a]pyrimidin-5-yl)-amine;
[3-(3,4-Dimethoxy-phenyl)-pyrazolo[1,5-a]pyrimidin-5-yl]-(6-methoxy-pyridin-3-yl)-amine;
Benzo[1,3]dioxol-5-ylmethyl-(3-benzo[1,3]dioxol-5-yl-pyrazolo[1,5-a]pyrimidin-5-yl)-amine;
(4-Morpholin-4-yl-phenyl)-(3-thiophen-3-yl-pyrazolo[1,5-a]pyrimidin-5-yl)-amine;
(4-Phenoxy-phenyl)-(3-pyridin-4-yl-pyrazolo[1,5-a] pyrimidin-5-yl)-amine;
(4-Phenoxy-phenyl)-(3-pyridin-3-yl-pyrazolo[1, 5-a]pyrimidin-5-yl)-amine;
(4-Isopropyl-phenyl)-(3-thiophen-3-yl-pyrazolo[1,5-a]pyrimidin-5-yl)-amine;
[3-(3-Trifluoromethyl-phenyl)-pyrazolo[1,5-a]pyrimidin-5-yl]-(3,4,5-trimethoxy-phenyl)-amine;
(3-Benzo[1,3]dioxol-5-yl-pyrazolo[1,5-a]pyrimidin-5-yl)-(4-morpholin-4-yl-phenyl)-amine;
(4-Phenoxy-phenyl)-(3-thiophen-3-yl-pyrazolo[1,5-a]pyrimidin-5-yl)-amine;
4-[3-(3,4,5-Trimethoxy-phenyl)-pyrazolo[1,5-a]pyrimidin-5-ylamino]-cyclohexanol;
Pyridin-3-ylmethyl-[3-(4-trifluoromethoxy-phenyl)-pyrazolo[1,5-a]pyrimidin-5-yl]-amine;
N-(4-Methoxy-phenyl)-4-[5-(4-morpholin-4-yl-phenylamino)-pyrazolo[1,5-a]pyrimidin-3-yl]-benzamide;
(3-Chloro-4-fluoro-phenyl)-[3-(2-methoxy-phenyl)-pyrazolo[1,5-a]pyrimidin-5-yl]-amine;
[3-(3,4-Dimethoxy-phenyl)-pyrazolo[1,5-a]pyrimidin-5-yl]-phenyl-amine;
N'-(3-Benzofuran-2-yl-pyrazolo[1,5-a]pyrimidin-5-yl)-N,N-dimethylethane-1,2-diamine;
[3-(4-Amino-phenyl)-pyrazolo[1,5-a]pyrimidin-5-yl]-(3-chloro-phenyl)-amine;
[3- (4-Dimethylamino-phenyl)-pyrazolo[1,5-a]pyrimidin-5-yl]-(4-phenoxyphenyl)-amine;
[3-(3,4-Dimethyl-phenyl)-pyrazolo[1,5-a]pyrimidin-5-yl]-(3,4,5-trimethoxy-phenyl)-amine;
4-Amino-N-{4-[3-(3-trifluoromethyl-phenyl)-pyrazoto[1,5-a]pyrimidin-5-ylamino]-phenyl}-benzamide;
[3-(3,4-Dimethyl-phenyl)-pyrazolo[1,5-a]pyrimidin-5-yl]-(4-morpholin-4-yl-phenyl)-amine;
N*4*-[3-(3-chloro-phenyl)-pyrazolo[1,5-a]pyrjmidin-5-yl]-N*1*,N*1*-diethyl-pentane-1,4-diamine;
N'-[3-(3-chloro-phenyl)-pyrazolo[1,5-a]pyrimidin-5-yl]-N,N-diethylpropane-1,3-diamine;

N*4*-[3-(3-Chloro-4-methyl-phenyl)-pyrazolo[1,5-a] pyrimidin-5-yl]-N*1*,N*1*-diethyl-pentane-1,4-diamine;
N'-[3-(3-Chloro-4-methyl-phenyl)-pyrazolo[1,5-a]pyrimidin-5-yl]-N,N-diethyl-propane-1,3-diamine.

**8.** A method of preparing a compound of general formula (I) according to any one of claims 1 to 7, wherein an intermediate of general formula 3 :

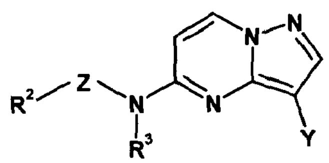

3 ,

in which Y represents a leaving group, such as a halogen atom for example, and Z, $R^2$ and $R^3$ have the meaning as given in claim 1 for general formula (I), is allowed to react with an intermediate of general formula 4 :

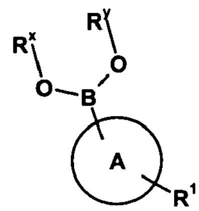

4 ,

in which $R^x$ and $R^y$ represent hydrogen, or, $R^x$ and $R^y$ are $C_1$-$C_6$-alkyl, chosen in such a way that, together with the oxygen atom to which they are attached, a 5 to 6 membered cyclic boronic acid ester is formed, and A and $R^1$ have the meaning as given in claim 1 for general formula (I),
to provide a compound of general formula (I) :

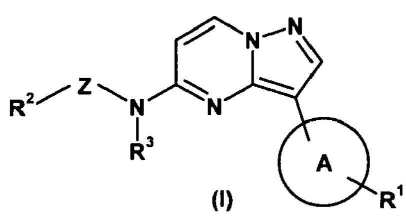

(I) ,

wherein A, $R^1$, $R^2$ and $R^3$ have the meaning as defined in claim 1 for general formula (I), it being understood that $R^1$, $R^2$ and $R^3$ may also incorporate one or more protecting groups, such as, for example $-C(O)OC(CH_3)_3$, wherein said protecting group is not incorporated in the final compound of general formula (I), as defined in claim 1, and may be cleaved to provide compounds of general formula (I).

**9.** A method of preparing a compound according to any one of claims 1 to 7, wherein an intermediate of general formula 7 :

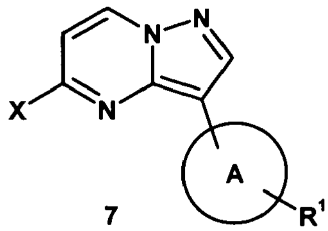

7 ,

in which X represents halogen or perfluor-$C_1$-$C_4$-alkyl sulfonyl, and A and $R^1$ have the meaning as defined in claim 1 for general formula (I),
is allowed to react with an intermediate of general formula 2 :

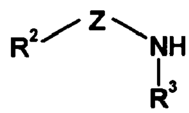

2 ,

in which Z, $R^2$ and $R^3$ have the meaning as defined in claim 1 for general formula (I),
to provide a compound of general formula (I) :

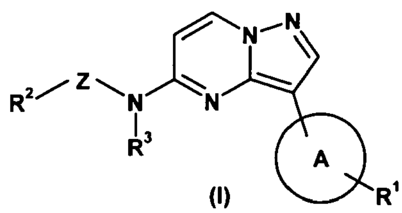

(I) ,

wherein A, $R^1$, $R^2$ and $R^3$ have the meaning as defined in claim 1 for general formula (I), it being understood that $R^1$, $R^2$ and $R^3$ may also incorporate one or more protecting groups, such as, for example $-C(O)OC(CH_3)_3$, wherein said protecting group is not incorporated in the final compound of general formula (I), as defined in claim 1, and may be cleaved to provide compounds of general formula (I).

**10.** A pharmaceutical composition which comprises a compound of general formula (I) according to any one of claims 1 to 7, or a pharmaceutically acceptable salt or an in *vivo* hydrolysable ester thereof, and a pharmaceutically-acceptable diluent or carrier.

**11.** Use of a compound of any one of claims 1 to 7 for manufacturing a pharmaceutical composition for the treatment or prophylaxis of diseases of dysregulated vascular growth or of diseases which are accompanied with dysregulated vascular growth.

**12.** Use according to claim 11, wherein said diseases are tumours and / or metastases thereof.

**13.** Use according to claim 11, wherein said diseases are retinopathy, other angiogenesis dependent diseases of the eye, rheumatoid arthritis, and other inflammatory diseases associated with angiogenesis.

**14.** Use of claim 13, wherein said angiogenesis dependent diseases of the eye are cornea transplant rejection, age-related macular degeneration.

**15.** Use according to claim 11, wherein said diseases are coronary and peripheral artery disease.

**16.** Use of claim 13, wherein said inflammatory diseases associated with angiogenesis are psoriasis, delayed type hypersensitivity, contact dermatitis, asthma, multiple sclerosis, restenosis, pulmonary hypertension, stroke, and diseases of the bowel.

**17.** Use according to claim 11, wherein said diseases are ascites, oedema such as brain tumour associated oedema, high altitude trauma, hypoxia induced cerebral oedema pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

**18.** A method of treating or prophylaxis of a disease of dysregulated vascular growth or diseases which are accompanied with dysregulated vascular growth by administering an effective amount of a compound of general formula (I) according to any one of claims 1 to 7.

**19.** The method according to claim 18, wherein said disease is tumour and / or metastases thereof.

**20.** The method according to claim 19, wherein said diseases are retinopathy, other angiogenesis dependent diseases of the eye rheumatoid arthritis, and other inflammatory diseases associated with angiogenesis.

**21.** The method according to claim 20, wherein said angiogenesis dependent diseases of the eye are cornea transplant rejection, age-related macular degeneration.

**22.** The method according to claim 18, wherein said diseases are coronary and peripheral artery disease.

**23.** The method according to claim 20 wherein said inflammatory diseases associated with angiogenesis are psoriasis, delayed type hypersensitivity, contact dermatitis, asthma, multiple sclerosis, restenosis, pulmonary hypertension, stroke, and diseases of the bowel.

**24.** The method according to claim 18, wherein said diseases are ascites, oedema such as brain tumour associated oedema, high altitude trauma, hypoxia induced cerebral oedema pulmonary oedema and macular oedema or oedema following burns and trauma, chronic lung disease, adult respiratory distress syndrome, bone resorption and for benign proliferating diseases such as myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

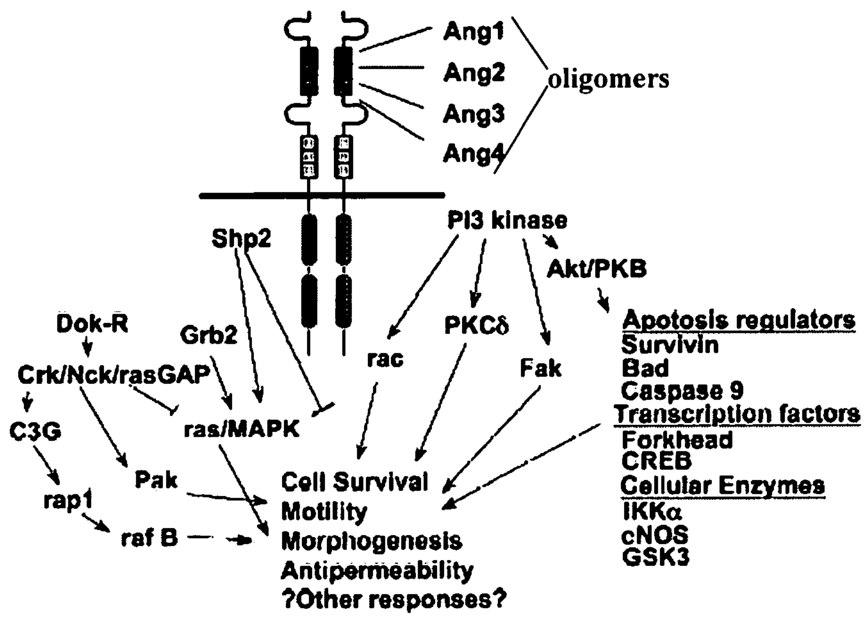
Ang1
Ang2
Ang3
Ang4
oligomers
Shp2
PI3 kinase
Akt/PKB
Dok-R
Grb2
PKCδ
rac
Fak
Apotosis regulators
Survivin
Bad
Caspase 9
Transcription factors
Forkhead
CREB
Cellular Enzymes
IKKα
cNOS
GSK3
Crk/Nck/rasGAP
C3G
ras/MAPK
Pak
rap1
raf B
Cell Survival
Motility
Morphogenesis
Antipermeability
?Other responses?

Fig. 1

European Patent Office

# PARTIAL EUROPEAN SEARCH REPORT

which under Rule 45 of the European Patent Convention shall be considered, for the purposes of subsequent proceedings, as the European search report

Application Number
EP 06 09 0113

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BULLOCK ALEX N ET AL: "Structural basis of inhibitor specificity of the human protooncogene proviral insertion site in moloney murine leukemia virus (PIM-1) kinase." JOURNAL OF MEDICINAL CHEMISTRY. 1 DEC 2005, vol. 48, no. 24, 1 December 2005 (2005-12-01), pages 7604-7614, XP002405915 ISSN: 0022-2623 * page 7608; figure 2 * ----- | 1-24 | INV. C07D487/04 A61K31/519 A61P35/00 |
| A | WILLIAMSON D S ET AL: "Structure-guided design of pyrazolo[1,5-a]pyrimidines as inhibitors of human cyclin-dependent kinase 2" BIOORGANIC & MEDICINAL CHEMISTRY LETTERS, OXFORD, GB, vol. 15, no. 4, 15 February 2005 (2005-02-15), pages 863-867, XP004730355 ISSN: 0960-894X * page 865; compound 9B * ----- -/-- | 1,11 | TECHNICAL FIELDS SEARCHED (IPC) C07D |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 8 November 2006 | SAHAGUN KRAUSE, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C07)

European Patent Office

# PARTIAL EUROPEAN SEARCH REPORT

Application Number
EP 06 09 0113

| DOCUMENTS CONSIDERED TO BE RELEVANT | | | CLASSIFICATION OF THE APPLICATION (IPC) |
|---|---|---|---|
| Category | Citation of document with indication, where appropriate, of relevant passages | Relevant to claim | |
| A | WO 2004/087707 A (VERNALIS CAMBRIDGE LTD [GB]; PARRATT MARTIN [GB]; BOWER JUSTIN FAIRFIE) 14 October 2004 (2004-10-14) compound 163 ----- | 1,11 | |
| A | WO 2004/022054 A (BIOGEN INC [US]; LEE WEN-CHERNG [US]; CARTER MARY BETH [US]; SUN LIHON) 18 March 2004 (2004-03-18) * abstract * ----- | 1,11 | |
| | | | TECHNICAL FIELDS SEARCHED (IPC) |

4

EPO FORM 1503 03.82 (P04C10)

European Patent Office

**INCOMPLETE SEARCH SHEET C**

Application Number
EP 06 09 0113

Claim(s) searched incompletely:
1-24

Claim(s) not searched:
-

Reason for the limitation of the search:

The wording "isomers" included in claims 1-6 as well as its definition in the description (page 15), specially for "constitutional isomers", would extend the protection to subject-matter not defined without undue burden (for example imidazo[1,2-b]pyridazines 1-6 in D1 would fall in the definition) and include compounds that very likely would not solve the problem posed.

## ANNEX TO THE EUROPEAN SEARCH REPORT ON EUROPEAN PATENT APPLICATION NO. EP 06 09 0113

This annex lists the patent family members relating to the patent documents cited in the above-mentioned European search report.
The members are as contained in the European Patent Office EDP file on
The European Patent Office is in no way liable for these particulars which are merely given for the purpose of information.

08-11-2006

| Patent document cited in search report | Publication date | Patent family member(s) | Publication date |
|---|---|---|---|
| WO 2004087707 A | 14-10-2004 | EP 1608652 A1 | 28-12-2005 |
| WO 2004022054 A | 18-03-2004 | AU 2003268447 A1 | 29-03-2004 |
| | | BR 0314053 A | 19-07-2005 |
| | | CA 2497970 A1 | 18-03-2004 |
| | | CN 1694698 A | 09-11-2005 |
| | | EP 1551398 A1 | 13-07-2005 |
| | | JP 2006502165 T | 19-01-2006 |
| | | MX PA05002443 A | 30-09-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **URNESS et al.** Arteriovenous malformations in mice lacking activin receptor-like kinase-1. *Nat Genet.,* 2000, vol. 26 (3), 328-31
- **HELDIN, C.H. et al.** TGF-beta signalling from cell membrane to nucleus through SMAD proteins. *Nature,* 1997, vol. 390 (6659), 465-71
- **LUX, A. et al.** Assignment of transforming growth factor beta1 and beta3 and a third new ligand to the type I receptor ALK1. *J Biol Chem,* 1999, vol. 274 (15), 9984-92
- **BROWN, M.A.** Crystal structure of BMP-9 and functional interactions with pro-region and receptors. *J Biol Chem,* 2005, vol. 280 (26), 25111-8
- **CARCAMO, J. et al.** Type I receptors specify growth-inhibitory and transcriptional responses to transforming growth factor beta and activin. *Mol Cell Biol,* 1994, vol. 14 (6), 3810-21
- **TEN DIJKE P ; M.K., HELDIN CH.** Signalling inputs converge on nuclear effectors in TGF-beta signalling. *Trends Biochem Sci,* 2000, vol. 25 (2), 64-70
- **CHEN, Y.G. ; J. MASSAGUE.** SMAD1 recognition and activation by the ALK1 group of transforming growth factor-beta family receptors. *J Biol Chem,* 1999, vol. 274 (6), 3672-7
- **GOUMANS, M.J. et al.** Balancing the activation state of the endothelium via two distinct TGF-beta type I receptors. *Embo J,* 2002, vol. 21 (7), 1743-53
- **LI, D.Y. et al.** Defective angiogenesis in mice lacking endoglin. *Science,* 1999, vol. 284 (5419), 1534-7
- **YANG, X. et al.** Angiogenesis defects and mesenchymal apoptosis in mice lacking SMAD5. *Development,* 1999, vol. 126 (8), 1571-80
- **ROMAN B.L. et al.** Disruption of acvrl1 increases endothelial cell number in zebrafish cranial vessels. *Development,* 2002, vol. 129, 3009-3019
- **MCDONALD et al.** Clinical manifestations in a large hereditary hemorrhagic telangiectasia (HHT) type 2 kindred. *Am. J. Med. Genet.,* 2000, vol. 93, 320-327
- **VAN DEN DRIESCHE S, M.C. ; WESTERMANN CJ.** Hereditary hemorrhagic telangiectasia: an update on transforming growth factor beta signalling in vasculogenesis and angiogenesis. *Cardiovasc Res,* 2003, vol. 58 (1), 20-31
- **GUTTMACHER et al.** Hereditary hemorrhagic telangiectasia. *N. Engl. J. Med.,* 1995, vol. 333, 918-924
- **SEKI, T. et al.** Arterial endothelium-specific activin receptor-like kinase 1 expression suggests its role in arterialisation and vascular remodelling. *Circ Res,* 2003, vol. 93 (7), 682-9
- *Pure Appl Chem,* 1976, vol. 45, 11-30